# EUROPEAN PATENT APPLICATION

(11) **EP 4 748 833 A1**
(43) Date of publication of application: **27.05.2026**
(21) Application number: 24844730.2
(22) Date of filing: 19.07.2024
(51) Int. Cl.: C07D 209/18, C07D 403/14, C07D 401/04, A61K 31/404, A61P 3/04, A61P 3/10, A61P 13/12

(54) **AMPK AGONIST AND USE THEREOF**

(30) Priority: 21.07.2023 CN 202310904807; 30.08.2023 CN 202311128823
(71) Applicant: Shenzhen Zhongge Biological Technology Co., Ltd., Shenzhen, Guangdong 518017 (CN)
(72) Inventor: ZHAO, Liyu, Shenzhen, Guangdong 518017 (CN); CAO, Bin, Shenzhen, Guangdong 518017 (CN); MA, Nannan, Shenzhen, Guangdong 518017 (CN); ZHANG, Qihua, Shenzhen, Guangdong 518017 (CN); CHEN, Zhaoqiang, Shenzhen, Guangdong 518017 (CN); WAN, Xiao, Shenzhen, Guangdong 518017 (CN); MA, Jin, Shenzhen, Guangdong 518017 (CN); XIE, Dewei, Shenzhen, Guangdong 518017 (CN); YANG, Yuwen, Shenzhen, Guangdong 518017 (CN); DOU, Guosheng, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN); WEN, Shuhao, Shenzhen, Guangdong 518017 (CN)
(74) Representative: Keil & Schaafhausen Patentanwälte PartGmbB
(86) International application number: PCT/CN2024/106470
(87) International publication number: WO 2025/021039

(57) **Abstract**

The present invention belongs to the field of drugs and cosmetics, and particularly relates to an AMPK agonist, and a preparation method therefor and the use thereof. In particular, the AMPK agonist is used in the preparation of a drug for treating and/or preventing alopecia, etc.

## Description

### Cross-Reference to Related Applications

The present application claims priority to Chinese patent application CN 202310904807.7 filed on July 21, 2023 and Chinese patent application CN 202311128823.8 filed on August 30, 2023, the contents of which are incorporated herein by reference in their entirety and for all purposes.

### Technical Field

The present invention belongs to the field of drugs and cosmetics, and particularly relates to an AMPK agonist, and a preparation method therefor and the use thereof.

### Background Art

AMPK (adenosine monophosphate (AMP)-activated protein kinase) is a serine/threonine protein kinase, and a heterotrimeric complex consisting of a kinase domain-containing α-subunit and two regulatory subunits (β- and γ-subunits). AMPK is regulated by upstream kinases LKB1 (liver kinase B1) and CAMKK2 (calcium/calmodulin-dependent protein kinase kinase 2). Phosphorylation of Thr174 in the activation loop (T-loop) of its α-subunit (Thr172 in the α2 isoform) leads to a 500- to 1000-fold increase in activity. AMPK acts as a metabolic switch; activation of AMPK can affect lipid, cholesterol, carbohydrate, and amino acid metabolism, as well as mitochondrial function, autophagy, and cell growth.

Hair has its own growth and metabolic cycle. Its growth is driven by hair follicle stem cells within the follicle. As these stem cells transition between active and resting states, hair undergoes a process of growth, rest, shedding, and regrowth. Therefore, the hair growth cycle consists of the anagen, catagen, and telogen phases.

Alopecia is the loss of hair from the head or part of the body, typically referring to hair loss from the scalp. It is generally classified into androgenetic alopecia, focal alopecia (alopecia areata), traction alopecia, telogen effluvium, and anagen effluvium, with the causes varying across different types. Currently, the most common and intensively studied types are androgenetic alopecia and alopecia areata.

At present, the range of products with proven efficacy in combating hair loss and stimulating hair growth is quite limited. Minoxidil and finasteride are the two approved drugs for the treatment of alopecia. Minoxidil is the only OTC drug. The main adverse effects of minoxidil are irritant dermatitis and hypertrichosis. Moreover, it takes a relatively long time to work and is effective in only 40% of the population. Consequently, its use is somewhat limited. There is a need to develop more products for combating hair loss and stimulating hair growth to meet demands.

Recently, Cell Reports (27, 3413-3421) reported that the AMPK agonist AICAR can activate hair follicle stem cells in telogen-phase mice, initiating a new round of hair growth cycle. Therefore, it is necessary to design and synthesise novel AMPK agonists that can activate AMPK, stimulate hair follicle stem cells, and promote hair growth, thereby providing more products for combating hair loss and stimulating hair growth to meet demands.

Type II diabetes, also known as non-insulin-dependent diabetes, is a chronic metabolic disease mainly characterised by hyperglycaemia, insulin deficiency, and insulin resistance. Besides genetic factors, lifestyle and diet greatly influence the incidence of type II diabetes. Currently, the main drugs for treating type II diabetes include biguanides, sulphonylureas, thiazolidinediones, DPP-4 inhibitors, SGLT-2 inhibitors, and GLP-1 analogues. However, all currently used drugs have certain side effects, so there is a need to develop hypoglycaemic drugs with novel mechanisms of action to meet the needs of diabetic patients.

AMPK reduces lipid storage by phosphorylating a variety of substrates. These pathways work together to promote fatty acid oxidation while inhibiting the synthesis of fatty acids and cholesterol. AMPK may also indirectly control the rate of fatty acid oxidation by regulating mitochondrial function. In addition to regulating fatty acid metabolism and indirectly affecting carbohydrate metabolism, AMPK also directly regulates carbohydrate metabolism pathways through a variety of mechanisms. Activation of AMPK can promote cellular glucose uptake by enhancing the translocation and expression of GLUT protein.

It has been reported in the relevant literature (Cell Metabolism 25, 1147-1159, ACS Med. Chem. Lett. 2018, 9, 1, 39-44) that AMPK agonists can significantly reduce blood glucose levels in mice, restoring them to normal; therefore, AMPK agonists have the potential to lower blood glucose. Therefore, it is necessary to design and synthesise novel AMPK agonists to regulate blood glucose levels in vivo by activating AMPK.

### Summary of the Invention

A first aspect of the present invention provides a compound of formula (I), or an enantiomer, a diastereomer, a racemate, a stereoisomer, a geometric isomer, a tautomer, a polymorph, a solvate, a hydrate, an N-oxide, an isotopically labelled compound, a metabolite, an ester, a prodrug or a pharmaceutically acceptable salt thereof, 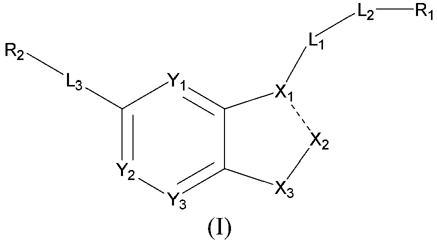 wherein,
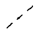 is selected from a single bond and a double bond;
when 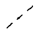 is a single bond, X₁ is selected from CH and N, and X₂ is selected from CR_{X2a}R_{X2b} and C(=O); or
X₁ is selected from CR_{X1} and N, and X₂ is selected from CR_{X2a}R_{X26} and C(=O); or
X₁ is C, L₁ is -CR_{L1b}-, and X₁ and L₁ are connected via a double bond;
when 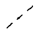 is a double bond, X₁ is C, and X₂ is selected from CR_{X2a} and N;
X₃ is selected from NR_{X3}, O and S;
Y₁ is selected from CR_{Y1}, N and an N-oxide;
Y₂ is selected from CR_{Y2} and N;
Y₃ is selected from CR_{Y3} and N;
L₁ is selected from -C(=O)- and -CR_{L1a}R_{L1b}-;
L₂ is selected from a bond and -C(=O)-;
L₃ is selected from a bond, -O-, -NR_{L3}-, -S-, -C₁₋₆ alkylene-O-, -C₁₋₆ alkylene-NR_{L3}-, -C₁₋₆ alkylene-S-, -C₁₋₆ alkylene-, -C₂₋₆ alkenylene- and -C₂₋₆ alkynylene-;
R₁ is selected from -OR₁ₐ, -NR_{1b}R_{1c}, tetrazolyl and oxodihydrooxadiazolyl 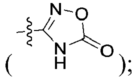
R₂ is selected from C₆₋₁₀ aryl, C₃₋₁₀ cyclic group, 4- to 10-membered heterocyclyl and 5- to 10-membered heteroaryl, and the C₆₋₁₀ aryl, C₃₋₁₀ cyclic group, 4- to 10-membered heterocyclyl and 5- to 10-membered heteroaryl are optionally substituted with 1, 2, 3, 4, or 5 groups each independently selected from R₂ₐ, R_{2b}, R_{2c}, R_{2d} and R₂ₑ;
Rₓ₁ is selected from H, C₁₋₆ alkyl and C₁₋₆ haloalkyl;
R_{X2a} and R_{X2b} are each independently selected from H, halogen, -NH₂, C₁₋₆ alkyl, C₁₋₆ haloalkyl, -NH-C₁₋₆ alkyl, -N(C₁₋₆ alkyl)-C₁₋₆ alkyl, C₁₋₆ alkoxy, C₁₋₆ haloalkoxy, -S-C₁₋₆ alkyl and -S-C₁₋₆ haloalkyl; or
R_{X2a} and R_{X2b} are each independently selected from H, halogen, hydroxyl, - NH₂, C₁₋₆ alkyl, C₁₋₆ haloalkyl, -NH-C₁₋₆ alkyl, -N(C₁₋₆ alkyl)-C₁₋₆ alkyl, C₁₋₆ alkoxy, C₁₋₆ haloalkoxy, -S-C₁₋₆ alkyl and -S-C₁₋₆ haloalkyl;
R_{X3} is selected from H, C₁₋₆ alkyl, C₁₋₆ haloalkyl, C₃₋₆ cycloalkyl and C₃₋₆ halocycloalkyl;
R_{Y1} is selected from H, D, halogen, hydroxyl, carboxyl, -CN, C₁₋₆ alkyl, C₁₋₆ haloalkyl, C₁₋₆ alkoxy, C₁₋₆ haloalkoxy, C₂₋₆ alkenyl, C₂₋₆ alkynyl, C₃₋₁₀ cycloalkyl and 4- to 10-membered heterocycloalkyl;
R_{Y2} and R_{Y3} are each independently selected from H, D, halogen, -CN, hydroxyl, carboxyl, C₁₋₆ alkyl, C₁₋₆ haloalkyl, C₁₋₆ alkoxy, C₁₋₆ haloalkoxy, C₂₋₆ alkenyl, C₂₋₆ alkynyl, C₃₋₁₀ cycloalkyl and 4- to 10-membered heterocycloalkyl; or
R_{Y2} and R_{Y3}, together with the atoms to which they are attached, form phenyl and 5- to 6-membered heteroaryl;
R_{L1a} and R_{L1b} are each independently selected from H, D, halogen, C₁₋₆ alkyl, C₁₋₆ haloalkyl, C₁₋₆ alkoxy and C₁₋₆ haloalkoxy; or
R_{L1a} and R_{L1b}, together with the atoms to which they are attached, form C₃₋₆ cycloalkyl;
R_{L3} is selected from H, D and C₁₋₄ alkyl;
R₁ₐ, R_{1b} and R_{1c} are each independently selected from H, C₁₋₆ alkyl, C₃₋₁₀ cycloalkyl, 4- to 10-membered heterocyclyl, 5- to 10-membered heteroaryl, C₆₋₁₀ aryl, -C₁₋₆ alkylene-C₃₋₁₀ cycloalkyl, -C₁₋₆ alkylene-4- to 10-membered heterocyclyl, -C₁₋₆ alkylene-C₆₋₁₀ aryl, -C₁₋₆ alkylene-5- to 10-membered heteroaryl, -C₁₋₆ haloalkylene-C₃₋₁₀ cycloalkyl, -C₁₋₆ haloalkylene-4- to 10-membered heterocyclyl, - C₁₋₆ haloalkylene-C₆₋₁₀ aryl and -C₁₋₆ haloalkylene-5- to 10-membered heteroaryl; or
R₁ₐ, R_{1b} and R_{1c} are each independently selected from H, C₁₋₆ alkyl, C₃₋₁₀ cycloalkyl, 4- to 10-membered heterocyclyl, 5- to 10-membered heteroaryl, C₆₋₁₀ aryl, -C₁₋₆ alkylene-C₃₋₁₀ cycloalkyl, -C₁₋₆ alkylene-4- to 10-membered heterocyclyl, -C₁₋₆ alkylene-C₆₋₁₀ aryl, -C₁₋₆ alkylene-5- to 10-membered heteroaryl, -C₁₋₆ haloalkylene-C₃₋₁₀ cycloalkyl, -C₁₋₆ haloalkylene-4- to 10-membered heterocyclyl, - C₁₋₆ haloalkylene-C₆₋₁₀ aryl and -C₁₋₆ haloalkylene-5- to 10-membered heteroaryl; wherein the C₁₋₆ alkyl, C₃₋₁₀ cycloalkyl, 4- to 10-membered heterocyclyl, 5- to 10-membered heteroaryl, C₆₋₁₀ aryl, -C₁₋₆ alkylene-C₃₋₁₀ cycloalkyl, -C₁₋₆ alkylene-4- to 10-membered heterocyclyl, -C₁₋₆ alkylene-C₆₋₁₀ aryl, -C₁₋₆ alkylene-5- to 10-membered heteroaryl, -C₁₋₆ haloalkylene-C₃₋₁₀ cycloalkyl, -C₁₋₆ haloalkylene-4- to 10-membered heterocyclyl, -C₁₋₆ haloalkylene-C₆₋₁₀ aryl and -C₁₋₆ haloalkylene-5-to 10-membered heteroaryl are optionally substituted with one or more substituents selected from oxo, hydroxyl, cyano, carboxyl, halogen, C₁₋₆ alkyl, C₁₋₆ haloalkyl, C₁₋₆ alkoxy, C₁₋₆ haloalkoxy, C₃₋₆ cycloalkyl, 4- to 7-membered heterocyclyl, C₆₋₁₀ aryl, and 5- to 10-membered heteroaryl;
R₂ₐ, R_{2b}, R_{2c}, R_{2d} and R₂ₑ are each independently selected from H, halogen, - OH, C₁₋₆ alkyl, C₁₋₆ haloalkyl, -NHC₁₋₆ alkyl, -N(C₁₋₆ alkyl)-C₁₋₆ alkyl, C₁₋₆ alkoxy, C₁₋₆ haloalkoxy, C₃₋₁₀ cyclic group, 4- to 10-membered heterocyclyl, C₆₋₁₀ aryl, 5- to 10-membered heteroaryl, -C₆₋₁₀ arylene-4- to 10-membered heterocyclyl, -C₆₋₁₀ arylene-5- to 10-membered heteroaryl, -C₆₋₁₀ arylene-C₆₋₁₀ aryl and -5- to 10-membered heteroarylene-5- to 10-membered heteroaryl, and the C₁₋₆ alkyl, C₁₋₆ haloalkyl, -NHC₁₋₆ alkyl, -N(C₁₋₆ alkyl)-C₁₋₆ alkyl, C₁₋₆ alkoxy, C₁₋₆ haloalkoxy, C₃₋₁₀ cyclic group, 4- to 10-membered heterocyclyl, C₆₋₁₀ aryl, 5- to 10-membered heteroaryl, -C₆₋₁₀ arylene-4- to 10-membered heterocyclyl, -C₆₋₁₀ arylene-5- to 10-membered heteroaryl, -C₆₋₁₀ arylene-C₆₋₁₀ aryl and -5- to 10-membered heteroarylene-5- to 10-membered heteroaryl are optionally substituted with one or more R;
each R is independently selected from halogen, -OH, -CN, -COOH, C₁₋₆ alkyl, -C₁₋₆ alkylene-OH, C₁₋₆ alkoxy, -C₁₋₆ alkyleneoxy-C₁₋₆ alkoxy, -C(=O)NH-C₁₋₆ alkyl, -C(=O)-C₁₋₆ alkyl, -NHC(=O)-C₁₋₆ alkyl, -N=S(=O)(C₁₋₆ alkyl)-C₁₋₆ alkyl, -NH-S(=O)₂C₁₋₆ alkyl, -S(=O)₂-C₁₋₆ alkyl, -S(=O)₂-4- to 10-membered heterocyclyl, - C(=O)-4- to 10-membered heterocyclyl, 4- to 10-membered heterocyclyl, 5- to 10-membered heteroaryl and 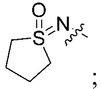
provided that when L₂ is a bond,
   1) L₁ is -CR_{L1a}R_{L1b}-, or
   2) R₁ is selected from -NR_{1b}R_{1c}, tetrazolyl and oxodihydrooxadiazolyl 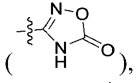 or
   3) 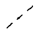 is a single bond.

In certain embodiments, each R is independently selected from halogen, -OH, -CN, -COOH, oxo, C₁₋₆ alkyl, -C₁₋₆ alkylene-OH, C₁₋₆ alkoxy, -C₁₋₆ alkyleneoxy-C₁₋₆ alkoxy, -C(=O)NH-C₁₋₆ alkyl, -C(=O)-C₁₋₆ alkyl, -NHC(=O)-C₁₋₆ alkyl, - N=S(=O)(C₁₋₆ alkyl)-C₁₋₆ alkyl, -NH-S(=O)₂C₁₋₆ alkyl, -S(=O)₂-C₁₋₆ alkyl, -S(=O)₂-4- to 10-membered heterocyclyl, -C(=O)-4- to 10-membered heterocyclyl, 4- to 10-membered heterocyclyl, 5- to 10-membered heteroaryl and

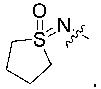

In certain embodiments, it is provided that when L₂ is a bond,
1) L₁ is -CR_{L1a}R_{L1b}-, or
2) R₁ is selected from -NR_{1b}R_{1c}, tetrazolyl and oxodihydrooxadiazolyl 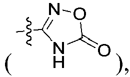 or
3) 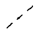 is a single bond, or
4) R₁ is -OR₁ₐ, wherein R₁ₐ is selected from 5- to 10-membered heteroaryl, -C₁₋₆ alkylene-5- to 10-membered heteroaryl, and -C₁₋₆ alkylene-4- to 10-membered heterocyclyl.

In certain embodiments, 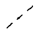 is a double bond.

In certain embodiments, L₂ is -C(=O)-.

In certain embodiments,
when 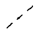 is a single bond, X₁ is selected from CR_{X1} and N, and X₂ is selected from CR_{X2a}R_{X2b} and C(=O), or
X₁ is C, L₁ is -CR_{L1b}-, and X₁ and L₁ are connected via a double bond;
when 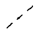 is a double bond, X₁ is C, and X₂ is selected from CR_{X2a} and N; Rₓ₁ is selected from H, C₁₋₃ alkyl, and C₁₋₃ haloalkyl.

In certain embodiments, Rₓ₁ is selected from H and C₁₋₃ alkyl.

In certain embodiments, Rₓ₁ is C₁₋₃ alkyl.

In certain embodiments, the Rₓ₁ is H.

In certain embodiments, 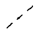 is a single bond, X₁ is C, L₁ is -CR_{L1b}-, and X₁ and L₁ are connected via a double bond.

In certain embodiments, 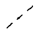 is a single bond, X₁ is selected from CR_{X1} and N, and X₂ is selected from CR_{X2a}R_{X2b} and C(=O).

In certain embodiments, 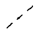 is a single bond, X₁ is N, and X₂ is C(=O); or X₁ is CR_{X1}, and X₂ is CR_{X2a}R_{X2b}.

In certain embodiments, 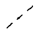 is a single bond, X₁ is CR_{X1}, and X₂ is C(=O).

In certain embodiments, 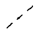 is a double bond, X₁ is C, and X₂ is selected from CR_{X2a} and N.

In certain embodiments, 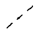 is a double bond, X₁ is C, and X₂ is CR_{X2a}.

In certain embodiments, 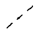 is a double bond, X₁ is C, and X₂ is selected from CH and C(OH).

In certain embodiments, R_{X2a} and R_{X2b} are each independently selected from H, halogen, hydroxyl, C₁₋₄ alkyl, C₁₋₄ haloalkyl, -NH-C₁₋₄ alkyl, -N(C₁₋₄ alkyl)-C₁₋₄ alkyl, C₁₋₄ alkoxy, C₁₋₄ haloalkoxy, -S-C₁₋₄ alkyl and -S-C₁₋₄ haloalkyl.

In certain embodiments, the R_{X2a} and R_{X2b} are each independently selected from H, halogen, C₁₋₄ alkyl, C₁₋₄ haloalkyl, -NH-C₁₋₄ alkyl, -N(C₁₋₄ alkyl)-C₁₋₄ alkyl, C₁₋₄ alkoxy, C₁₋₄ haloalkoxy, -S-C₁₋₄ alkyl and -S-C₁₋₄ haloalkyl.

In certain embodiments, R_{X2a} and R_{X2b} are each independently selected from H, halogen, hydroxyl, C₁₋₄ alkoxy, C₁₋₄ haloalkoxy, and -S-C₁₋₄ alkyl.

In certain embodiments, R_{X2a} and R_{X2b} are each independently selected from H, halogen, hydroxyl, methyl, ethyl, propyl, butyl, halomethyl, haloethyl, halopropyl, halobutyl, -NH-methyl, -NH-ethyl, -NH-propyl, -NH-butyl, -N(methyl)-methyl, - N(methyl)-ethyl, -N(methyl)-propyl, -N(ethyl)-ethyl, -N(ethyl)-propyl, methoxy, ethoxy, propoxy, butoxy, halomethoxy, haloethoxy, halopropoxy, halobutoxy, -S-methyl, -S-ethyl, -S-propyl, -S-butyl, -S-halomethyl, -S-haloethyl, -S-halopropyl and -S-halobutyl.

In certain embodiments, the R_{X2a} and R_{X2b} are each independently selected from H, halogen, methyl, ethyl, propyl, butyl, halomethyl, haloethyl, halopropyl, halobutyl, -NH-methyl, -NH-ethyl, -NH-propyl, -NH-butyl, -N(methyl)-methyl, - N(methyl)-ethyl, -N(methyl)-propyl, -N(ethyl)-ethyl, -N(ethyl)-propyl, methoxy, ethoxy, propoxy, butoxy, halomethoxy, haloethoxy, halopropoxy, halobutoxy, -S-methyl, -S-ethyl, -S-propyl, -S-butyl, -S-halomethyl, -S-haloethyl, -S-halopropyl and -S-halobutyl.

In certain embodiments, R_{X2a} and R_{X2b} are each independently selected from H, F, Cl, Br, hydroxyl, -CH₃, -CH₂CH₃, -CH₂F, -CHF₂, -CF₃, -CF₂CF₃, -NHCH₃, - N(CH₃)₂, -N(CH₂CH₃)₂, -OCH₃, -OCH₂CH₃, -OCH₂CH₂CH₃, -OCH(CH₃)₂, -OCF₃, -OCF₂CF₃, -S-CH₃, -S-CH₂CH₃, -S-CH₂CH₂CH₃ and -S-CH(CH₃)₂.

In certain embodiments, the R_{X2a} and R_{X2b} are each independently selected from H, F, Cl, Br, -CH₃, -CH₂CH₃, -CH₂F, -CHF₂, -CF₃, -CF₂CF₃, -NHCH₃, -N(CH₃)₂, - N(CH₂CH₃)₂, -OCH₃, -OCH₂CH₃, -OCH₂CH₂CH₃, -OCH(CH₃)₂, -OCF₃, -OCF₂CF₃, -S-CH₃, -S-CH₂CH₃, -S-CH₂CH₂CH₃ and -S-CH(CH₃)₂.

In certain embodiments, R_{X2a} is selected from H, F, Cl, hydroxyl, -OCH₃, - OCF₃ and -SCH₃.

In certain embodiments, the R_{X2a} is selected from H, F, Cl, -OCH₃, -OCF₃ and -SCH₃.

In certain embodiments, the R_{X2a} is selected from H and hydroxyl.

In certain embodiments, the R_{X2b} is H.

In certain embodiments, the R_{X3} is selected from H, C₁₋₄ alkyl, C₁₋₄ haloalkyl, C₃₋₅ cycloalkyl and C₃₋₅ halocycloalkyl.

In certain embodiments, the R_{X3} is selected from H, C₁₋₄ alkyl, and C₁₋₄ haloalkyl.

In certain embodiments, the X₃ is selected from NR_{X3}, O and S; the R_{X3} is selected from H, C₁₋₄ alkyl, C₁₋₄ haloalkyl, C₃₋₅ cycloalkyl and C₃₋₅ halocycloalkyl.

In certain embodiments, the R_{X3} is selected from H, methyl, ethyl, propyl, halomethyl, haloethyl, halopropyl, cyclopropyl, cyclobutyl, cyclopentyl, halocyclopropyl, halocyclobutyl and halocyclopentyl.

In certain embodiments, the R_{X3} is H.

In certain embodiments, the X₃ is NR_{X3}.

In certain embodiments, the X₃ is selected from O and S.

In certain embodiments, the Y₁ is selected from CR_{Y1} and N; Y₂ is selected from CR_{Y2} and N; Y₃ is selected from CR_{Y3} and N; R_{Y1}, R_{Y2} and R_{Y3} are each independently selected from H, D, halogen, -CN, hydroxyl, carboxyl, C₁₋₄ alkyl, C₁₋₄ haloalkyl, C₁₋₄ alkoxy, C₁₋₄ haloalkoxy, C₂₋₄ alkenyl, C₂₋₄ alkynyl, C₃₋₆ cycloalkyl and 4- to 6-membered heterocycloalkyl, or R_{Y2} and R_{Y3}, together with the atoms to which they are attached, form phenyl or 5- to 6-membered heteroaryl.

In certain embodiments, the R_{Y1}, R_{Y2} and R_{Y3} are each independently selected from H, D, halogen, -CN, hydroxyl, carboxyl, C₁₋₄ alkyl, C₁₋₄ haloalkyl, C₁₋₄ alkoxy, C₁₋₄ haloalkoxy, C₂₋₄ alkenyl, C₂₋₄ alkynyl, C₃₋₆ cycloalkyl and 4- to 6-membered heterocycloalkyl.

In certain embodiments, Y₁ is selected from CR_{Y1} and N.

In certain embodiments, Y₁ is CR_{Y1}.

In certain embodiments, Y₂ is selected from CR_{Y2} and N.

In certain embodiments, Y₂ is CR_{Y2}.

In certain embodiments, Y₃ is selected from CR_{Y3} and N.

In certain embodiments, Y₃ is CR_{Y3}.

In certain embodiments, the R_{Y1}, R_{Y2} and R_{Y3} are each independently selected from H, D, halogen, -CN, methyl, ethyl, propyl, butyl, halomethyl, haloethyl, halopropyl, halobutyl, methoxy, ethoxy, propoxy, butoxy, halomethoxy, haloethoxy, halopropoxy, halobutoxy, vinyl, propenyl, butenyl, ethynyl, propynyl, butynyl, cyclopropyl, cyclobutyl, cyclopentyl, cyclohexyl, oxetanyl, azetidinyl, tetrahydrofuryl, pyrrolidyl and morpholinyl.

In certain embodiments, the R_{Y1}, R_{Y2} and R_{Y3} are each independently selected from H, D, F, Cl, Br, -CN, -CH₃, -CH₂CH₃, -CH₂CH₂CH₃, -CH(CH₃)₂, -CH₂F, - CHF₂, -CF₃, -CF₂CF₃, -OCH₃, -OCH₂CH₃, -OCH₂CH₂CH₃, -OCH(CH₃)₂, -OCF₃, vinyl, propenyl, ethynyl, propynyl, cyclopropyl, cyclobutyl, oxetanyl and azetidinyl.

In certain embodiments, R_{Y1} is selected from H, D, halogen, C₁₋₄ alkyl, C₁₋₄ haloalkyl, C₁₋₄ alkoxy, and C₁₋₄ haloalkoxy.

In certain embodiments, R_{Y1} is selected from H, D, and halogen.

In certain embodiments, R_{Y2} is selected from H, D, halogen, -CN, C₁₋₄ alkyl, C₁₋₄ haloalkyl, C₁₋₄ alkoxy, C₁₋₄ haloalkoxy, C₂₋₄ alkynyl, C₃₋₆ cycloalkyl and 4- to 6-membered heterocycloalkyl.

In certain embodiments, R_{Y2} is halogen.

In certain embodiments, R_{Y3} is selected from H, D, halogen, and C₁₋₄ alkyl.

In certain embodiments, the R_{Y1} is selected from H and F.

In certain embodiments, the R_{Y1} is H.

In certain embodiments, the R_{Y2} is selected from F, Cl, -CN, -CH₃, -CH₂CH₃, -CH(CH₃)₂, -OCH₃, -OCF₃, ethynyl, cyclopropyl and

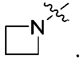

In certain embodiments, the R_{Y2} is Cl.

In certain embodiments, the R_{Y3} is H.

In certain embodiments, the R_{Y2} and R_{Y3}, together with the atoms to which they are attached, form phenyl or 5- to 6-membered heteroaryl.

In certain embodiments, the R_{Y2} and R_{Y3}, together with the atoms to which they are attached, form phenyl or 5-membered heteroaryl.

In certain embodiments, the R_{Y2} and R_{Y3}, together with the atoms to which they are attached, form thienyl, furyl, pyrrolyl, thiazolyl, oxazolyl, pyridyl, pyrimidyl, pyrazinyl and phenyl.

In certain embodiments, the R_{Y2} and R_{Y3}, together with the atoms to which they are attached, form thienyl and phenyl.

In certain embodiments, the R_{Y1} is H.

In certain embodiments, the structural unit 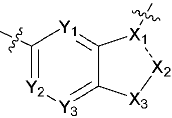 is selected from

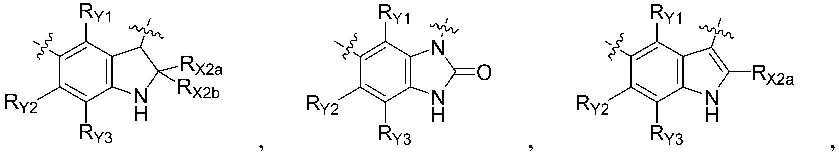

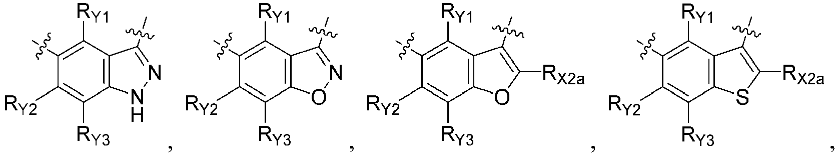

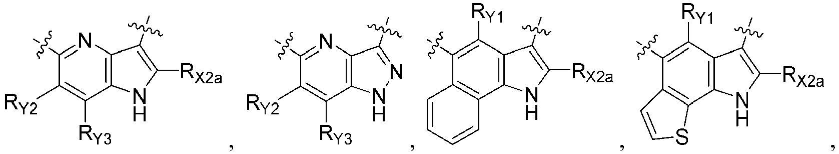

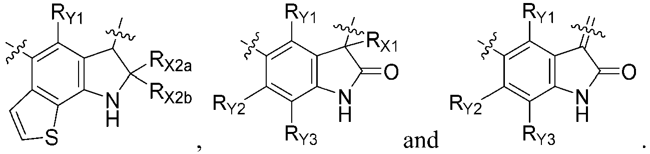

In certain embodiments, the structural unit 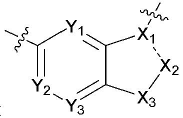 is selected from 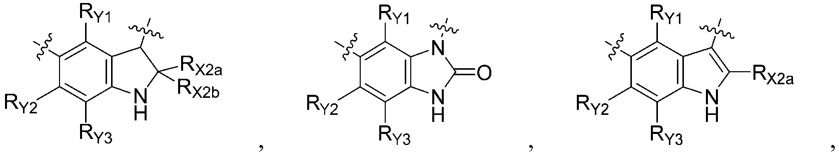 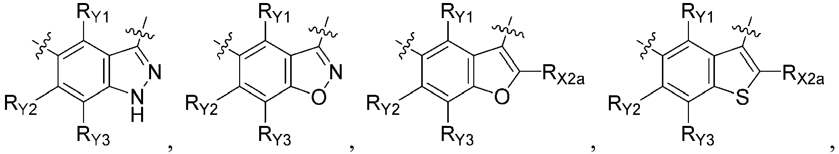 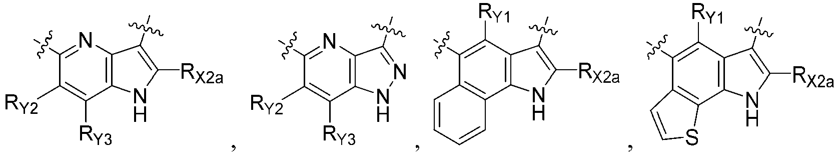 and 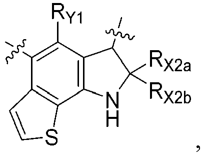 wherein R_{X2a}, R_{X2b}, R_{Y1}, R_{Y2} and R_{Y3} are as defined in the present invention.

In certain embodiments, structural unit 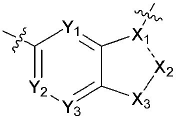 is selected from 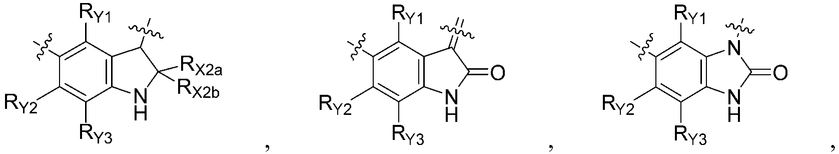 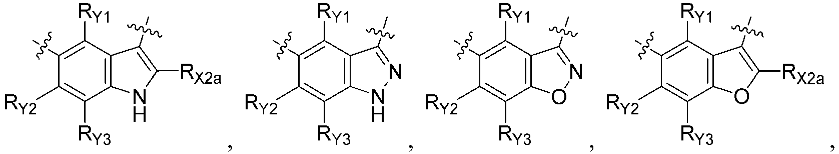 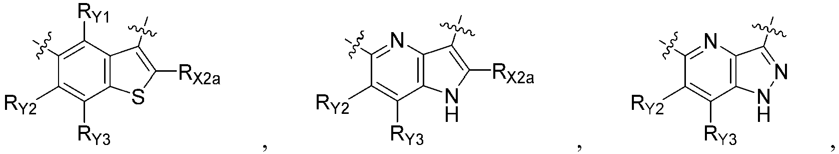 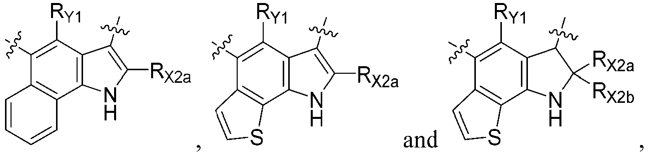 wherein R_{X2a}, R_{X2b}, R_{Y1}, R_{Y2} and R_{Y3} are as defined in the present invention.

In certain embodiments, structural unit 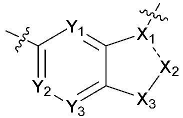 is selected from 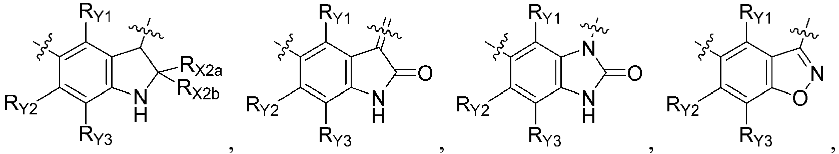 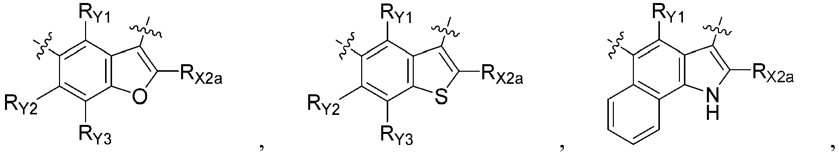 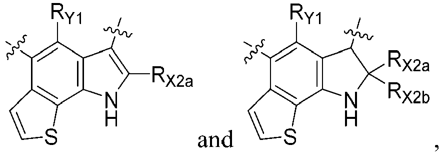 wherein R_{X2a}, R_{X2b}, R_{Y1}, R_{Y2} and R_{Y3} are as defined in the present invention.

In certain embodiments, structural unit 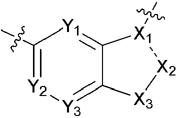 is selected from 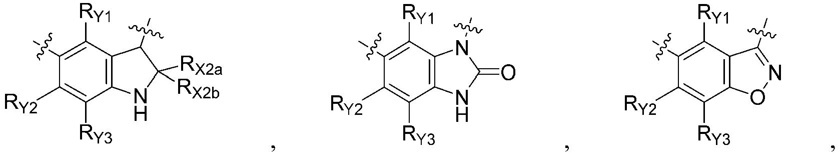 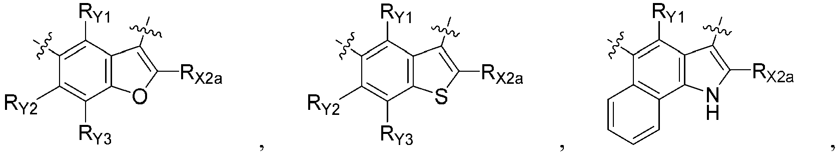 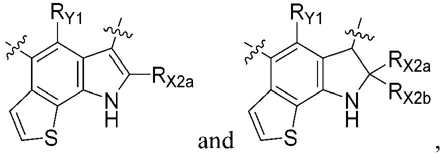 wherein R_{X2a}, R_{X2b}, R_{Y1}, R_{Y2} and R_{Y3} are as defined in the present invention.

In certain embodiments, structural unit 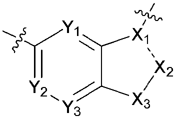 is selected from 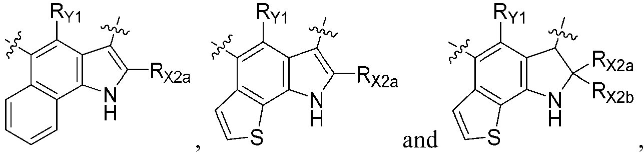 wherein R_{X2a}, R_{X2b} and R_{Y1} are as defined in the present invention.

In certain embodiments, the structural unit 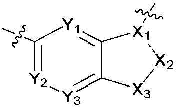 is selected from

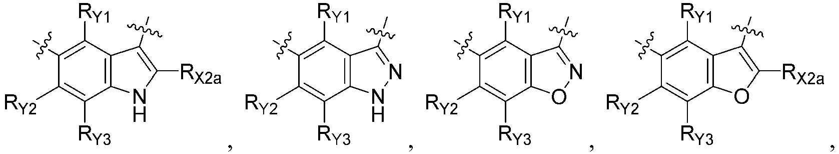

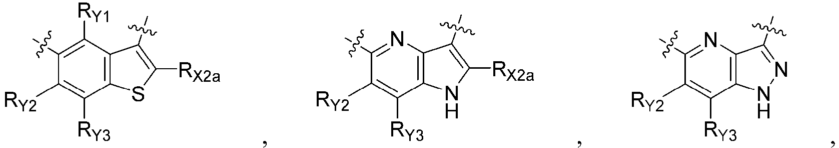

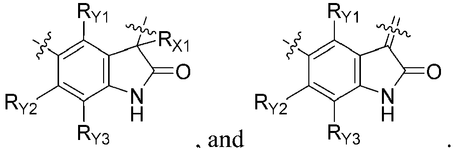

In certain embodiments, the structural unit 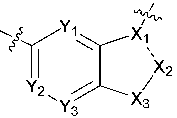 is selected from 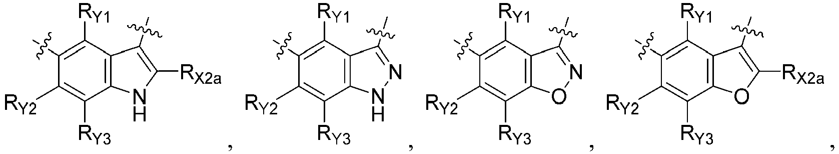 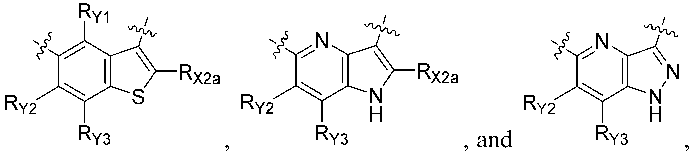 wherein R_{X2a}, R_{Y1}, R_{Y2} and R_{Y3} are as defined in the present invention.

In certain embodiments, the structural unit 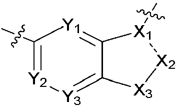 is selected from 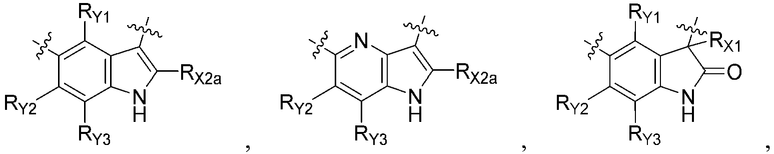 and

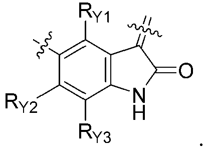

In certain embodiments, the structural unit 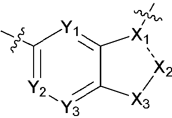 is selected from 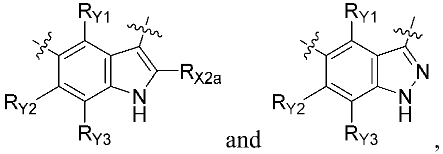 wherein R_{X2a}, R_{Y1}, R_{Y2} and R_{Y3} are as defined in the present invention.

In certain embodiments, the structural unit 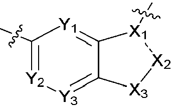 is selected from

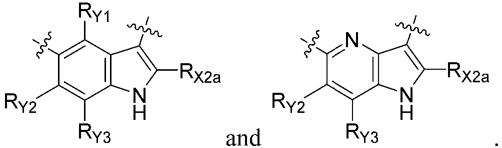

In certain embodiments, the structural unit 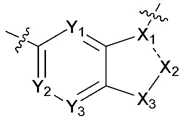 is 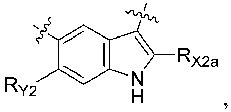 wherein R_{X2a} and R_{Y2} are as defined in the present invention.

In certain embodiments, the L₁ is selected from -C(=O)- and -CR_{L1a}R_{L1b}-; the R_{L1a} and R_{L1b} are each independently selected from H, D, halogen, C₁₋₄ alkyl, C₁₋₄ haloalkyl, C₁₋₄ alkoxy and C₁₋₄ haloalkoxy, or R_{L1a} and R_{L1b}, together with the atoms to which they are attached, form C₃₋₆ cycloalkyl.

In certain embodiments, the R_{L1a} and R_{L1b} are each independently selected from H, D, halogen, C₁₋₄ alkyl, C₁₋₄ haloalkyl, C₁₋₄ alkoxy and C₁₋₄ haloalkoxy.

In certain embodiments, R_{L1a} and R_{L1b} are each independently selected from H, D, and halogen.

In certain embodiments, L₁ is -C(=O)-.

In certain embodiments, L₁ is -CR_{L1a}R_{L1b}-.

In certain embodiments, the L₁ is -CR_{L1a}R_{L1b}-; the R_{L1a} and R_{L1b} are each independently selected from H, D, halogen, C₁₋₄ alkyl, C₁₋₄ haloalkyl, C₁₋₄ alkoxy and C₁₋₄ haloalkoxy.

In certain embodiments, the R_{L1a} and R_{L1b} are each independently selected from H, D, F, Cl, Br, -CH₃, -CH₂CH₃, -CH₂F, -CHF₂, -CF₃, -CF₂CF₃, -OCH₃, -OCH₂CH₃, -OCH₂CH₂CH₃ and -OCF₃.

In certain embodiments, the R_{L1a} and R_{L1b} are each independently selected from H and F.

In certain embodiments, the R_{L1a} and R_{L1b} are each independently H.

In certain embodiments, the R_{L1a} and R_{L1B}, together with the atoms to which they are attached, form cyclopropyl, cyclobutyl, cyclopentyl or cyclohexyl.

In certain embodiments, the R_{L1a} and R_{L1b}, together with the atoms to which they are attached, form cyclopropyl or cyclobutyl.

In certain embodiments, the L₁ is selected from -C(=O)-, -CH₂-, -CHF-, =CH-and -CF₂-.

In certain embodiments, the L₁ is selected from -C(=O)-, -CH₂-, -CHF- and - CF₂-.

In certain embodiments, L₁ is selected from -C(=O)-, -CH₂-, and =CH-.

In certain embodiments, the L₁ is -C(=O)-.

In certain embodiments, the L₂ is a bond.

In certain embodiments, the L₂ is -C(=O)-.

In certain embodiments, the R₁ is selected from -OR₁ₐ, -NR_{1b}R_{1c}, tetrazolyl and oxodihydrooxadiazolyl 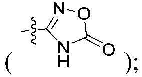 R₁ₐ, R_{1b} and R_{1c} are each independently selected from H, C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl; wherein the C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl are optionally substituted with one or more substituents selected from oxo, hydroxyl, cyano, carboxyl, and halogen.

In certain embodiments, the R₁ₐ, R_{1b} and R_{1c} are each independently selected from H, C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl; wherein the C₁₋₄ alkyl is optionally substituted with one or more substituents selected from oxo, hydroxyl, and carboxyl.

In certain embodiments, R₁ₐ is selected from H, C₁₋₃ alkyl, C₃₋₆ cycloalkyl, 4-to 6-membered heterocyclyl, 5- to 6-membered heteroaryl, phenyl, -C₁₋₃ alkylene-4-to 6-membered heterocyclyl, and -C₁₋₃ alkylene-5- to 6-membered heteroaryl.

In certain embodiments, the R₁ₐ is selected from H and C₁₋₆ alkyl (such as C₁₋₃ alkyl).

In certain embodiments, the R₁ₐ is selected from H, methyl, cyclopropyl, cyclobutyl, oxetanyl (such as 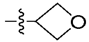 ), oxanyl (such as 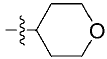 ), pyrazolyl (such as 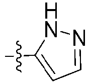 ), oxazolyl (such as 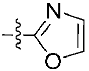 ), phenyl, -C₁₋₃ alkylene-4- to 6-membered heterocyclyl (such as 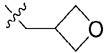 ), and -C₁₋₃ alkylene-5-membered heteroaryl (such as 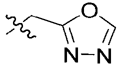 and 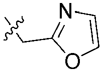 ).

In certain embodiments, the R₁ₐ is H and methyl.

In certain embodiments, the R₁ₐ is methyl.

In certain embodiments, the R_{1b} is selected from H and C₁₋₃ alkyl; the R_{1c} is selected from C₁₋₃ alkyl, C₃₋₆ cycloalkyl, 5- to 6-membered heteroaryl, phenyl, -C₁₋₃ alkylene-4- to 6-membered heterocyclyl, -C₁₋₃ alkylene-phenyl, and -C₁₋₃ alkylene-5- to 6-membered heteroaryl, wherein the C₁₋₃ alkyl is optionally substituted with 1 or 2 substituents selected from oxo, hydroxyl, and carboxyl.

In certain embodiments, the R_{1c} is selected from methyl, -CH₂C(=O)OH, cyclopropyl, pyrrolyl (such as 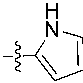 ), furyl (such as 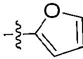 ), thienyl (such as 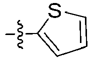 ), imidazolyl (such as 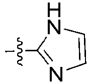 ), pyrazolyl (such as 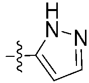 ), oxazolyl (such as 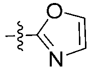 ), tetrazolyl (such as 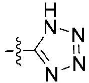 ), phenyl, -CH₂-phenyl, -C₁₋₃ alkylene-4- to 6-membered heterocyclyl (such as 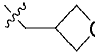 and 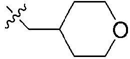 ), and -C₁₋₃ alkylene-5- to 6-membered heteroaryl (such as 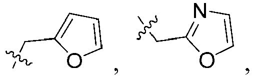 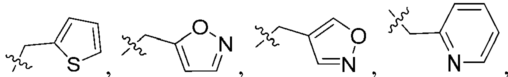 and 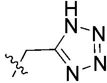 ).

In certain embodiments, the R_{1c} is selected from methyl, -CH₂C(=O)OH, cyclopropyl, pyrrolyl (such as 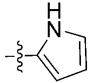 ), furyl (such as 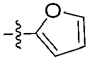 ), thienyl (such as 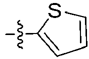 ), imidazolyl (such as 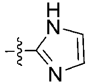 ), pyrazolyl (such as 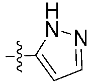 ), oxazolyl (such as 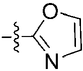 ), tetrazolyl (such as 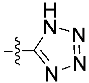 ), phenyl, -CH₂-phenyl, -C₁₋₃ alkylene-4- to 6-membered heterocyclyl (such as 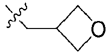 and 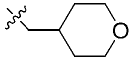 ), and -C₁₋₃ alkylene-5- to 6-membered heteroaryl (such as 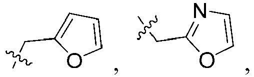 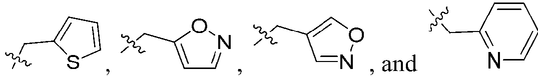 ).

In certain embodiments, the R_{1b} is selected from H and C₁₋₃ alkyl; R_{1c} is selected from C₁₋₃ alkyl, 5-membered heteroaryl, -C₁₋₃ alkylene-4- to 6-membered heterocyclyl, and -C₁₋₃ alkylene-5- to 6-membered heteroaryl, wherein the C₁₋₃ alkyl is optionally substituted with carboxyl.

In certain embodiments, R_{1b} is selected from H and C₁₋₃ alkyl; R_{1c} is selected from C₁₋₃ alkyl, 5-membered heteroaryl, and -C₁₋₃ alkylene-5-membered heteroaryl, wherein the C₁₋₃ alkyl is substituted with one carboxyl.

In certain embodiments, the R_{1b} is selected from H and C₁₋₃ alkyl; the R_{1c} is selected from C₁₋₃ alkyl, 5-membered heteroaryl, and -C₁₋₃ alkylene-5-membered heteroaryl, wherein the C₁₋₃ alkyl is optionally substituted with carboxyl.

In certain embodiments, the R_{1b} is H; R_{1c} is selected from -CH₂C(=O)OH, pyrazolyl (such as 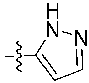 ), and -C₁₋₃ alkylene-5-membered heteroaryl (such as 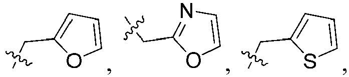 and 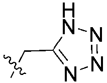 ).

In certain embodiments, the R_{1b} is H; R_{1c} is selected from methyl, imidazolyl (such as 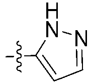 ), and -C₁₋₃ alkylene-5-membered heteroaryl (such as 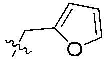 and 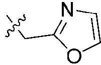 ).

In certain embodiments, the R₁ₐ, R_{1b} and R_{1c} are each independently selected from H, C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl.

In certain embodiments, the R₁ₐ, R_{1b} and R_{1c} are each independently selected from H, C₁₋₄ alkyl, -(CR₁ₐₐR_{1bb})ₘ-C₃₋₆ cycloalkyl, -(CR₁ₐₐR_{1bb})ₘ-4- to 6-membered heterocyclyl, -(CR₁ₐₐR_{1bb})ₘ-5- to 6-membered heteroaryl and -(CR₁ₐₐR_{1bb})ₘ-phenyl, wherein R₁ₐₐ and R_{1bb} are each independently selected from H, F and -CH₃, and m is selected from 0, 1 and 2.

In certain embodiments, the R₁ₐ, R_{1b} and R_{1c} are each independently selected from H, methyl, ethyl, propyl, butyl, -(CR₁ₐₐR_{1bb})ₘ-cyclopropyl, -(CR₁ₐₐR_{1bb})ₘ-cyclobutyl, -(CR₁ₐₐR_{1bb})ₘ-cyclopentyl, -(CR₁ₐₐR_{1bb})ₘ-cyclohexyl, -(CR₁ₐₐR_{1bb} )ₘ-oxetanyl, -(CR₁ₐₐR_{1bb})ₘ-azetidinyl, -(CR₁ₐₐR_{1bb})ₘ-tetrahydrofuryl, -(CR₁ₐₐR_{1bb})ₘ-pyrrolidyl, -(CR₁ₐₐR_{1bb})ₘ-tetrahydropyranyl, -(CR₁ₐₐR_{1bb})ₘ-piperidyl, - (CR₁ₐₐR_{1bb})ₘ-morpholinyl, -(CR₁ₐₐR_{1bb})ₘ-phenyl, -(CR₁ₐₐR_{1bb})ₘ-thienyl, - (CR₁ₐₐR_{1bb})ₘ-pyrazolyl, -(CR₁ₐₐR_{1bb})ₘ-imidazolyl, -(CR₁ₐₐR_{1bb})ₘ-tetrazolyl, - (CR₁ₐₐR_{1bb})ₘ-furyl, -(CR₁ₐₐR_{1bb})ₘ-pyrrolyl, -(CR₁ₐₐR_{1bb})ₘ-thiazolyl, -(CR₁ₐₐR_{1bb})ₘ-oxazolyl, -(CR₁ₐₐR_{1bb})ₘ-isoxazolyl, -(CR₁ₐₐR_{1bb})ₘ-oxadiazolyl, -(CR₁ₐₐR_{1bb})ₘ-pyridyl, -(CR₁ₐₐR_{1bb})ₘ-pyrimidyl and -(CR₁ₐₐR_{1bb})ₘ-pyrazinyl, wherein R₁ₐₐ and R_{1bb} are each independently selected from H, F and -CH₃, and m is selected from 0, 1 and 2.

In certain embodiments, the R₁ₐ, R_{1b} and R_{1c} are each independently selected from H, methyl, ethyl, propyl, butyl, -(CR₁ₐₐR_{1bb})ₘ-cyclopropyl, -(CR₁ₐₐR_{1bb})ₘ-cyclobutyl, -(CR₁ₐₐR_{1bb})ₘ-cyclopentyl, -(CR₁ₐₐR_{1bb})ₘ-cyclohexyl, -(CR₁ₐₐR_{1bb})ₘ-oxetanyl, -(CR₁ₐₐR_{1bb})ₘ-azetidinyl, -(CR₁ₐₐR_{1bb})ₘ-tetrahydrofuryl, -(CR₁ₐₐR_{1bb})ₘ-pyrrolidyl, -(CR₁ₐₐR_{1bb})ₘ-tetrahydropyranyl, -(CR₁ₐₐR_{1bb})ₘ-piperidyl, - (CR₁ₐₐR_{1bb})ₘ-morpholinyl, -(CR₁ₐₐR_{1bb})ₘ-phenyl, -(CR₁ₐₐR_{1bb})ₘ-thienyl, - (CR₁ₐₐR_{1bb})ₘ-furyl, -(CR₁ₐₐR_{1bb})ₘ-pyrrolyl, -(CR₁ₐₐR_{1bb})ₘ-thiazolyl, -(CR₁ₐₐR_{1bb})ₘ-oxazolyl, -(CR₁ₐₐR_{1bb})ₘ-oxadiazolyl, -(CR₁ₐₐR_{1bb})ₘ-pyridyl, -(CR₁ₐₐR_{1bb})ₘ-pyrimidyl and -(CR₁ₐₐR_{1bb})ₘ-pyrazinyl, wherein R₁ₐₐ and R_{1bb} are each independently selected from H, F and -CH₃, and m is selected from 0, 1 and 2.

In certain embodiments, the R₁ₐ and R_{1c} are each independently selected from H, -CH₃, cyclopropyl, cyclobutyl, oxetanyl, tetrahydropyranyl, phenyl, pyrazolyl, imidazolyl, oxazolyl, tetrazolyl, pyrrolyl, thienyl, furyl, -CH₂-oxetanyl, -CH₂-tetrahydropyranyl, -CH₂-phenyl, -CH₂-oxadiazolyl, -CH₂-oxazolyl, -CH₂-furyl, - CH₂-thienyl, -CH₂-pyridyl, -CH₂-isoxazolyl, and -CH₂-tetrazolyl.

In certain embodiments, the R₁ₐ and R_{1c} are each independently selected from H, -CH₃, cyclopropyl, cyclobutyl, oxetanyl, tetrahydropyranyl, phenyl, -CH₂-oxetanyl, -CH₂-tetrahydropyranyl, -CH₂-phenyl and -CH₂-oxadiazolyl.

In certain embodiments, R₁ₐ is selected from H, -CH₃, cyclopropyl, cyclobutyl, oxetanyl, tetrahydropyranyl, pyrazolyl, phenyl, -CH₂-oxetanyl, -CH₂-oxadiazolyl, and -CH₂-oxazolyl; R_{1c} is selected from methyl, cyclopropyl, phenyl, -CH₂-phenyl, -CH₂-tetrahydropyranyl, pyrazolyl, imidazolyl, oxazolyl, tetrazolyl, pyrrolyl, thienyl, furyl, -CH₂-oxazolyl, -CH₂-furyl, -CH₂-thienyl, -CH₂-pyridyl, -CH₂-oxetanyl, -CH₂-isoxazolyl, and -CH₂-tetrazolyl.

In certain embodiments, the R₁ₐ and R_{1c} are each independently selected from H, methyl, pyrazolyl, -CH₂-thienyl, -CH₂-oxazolyl, -CH₂-furyl, and -CH₂-tetrazolyl.

In certain embodiments, the R₁ₐ is selected from H and methyl; R_{1c} is selected from pyrazolyl, -CH₂-thienyl, -CH₂-oxazolyl, -CH₂-furyl, and -CH₂-tetrazolyl.

In certain embodiments, the R_{1b} is H.

In certain embodiments, the R₁ is selected from -OR₁, and -NR_{1b}R_{1c}.

In certain embodiments, the R₁ is selected from -OH, -OCH₃, -O-cyclopropyl, -O-cyclobutyl, 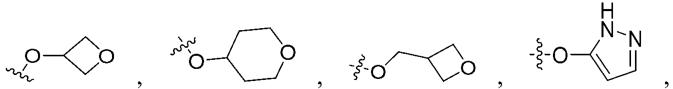 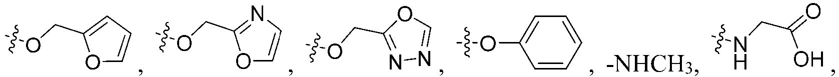 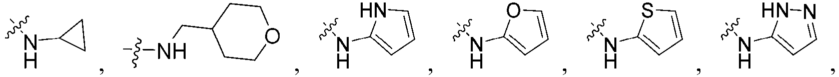 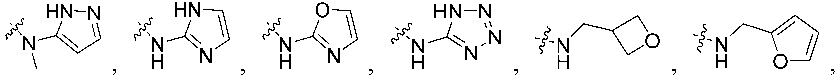 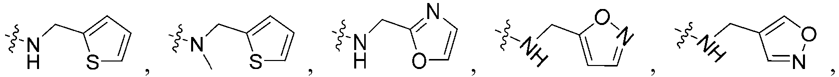 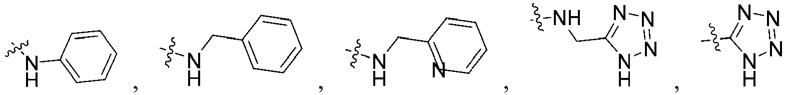 and

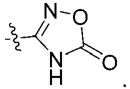

In certain embodiments, the R₁ is selected from -OH, -OCH₃, -O-cyclopropyl, -O-cyclobutyl, -O-oxetanyl (such as

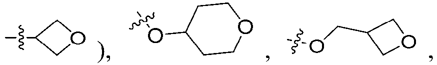

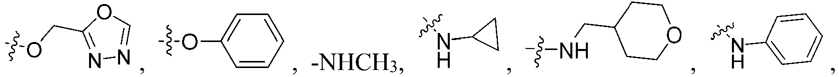

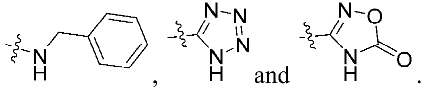

In certain embodiments, the R₁ is selected from -OH, -OCH₃, -O-cyclopropyl, -O-cyclobutyl, -O-oxetanyl (such as

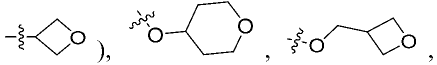

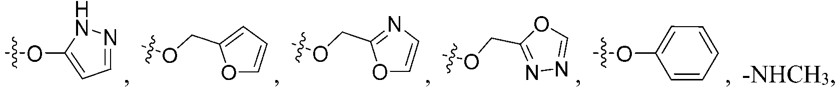

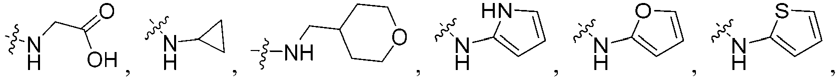

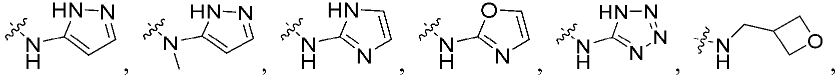

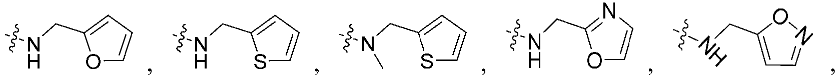

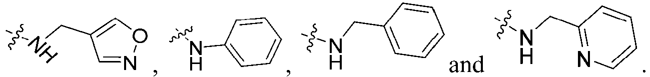

In certain embodiments, the R₁ is selected from -OH, -OCH₃, -O-cyclopropyl, -O-cyclobutyl,

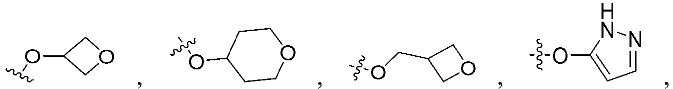

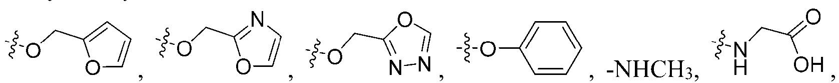

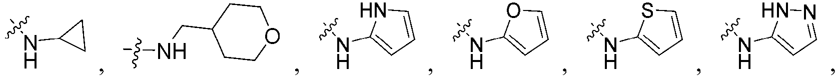

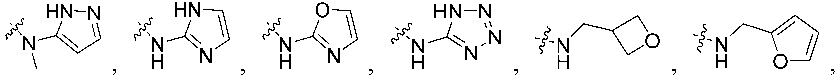

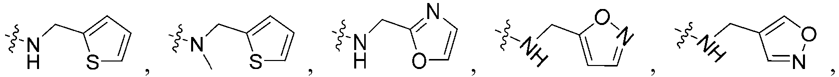

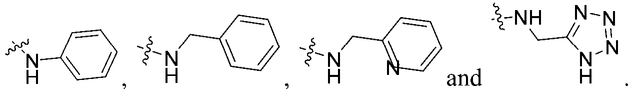

In certain embodiments, the R₁ is selected from -OH, -OCH₃, -NHCH₃,

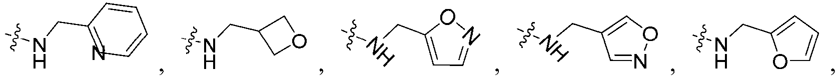

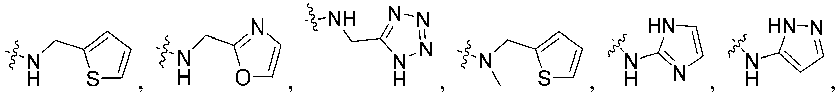

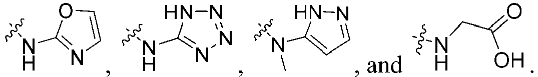

In certain embodiments, the R₁ is selected from -OH, -OCH₃,

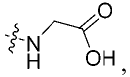

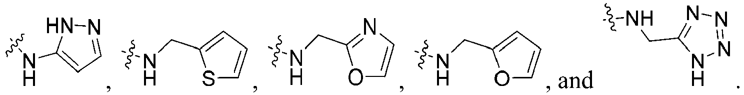

In certain embodiments, the R₁ is -OH.

In certain embodiments, the -L₁-L₂- is selected from -C(=O)C(=O)-, #X1-CR_{L1a}R_{L1b}-C(=O)-$R1, -C(=O)-, -CR_{L1a}R_{L1b}-, and #X1=CR_{L1b}-$R1.

In certain embodiments, the -L₁-L₂- is selected from -C(=O)C(=O)-, #X1-CH₂-C(=O)-$R1, #X1-CF₂-C(=O)-$R1, -C(=O)-, -CHF-, -CH₂-, and #X1=CH-$R1; wherein #X1 is the point of attachment to X₁, and $R1 is the point of attachment to R₁.

In certain embodiments, the -L₁-L₂- is selected from -C(=O)C(=O)-, #X1-CH₂-C(=O)-$R1, #X1-CF₂-C(=O)-$R1, -C(=O)-, -CHF-, and -CH₂-; wherein #X1 is the point of attachment to X₁, and $R1 is the point of attachment to R₁.

In certain embodiments, the -L₁-L₂- is selected from -C(=O)C(=O)-, #X1-CH₂-C(=O)-$R1, -C(=O)-, and #X1=CH-$R1.

In certain embodiments, the -L₁-L₂- is selected from -C(=O)- and - C(=O)C(=O)-.

In certain embodiments, the -L₁-L₂- is -C(=O)-.

In certain embodiments, the -L₁-L₂-R₁ is selected from -C(=O)-OR₁ₐ, - CR_{L1a}R_{L1b}-C(=O)-OR₁ₐ, -C(=O)-C(=O)-OR₁ₐ, -C(=O)-C(=O)-NR_{1b}R_{1c}, 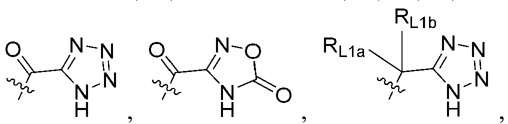 -C(=O)-NR_{1b}R_{1c}, and =CR_{L1b}-NR_{1b}R_{1c}.

In certain embodiments, the -L₁-L₂-R₁ is selected from -CR_{L1a}R_{L1b}-C(=O)-OR₁ₐ, -C(=O)-C(=O)-OR₁ₐ, -C(=O)-C(=O)-NR_{1b}R_{1c}, -C(=O)-NR_{1b}R_{1c}, and =CR_{L1b}-NR_{1b}R_{1c}.

In certain embodiments, the -L₁-L₂-R₁ is selected from -C(=O)-C(=O)-OR₁ₐ and -C(=O)-NR_{1b}R_{1c}.

In certain embodiments, the -L₁-L₂-R₁ is -C(=O)-NR_{1b}R_{1c}.

In certain embodiments, the -L₁-L₂-R₁ is selected from 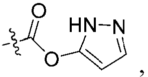 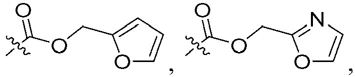 -COOH, -C(=O)-C(=O)-OH, -CH₂-C(=O)-OH, -CF₂-C(=O)-OH, -C(=O)-C(=O)-OCH₃, -C(=O)-C(=O)-NHCH₃,

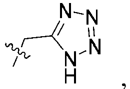

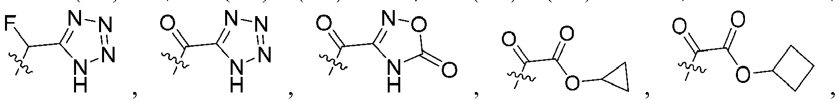

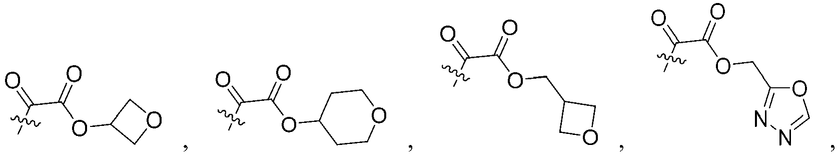

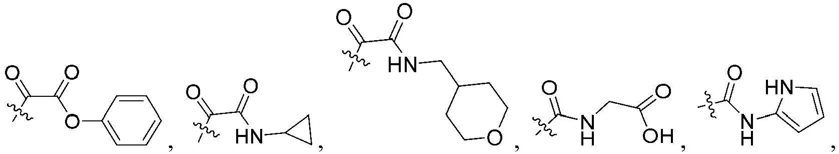

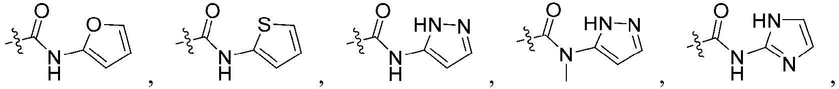

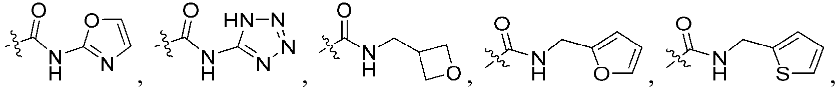

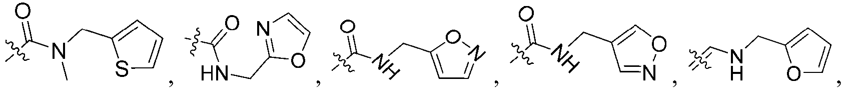

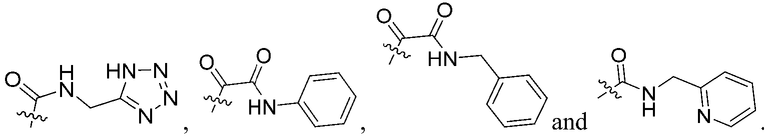

In certain embodiments, the -L₁-L₂-R₁ is selected from 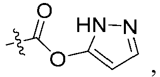 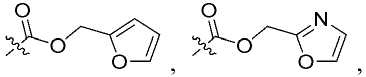 -C(=O)-C(=O)-OH, -CH₂-C(=O)-OH, -CF₂-C(=O)-OH, -C(=O)-C(=O)-OCH₃, -C(=O)-C(=O)-NHCH₃,

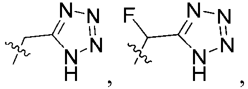

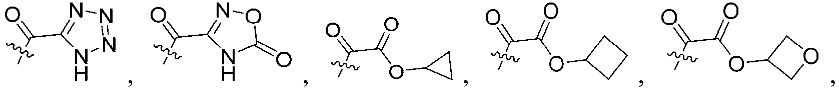

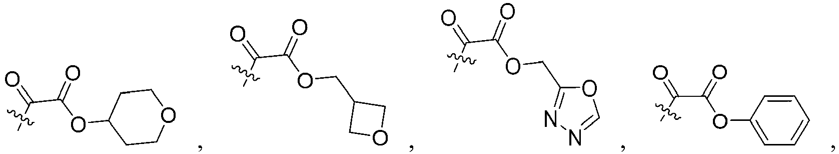

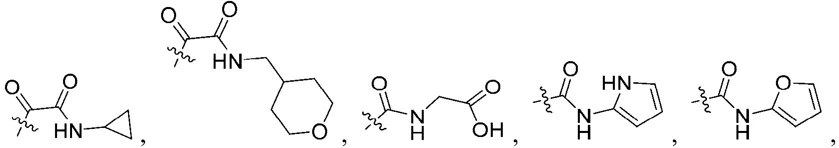

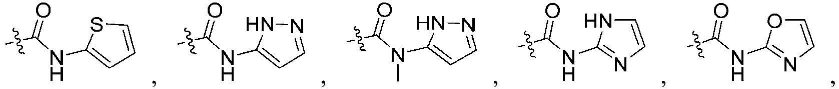

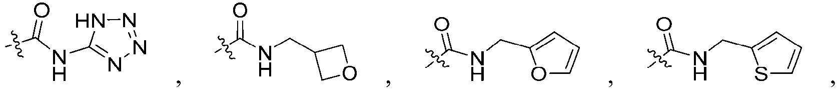

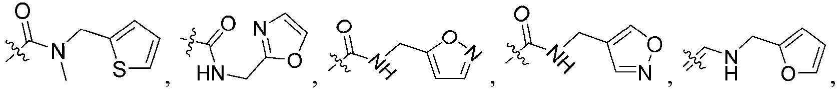

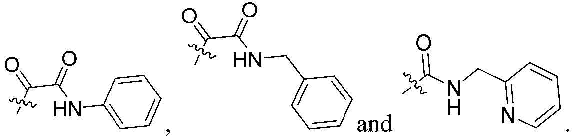

In certain embodiments, the -L₁-L₂-R₁ is selected from -COOH, -C(=O)-C(=O)-OH, -CH₂-C(=O)-OH, -CF₂-C(=O)-OH, -C(=O)-C(=O)-OCH₃, -C(=O)-C(=O)-NHCH₃, 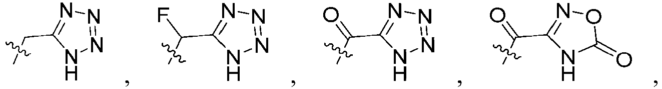 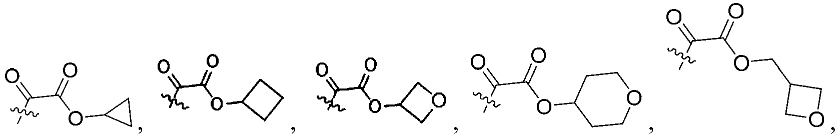 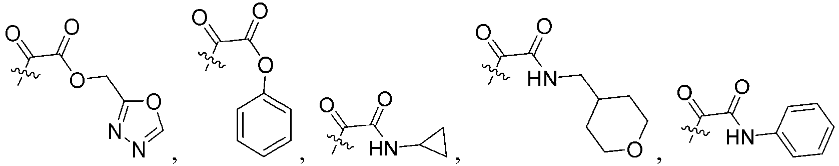 and

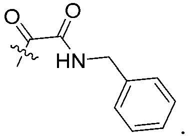

In certain embodiments, the -L₁-L₂-R₁ is selected from -CH₂-C(=O)-OH, - C(=O)-C(=O)-OH, -C(=O)-C(=O)-OCH₃, -C(=O)-C(=O)-NHCH₃,

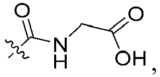

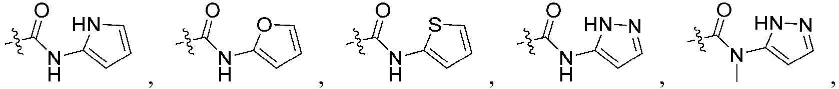

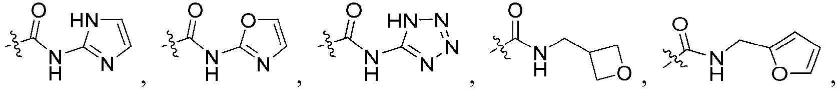

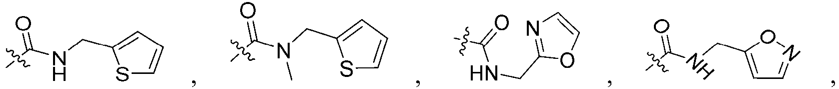

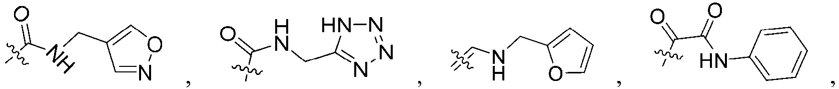

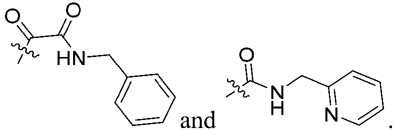

In certain embodiments, the -L₁-L₂-R₁ is selected from -CH₂-C(=O)-OH, - C(=O)-C(=O)-OH, -C(=O)-C(=O)-OCH₃, -C(=O)-C(=O)-NHCH₃, 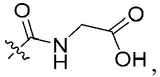 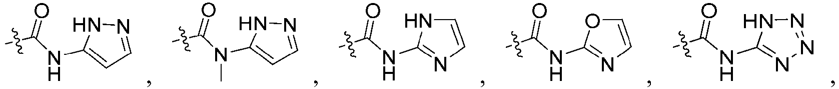 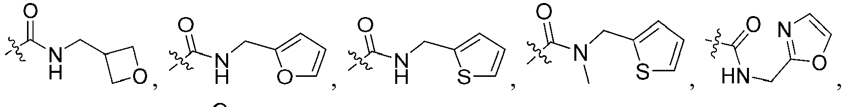 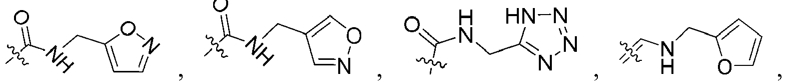 and

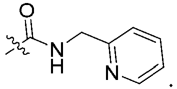

In certain embodiments, the -L₁-L₂-R₁ is selected from -C(=O)-C(=O)-OH, - C(=O)-C(=O)-OCH₃,

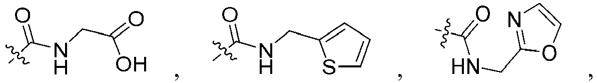

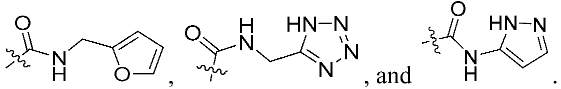

In certain embodiments, the L₁ is -C(=O)-, L₂ is a bond or -C(=O)-, R₁ is selected from -OR₁ₐ and -NR_{1b}R_{1c}, R₁ₐ is C₁₋₃ alkyl, R_{1b} is H, and R_{1c} is selected from C₁₋₃ alkyl, 5-membered heteroaryl (such as pyrrolyl, furyl, thienyl, imidazolyl, pyrazolyl, oxazolyl, and tetrazolyl), and -C₁₋₃ alkylene-5-membered heteroaryl (such as -CH₂-pyrazolyl, -CH₂-oxazolyl, -CH₂-furyl, -CH₂-thienyl, and -CH₂-tetrazolyl), wherein the C₁₋₃ alkyl is optionally substituted with carboxyl.

In certain embodiments, the L₁ is -C(=O)-, L₂ is a bond or -C(=O)-, R₁ is selected from -OR_{1A} and -NR_{1b}R_{1c}, R₁ₐ is C₁₋₃ alkyl, R_{1b} is H, and R_{1c} is selected from C₁₋₃ alkyl, 5-membered heteroaryl (such as pyrrolyl, furyl, thienyl, imidazolyl, pyrazolyl, and oxazolyl), and -C₁₋₃ alkylene-5-membered heteroaryl (such as -CH₂-pyrazolyl, -CH₂-oxazolyl, and -CH₂-furyl), wherein the C₁₋₃ alkyl is optionally substituted with carboxyl.

In certain embodiments, the L₂ is a bond.

In certain embodiments, the -L₁-L₂-R₁ is -C(=O)-C(=O)-OH.

In certain embodiments, the -L₁-L₂-R₁ is selected from -C(=O)-C(=O)-OCH₃, -C(=O)-C(=O)-NHCH₃,

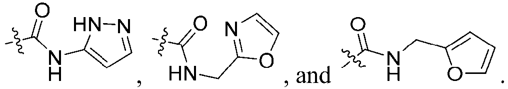

In certain embodiments, the R_{L3} is selected from H, D, -CH₃, -CH₂CH₃ and - CH(CH₃)₂.

In certain embodiments, the R_{L3} is H.

In certain embodiments, the L₃ is selected from a bond, -O-, -NR_{L3}-, -S-, #-C₁₋₆ alkylene-O-*, #-C₁₋₆ alkylene-NR_{L3}-*, #-C₁₋₆ alkylene-S-*, -C₁₋₆ alkylene-, -C₂₋₆ alkenylene- and -C₂₋₆ alkynylene-, wherein the end indicated by "#" is attached to R₂, and the end indicated by "*" is attached to

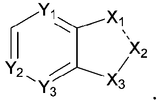

In certain embodiments, the L₃ is selected from a bond, -O-, -NR_{L3}-, -S-, #-C₁₋₄ alkylene-O-*, #-C₁₋₄ alkylene-NR_{L3}-*, #-C₁₋₄ alkylene-S-*, -C₁₋₄ alkylene-, -C₂₋₄ alkenylene- and -C₂₋₄ alkynylene-, wherein the end indicated by "#" is attached to R₂, the end indicated by "*" is attached to 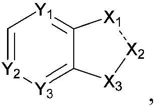 and R_{L3} is as defined in the present invention.

In certain embodiments, the L₃ is selected from a bond, -O-, -NR_{L3}-, -S-, #-(CH₂)ₙ-O-*, #-(CH₂)ₙ-NR_{L3}-*, #-(CH₂)ₙ-S-*, vinylene, propenylene, ethynylene and propynylene, wherein the end indicated by "#" is attached to R₂, the end indicated by "*" is attached to 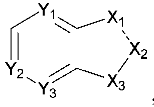 , n is selected from 1 and 2, and R_{L3} is as defined in the present invention.

In certain embodiments, the L₃ is selected from a bond, -O-, -NH-, #-CH₂-NH-*, #-CH₂-S-* and -C≡C-, wherein the end indicated by "#" is attached to R₂, and the end indicated by "*" is attached to

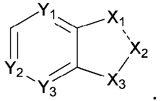

In certain embodiments, the L₃ is a bond.

In certain embodiments, each of the R is independently selected from halogen, -OH, -CN, -COOH, oxo, C₁₋₄ alkyl, -C₁₋₄ alkylene-OH, C₁₋₄ alkoxy, -C₁₋₄ alkyleneoxy-C₁₋₄ alkoxy, -C(=O)NH-C₁₋₄ alkyl, -C(=O)-C₁₋₄ alkyl, -NHC(=O)-C₁₋₄ alkyl, -N=S(=O)(C₁₋₄ alkyl)-C₁₋₄ alkyl, -NH-S(=O)₂C₁₋₄ alkyl, -S(=O)₂-C₁₋₄ alkyl,-S(=O)₂-4- to 6-membered heterocyclyl, -C(=O)-4- to 6-membered heterocyclyl, 4-to 6-membered heterocyclyl, 5- to 6-membered heteroaryl and

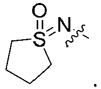

In certain embodiments, each of the R is independently selected from halogen, -OH, -CN, -COOH, C₁₋₄ alkyl, -C₁₋₄ alkylene-OH, C₁₋₄ alkoxy, -C₁₋₄ alkyleneoxy-C₁₋₄ alkoxy, -C(=O)NH-C₁₋₄ alkyl, -C(=O)-C₁₋₄ alkyl, -NHC(=O)-C₁₋₄ alkyl, - N=S(=O)(C₁₋₄ alkyl)-C₁₋₄ alkyl, -NH-S(=O)₂C₁₋₄ alkyl, -S(=O)₂-C₁₋₄ alkyl, -S(=O)₂-4- to 6-membered heterocyclyl, -C(=O)-4- to 6-membered heterocyclyl, 4- to 6-membered heterocyclyl, 5- to 6-membered heteroaryl and

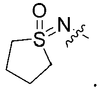

In certain embodiments, the R is selected from -OH and -C₁₋₄ alkylene-OH.

In certain embodiments, the R is selected from halogen, -OH, -CN, -COOH, - CH₂OH, methyl, ethyl, propyl, butyl, -ethylene-OH, -propylene-OH, -butylene-OH, methoxy, ethoxy, propoxy, butoxy, -methyleneoxy-methoxy, -methyleneoxy-ethoxy, -methyleneoxy-propoxy, -ethyleneoxy-methoxy, -ethyleneoxy-ethoxy, - ethyleneoxy-propoxy, -C(=O)-NH-methyl, -C(=O)-NH-ethyl, -C(=O)-NH-propyl, - C(=O)-NH-butyl, -N=S(=O)(methyl)-methyl, -N=S(=O)(methyl)-ethyl, - N=S(=O)(methyl)-propyl, -N=S(=O)(methyl)-butyl, -N=S(=O)(ethyl)-ethyl, - N=S(=O)(ethyl)-propyl, -(NH)ₚ-W-methyl, -(NH)ₚ-W-ethyl, -(NH)ₚ-W-propyl, - (NH)ₚ-W-butyl, -(W)_{q}-oxetanyl, -(W)_{q}-azetidinyl, -(W)_{q}-tetrahydrofuryl, -(W)_{q}-pyrrolidyl, -(W)_{q}-tetrahydropyranyl, -(W)_{q}-piperidyl, -(W)_{q}-morpholinyl, thienyl, furyl, pyrrolyl, thiazolyl, oxazolyl, pyridyl, pyrimidyl, pyrazinyl and 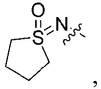 wherein W is C(=O) or S(=O)₂, and each p or q is independently 0 or 1.

In certain embodiments, the R is selected from F, -OH, -CN, -COOH, -CH₃, - CH₂OH, -C(CH₃)₂OH, -CH₂C(CH₃)₂OH, -OCH₃, -OCH₂CH₂OCH₃, -C(=O)CH₃, - C(=O)NHCH₃, -NHC(=O)CH₃, -N=S(=O)(CH₃)₂, -NH-S(=O)₂CH₃, -S(=O)₂CH₃, - S(=O)₂-piperidyl (such as 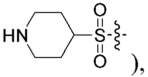 -C(=O)-pyrrolidyl (such as 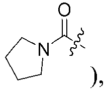 C(=O)-morpholinyl (such as 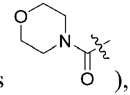 morpholinyl (such as 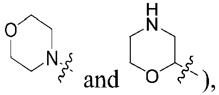 pyrazolyl (such as

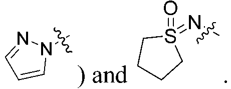

In certain embodiments, R is -OH.

In certain embodiments, R is selected from -OH and -CH₂OH.

In certain embodiments, the R₂ₐ, R_{2b}, R_{2c}, R_{2d} and R₂ₑ are each independently selected from H, halogen, -OH, C₁₋₄ alkyl, C₁₋₄ haloalkyl, -NHC₁₋₄ alkyl, -N(C₁₋₄ alkyl)-C₁₋₄ alkyl, C₁₋₄ alkoxy, C₁₋₄ haloalkoxy, C₃₋₆ cycloalkyl, C₃₋₆ cycloalkenyl, 4-to 6-membered heterocyclyl, phenyl, 5- to 6-membered heteroaryl, -phenylene-4- to 6-membered heterocyclyl, -phenylene-5- to 6-membered heteroaryl, -phenylene-phenyl and -5- to 6-membered heteroarylene-5- to 6-membered heteroaryl, and the C₁₋₄ alkyl, C₁₋₄ haloalkyl, -NHC₁₋₄ alkyl, -N(C₁₋₄ alkyl)-C₁₋₄ alkyl, C₁₋₄ alkoxy, C₁₋₄ haloalkoxy, C₃₋₆ cycloalkyl, C₃₋₆ cycloalkenyl, 4- to 6-membered heterocyclyl, phenyl, 5- to 6-membered heteroaryl, -phenylene-4- to 6-membered heterocyclyl, - phenylene-5- to 6-membered heteroaryl, -phenylene-phenyl and -5- to 6-membered heteroarylene-5- to 6-membered heteroaryl are optionally substituted with 1, 2 or 3 R, wherein R is as defined in the present invention.

In certain embodiments, the R₂ₐ, R_{2b}, R_{2c}, R_{2d} and R₂ₑ are each independently selected from H, C₁₋₄ alkyl, C₁₋₄ alkoxy, C₃₋₆ cycloalkyl, 4- to 6-membered heterocyclyl, phenyl, and 5- to 6-membered heteroaryl; the C₁₋₄ alkyl, C₁₋₄ alkoxy, C₃₋₆ cycloalkyl, 4- to 6-membered heterocyclyl, phenyl, and 5- to 6-membered heteroaryl are optionally substituted with 1, 2 or 3 R.

In certain embodiments, the R₂ₐ, R_{2b}, R_{2c}, R_{2d} and R₂ₑ are each independently selected from H, C₁₋₄ alkyl, C₁₋₄ alkoxy, C₃₋₆ cycloalkyl, and phenyl; the C₁₋₄ alkyl, C₁₋₄ alkoxy, C₃₋₆ cycloalkyl, and phenyl are optionally substituted with 1, 2 or 3 R.

In certain embodiments, the R₂ₐ, R_{2b}, R_{2c}, R_{2d} and R₂ₑ are each independently selected from H, C₁₋₄ alkyl, C₃₋₆ cycloalkyl, and phenyl; the C₁₋₄ alkyl, C₃₋₆ cycloalkyl, and phenyl are optionally substituted with 1, 2 or 3 R.

In certain embodiments, the R₂ₐ, R_{2b}, R_{2c}, R_{2d} and R₂ₑ are each independently selected from H, F, Cl, Br, -OH, methyl, ethyl, propyl, butyl, halomethyl, haloethyl, halopropyl, halobutyl, -NH-methyl, -NH-ethyl, -NH-propyl, -NH-butyl, -N(CH₃)₂, - N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, -(phenylene)ᵣ-oxetanyl, -(phenylene)ᵣ-azetidinyl, -(phenylene)ᵣ-tetrahydrofuryl, -(phenylene)ᵣ-pyrrolidyl, -(phenylene)ᵣ-tetrahydropyranyl, -(phenylene)ᵣ-piperidyl, -(phenylene)ᵣ-morpholinyl, - (phenylene)ᵣ-oxopyrrolidyl, -(phenylene)ᵣ-oxoimidazolyl, -(phenylene)ᵣ-oxopyrazolyl, -(phenylene)ᵣ-oxodihydrotriazolyl, -(phenylene)ᵣ-phenyl, -(V)ᵣ-thienyl, -(V)ᵣ-furyl, -(V)ᵣ-pyrrolyl, -(V)ᵣ-imidazolyl, -(V)ᵣ-pyrazolyl, -(V)ᵣ-thiazolyl, -(V)ᵣ-oxazolyl, -(V)ᵣ-oxadiazolyl, -(V)ᵣ-triazolyl, -(V)ᵣ-tetrazolyl, -(V)ᵣ-pyridyl,-(V)ᵣ-pyrimidyl and -(V)ᵣ-pyrazinyl, and the methyl, ethyl, propyl, butyl, halomethyl, haloethyl, halopropyl, halobutyl, -NH-methyl, -NH-ethyl, -NH-propyl, -NH-butyl, - N(CH₃)₂, -N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, -(phenylene)ᵣ-oxetanyl, - (phenylene)ᵣ-azetidinyl, -(phenylene)ᵣ-tetrahydrofuryl, -(phenylene)ᵣ-pyrrolidyl, - (phenylene)ᵣ-tetrahydropyranyl, -(phenylene)ᵣ-piperidyl, -(phenylene)ᵣ-morpholinyl, -(phenylene)ᵣ-oxopyrrolidyl, -(phenylene)ᵣ-oxoimidazolyl, -(phenylene)ᵣ-oxopyrazolyl, -(phenylene)ᵣ-oxodihydrotriazolyl, -(phenylene)ᵣ-phenyl, -(V)ᵣ-thienyl, -(V)ᵣ-furyl, -(V)ᵣ-pyrrolyl, -(V)ᵣ-imidazolyl, -(V)ᵣ-pyrazolyl, -(V)ᵣ-thiazolyl, -(V)ᵣ-oxazolyl, -(V)ᵣ-oxadiazolyl, -(V)ᵣ-triazolyl, -(V)ᵣ-tetrazolyl, -(V)ᵣ-pyridyl, - (V)ᵣ-pyrimidyl and -(V)ᵣ-pyrazinyl are optionally substituted with 1, 2 or 3 R, wherein V is selected from phenylene and pyridylene, r is 0 or 1, and R is as defined in the present invention.

In certain embodiments, the R₂ₐ, R_{2b}, R_{2c}, R_{2d} and R₂ₑ are each independently selected from H, F, -OH, -CH₃, -CH₂CH₃, -CH₂CH₂CH₃, -CH(CH₃)₂, -C(CH₃)₃,-N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, morpholinyl, tetrahydrofuryl, pyrrolidyl, tetrahydropyranyl, piperidyl, phenyl, pyridyl, -phenylene-oxetanyl, - phenylene-morpholinyl, -phenylene-oxopyrrolidyl, -phenylene-oxoimidazolyl, - phenylene-oxopyrazolyl, -phenylene-oxodihydrotriazolyl, -phenylene-phenyl, - phenylene-imidazolyl, -phenylene-pyrazolyl, -phenylene-oxadiazolyl, -phenylene-triazolyl, -phenylene-tetrazolyl, -phenylene-pyrimidyl and -pyridylene-pyrazolyl, and the -CH₃, -CH₂CH₃, -CH₂CH₂CH₃, -CH(CH₃)₂, -C(CH₃)₃, -N(CH₃)₂, -OCH₃,-OCH₂CH₃, -OCH₂CH₂CH₃, -OCH(CH₃)₂, -OCF₃, cyclopropyl, cyclobutyl, cyclohexenyl, azetidinyl, oxetanyl, morpholinyl, tetrahydrofuryl, pyrrolidyl, tetrahydropyranyl, piperidyl, phenyl, pyridyl, -phenylene-oxetanyl, -phenylene-morpholinyl, -phenylene-oxopyrrolidyl, -phenylene-oxoimidazolyl, -phenylene-oxopyrazolyl, -phenylene-oxodihydrotriazolyl, -phenylene-phenyl, -phenylene-imidazolyl, -phenylene-pyrazolyl, -phenylene-oxadiazolyl, -phenylene-triazolyl, - phenylene-tetrazolyl, -phenylene-pyrimidyl and -pyridylene-pyrazolyl are optionally substituted with 1, 2 or 3 R, wherein R is as defined in the present invention.

In certain embodiments, the R₂ₐ, R_{2b}, R_{2c}, R_{2d} and R₂ₑ are each independently selected from H, methyl, methoxy, cyclopropyl, cyclobutyl, and phenyl, and the methyl, methoxy, cyclopropyl, cyclobutyl, and phenyl are optionally substituted with 1, 2 or 3 R.

In certain embodiments, the R₂ₐ, R_{2b}, R_{2c}, R_{2d} and R₂ₑ are each independently selected from H, methyl, cyclopropyl, cyclobutyl, and phenyl, and the methyl, cyclopropyl, cyclobutyl, and phenyl are optionally substituted with 1, 2 or 3 R.

In certain embodiments, the R₂ is selected from C₃₋₆ cycloalkyl, phenyl, 5- to 6-membered heteroaryl, 9-membered heteroaryl, 9- to 10-membered heterocyclyl and C₉₋₁₀ cyclic group, and the C₃₋₆ cycloalkyl, phenyl, 5- to 6-membered heteroaryl, 9-membered heteroaryl, 9- to 10-membered heterocyclyl and C₉₋₁₀ cyclic group are optionally substituted with 1, 2, 3, 4, or 5 groups each independently selected from R₂ₐ, R_{2b}, R_{2c}, R_{2d} and R₂ₑ, wherein R₂ₐ, R_{2b}, R_{2c}, R_{2d} and R₂ₑ are as defined in the present invention.

In certain embodiments, the R₂ is selected from phenyl and 9-membered heteroaryl, and the phenyl and 9-membered heteroaryl are optionally substituted with 1, 2, 3, 4, or 5 groups each independently selected from R₂ₐ, R_{2b}, R_{2c}, R_{2d} and R₂ₑ.

In certain embodiments, the R₂ is selected from cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, 2,3-dihydrobenzofuryl, 2,3-dihydrobenzodioxanyl and 2,3-dihydroindenyl, and the cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, 2,3-dihydrobenzofuryl, 2,3-dihydrobenzodioxanyl and 2,3-dihydroindenyl are optionally substituted with 1, 2, 3, 4, or 5 groups each independently selected from R₂ₐ, R_{2b}, R_{2c}, R_{2d} and R₂ₑ, wherein R₂ₐ, R_{2b}, R_{2c}, R_{2d} and R₂ₑ are as defined in the present invention.

In certain embodiments, R₂ is selected from phenyl and indolyl, and the phenyl and indolyl are optionally substituted with 1, 2, 3, 4, or 5 groups each independently selected from R₂ₐ, R_{2b}, R_{2c}, R_{2d} and R₂ₑ.

In certain embodiments, the R₂ is selected from cyclopropyl, cyclobutyl, phenyl, thienyl, thiazolyl, oxazolyl, pyridyl, indolyl, indolinyl, 2,3-dihydrobenzofuryl, 2,3-dihydrobenzodioxanyl and 2,3-dihydroindenyl, and the cyclopropyl, cyclobutyl, phenyl, thienyl, thiazolyl, oxazolyl, pyridyl, indolyl, indolinyl, 2,3-dihydrobenzofuryl, 2,3-dihydrobenzodioxanyl and 2,3-dihydroindenyl are optionally substituted with 1, 2, 3, 4, or 5 groups each independently selected from R₂ₐ, R_{2b}, R_{2c}, R_{2d} and R₂ₑ, wherein R₂ₐ, R_{2b}, R_{2c}, R_{2d} and R₂ₑ are as defined in the present invention.

In certain embodiments, the R₂ is selected from 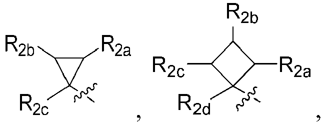 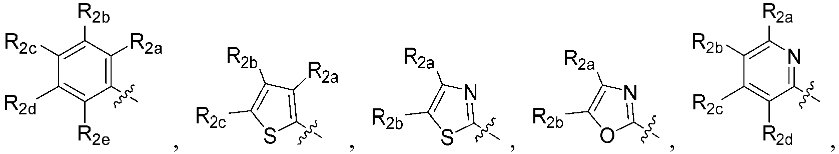 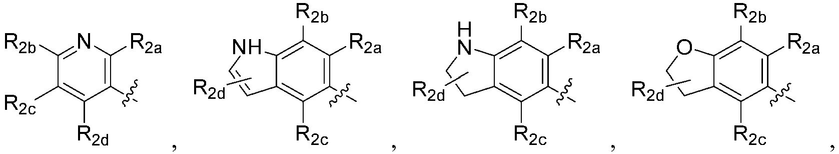 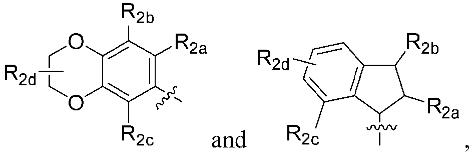 **wherein** R₂ₐ, R_{2b}, R_{2c}, R_{2d} and R₂ₑ are as defined in the present invention.

In certain embodiments, the R₂ is selected from 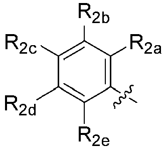 and

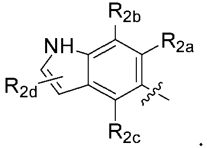

In certain embodiments, the R₂ is selected from 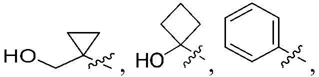 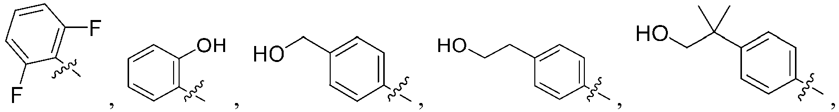 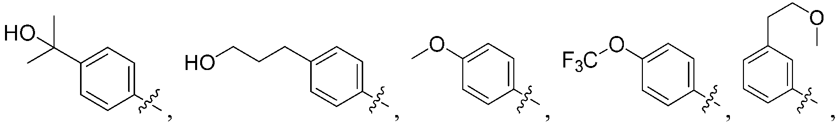 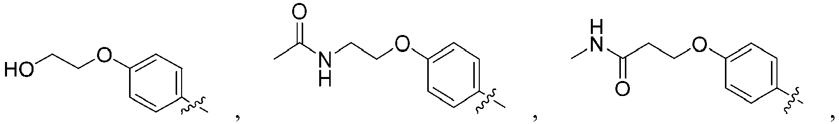 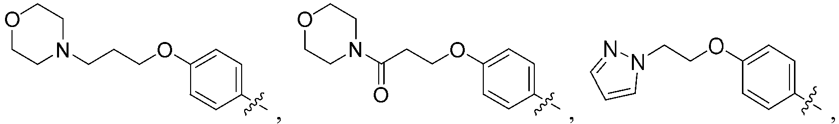 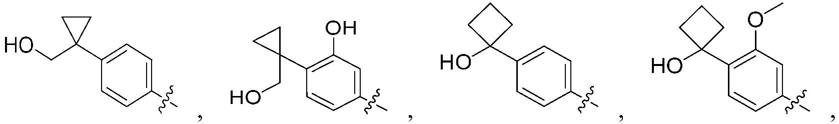 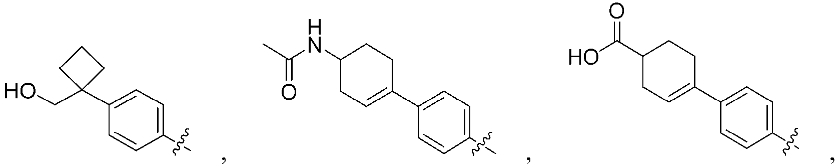 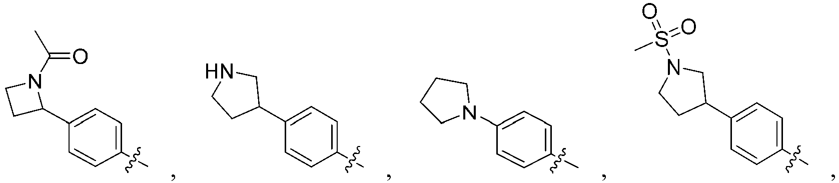 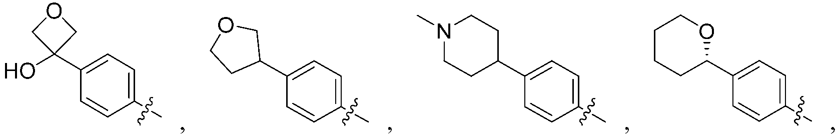 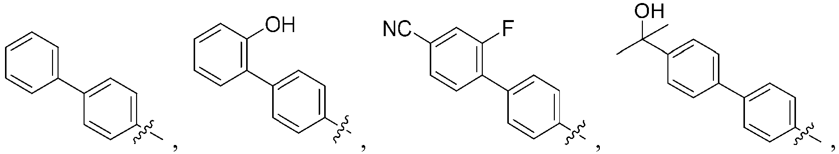 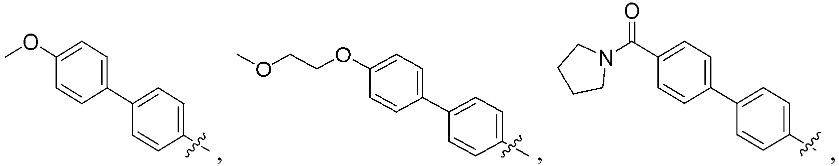 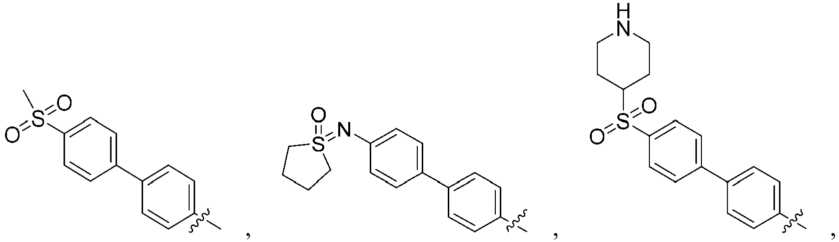 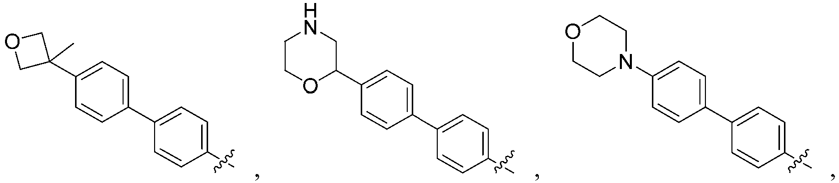 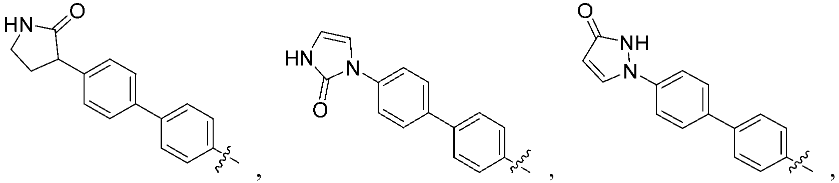 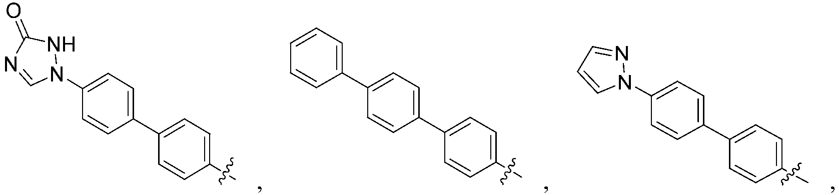 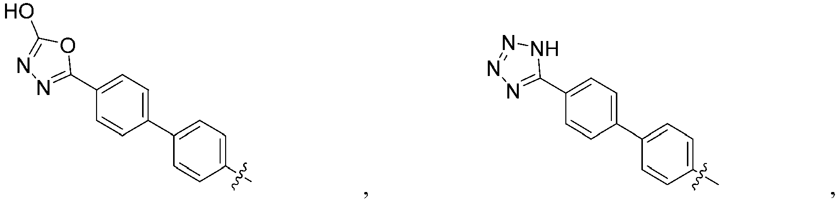 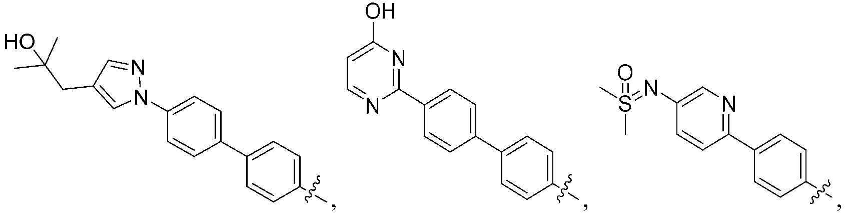 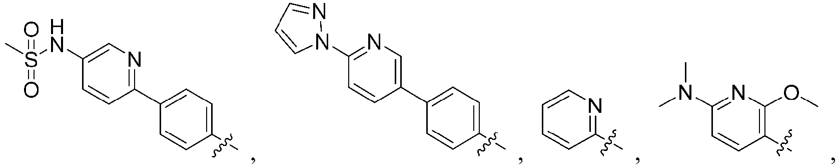 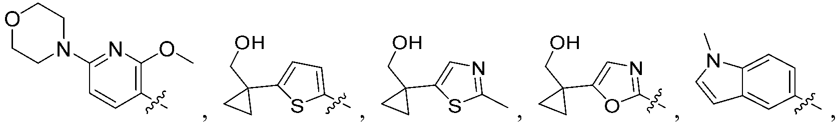 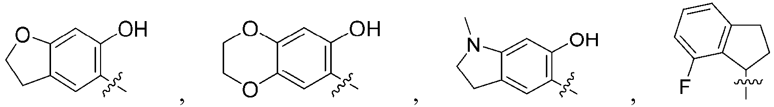 and

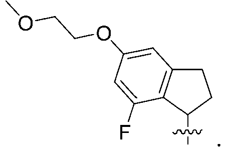

In certain embodiments, the R₂ is selected from 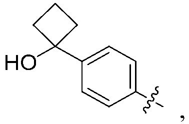 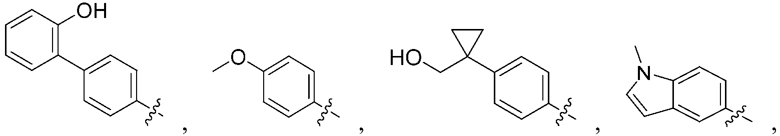 and

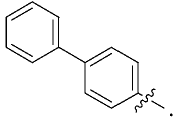

In certain embodiments, the R₂ is selected from

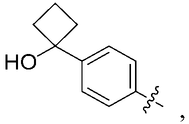

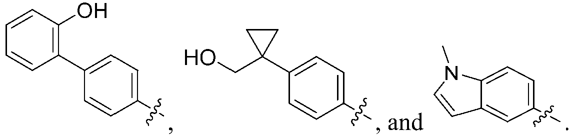

In certain embodiments, the R₂ is selected from 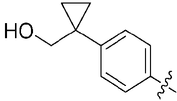 and

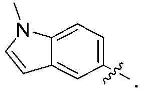

In certain embodiments, the compound is selected from formula (I-1) and (I-2), 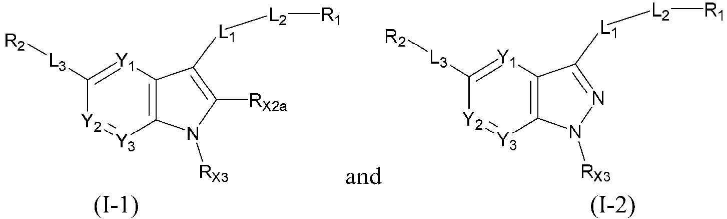 wherein,
Y₁, Y₂, Y₃, L₁, L₂, L₃, R₁, R₂, R_{X2a} and R_{X3} are as defined in the present invention.

In certain embodiments, the compound is selected from formula (I-3), (I-4) and (I-5) 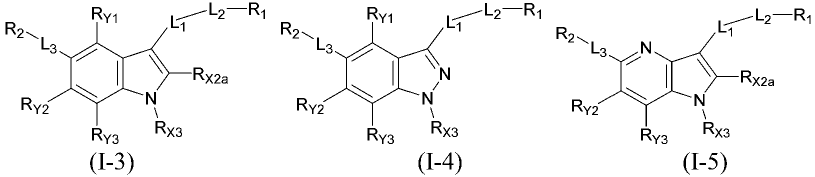 wherein,
L₁, L₂, L₃, R₁, R₂, R_{X2a}, R_{X3}, R_{Y1}, R_{Y2} and R_{Y3} are as defined in the present invention.

In certain embodiments, the compound is selected from formula (1-6), (I-7), (I-8), (1-9), (I-10), (I-11) and (I-12), 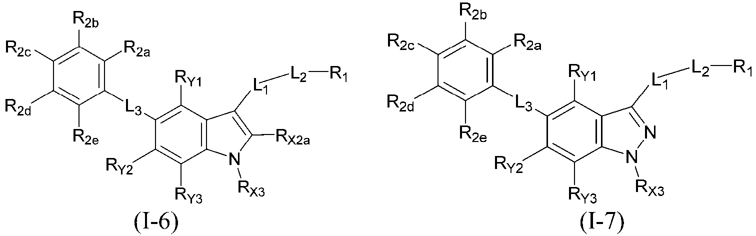 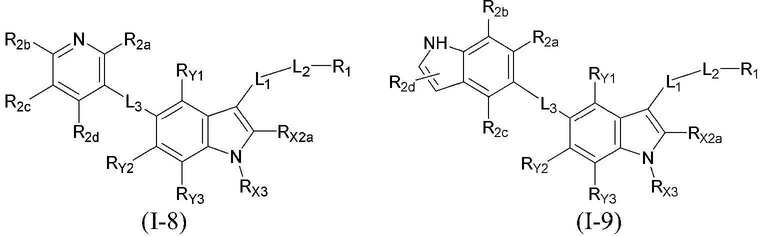 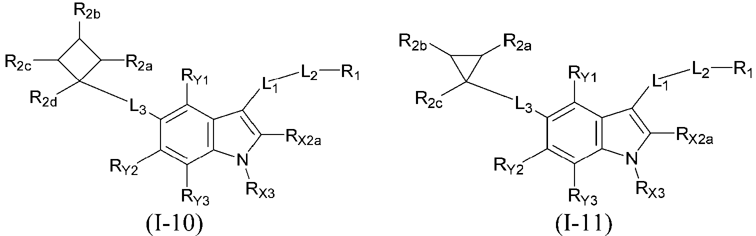 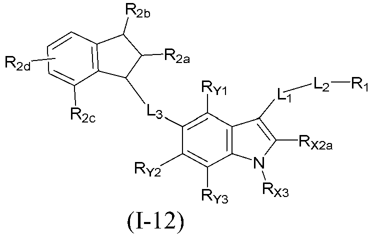 wherein,
L₁, L₂, L₃, R₁, R_{X2a}, R_{X3}, R_{Y1}, R_{Y2}, R_{Y3}, R₂ₐ, R_{2b}, R_{2c}, R_{2d} and R₂ₑ are as defined in the present invention.

In certain embodiments, the compound is selected from formula (1-13), (I-14), (I-15), (I-16), (I-17), (I-18), (I-19), (I-20) and (I-21), 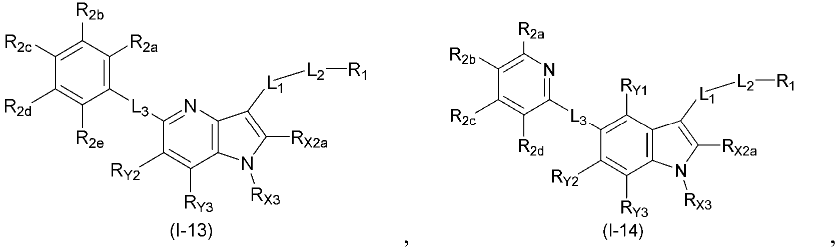 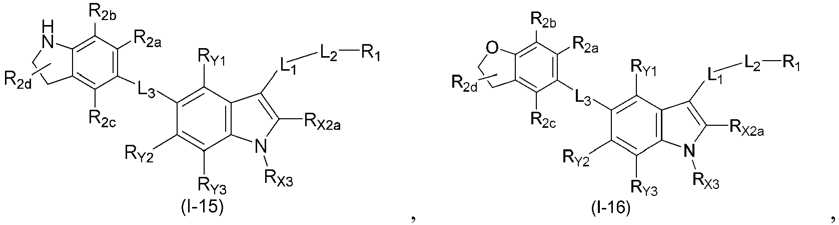 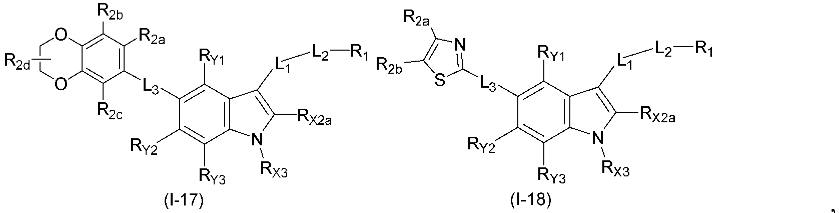 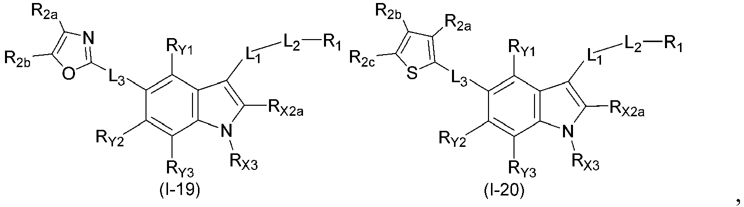 and

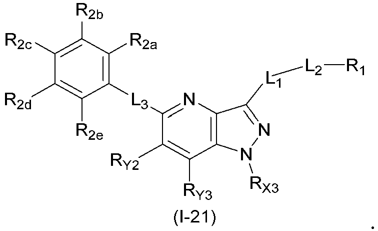

In certain embodiments, the compound is selected from formula (I-A), (I-B) and (I-C), 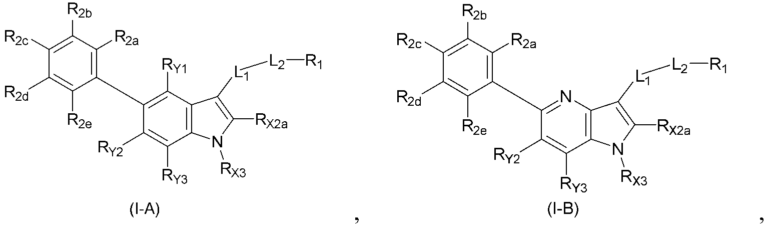 and

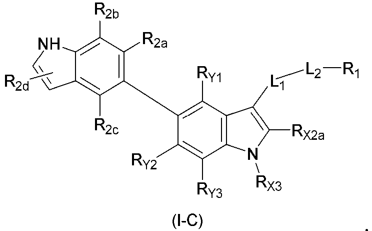

In certain embodiments, the L₂ is -C(=O)-.

In certain embodiments, the compound has a structure as shown in formula (II-1), 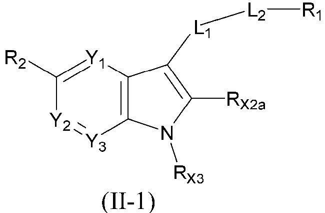 wherein Y₁, Y₂, Y₃, L₁, L₂, R₁, R₂, R_{X2a} and R_{X3} are as defined in the present invention.

In certain embodiments, the compound is selected from formula (II-2) and (II-3), 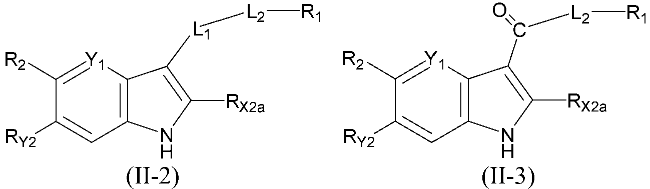 wherein Y₁, L₁, L₂, R₁, R₂, R_{X2a} and R_{Y2} are as defined in the present invention.

In certain embodiments, the compound is selected from formula (II-2') and (II-3),

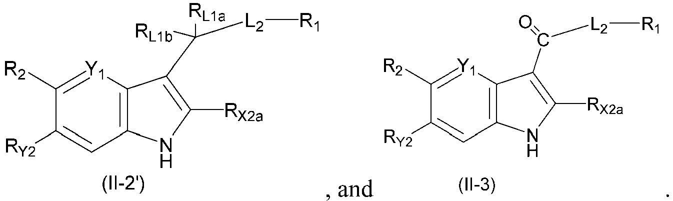

In certain embodiments, the compound is selected from formula (II-4), (II-5), (II-6) and (II-7), 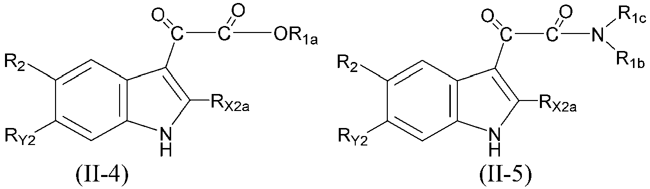 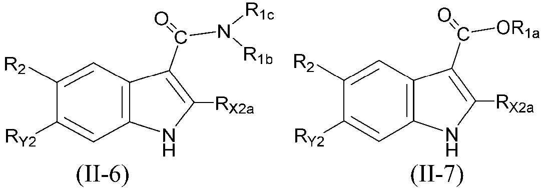 wherein R₂, R_{X2a}, R_{Y2}, R₁ₐ, R_{1b} and R_{1c} are as defined in the present invention.

In certain embodiments, the compound is selected from formula (II-8) and (II-9),

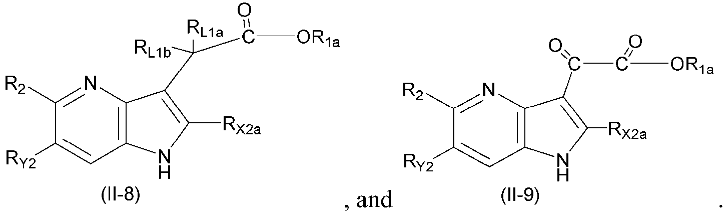

In certain embodiments, the compound has a structure as shown in formula (II-6), 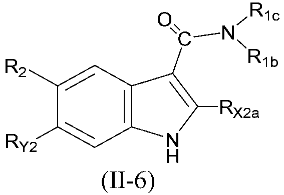
wherein R_{X2a} is selected from H, -OH, and -C₁₋₃ alkoxy;
R_{Y2} is halogen;
R_{1b} is selected from H and C₁₋₃ alkyl;
R_{1c} is selected from C₁₋₃ alkyl, 5- to 6-membered heteroaryl, -C₁₋₃ alkylene-5-to 6-membered heteroaryl, and -C₁₋₃ alkylene-4- to 7-membered heterocyclyl, and the C₁₋₃ alkyl is optionally substituted with carboxyl;
R₂ is selected from phenyl and 5- to 10-membered heteroaryl, wherein the phenyl and 5-10 heteroaryl are optionally substituted with a substituent selected from C₁₋₃ alkyl, C₁₋₃ alkoxy, C₃₋₆ cycloalkyl, and phenyl, and the C₃₋₆ cycloalkyl and phenyl serving as substituents on the phenyl and 5- to 10-membered heteroaryl are optionally substituted with a substituent selected from -OH and -C₁₋₃ alkylene-OH.

In certain embodiments, R_{X2a} is selected from H and -OH.

In certain embodiments, R_{X2a} is H.

In certain embodiments, R_{Y2} is Cl.

In certain embodiments, R_{1b} is selected from H and methyl.

In certain embodiments, R_{1b} is H.

In certain embodiments, R_{1c} is selected from C₁₋₃ alkyl, C₃₋₆ cycloalkyl, phenyl, 5- to 6-membered heteroaryl, 4- to 7-membered heterocyclyl, -C₁₋₃ alkylene-C₃₋₆ cycloalkyl, -C₁₋₃ alkylene-phenyl, -C₁₋₃ alkylene-5- to 6-membered heteroaryl, and - C₁₋₃ alkylene-4- to 7-membered heterocyclyl, and the C₁₋₃ alkyl is optionally substituted with carboxyl.

In certain embodiments, R_{1c} is selected from C₁₋₃ alkyl, C₃₋₆ cycloalkyl, phenyl, 5- to 6-membered heteroaryl, -C₁₋₃ alkylene-phenyl, -C₁₋₃ alkylene-5- to 6-membered heteroaryl, and -C₁₋₃ alkylene-4- to 7-membered heterocyclyl, and the C₁₋₃ alkyl is optionally substituted with carboxyl.

In certain embodiments, R_{1c} is selected from C₁₋₃ alkyl, 5- to 6-membered heteroaryl, -C₁₋₃ alkylene-5- to 6-membered heteroaryl, and -C₁₋₃ alkylene-4- to 7-membered heterocyclyl, and the C₁₋₃ alkyl is optionally substituted with carboxyl.

In certain embodiments, R_{1c} is selected from CH₂C(=O)OH, pyrazolyl (such as 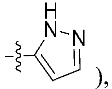 imidazolyl (such as 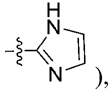 oxazolyl (such as 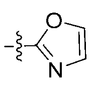 ), 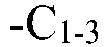 alkylene-pyridyl (such as 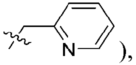 -C₁₋₃ alkylene-furyl (such as 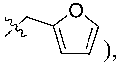 C₁₋₃ alkylene-thienyl (such as 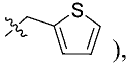 -C₁₋₃ alkylene-oxazolyl (such as 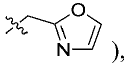 -C₁₋₃ alkylene-isoxazolyl (such as 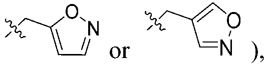 -C₁₋₃ alkylene-tetrazolyl (such as 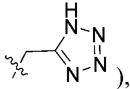 and -C₁₋₃ alkylene-oxetanyl (such as

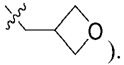

In certain embodiments, R_{1c} is selected from pyrazolyl (such as 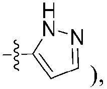 imidazolyl (such as 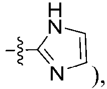 oxazolyl (such as 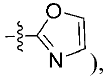 -C₁₋₃ alkylene-pyridyl (such as 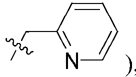 , -C₁₋₃ alkylene-furyl (such as 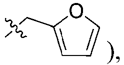 -C₁₋₃ alkylene-thienyl (such as 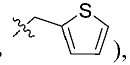 -C₁₋₃ alkylene-oxazolyl (such as 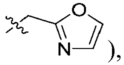 and -C₁₋₃ alkylene-isoxazolyl (such as

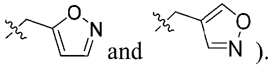

In certain embodiments, R₂ is selected from phenyl and 9-membered heteroaryl (such as indolyl), wherein the phenyl and 9-membered heteroaryl are optionally substituted with a substituent selected from C₁₋₃ alkyl, C₁₋₃ alkoxy, C₃₋₆ cycloalkyl, and phenyl, and the C₃₋₆ cycloalkyl and phenyl serving as substituents on the phenyl and 9-membered heteroaryl are optionally substituted with a substituent selected from -OH and -C₁₋₃ alkylene-OH.

In certain embodiments, R₂ is phenyl, the phenyl is substituted with a substituent selected from C₁₋₃ alkyl, C₁₋₃ alkoxy, C₃₋₆ cycloalkyl, and phenyl, and the C₃₋₆ cycloalkyl and phenyl serving as substituents on the phenyl are optionally substituted with a substituent selected from -OH and -C₁₋₃ alkylene-OH.

In certain embodiments, R₂ is selected from 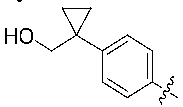 and

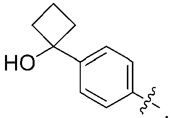

In certain embodiments, R₂ is indolyl, and the indolyl is substituted with C₁₋₃ alkyl.

In certain embodiments, R₂ is

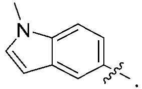

In certain embodiments, R_{X2a} is H; R_{Y2} is Cl; R_{1b} is H.

In certain embodiments, the compound is selected from (V-1), 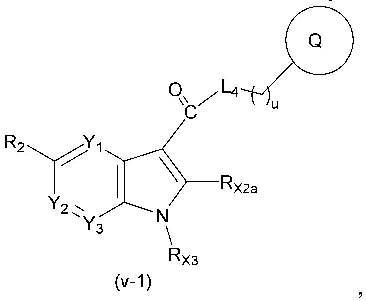 wherein,
L₄ is selected from -O- and -NR_{1b}-;
u is selected from 0, 1 and 2;
Q is selected from C₃₋₁₀ cycloalkyl, 4- to 10-membered heterocyclyl, 5- to 10-membered heteroaryl, and C₆₋₁₀ aryl, and the C₃₋₁₀ cycloalkyl, 4- to 10-membered heterocyclyl, 5- to 10-membered heteroaryl, and C₆₋₁₀ aryl are optionally substituted with one or more substituents selected from oxo, hydroxyl, cyano, carboxyl, halogen, C₁₋₆ alkyl, C₁₋₆ haloalkyl, C₁₋₆ alkoxy, C₁₋₆ haloalkoxy, C₃₋₆ cycloalkyl, 4- to 7-membered heterocyclyl, C₆₋₁₀ aryl, and 5- to 10-membered heteroaryl.

In certain embodiments, L₄ is -NR_{1b}-.

In certain embodiments, u is selected from 0 and 1.

In certain embodiments, Q is selected from C₃₋₆ cycloalkyl, 4- to 6-membered heterocyclyl, 5- to 6-membered heteroaryl, and phenyl.

In certain embodiments, Q is selected from 4- to 6-membered heterocyclyl and 5- to 6-membered heteroaryl.

In certain embodiments, the compound is selected from formula (V-2) or (V-3),

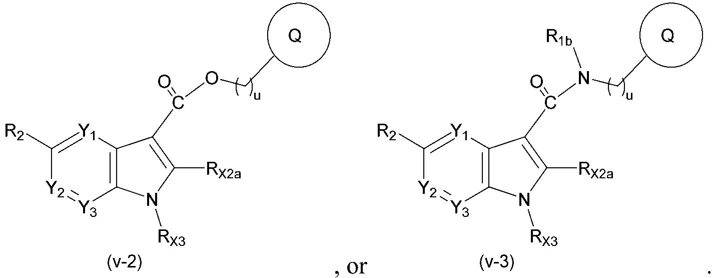

In certain embodiments, the compound is formula (V-4),

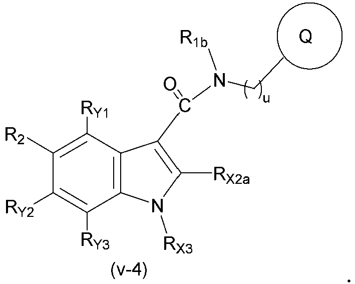

In certain embodiments, the compound is formula (VI-1),

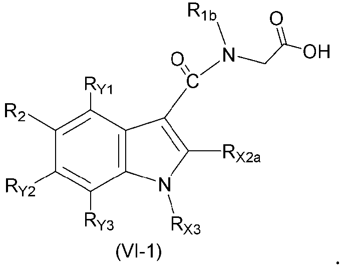

In certain embodiments, the compound is selected from formula (III-1) and (III-2),

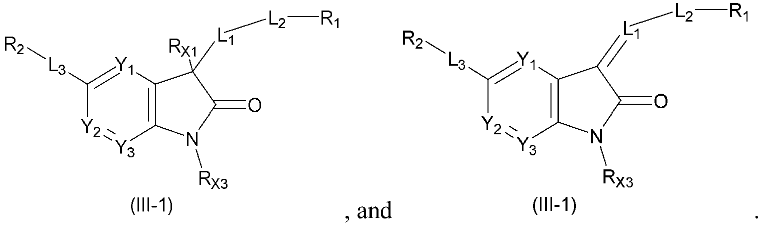

In certain embodiments, the compound is selected from formula (III-3) and (III-4),

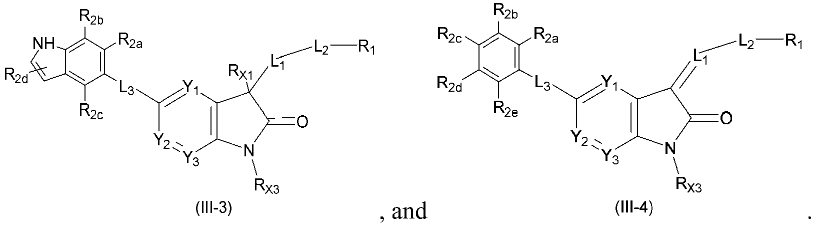

In certain embodiments, the compound is selected from formula (III-5) and (III-6),

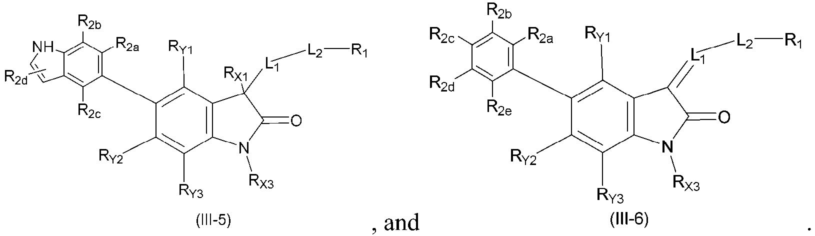

In certain embodiments, it is provided that:
1) when structural unit 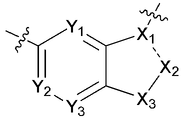 is 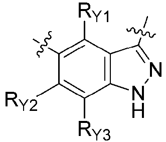 or 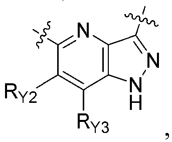 -L₁-L₂-R₁ is not -C(=O)-C(=O)-OH; or
2) when structural unit 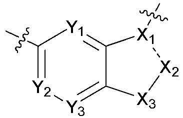 is 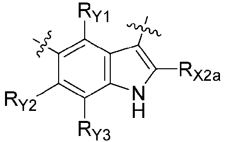 or 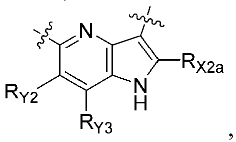 R_{X2a} is not H; or
3) when structural unit 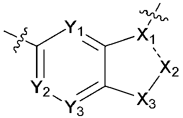 is 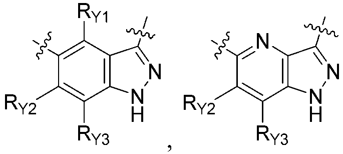 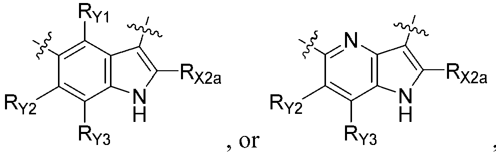 R_{Y2} is not H, halogen, C₁₋₃ alkyl, or -CF₃;
4) when -L₁-L₂-R₁ is -C(=O)-C(=O)-OH, L₃ is not a bond; or
5) when -L₁-L₂-R₁ is -C(=O)-C(=O)-OH, R₂ is not phenyl or 5-membered heteroaryl, and the phenyl and 5-membered heteroaryl are optionally substituted with 1, 2, 3, 4, or 5 groups each independently selected from R₂ₐ, R_{2b}, R_{2c}, R_{2d} and R₂ₑ;
   wherein L₁, L₂, L₃, R₁, R₂, R_{Y1}, R_{Y2}, R_{Y3}, R_{X2a}, R₂ₐ, R_{2b}, R_{2c}, R_{2d} and R₂ₑ are as defined in the present invention.

In certain embodiments, it is provided that:
1) when structural unit 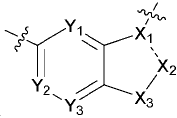 is 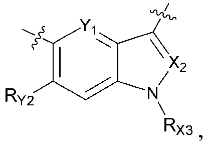 -L₁-L₂-R₁ is not -C(=O)-C(=O)-OH; or
2) when structural unit 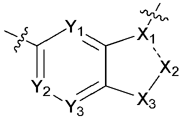 is 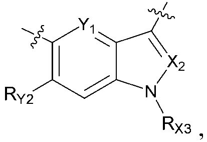 and X₂ is CR_{X2a}, R_{X2a} is not H;
3) when structural unit 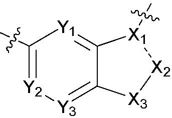 is 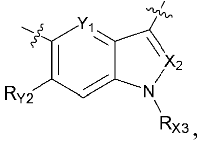 R_{Y2} is not H, halogen, C₁₋₃ alkyl, or -CF₃;
4) when -L₁-L₂-R₁ is -C(=O)-C(=O)-OH, L₃ is not a bond; or
5) when -L₁-L₂-R₁ is -C(=O)-C(=O)-OH, R₂ is not phenyl or 5-membered heteroaryl, and the phenyl and 5-membered heteroaryl are optionally substituted with 1, 2, 3, 4, or 5 groups each independently selected from R₂ₐ, R_{2b}, R_{2c}, R_{2d} and R₂ₑ;
wherein X₂, Y₁, L₁, L₂, L₃, R₁, R₂, R_{X3}, R₂ₐ, R_{2b}, R_{2c}, R_{2d} and R₂ₑ are as defined in any of claims 1-20.

In certain embodiments, it is provided that: -L₁-L₂-R₁ is not -COOR₁ₐ .

In certain embodiments, it is provided that: -L₁-L₂-R₁ is not -CR_{L1a}R_{L1b}-COOR₁ₐ.

In certain embodiments, it is provided that: -L₁-L₂-R₁ is not -CO-CO-OR₁ₐ.

In certain embodiments, it is provided that: -L₁-L₂-R₁ is not -CO-CO-NR_{1b}R_{1c}.

In certain embodiments, it is provided that: the compound is not

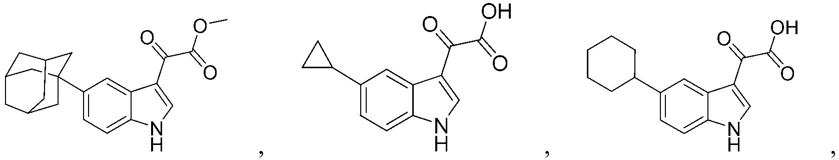

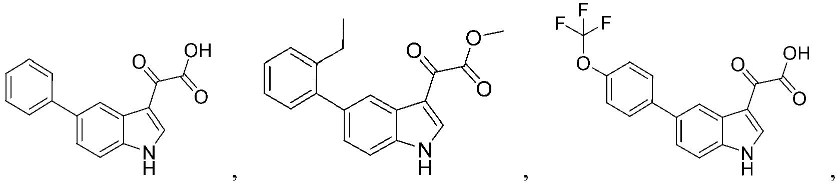

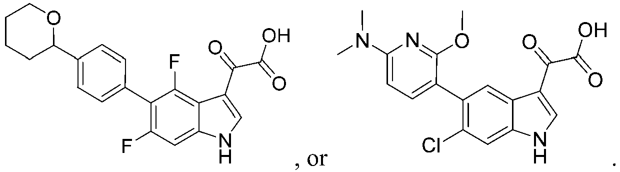

In certain embodiments, the compound is selected from 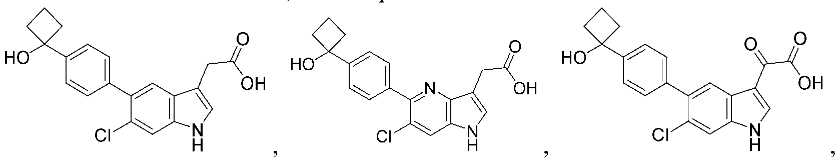 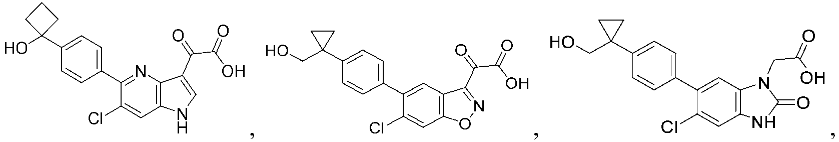 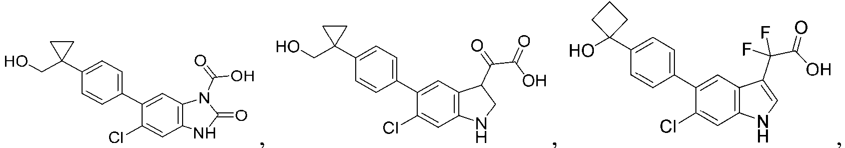 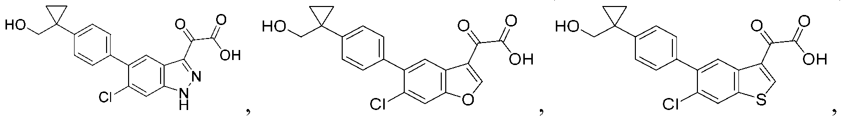 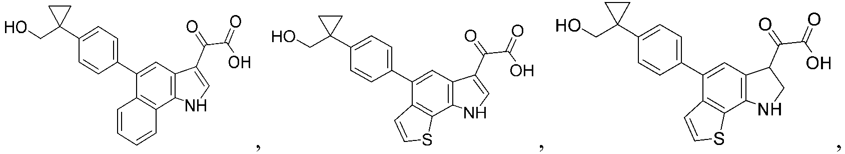 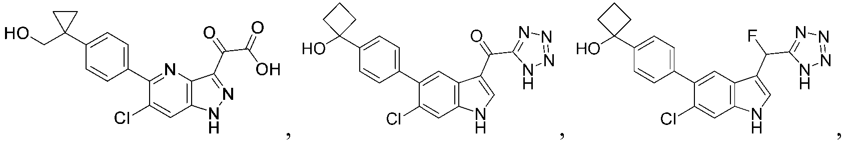 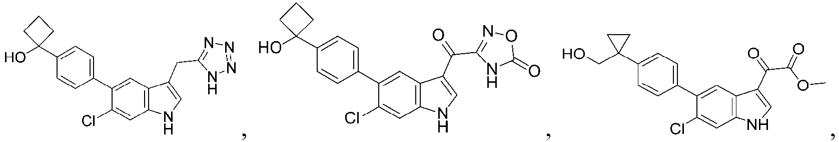 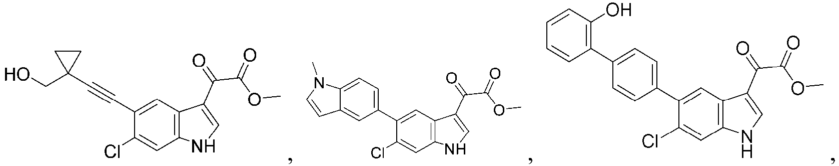 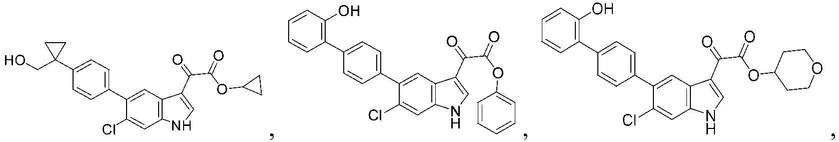 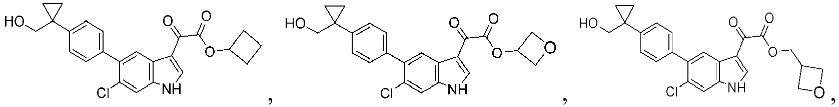 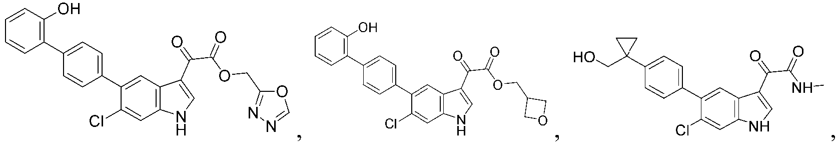 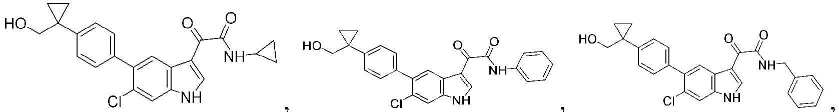 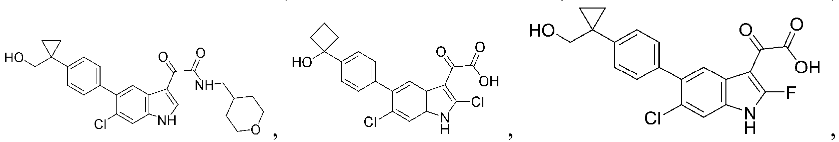 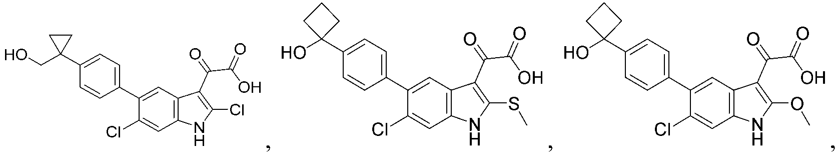 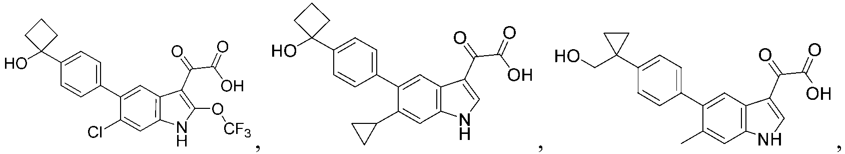 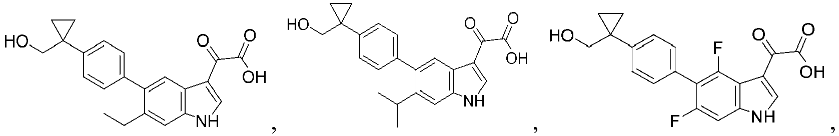 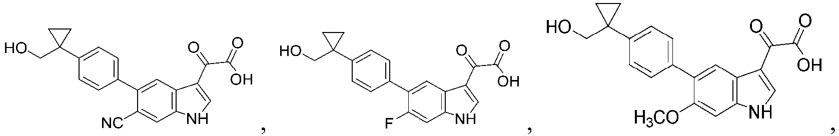 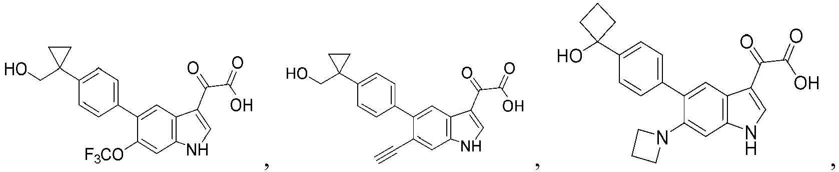 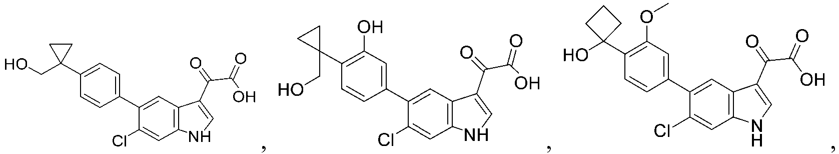 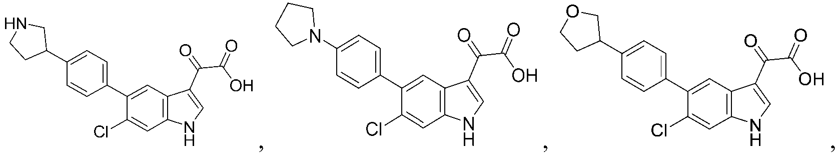 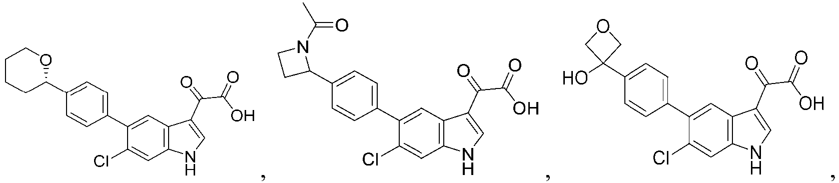 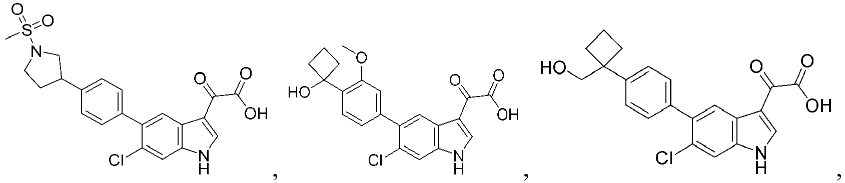 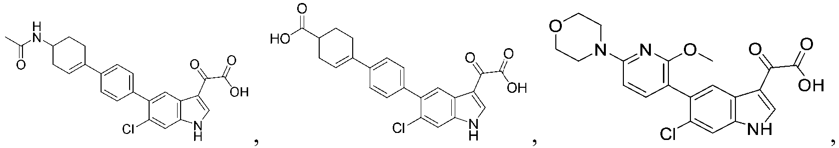 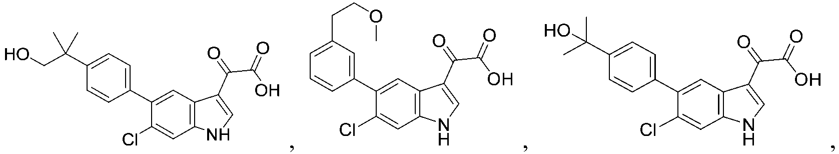 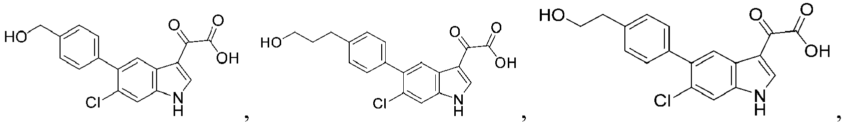 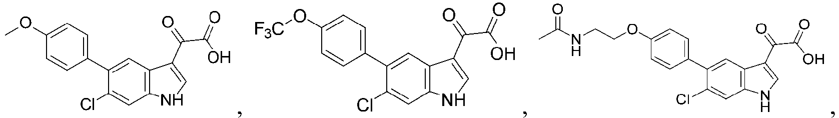 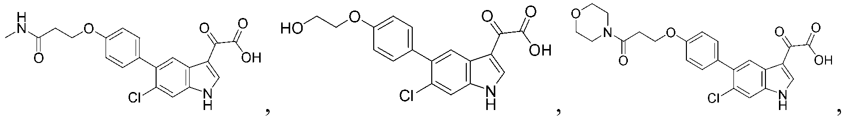 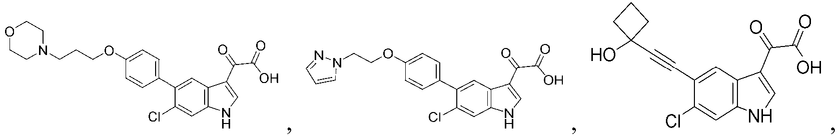 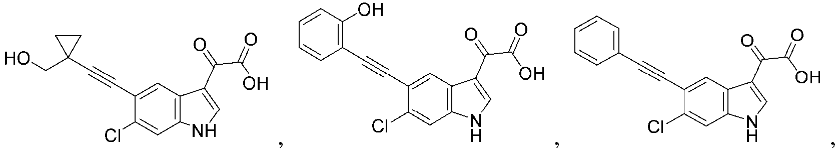 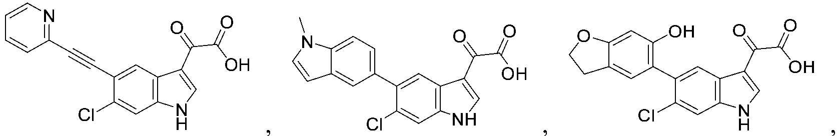 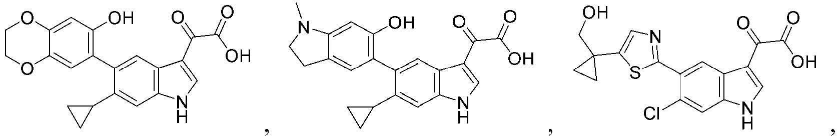 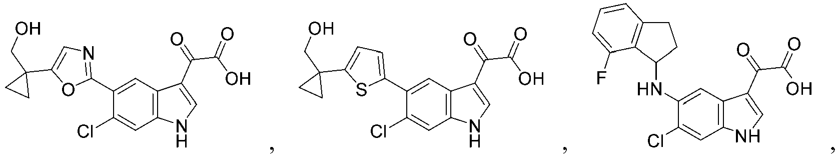 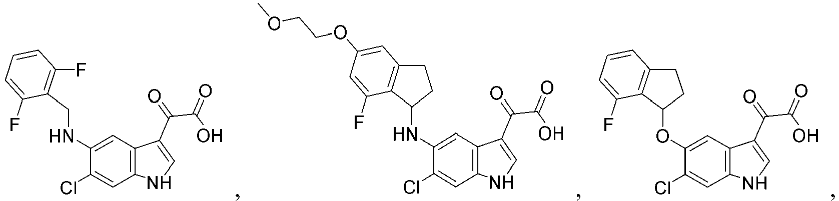 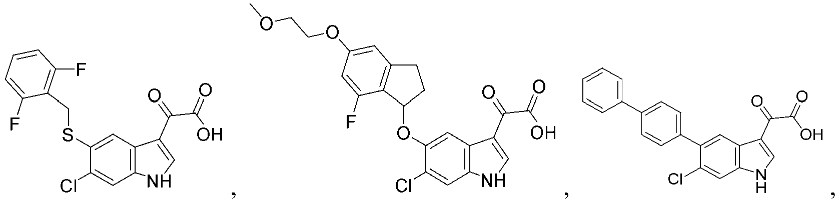 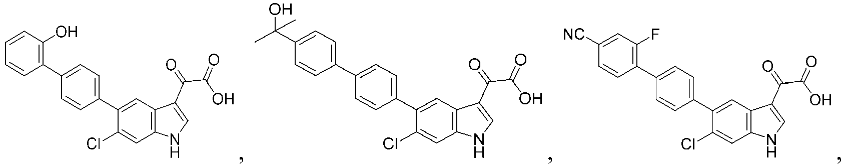 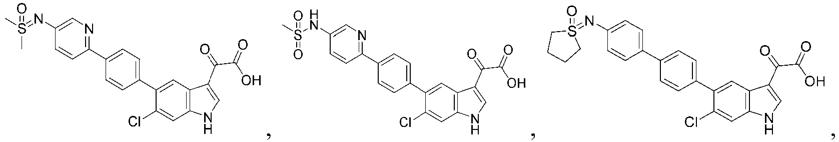 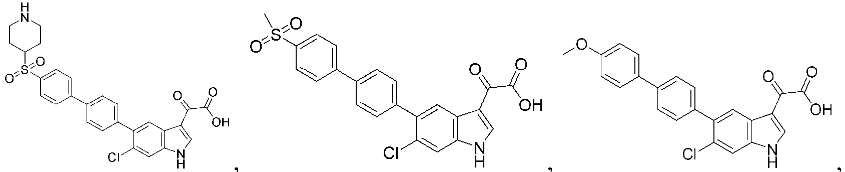 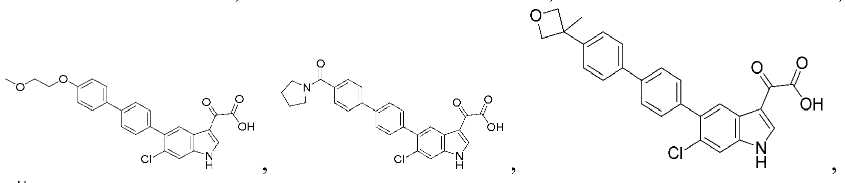 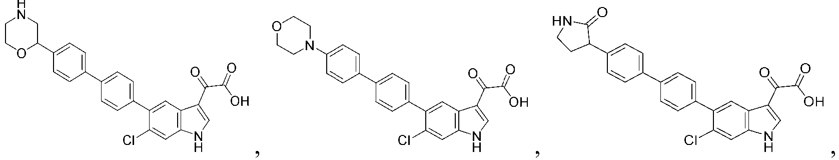 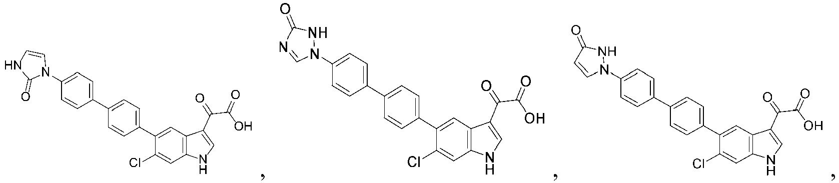 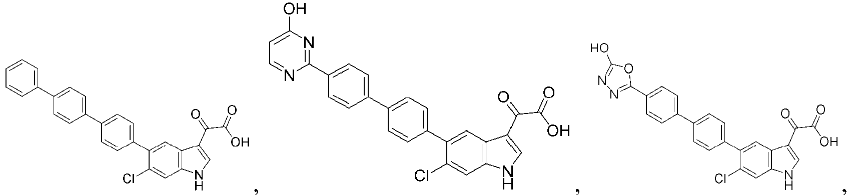 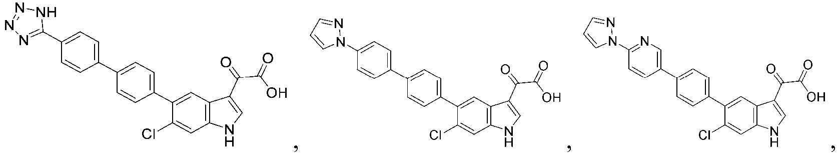 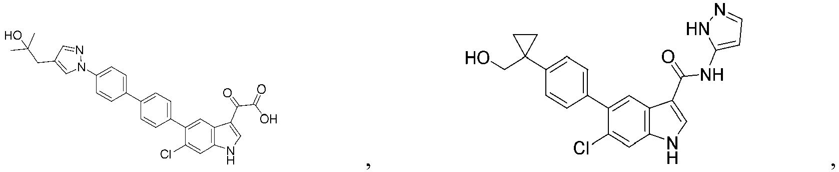 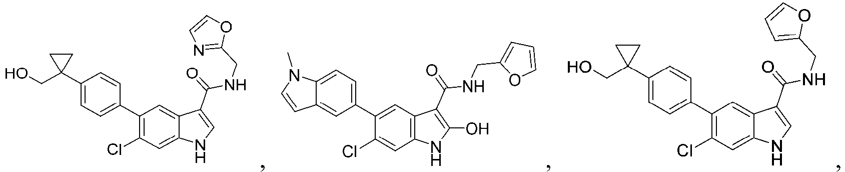 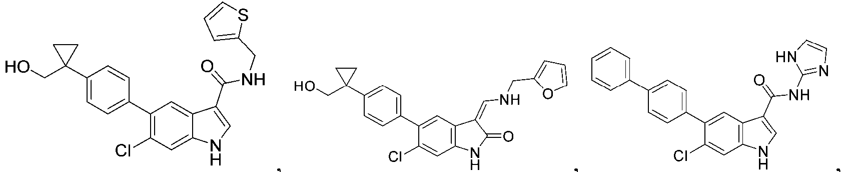 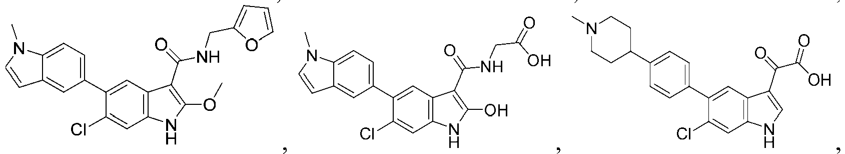 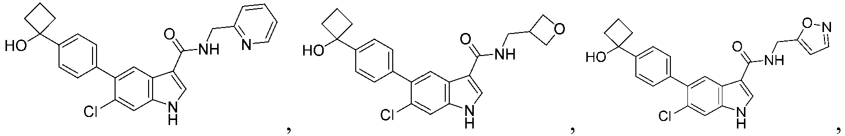 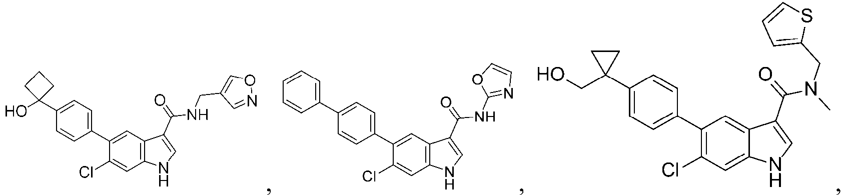 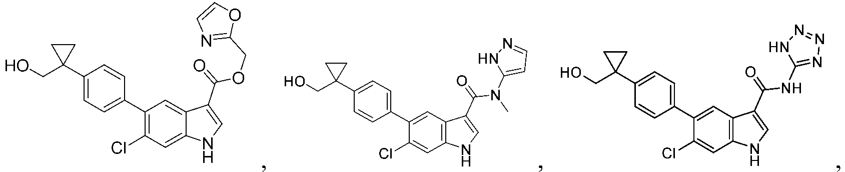 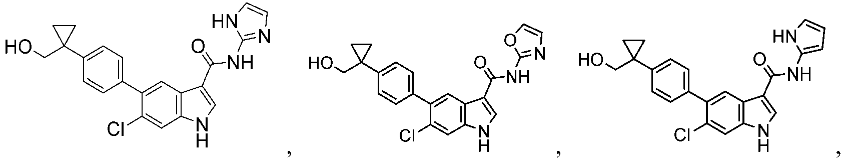 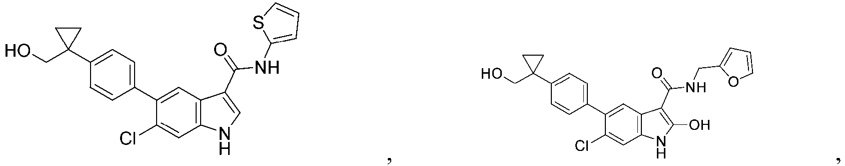 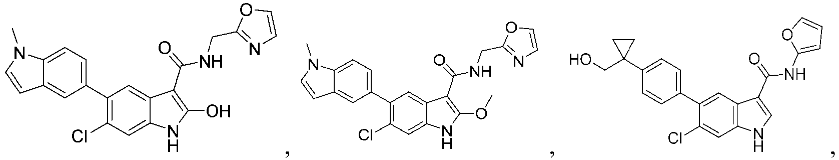 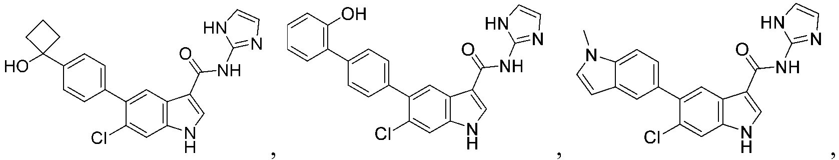 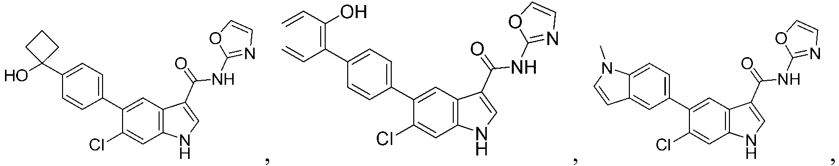 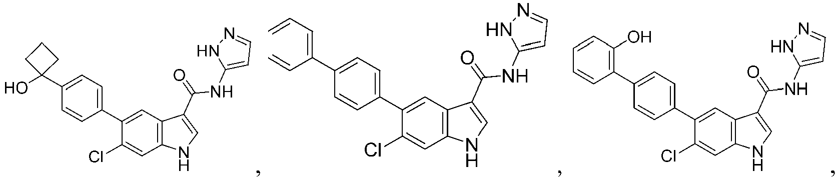 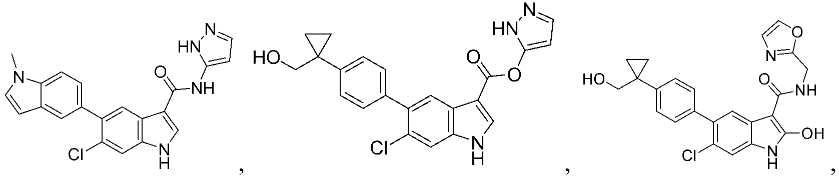 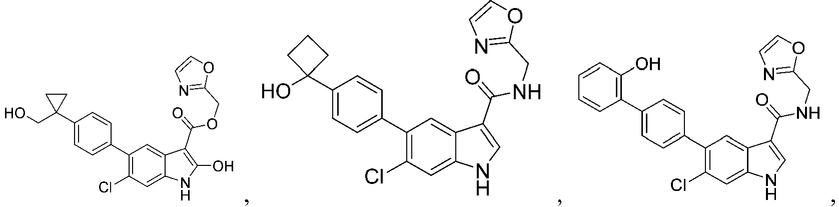 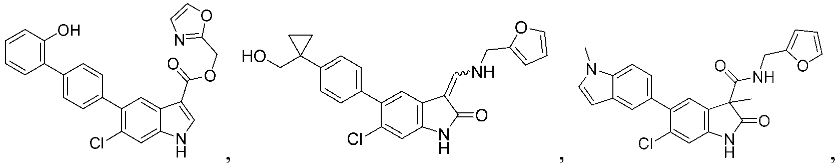 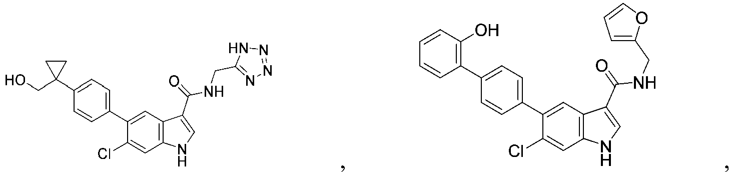 and

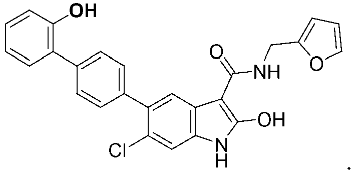

In certain embodiments, the compound is selected from

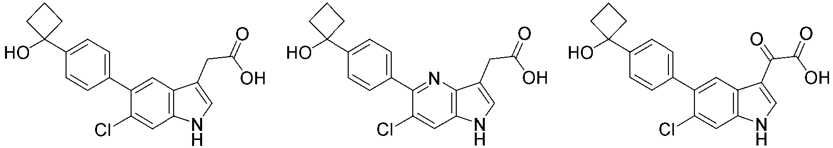

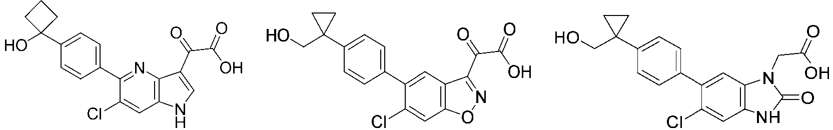

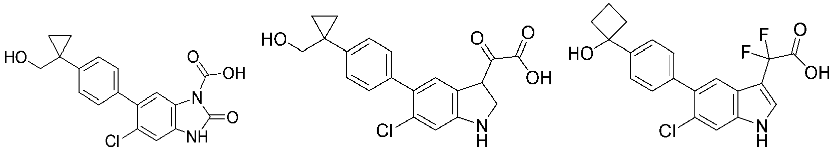

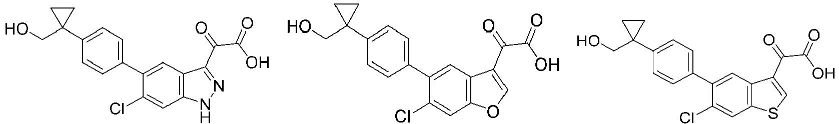

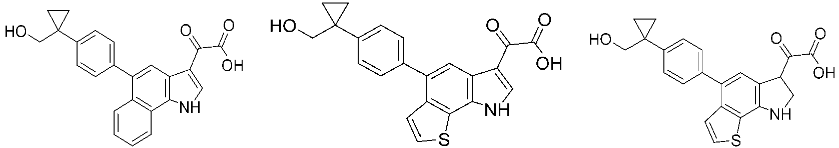

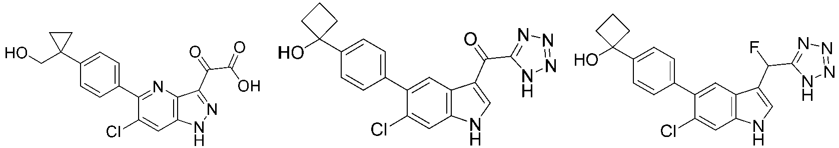

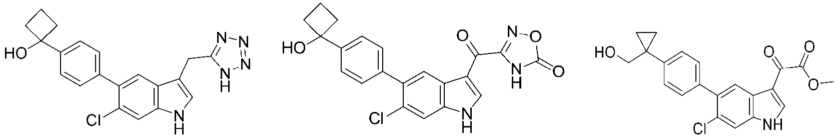

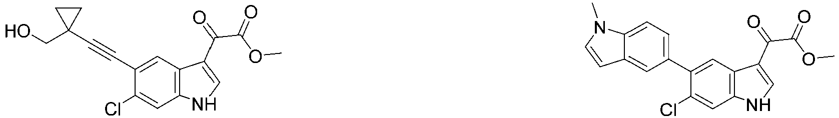

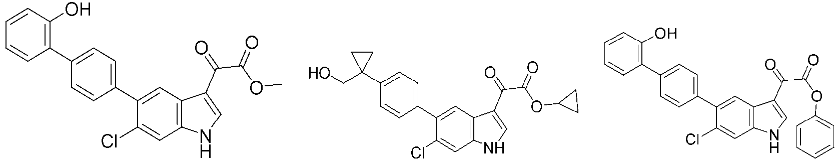

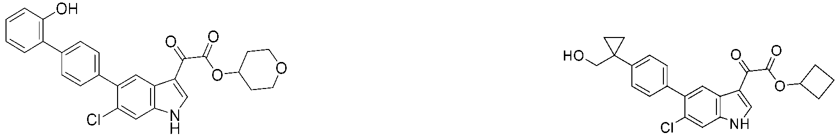

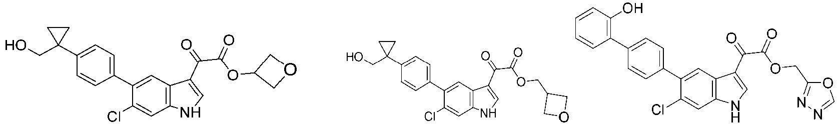

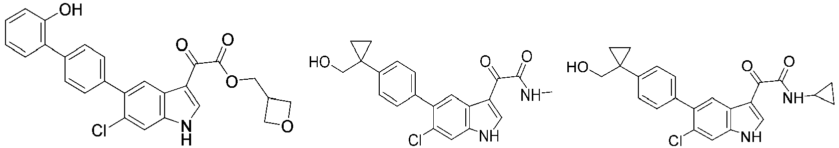

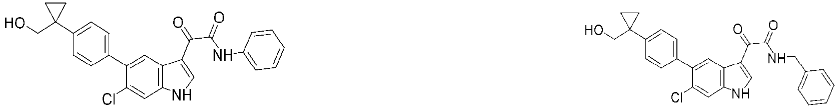

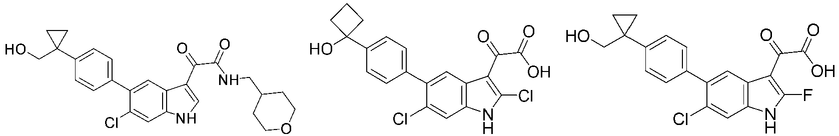

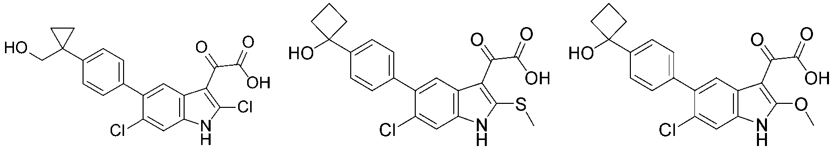

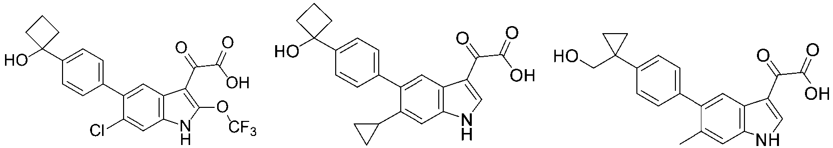

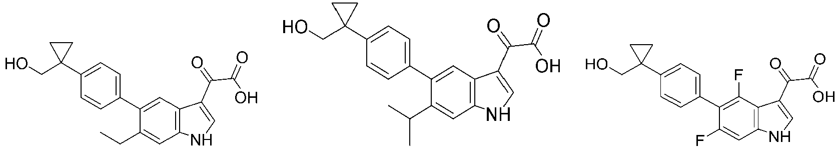

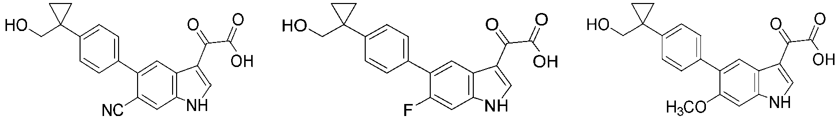

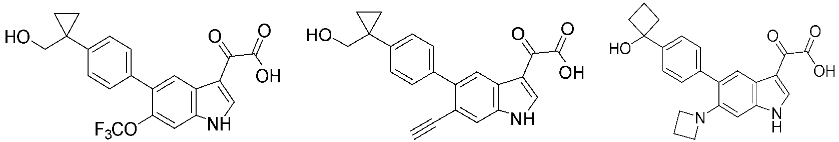

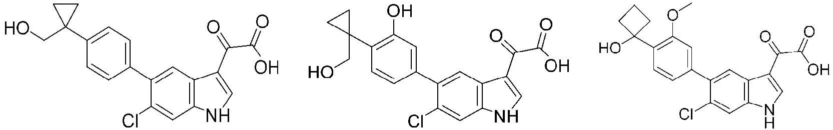

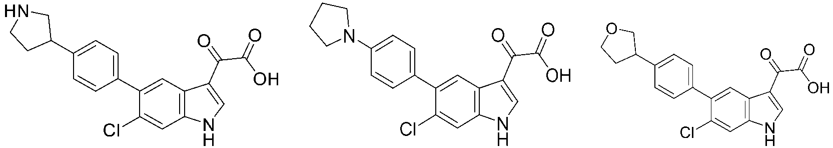

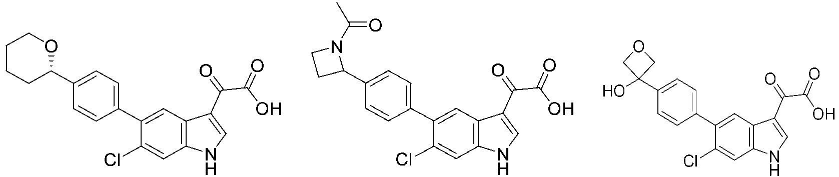

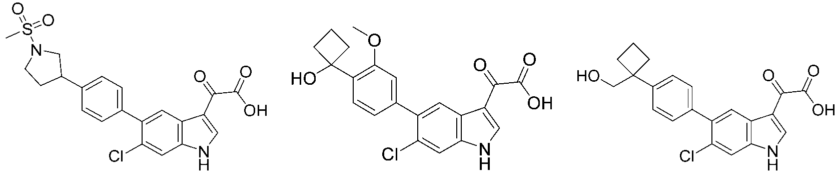

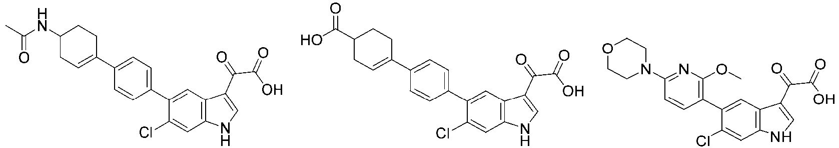

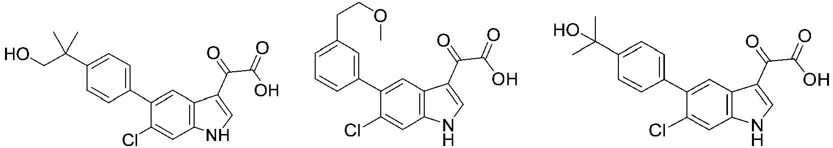

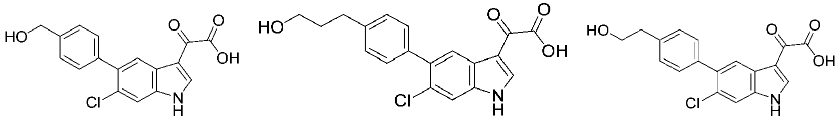

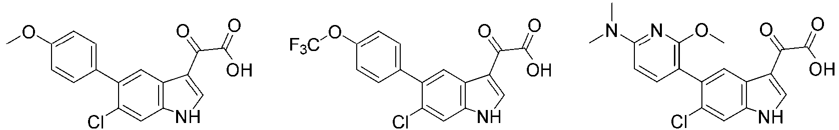

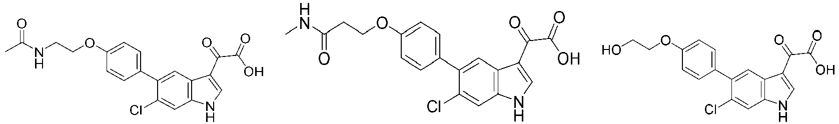

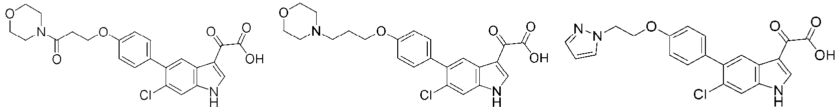

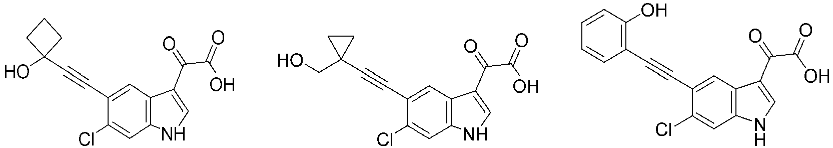

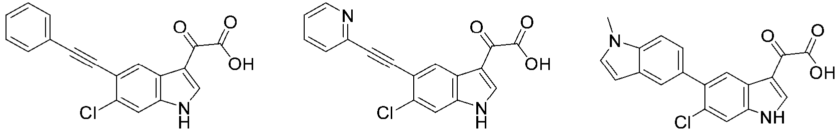

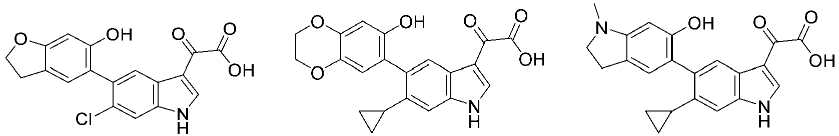

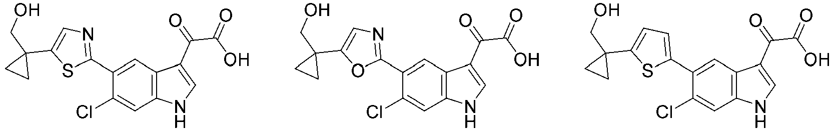

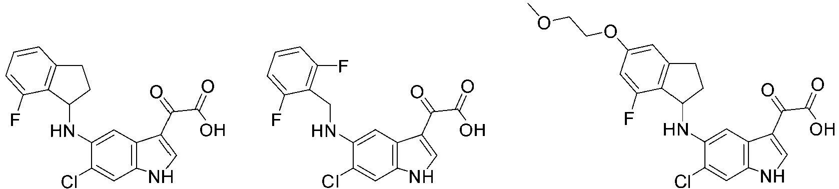

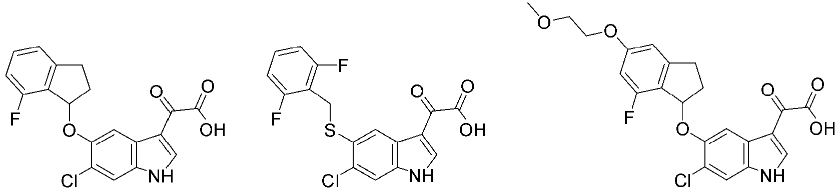

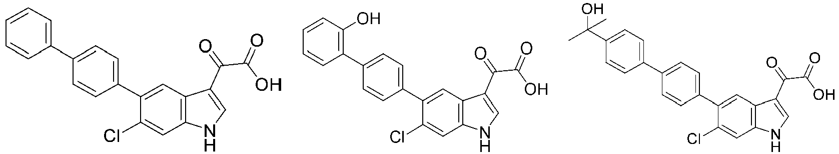

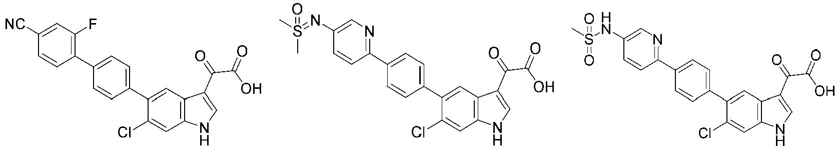

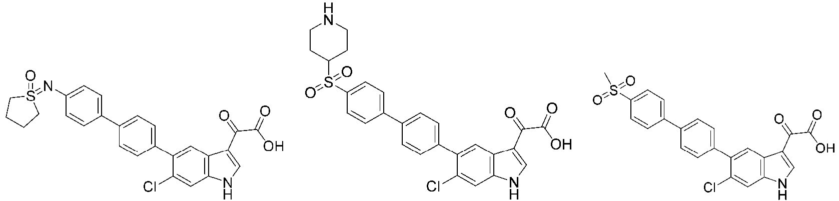

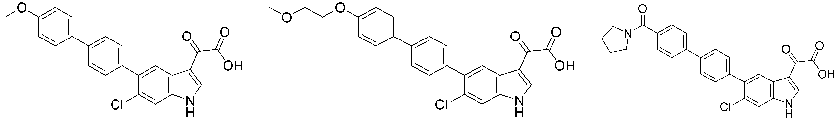

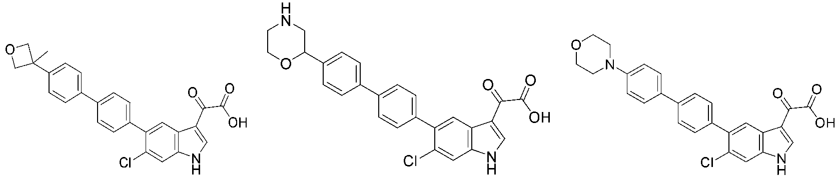

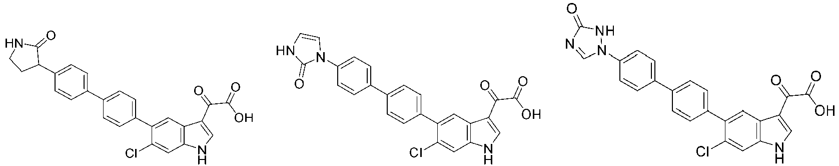

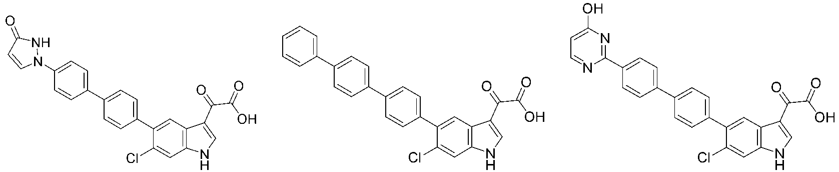

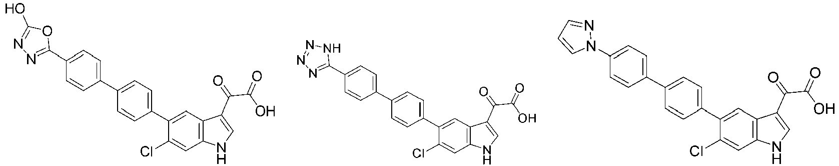

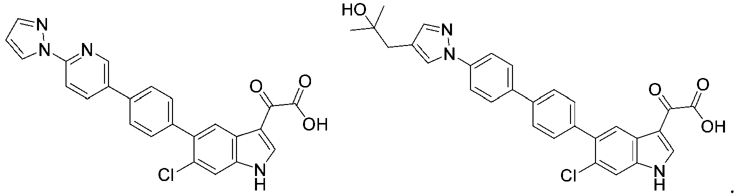

In certain embodiments, the compound is selected from

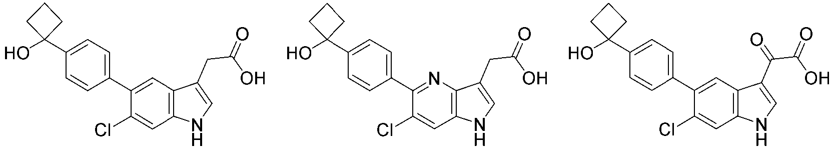

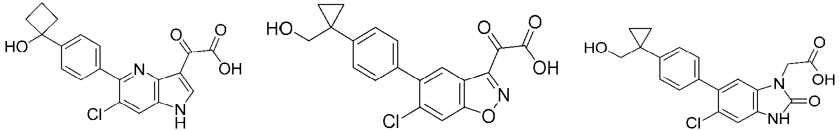

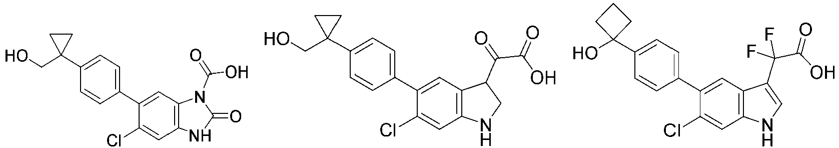

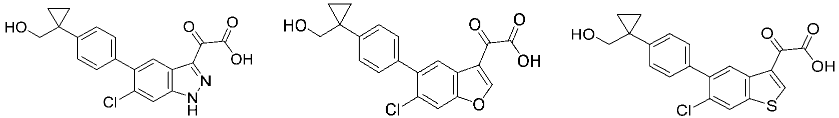

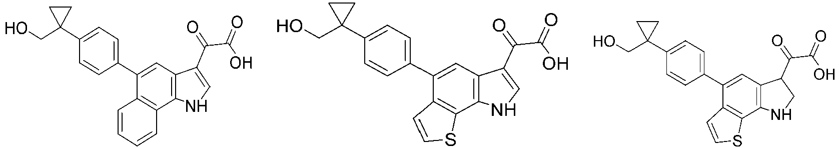

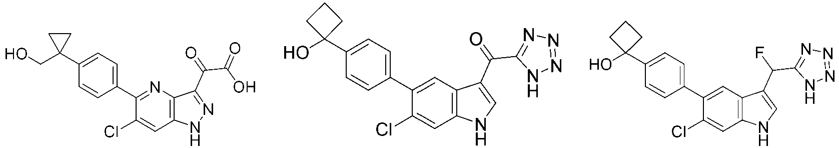

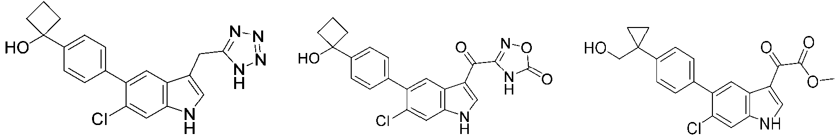

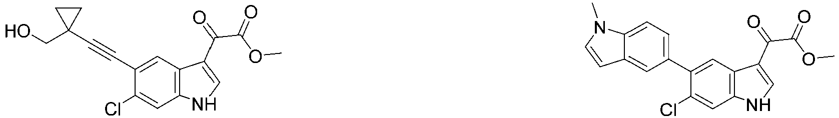

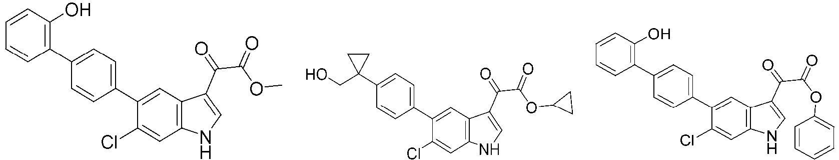

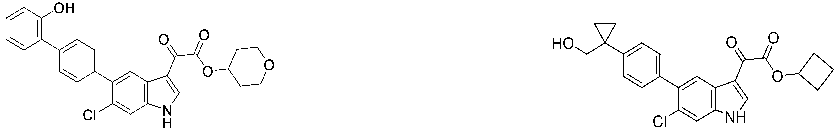

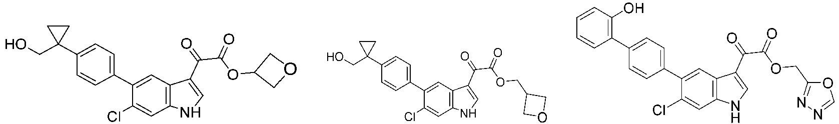

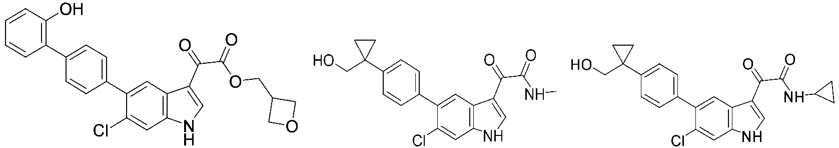

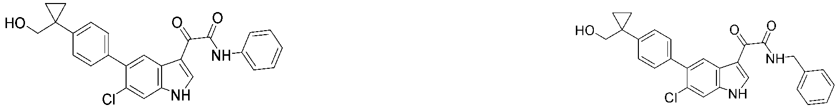

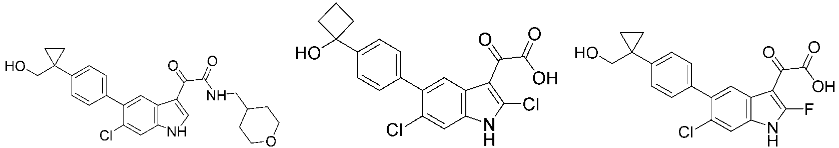

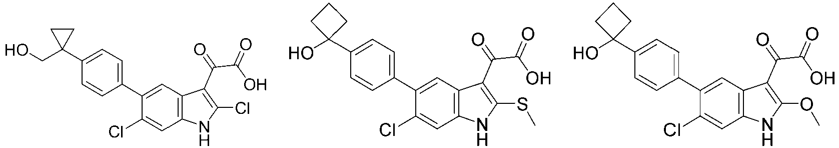

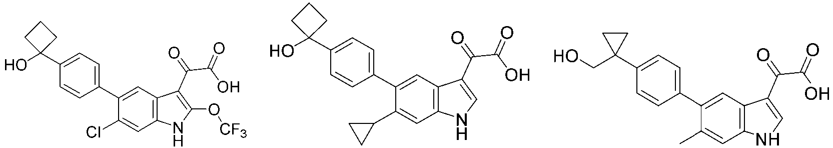

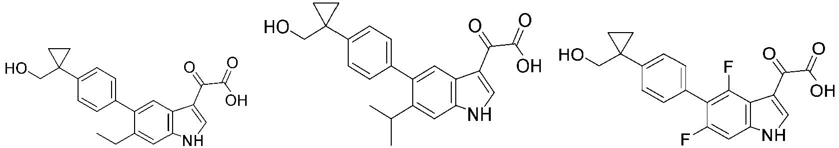

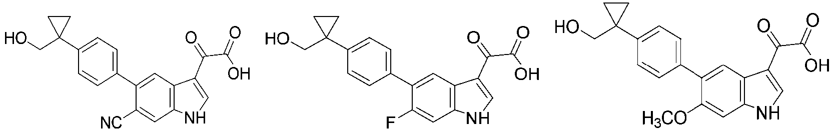

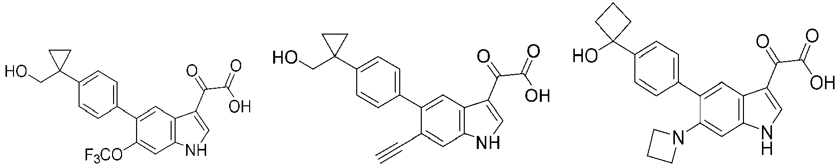

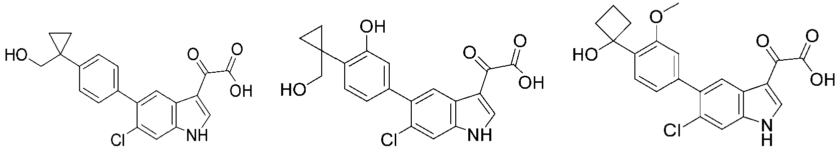

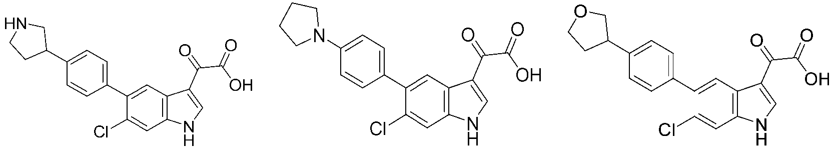

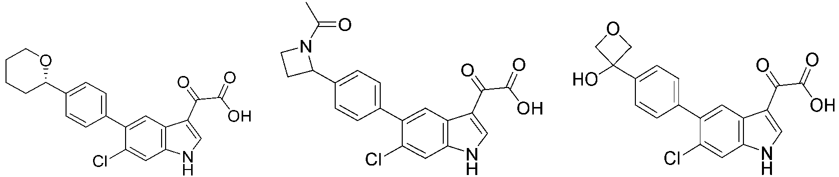

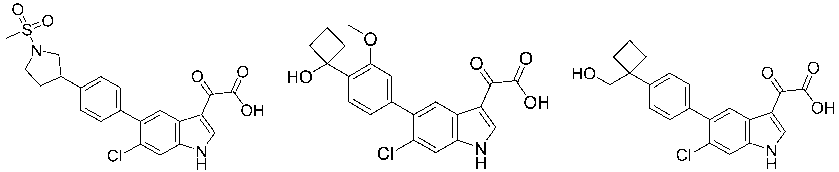

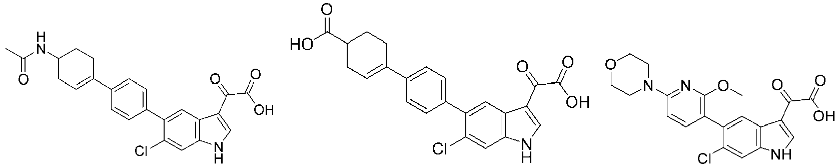

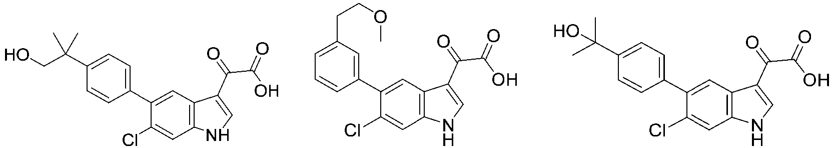

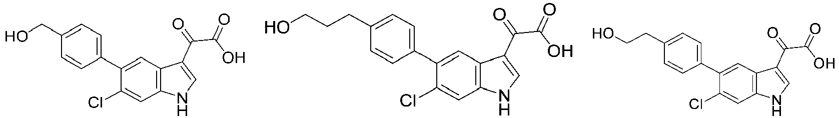

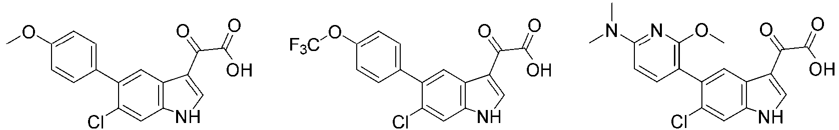

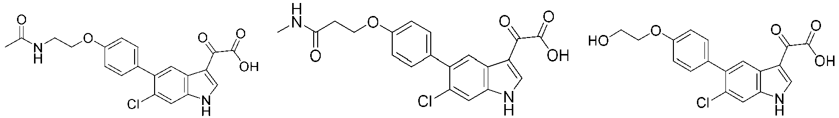

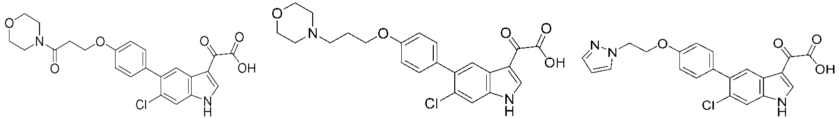

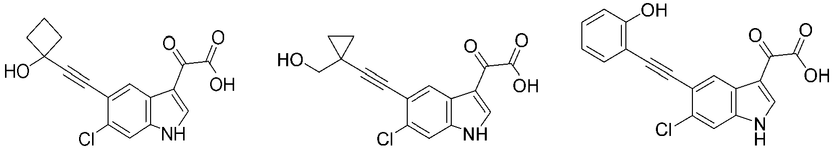

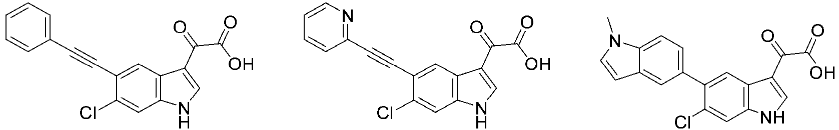

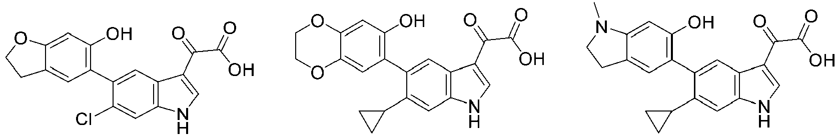

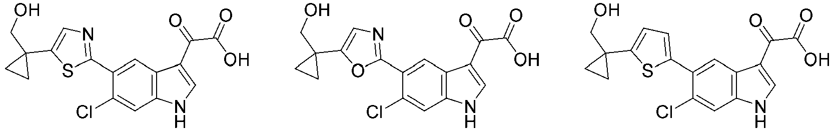

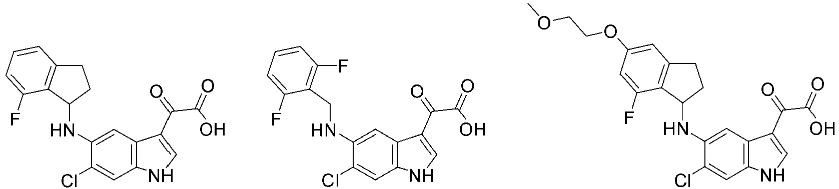

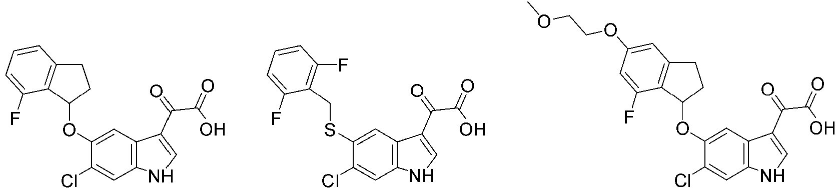

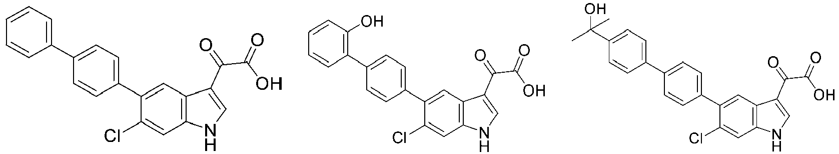

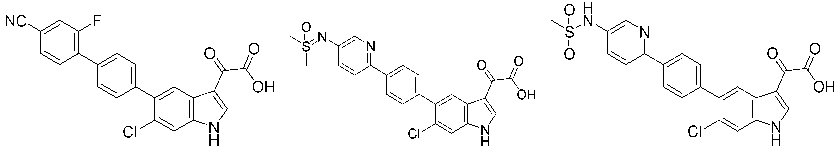

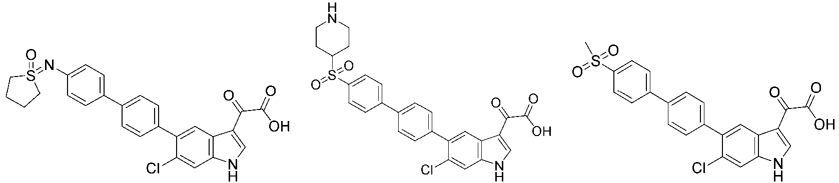

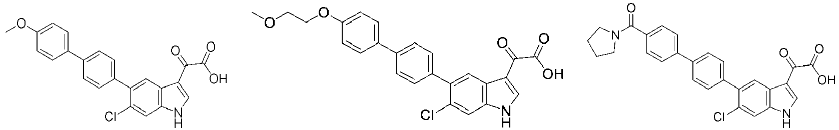

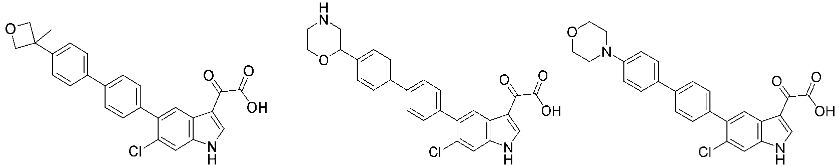

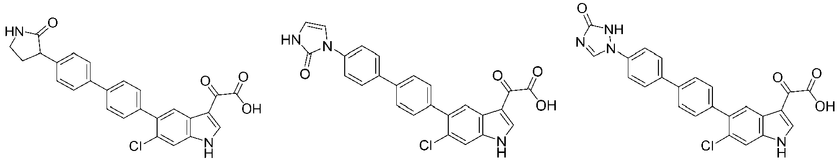

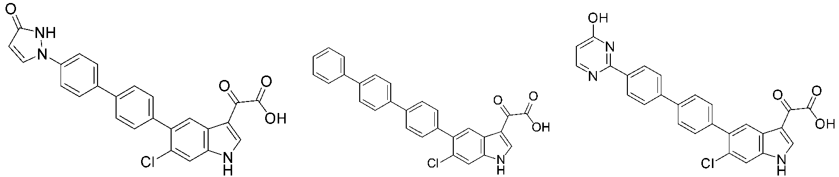

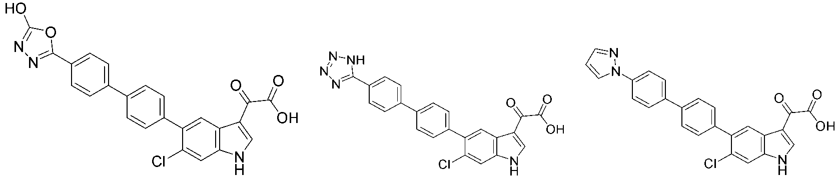

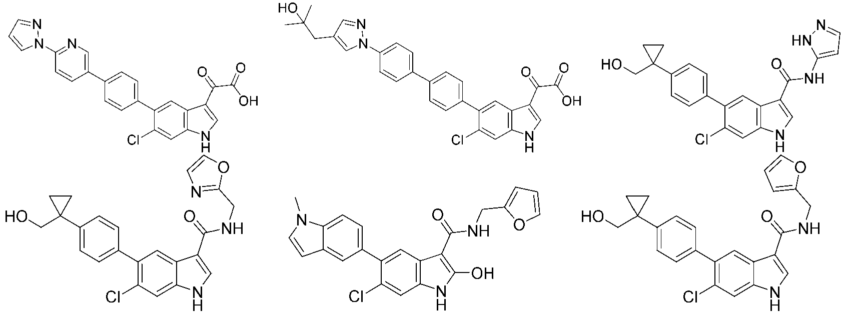

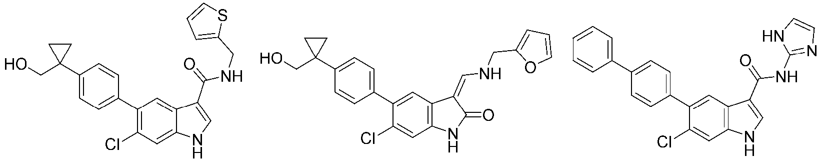

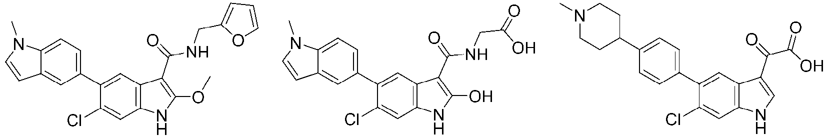

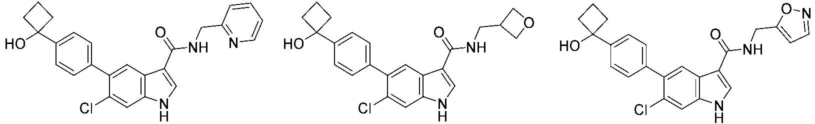

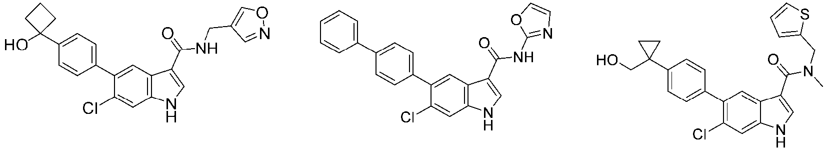

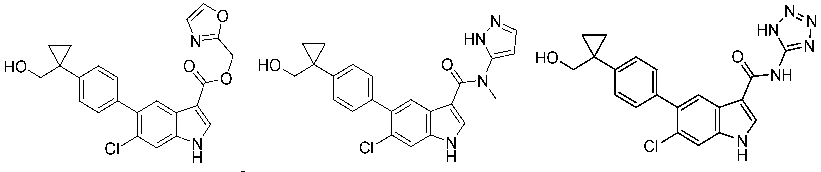

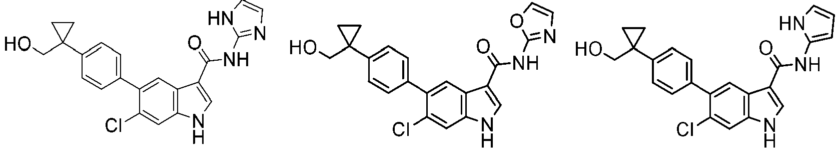

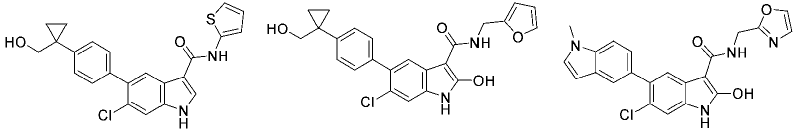

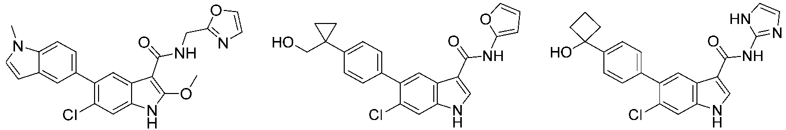

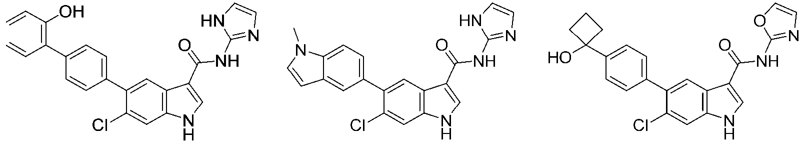

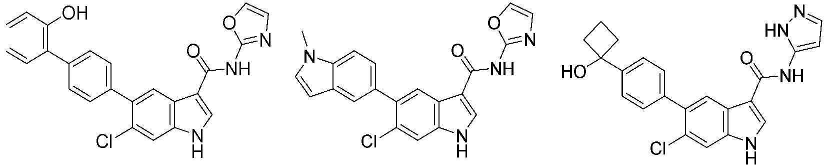

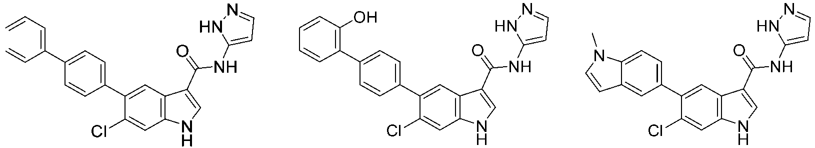

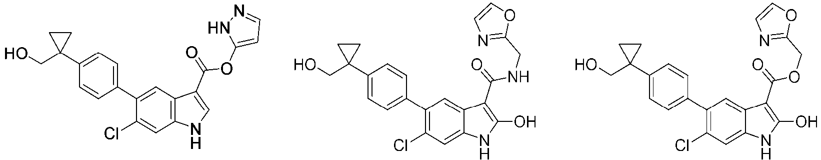

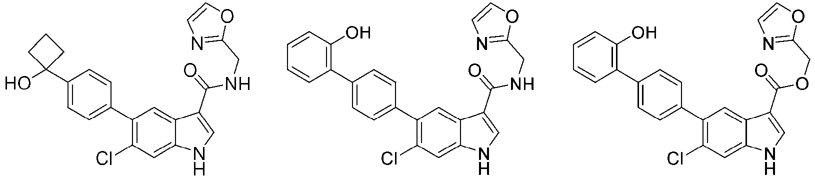

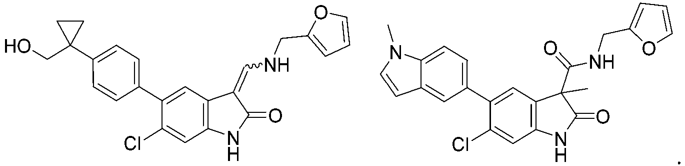

In certain embodiments, the compound is selected from

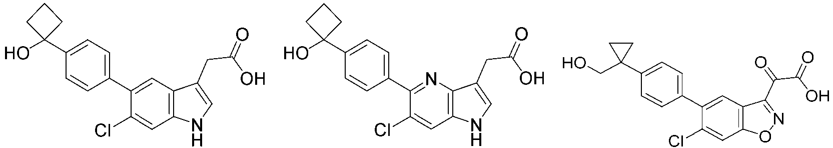

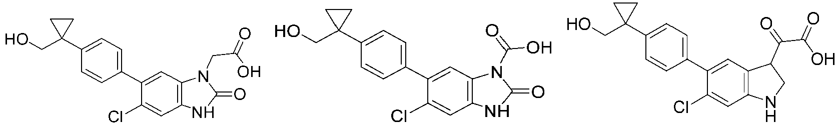

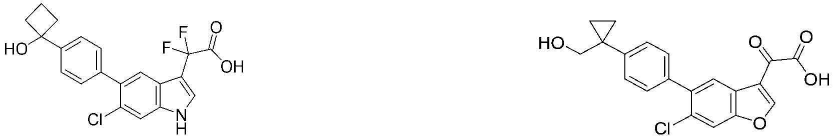

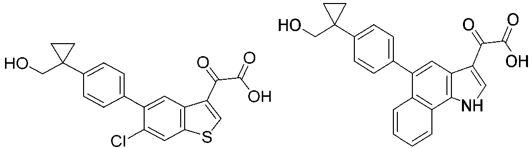

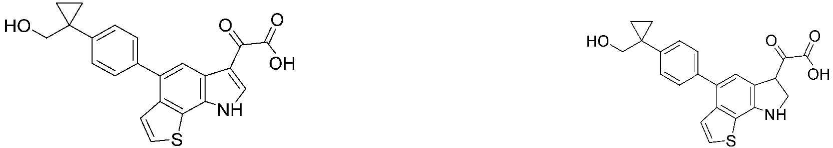

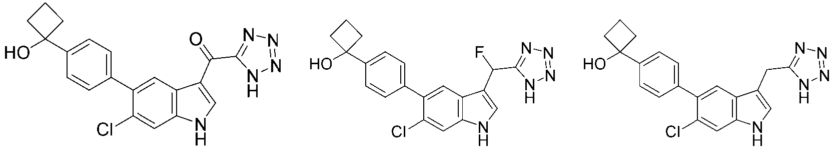

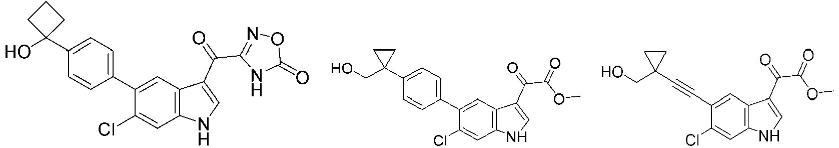

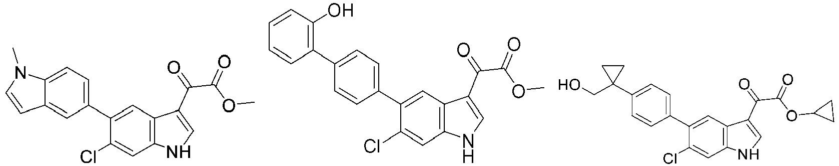

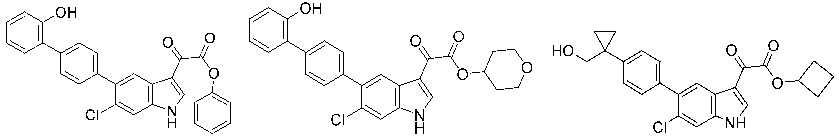

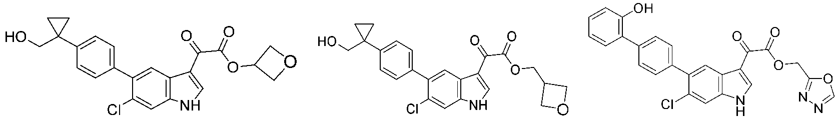

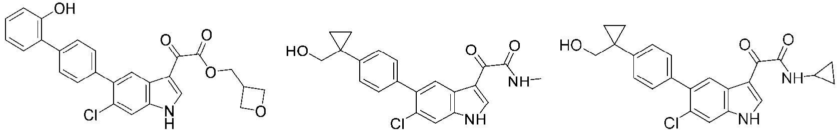

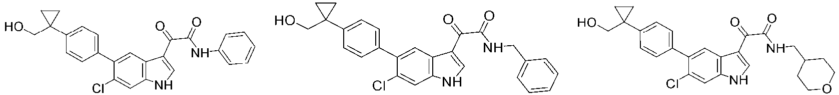

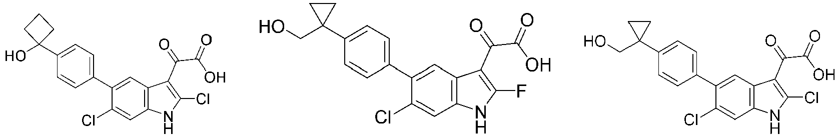

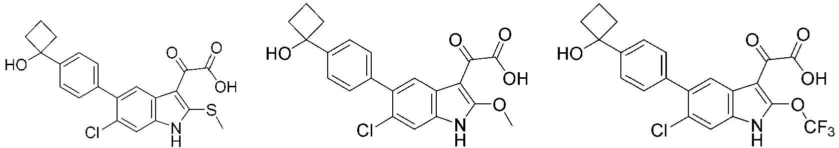

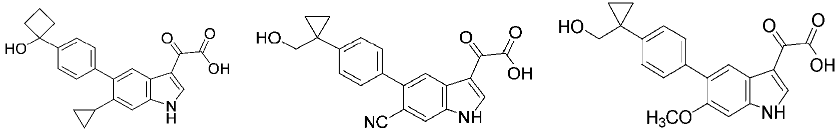

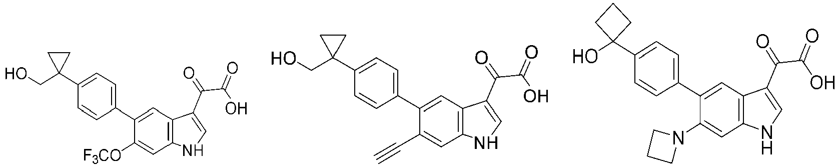

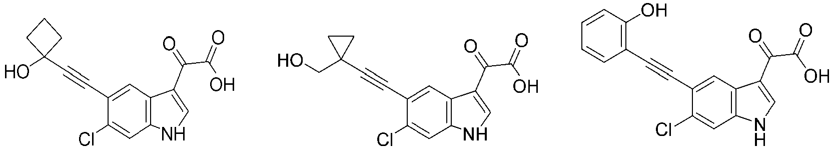

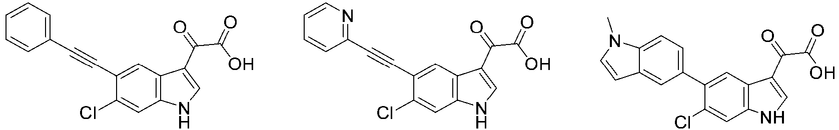

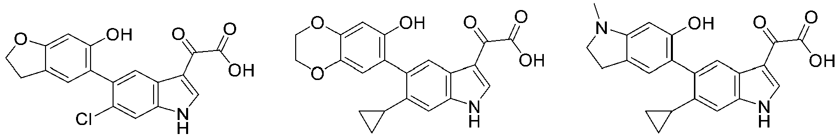

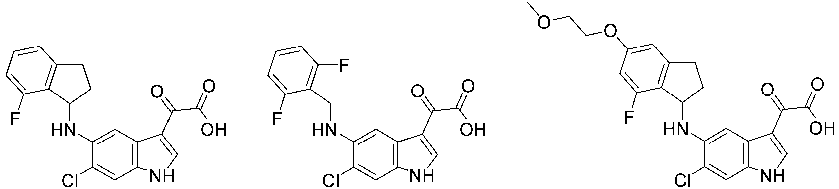

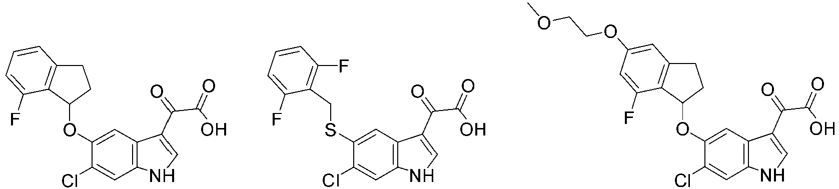

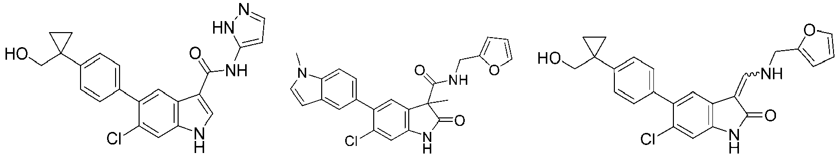

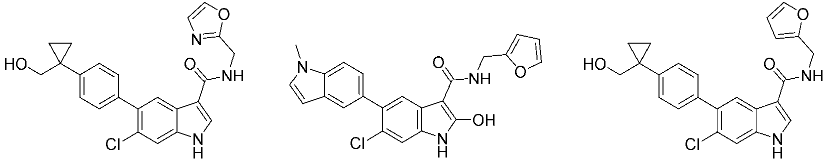

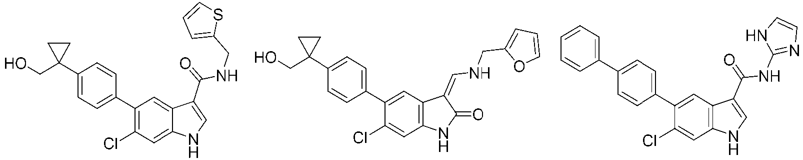

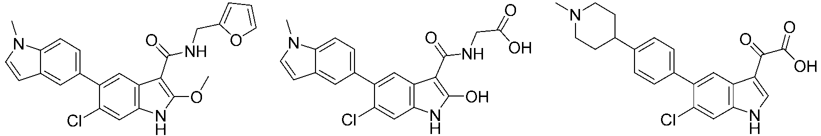

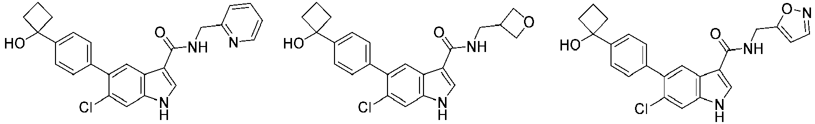

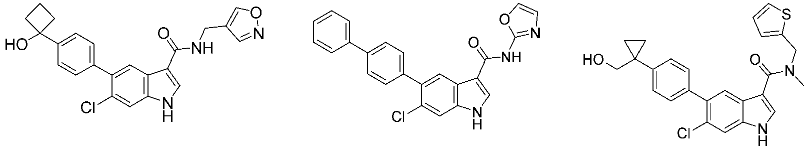

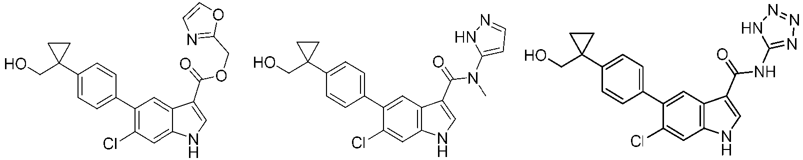

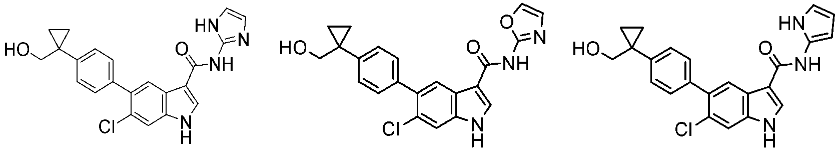

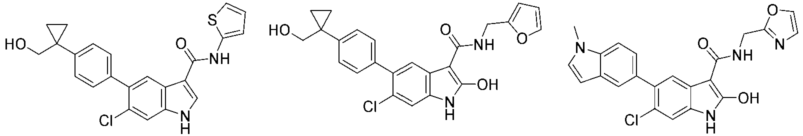

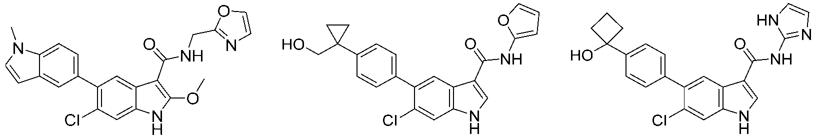

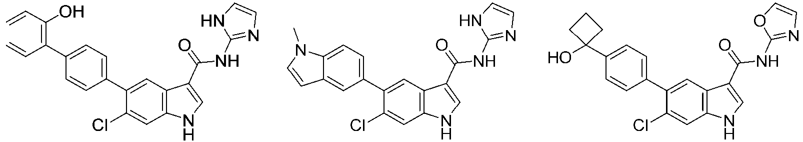

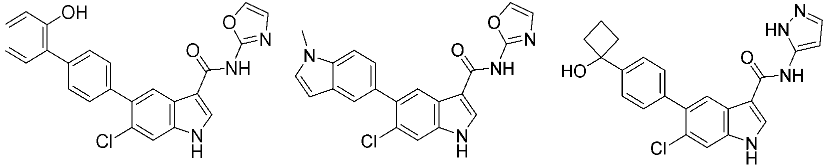

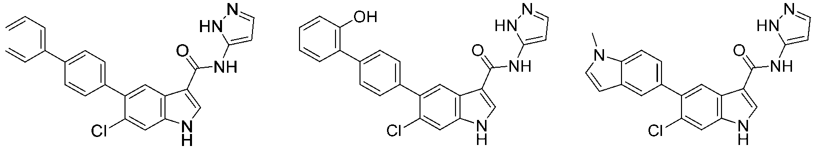

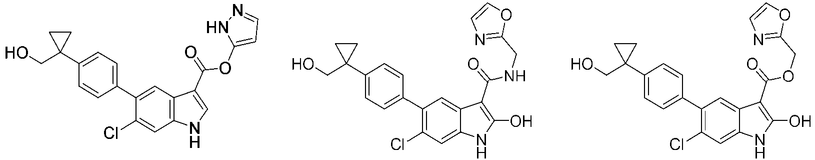

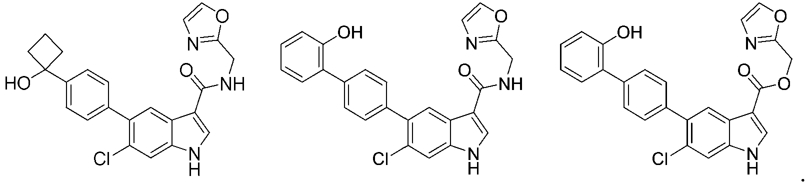

The present invention encompasses compounds obtained by combining any of the embodiments.

A second aspect of the present invention provides a pharmaceutical composition, comprising the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, and one or more pharmaceutically acceptable excipients or carriers. In certain embodiments, the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention is present in an effective amount.

A third aspect of the present invention provides the use of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, or the composition of the present invention in the preparation of a medicament as an AMPK agonist.

A fourth aspect of the present invention provides the use of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, or the composition of the present invention in the preparation of a medicament for treating and/or preventing type II diabetes, dyslipidaemia, obesity, chronic kidney disease, diabetic nephropathy, acute kidney injury, polycystic kidney disease, or alopecia.

A fifth aspect of the present invention provides the use of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, or the composition of the present invention for treating and/or preventing type II diabetes, dyslipidaemia, obesity, chronic kidney disease, diabetic nephropathy, acute kidney injury, polycystic kidney disease, or alopecia.

A sixth aspect of the present invention provides the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, or the composition of the present invention, for use as an AMPK agonist.

A seventh aspect of the present invention provides a method for treating and/or preventing a disease, comprising administering to an individual in need thereof a therapeutically or prophylactically effective amount of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, or the composition of the present invention, wherein the disease is type II diabetes, dyslipidaemia, obesity, chronic kidney disease, diabetic nephropathy, acute kidney injury, polycystic kidney disease, or alopecia.

In terms of drug absorption, replacing carboxylic acid groups with groups such as amides helps enhance the in vivo absorption of molecules, improve bioavailability, and further facilitate the exertion of pharmacological effects. The compounds of the present invention can be synthesised using common chemical synthesis knowledge familiar to those of ordinary skill in the art or by various similar known methods in the art. The separation and purification of the product can be achieved through standard procedures known to those of ordinary skill in the art.

### Definition of Terms

Various terms and phrases used in the present invention have the general meanings well-known to those skilled in the art; nevertheless, the present invention intends to provide a more detailed description and explanation of these terms and phrases herein. In case any of the mentioned terms and phrases is inconsistent with their well-known meanings, the meanings expressed in the present invention shall prevail.

In case of any inconsistency between the compound names and chemical structural formulas used in the present invention, the chemical structural formulas shall prevail.

In the structural formulas of the present invention, the nitrogen-containing heteroaryl moiety substituted with hydroxyl may undergo tautomerism, such as

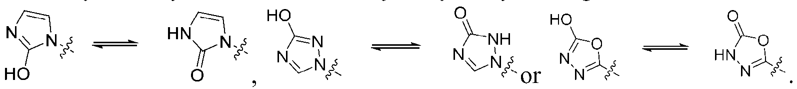

Unless otherwise defined, divalent groups are not restricted by directionality; for example, in A-L-B, when L is -C(=O)-NH-, A-L-B is A-C(=O)-NH-B or A-NH-C(=O)-B.

As used in the present invention, "selected from a bond" means that the groups on both sides of the variable are directly connected; for example, in A-L-B, when L is a bond, A-L-B is A-B.

As used in the present invention, "pharmaceutically acceptable salts" refer to salts of the compounds of the present invention that are pharmaceutically acceptable and possess the desired pharmacological activity of the parent compound. Such salts include: acid addition salts formed with inorganic or organic acids, or salts formed when an acidic proton on the parent compound is replaced by a metal ion, or coordination compounds formed with organic bases.

As used in the present invention, the term "stereoisomers" refers to isomers resulting from at least one asymmetric centre. In compounds having one or more (e.g., 1, 2, 3, or 4) asymmetric centres, racemic mixtures, single enantiomers, diastereomer mixtures, and individual diastereomers can be generated. Specific individual molecules may also exist in geometric isomers (cis/trans). Similarly, the compounds of the present invention may exist as mixtures of two or more structurally distinct forms in rapid equilibrium, commonly referred to as tautomers. Representative examples of tautomers include keto-enol tautomers, phenol-ketone tautomers, nitroso-oxime tautomers, imine-enamine tautomers, etc. It is to be understood that the scope of the present application encompasses all such isomers or mixtures thereof in any ratio (e.g., 60%, 65%, 70%, 75%, 80%, 85%, 90%, 95%, 96%, 97%, 98%, or 99%).

Unless otherwise indicated, the compounds of the present invention may be intended to exist in forms of stereoisomers, including cis and trans isomers, optical isomers (e.g., R- and S-enantiomers), diastereomers, geometric isomers, rotamers, conformers, atropisomers, and mixtures thereof. The compounds of the present invention may exhibit more than one type of isomerism, and consist of mixtures thereof (such as racemic mixtures and diastereomeric pairs).

In the present invention, solid lines ( 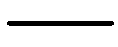 ), solid wedges ( 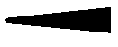 ), dashed wedges ( 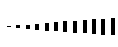 ), and wavy lines ( 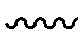 ) may be used to depict the chemical bonds of the compounds of the present invention. The use of solid lines to depict the bonds connected to asymmetric carbon atoms is intended to indicate that all possible stereoisomers at the carbon atoms (such as specific enantiomers and racemic mixtures) are included. The use of solid or dashed wedges to depict the bonds connected to asymmetric carbon atoms is intended to indicate the presence of the stereoisomers shown. When present in a racemic mixture, solid and dashed wedges are used to define the relative stereochemistry rather than the absolute stereochemistry. The use of wavy lines to depict the bonds connected to one carbon atom in a carbon-carbon double bond is intended to indicate the presence of cis-trans isomers in the compound. Unless otherwise indicated, the compounds of the present invention may exist in forms of stereoisomers, including cis and trans isomers, optical isomers (e.g., R- and S-enantiomers), diastereomers, geometric isomers, rotamers, conformers, atropisomers, and mixtures thereof. The compounds of the present invention may exhibit more than one type of isomerism, and consist of mixtures thereof (such as racemic mixtures and diastereomeric pairs).

As used in the present invention, the term "isotopically labelled compound" refers to a compound in which one or more atoms are replaced by atoms having the same atomic number but having an atomic mass or mass number different from the atomic mass or mass number prevailing in nature. Examples of isotopes suitable for inclusion in the compound of the present invention include, but are not limited to, isotopes of hydrogen (such as ²H and ³H); isotopes of carbon (such as ¹¹C, ¹³C and ¹⁴C); isotopes of chlorine (such as ³⁶Cl); isotopes of fluorine (such as ¹⁸F); isotopes of iodine (such as ¹²³I and ¹²⁵I); isotopes of nitrogen (such as ¹³N and ¹⁵N); isotopes of oxygen (such as ¹⁵O, ¹⁷O and ¹⁸O); and isotopes of sulphur (such as ³⁵S).

As used in the present invention, the term "prodrug" refers to a derivative that can undergo hydrolysis, oxidation, or other reactions under biological conditions (either in vitro or in vivo) to provide the compounds disclosed in the present invention. The prodrugs only become active compounds through this reaction under biological conditions, or they exhibit no or little activity in their unreactive forms. Prodrugs can generally be prepared using well-known methods, for example, those described in Burger's Medicinal Chemistry and Drug Discovery (1995) 172-178, 949-982 (Manfred E. Wolff ed., 5th edition).

As used in the present invention, the term "ester" refers to esters capable of undergoing hydrolysis in vivo, including those that are readily decomposed in the human body to dissociate from the parent compound as described. The hydroxylcontaining compounds of the present invention may form esters with organic acids or inorganic acids, or the carboxyl-containing compounds of the present invention may form esters with alcohols such as methanol, ethanol, or propanol.

The compounds of the present invention may exist in the form of solvates (such as hydrates), wherein the compounds of the present application comprise a solvent (e.g., water, methanol, or ethanol) as a structural element of the crystal lattice of the compounds. The amount of the solvent may be present in a stoichiometric or nonstoichiometric ratio.

The present invention encompasses all possible crystal forms or polymorphs of the compounds, which may be a single polymorph or a mixture of more than one polymorph in any ratio.

As used in the present invention, the term "N-oxide" refers to a compound containing an amine oxide moiety (i.e., the oxide of a tertiary amine group) formed by the oxidation of at least one nitrogen atom in the compound. Those skilled in the art will appreciate that not all nitrogen-containing heterocyclic rings are capable of forming N-oxides, as nitrogen requires an available lone pair of electrons to be oxidised to an oxide. Those skilled in the art will recognise nitrogen-containing heterocyclic rings that are capable of forming N-oxides. Those skilled in the art will also recognise that tertiary amines are capable of forming N-oxides. Synthetic methods for preparing N-oxides of nitrogen-containing heterocyclic rings and tertiary amines are well known to those skilled in the art; for example, nitrogen atoms (e.g., trivalent nitrogen) can be converted into the corresponding N-oxide forms by known methods such as treatment with oxidising agents. Specific examples include, but are not limited to

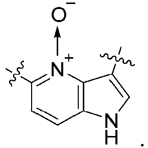

As used in the present invention, the term "optionally substituted with..." when used to modify a substituent means that the substituent may be substituted or unsubstituted.

As used in the present invention, the term "halogen" refers to fluorine, chlorine, bromine, and iodine, preferably fluorine, chlorine, and bromine.

As used in the present invention, the term "C₁₋₆ alkyl" refers to a linear or branched monovalent saturated hydrocarbon group having 1 to 6 carbon atoms, such as 1, 2, 3, 4, 5, or 6 carbon atoms. The C₁₋₆ alkyl includes C₁₋₅ alkyl, C₁₋₄ alkyl, C₁₋₃ alkyl, etc., and specific examples include, but are not limited to methyl, ethyl, propyl, butyl, pentyl or hexyl; the propyl, butyl, pentyl or hexyl includes a linear or branched saturated hydrocarbon group. For example, specific examples of propyl include n-propyl or isopropyl; specific examples of butyl include, but are not limited to - CH₂CH₂CH₂CH₃, -CH(CH₃)CH₂CH₃, -CH₂CH(CH₃)CH₃ or -CH(CH₃)₃.

As used in the present invention, the term "C₂₋₆ alkenyl" refers to a linear or branched unsaturated hydrocarbon group containing at least one double bond and having 2 to 6 (e.g., 2, 3, 4, 5, or 6) carbon atoms. The C₂₋₆ alkenyl includes C₂₋₅ alkenyl, C₂₋₄ alkenyl, C₂₋₃ alkenyl, etc., and specific examples include, but are not limited to -CH=CH₂, -CH=CH-CH=CH₂ or -CH=C(CH)₃-CH₃.

As used in the present invention, the term "C₂₋₆ alkynyl" refers to a linear or branched unsaturated hydrocarbon group containing at least one triple bond and having 2 to 6 (e.g., 2, 3, 4, 5, or 6) carbon atoms. The C₂₋₆ alkynyl includes C₂₋₅ alkynyl, C₂₋₄ alkynyl, C₂₋₃ alkynyl, etc., and specific examples include, but are not limited to ethynyl (-C≡CH) or propynyl (-C≡C-CH₃).

As used in the present invention, the term "C₁₋₆ alkylene" refers to a linear or branched divalent saturated hydrocarbon group having 1 to 6 carbon atoms, such as 1, 2, 3, 4, 5, or 6 carbon atoms. The C₁₋₆ alkylene includes C₁₋₅ alkylene, C₁₋₄ alkylene, C₁₋₃ alkylene, etc., and specific examples include, but are not limited to methylene, ethylene, propylene or isopropylene.

As used in the present invention, the term "C₂₋₆ alkenylene" refers to a linear or branched divalent unsaturated hydrocarbon group containing at least one double bond and having 2 to 6 (e.g., 2, 3, 4, 5, or 6) carbon atoms. The C₂₋₆ alkenylene includes C₂₋₅ alkenylene, C₂₋₄ alkenylene, C₂₋₃ alkenylene, etc., and specific examples include, but are not limited to -CH=CH-, -CH=CH-CH=CH- or -CH=CH-CH₂-.

As used in the present invention, the term "C₂₋₆ alkynylene" refers to a linear or branched divalent unsaturated hydrocarbon group containing at least one triple bond and having 2 to 6 (e.g., 2, 3, 4, 5, or 6) carbon atoms. The C₂₋₆ alkynylene includes C₂₋₅ alkynylene, C₂₋₄ alkynylene, C₂₋₃ alkynylene, etc., and specific examples include, but are not limited to -C≡C- or -C≡C -C≡C-.

As used in the present invention, the term "C₁₋₆ alkoxy" refers to -O-C₁₋₆ alkyl, i.e., a group obtained by connecting the carbon atom of the C₁₋₆ alkyl as defined above to an oxygen atom. The C₁₋₆ alkoxy includes C₁₋₅ alkoxy, C₁₋₄ alkoxy, C₁₋₃ alkoxy, etc., and specific examples include, but are not limited to methoxy, ethoxy, propoxy or butoxy.

As used in the present invention, the term "C₁₋₆ alkyleneoxy" refers to -O-C₁₋₆ alkylene, i.e., a group obtained by connecting the carbon atom of the C₁₋₆ alkylene as defined above to an oxygen atom. The C₁₋₆ alkyleneoxy includes C₁₋₅ alkyleneoxy, C₁₋₄ alkyleneoxy, C₁₋₃ alkyleneoxy, etc., and specific examples include, but are not limited to methyleneoxy (-OCH₂-) or ethyleneoxy (-OCH₂CH₂-).

As used in the present invention, the term "halo" refers to the group it modifies being substituted with one or more halogen, for example, substituted with 1, 2, 3, 4, 5, or 6 halogen. For example, "C₁₋₆ haloalkyl" refers to the C₁₋₆ alkyl as defined above substituted with one or more halogen, and specific examples include, but are not limited to -CF₃, -CHF₂, -CH₂F or -CF₂CF₃; "C₁₋₆ haloalkoxy" refers to the C₁₋₆ alkoxy as defined above substituted with one or more halogen, and specific examples include, but are not limited to -OCF₃ or -OCF₂CF₃.

As used in the present invention, the term "C₆₋₁₀ aryl" refers to an unsaturated aromatic carbocyclic group with a conjugated π-electron system, which consists of 6 to 10 (e.g., 6, 7, 8, 9, or 10) carbon atoms and has one monocyclic ring or two or more fused rings. Specific examples include, but are not limited to phenyl or naphthyl.

As used in the present invention, the term "5- to 10-membered heteroaryl" refers to a group with a conjugated π-electron system, which consists of 5 to 10 (e.g., 5, 6, 7, 8, 9 or 10) ring atoms, including monocyclic heteroaromatic rings and polycyclic aromatic rings, wherein 1, 2, 3 or 4 ring atoms are heteroatoms, and the remainder are carbon atoms; preferably, the heteroatoms are selected from N, O or S, wherein the nitrogen atom is optionally quaternised, and the nitrogen and sulphur heteroatoms may optionally be oxidised. The 5- to 10-membered heteroaryl includes 5- to 9-membered, 6- to 9-membered, 5- to 6-membered heteroaryl, etc., and specific examples include, but are not limited to imidazolyl, thiazolyl, pyridyl, thienyl, furyl, pyrrolyl, thiazolyl, oxazolyl or pyrazinyl.

As used in the present invention, the term "C₃₋₁₀ cyclic group" or "C₃₋₁₀ cyclohydrocarbyl" refers to a saturated cyclic hydrocarbon group or a partially unsaturated cyclic hydrocarbon group consisting of 3 to 10 (e.g., 3, 4, 5, 6, 7, 8, 9, or 10) carbon atoms. The C₃₋₁₀ cyclic group includes C₄₋₈ cyclic group, C₄₋₆ cyclic group, C₄₋₇ cyclic group, C₅₋₆ cyclic group, C₅₋₇ cyclic group or C₆₋₇ cyclic group, etc. The C₃₋₁₀ cyclic group includes monocyclic, bicyclic or polycyclic rings, including spiro, fused, or bridged rings. Specific examples include, but are not limited to cyclobutyl, cyclopentyl, cyclohexyl, cyclohexenyl, 1,3-cyclohexadienyl or 1,4-cyclohexadienyl.

As used in the present invention, the term "C₃₋₁₀ cycloalkyl" refers to a saturated hydrocarbon group consisting of 3 to 10 (e.g., 3, 4, 5, 6, 7, 8, 9, or 10) carbon atoms. The C₃₋₁₀ cycloalkyl includes C₃₋₆ cycloalkyl, C₄₋₈ cycloalkyl, C₄₋₆ cycloalkyl or C₅₋₉ cycloalkyl, etc. The C₃₋₁₀ cycloalkyl includes monocyclic, bicyclic or polycyclic rings, including spiro, fused, or bridged rings. Specific examples include, but are not limited to cyclobutyl, cyclopentyl or cyclohexyl.

As used in the present invention, the term "C₃₋₆ cycloalkenyl" refers to a partially unsaturated cyclic hydrocarbon group consisting of 3 to 6 (e.g., 3, 4, 5, or 6) carbon atoms and containing at least one carbon-carbon double bond. The C₃₋₆ cycloalkenyl includes C₄₋₆ cycloalkenyl, C₅ cycloalkenyl or C₆ cycloalkenyl, etc. Specific examples of the C₃₋₆ cycloalkenyl include, but are not limited to cyclopentenyl, cyclohexenyl, 1,3-cyclohexadienyl or 1,4-cyclohexadienyl.

As used in the present invention, the term "4- to 10-membered heterocyclyl" refers to a saturated or partially unsaturated cyclic group consisting of 4 to 10 (e.g., 4, 5, 6, 7, 8, 9 or 10) ring atoms, wherein 1, 2, 3 or 4 ring atoms are heteroatoms, and the remainder are carbon atoms; preferably, the heteroatoms are selected from N, O or S, wherein the nitrogen atom is optionally quaternised, and the nitrogen and sulphur heteroatoms may optionally be oxidised; preferably, the carbon atom is optionally substituted with =O. The 4- to 10-membered heterocyclyl includes 4- to 8-membered, 4- to 6-membered, 4- to 5-membered, 5- to 6-membered, 9- to 10-membered heterocyclyl, etc. The 4- to 10-membered heterocyclyl includes monocyclic, bicyclic or polycyclic rings, including spiro, fused, or bridged rings. Specific examples of the 4- to 10-membered heterocyclyl include, but are not limited to azetidinyl, oxetanyl, pyrrolidyl, 2,3-dihydrofuryl, 3,4-dihydro-2H-pyranyl or 2-oxopyrrolidyl.

As used in the present invention, the term "C₆₋₁₀ arylene" refers to a divalent unsaturated aromatic carbocyclic group with a conjugated π-electron system, which consists of 6 to 10 (e.g., 6, 7, 8, 9, or 10) carbon atoms. Specific examples include, but are not limited to phenylene.

As used in the present invention, the term "5- to 10-membered heteroarylene" refers to a divalent group with a conjugated π-electron system, which consists of 5 to 10 (e.g., 5, 6, 7, 8, 9 or 10) ring atoms, including monocyclic heteroaromatic rings and polycyclic aromatic rings, wherein 1, 2, 3 or 4 ring atoms are heteroatoms, and the remainder are carbon atoms; preferably, the heteroatoms are selected from N, O or S, wherein the nitrogen atom is optionally quaternised, and the nitrogen and sulphur heteroatoms may optionally be oxidised. The 5- to 10-membered heteroarylene includes 5- to 9-membered, 6- to 9-membered, 5- to 6-membered heteroarylene, etc., and specific examples include, but are not limited to imidazolylene or pyridylene.

In the present invention, the purpose of the pharmaceutical composition is to facilitate the administration to an organism, which will enhance the absorption of active ingredients and thus exert biological activities. The carrier includes, but is not limited to an ion exchanger, alumina, aluminium stearate, lecithin, serum proteins such as human albumin, buffer substances such as phosphate, glycerol, sorbic acid, potassium sorbate, a mixture of partial glycerides of saturated vegetable fatty acids, water, salts or electrolytes such as protamine sulphate, disodium hydrogen phosphate, potassium hydrogen phosphate, sodium chloride, zinc salts, colloidal silica, magnesium trisilicate, polyvinylpyrrolidone, cellulose substances, polyethylene glycols, sodium carboxymethyl cellulose, polyacrylates, beeswax, and lanolin. The excipient refers to the additives in a pharmaceutical preparation other than the active pharmaceutical ingredient. These additives are stable, exhibit no incompatibility with the active pharmaceutical ingredient, produce no side effects, and do not impact therapeutic efficacy. They remain resistant to deformation, cracking, mold, or insect infestation under ambient conditions, are non-toxic to the human body and physiologically inert, do not interact chemically or physically with the active pharmaceutical ingredient, and do not interfere with the assay of the active pharmaceutical ingredient. For example, binders, fillers, disintegrants, and lubricants in tablets; wine, vinegar, and medicinal juices in traditional Chinese medicinal pills; the base components in semisolid preparations such as ointments and creams; as well as preservatives, antioxidants, flavouring agents, aromatic agents, cosolvents, emulsifiers, solubilizers, osmotic pressure regulators, colouring agents, etc. in liquid preparations-all of these can be referred to as excipients.

In the present invention, the pharmaceutical composition can be formulated into various suitable dosage forms according to the route of administration, such as tablets, capsules, granules, oral solutions, oral suspensions, oral emulsions, powders, tinctures, syrups, injections, suppositories, ointments, creams, pastes, ophthalmic preparations, pills, implants, aerosols, powder inhalers, and sprays. The pharmaceutical composition or suitable dosage form may contain 0.01 mg to 1000 mg of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the present invention, suitably 0.1 mg to 800 mg, preferably 0.5-500 mg, preferably 0.5-350 mg, particularly preferably 1-250 mg.

As used in the present invention, the term "individual" includes humans or non-human animals. Exemplary human individuals include human individuals suffering from diseases (e.g., diseases described herein) (referred to as patients) or normal individuals. In the present invention, the term "non-human animal" includes all vertebrates, such as non-mammals (e.g., birds, amphibians, and reptiles) and mammals, such as non-human primates, livestock and/or domesticated animals (e.g., sheep, dogs, cats, cows, and pigs).

As used in the present invention, the term "treatment" is intended to alleviate, reduce, ameliorate, or eliminate the targeted disease state or condition. A subject is successfully "treated" if the subject receives a therapeutic amount of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to the methods described herein, and one or more signs and symptoms of the subject exhibit an observable and/or detectable reduction or improvement. It should also be understood that the treatment of the disease state or condition includes not only complete treatment, but also cases where complete treatment is not achieved, yet some biologically or medically relevant outcomes are attained.

As used in the present invention, the term "prevention" is intended to avoid, reduce, inhibit, or delay the onset of a disease or disease-related symptoms, wherein the disease or disease-related symptoms have not yet occurred prior to the administration of the relevant drug. "Prevention" does not necessarily require the complete prevention of the onset of a disease or disease-related symptoms; for example, reducing the risk of a subject developing a specific disease or disease-related symptoms after administration of the relevant drug, or attenuating the severity of the relevant symptoms that subsequently occur, may both be considered as "preventing" the onset or progression of the disease.

As used in the present invention, the term "effective amount" refers to an amount sufficient to achieve the desired therapeutic or prophylactic effect, for example, an amount that reduces the symptoms associated with the disease to be treated, or an amount that can effectively prevent, inhibit, or delay the onset of the disease. It is within the competence of those skilled in the art to determine such effective amounts.

It should also be noted that the dosage and method of use of the compounds of the present invention depend on many factors, including the patient's age, weight, gender, natural health condition, nutritional status, the activity intensity of the compound, the time of administration, the metabolic rate, the severity of the condition, and the subjective judgment of the attending physician. The preferred dosage used is between 0.01 and 100 mg/kg body weight/day.

Unless otherwise specified, the substitutions or combinations of groups in the Markush structures described herein are those that are stable or chemically feasible.

### Beneficial Effects

The compounds of the present invention exhibit AMPK agonist activity, and in particular, some compounds demonstrate more excellent AMPK agonist activity.

### Detailed Description of Embodiments

Embodiments of the present invention will be described in detail below with reference to examples; however, those skilled in the art will understand that the following examples are only used to illustrate the present invention and should not be regarded as limiting the scope of the present invention. If no specific conditions are specified in the examples, they are carried out according to conventional conditions or conditions recommended by the manufacturer. If no manufacturers of the reagents or instruments used are specified, they are conventional products commercially available.

### Example 1: Synthesis of compound 1

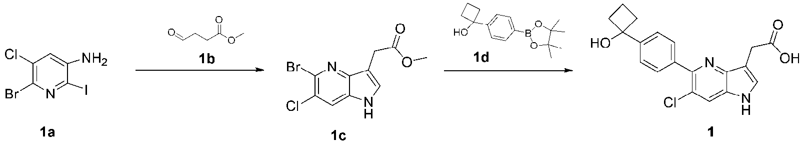

Step 1: At room temperature, intermediate 1a (50 mg, 0.16 mmol), intermediate 1b (50.83 mg, 0.20 mmol), tri-tert-butylphosphonium tetrafluoroborate (90.0 mg, 0.3mmol), triethylenediamine (1.01 g, 9.0 mmol), tris(dibenzylideneacetone)dipalladium (0.14 g, 0.15 mmol) and N,N-dimethylaniline (10 mL) were added to a single-necked flask. The mixture was reacted at 120°C under nitrogen atmosphere for 16 hours. After the reaction was completed, the reaction solution was poured into water (20 mL), and the mixture was extracted with ethyl acetate (3 × 10 mL). The organic phases were combined, washed with saturated aqueous sodium chloride solution (20 mL), dried over anhydrous sodium sulphate, and filtered. The filtrate was concentrated under reduced pressure to afford a crude product, and the crude product was separated and purified by silica gel column chromatography to afford intermediate 1c. LC-MS: m/z: 303.0 (M+H)⁺.

Step 2: At room temperature, intermediate 1c (105 mg, 0.35 mmol), intermediate 1d (Bide Pharmatech Ltd., 113.8 mg, 0.42 mmol), [1,1'-bis(diphenylphosphino)ferrocene]palladium dichloride-dichloromethane complex (28.25 mg, 0.03 mmol), caesium carbonate (338.12 mg, 1.04 mmol), 1,4-dioxane (3.0 mL) and water (1.0 mL) were added to a single-necked flask. The mixture was reacted at 100°C under nitrogen atmosphere for 16 hours. After the reaction was completed, the reaction system was filtered, the filtrate was concentrated under reduced pressure to afford a crude product, and the crude product was separated and purified by high performance liquid chromatography (mobile phase: A: 0.05% TFA/H₂O B: ACN, chromatographic column: Waters-SunFire-C₁₈-10 µm-19*250 mm) to afford compound 1. LC-MS: m/z: 357.2 (M+H)⁺. ¹H NMR (400 MHz, DMSO-*d*₆) δ 11.29 (s, 1H), 7.95 (s, 1H), 7.60 (m, 6H), 5.54 (s, 1H), 3.69 (s, 2H), 2.44 - 2.42 (m, 1H), 2.37 - 2.24 (m, 3H), 1.94 (m, 1H), 1.70 (m, 1H).

### Example 2: Synthesis of compound 2

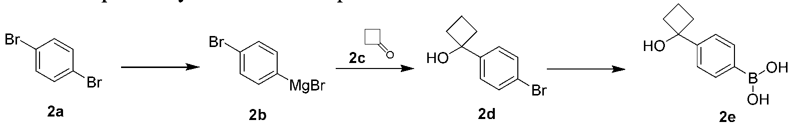

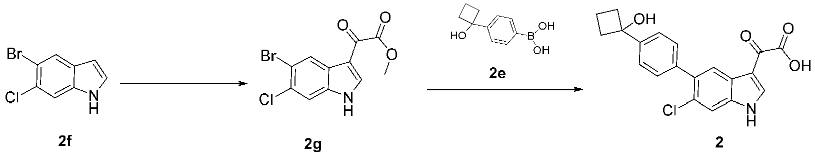

Step 1: Under nitrogen atmosphere, magnesium turnings (0.24 g, 9.96 mmol) and THF (10 mL) were added to a three-necked flask, and intermediate 2a (2.35 g, 9.96 mmol), 1,2-dibromoethane (0.02 g, 0.10 mmol) and THF (10 ml) were sequentially added under stirring. The mixture was reacted at room temperature for 1 hour to afford a reaction solution containing intermediate 2b, which was directly used in the next step.

Step 2: Under nitrogen atmosphere at -40°C, intermediate 2c (700 mg, 9.99 mmol) and THF (5 mL) were added to a three-necked flask, and the reaction solution containing intermediate 2b (20 ml, 7.69 mmol) was added under stirring. The mixture was reacted at 0°C for two hours. The reaction solution was quenched with water, and the mixture was extracted with ethyl acetate. The organic phase was concentrated under reduced pressure to afford a crude product, and the crude product was separated and purified by silica gel column chromatography to afford intermediate 2d. LC-MS: m/z: 209.0 (M+H-18)⁺.

Step 3: Under nitrogen atmosphere, intermediate 2d (500 mg, 2.20 mmol) and THF (5 mL) were added to a three-necked flask, and n-butyllithium (1.76 mL, 4.40 mmol) was added under stirring. The mixture was stirred at -78°C for half an hour, and then triisopropyl borate (496.88 mg, 2.64 mmol) and THF (2 mL) were added. The resulting mixture was reacted for two hours until completion. The reaction solution was quenched with water, and the mixture was extracted with ethyl acetate. The organic phase was dried over anhydrous NaSO₄, and concentrated under reduced pressure to afford a crude product. The crude product was slurried with petroleum ether to finally afford intermediate 2e. LC-MS: m/z: 175.0 (M+H-18)⁺.

Step 4: Under nitrogen atmosphere, intermediate 2f (1.0 g, 4.34 mmol) and dichloromethane (10 mL) were added to a three-necked flask, and aluminium trichloride (2.31 g, 17.36 mmol) was added under stirring, followed by dropwise addition of a solution of methyl oxalyl chloride (1.85 mg, 15.12 mmol) in dichloromethane (4 mL). The mixture was reacted at room temperature for 24 hours. The reaction solution was poured into water, and the mixture was extracted with ethyl acetate three times. The organic phase was dried over anhydrous sodium sulphate, and filtered. The filtrate was concentrated to afford a crude product, and the crude product was slurried with petroleum ether/ethyl acetate (V/V=5/1) to afford intermediate 2g. LC-MS: m/z: 316.0 (M+H)⁺. ¹H NMR (400 MHz, DMSO-*d₆*) δ 12.78 (s, 1H), 8.57 (s, 1H), 8.45 (s, 1H), 7.85 (s, 1H), 3.90 (s, 3H).

Step 5: To a microwave tube were added intermediate 2g (100 mg, 0.32 mmol), intermediate 2e (72.79 mg, 0.38 mmol), [1,1'-bis(diphenylphosphino)ferrocene]palladium dichloride-dichloromethane complex (25.80 mg, 0.03 mmol), potassium carbonate (130.98 mg, 0.95 mmol), 1,4-dioxane (3 mL) and water (0.75 mL). The mixture was reacted at 100°C under microwave conditions for two hours until completion. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 2. LC-MS: m/z: 370.0 (M+H)⁺. ¹H NMR (400 MHz, DMSO-*d₆*) δ 12.41 (s, 1H), 8.51 (s, 1H), 8.12 (s, 1H), 7.73 (s, 1H), 7.59 (d, 2H), 7.43 (d, 2H), 5.53 (s, 1H), 2.47-2.32 (m, 2H), 2.30-2.27 (m, 2H), 2.01-1.97 (m, 1H), 1.94-1.64 (m, 1H).

### Example 3: Synthesis of compound 3

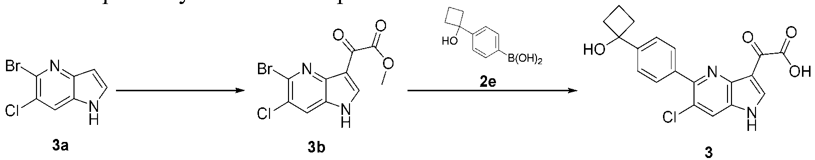

Step 1: At room temperature, intermediate 3a (500 mg, 2.16 mmol), dichloromethane (15 mL) and aluminium trichloride (1.01 g, 7.56 mmol) were sequentially added to a three-necked flask. After purging with nitrogen, the mixture was stirred and reacted at room temperature for 1 hour. Subsequently, methyl oxalyl chloride (926 mg, 7.56 mmol) dissolved in dichloromethane (2 mL) was slowly added dropwise to the three-necked flask, and the mixture was reacted at room temperature overnight until completion. After the reaction was completed, the reaction solution was poured into ice water to quench the reaction, and the mixture was extracted with ethyl acetate 3 times. The organic phases were collected, dried over anhydrous sodium sulphate, and concentrated under reduced pressure. The crude product was slurried with petroleum ether/ethyl acetate (V/V=5/1) to afford intermediate 3b. LC-MS: m/z: 317.0 (M+H)⁺. ¹H NMR (400 MHz, DMSO-*d*₆) δ 12.89 (s, 1H), 8.71 (s, 1H), 8.26 (s, 1H), 3.92 (s, 3H).

Step 2: At room temperature, intermediate 3b (50 mg, 0.16 mmol), intermediate 2e (45 mg, 0.24 mmol), [1,1'-bis(diphenylphosphino)ferrocene]palladium dichloride-dichloromethane complex (12.86 mg, 0.02 mmol), potassium carbonate (65 mg, 0.47 mmol), 1,4-dioxane (2 mL) and water (0.50 mL) were added to a microwave tube. The mixture was subjected to a microwave reaction at 100°C under nitrogen atmosphere for two hours. After the reaction was completed, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 3. LC-MS: m/z: 371.0 (M+H)⁺. ¹H NMR (400 MHz, DMSO-*d*₆) δ 8.41 (s, 1H), 8.07 (s, 1H), 7.63 - 7.57 (m, 4H), 7.25 (d, *J* = 8.6 Hz, 1H), 5.58 (s, 1H), 2.48 - 2.41 (m, 2H), 2.35 - 2.28 (m, 2H), 1.99 - 1.89 (m, 1H), 1.76 - 1.65 (m, 1H).

### Example 4: Synthesis of compound 4

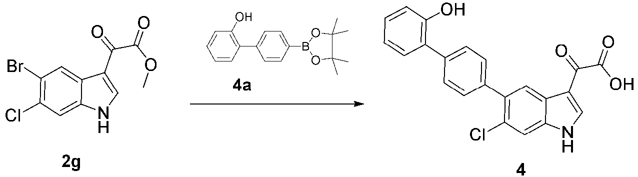

Step 1: To a microwave tube were added intermediate 2g (100 mg, 0.32 mmol), intermediate 4a (102.92 mg, 0.35 mmol), [1,1'-bis(diphenylphosphino)ferrocene]palladium dichloride-dichloromethane complex (25.80 mg, 0.03 mmol), potassium carbonate (130.98 mg, 0.95 mmol), 1,4-dioxane (4 mL) and water (1 mL). The mixture was reacted at 100°C under microwave conditions for two hours. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 4. LC-MS: m/z: 392.1 (M+H)⁺. ¹H NMR (400 MHz, DMSO-*d₆*) δ 9.64 (s, 1H), 8.15 (m, 2H), 7.65-7.63 (m, 3H), 7.47-7.44 (m, 2H), 7.36-7.33 (m, 1H), 7.20-7.16 (m, 2H), 6.99-6.97 (m, 1H), 6.93-6.89 (m, 1H).

### Example 5: Synthesis of compound 5

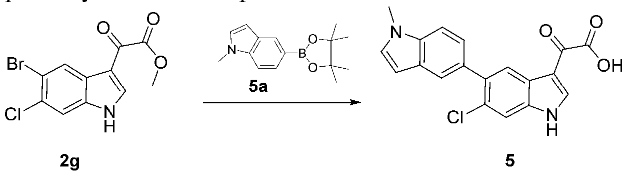

Step 1: To a microwave tube were added intermediate 2g (50 mg, 0.16 mmol), intermediate 5a (50.83 mg, 0.20 mmol), [1,1'-bis(diphenylphosphino)ferrocene]palladium dichloride-dichloromethane complex (13.07 mg, 0.02 mmol), potassium carbonate (66.34 mg, 0.48 mmol), 1,4-dioxane (2 mL) and water (0.5 mL). The mixture was reacted at 100°C under microwave conditions for two hours. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 5. LC-MS: m/z: 353.0 (M+H)⁺. ¹H NMR (400 MHz, DMSO-*d₆*) δ 8.31 (s, 1H), 8.16 (s, 1H), 7.66 (s, 1H), 7.57 (s, 1H), 7.56-7.49 (m, 1H), 7.39-7.38 (m, 1H), 7.22-7.17 (m, 2H), 6.49-6.48 (m, 1H), 3.84 (s, 3H).

### Example 6: Synthesis of compound 6

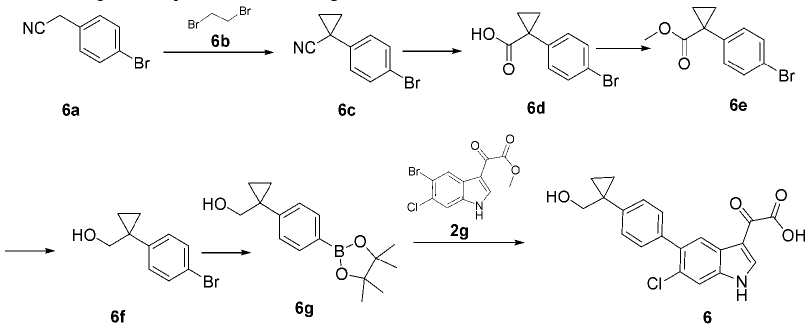

Step 1: Sodium hydride (24 g, 1.02 mol) was dissolved in a three-necked flask containing DMF (300 ml), and the mixture was cooled to 0°C and purged with nitrogen. Subsequently, intermediate 6a (20 g, 0.102 mol) and intermediate 6b (134 g, 0.714 mol) were dissolved in DMF (200 ml), and the mixture was added dropwise to the three-necked flask at 0°C. The resulting mixture was warmed to room temperature and reacted at room temperature for two hours. The reaction solution was poured into ice water (500 ml), and the mixture was extracted with ethyl acetate (500 ml). The organic phase was washed with aqueous sodium chloride solution, dried overnight over anhydrous sodium sulphate, and filtered to afford the filtrate. The filtrate was concentrated to afford a crude product, which was subjected to silica gel column chromatography to afford intermediate 6c. LC-MS: m/z:222.0 (M+H)⁺. ¹H NMR (400 MHz, CDCl₃) δ 7.48 (d, J = 8.6 Hz, 2H), 7.17 (d, J = 8.6 Hz, 2H), 1.77-1.72 (m, 2H), 1.41-1.36 (m, 2H).

Step 2: Intermediate 6c (20 g, 90.05 mmol) and barium hydroxide (62 g, 360.21 mmol) were dissolved in isopropanol (200 ml) and water (600 ml), and the mixture was reacted at 100°C for sixteen hours. The reaction solution was adjusted to pH 3-4 with 1 mol/L dilute hydrochloric acid, and the mixture was extracted with ethyl acetate. The organic phase was washed with aqueous sodium chloride solution, dried overnight over anhydrous sodium sulphate, filtered, and concentrated to afford intermediate 6d. LC-MS: m/z:241.0 (M+H)⁺. ¹H NMR (400 MHz, CDCl₃) δ 7.41 (d, J = 7.9 Hz, 2H), 7.19 (d, J = 7.9 Hz, 2H), 1.58 (d, J = 5.2 Hz, 2H), 1.16 (s, 2H).

Step 3: Intermediate 6d (23 g, 95.40 mmol) was dissolved in methanol (300 ml), and the mixture was cooled to 0°C, and then thionyl chloride (34 g, 286.21 mmol) was added dropwise. The mixture was reacted at 80°C for sixteen hours. The reaction system was concentrated under reduced pressure to afford a crude product, which was separated and purified by silica gel column chromatography to afford intermediate 6e. LC-MS: m/z:255.0 (M+H)⁺. ¹H NMR (400 MHz, DMSO-*d₆*) δ 7.53-7.47 (m, 2H), 7.33 - 7.27 (m, 2H), 3.55 (s, 3H), 1.49 (q, m, 2H), 1.21-1.17 (m, 2H).

Step 4: Intermediate 6e (13.8 g, 54.09 mmol) was dissolved in tetrahydrofuran (150 ml), and the mixture was purged with nitrogen and cooled to 0°C. Subsequently, 1 mol/L solution of lithium aluminium hydride in tetrahydrofuran (64.91 ml, 64.91 mmol) was added dropwise at 0°C, and the resulting mixture was warmed to room temperature and reacted for two hours until completion. Subsequently, the reaction solution was cooled to 0°C, and water (2.5 mL), 15% aqueous sodium hydroxide solution (2.5 mL), and water (7.5 mL) were sequentially added dropwise thereto. The mixture was warmed to room temperature and stirred for 30 minutes, and an appropriate amount of anhydrous sodium sulphate was added. The resulting mixture was stirred at room temperature for 30 minutes. Finally, the reaction system was filtered, and the filtrate was concentrated to afford intermediate 6f. LC-MS: m/z:210 (M+H-OH)⁺. ¹H NMR (400 MHz, DMSO-*d₆*) δ 7.49-7.42 (m, 2H), 7.28-7.22 (m, 2H), 4.72 (t, *J* = 5.6 Hz, 1H), 3.50 (d, *J=* 5.5 Hz, 2H), 0.86-0.80 (m, 2H), 0.75-0.69 (m, 2H).

Step 5: Intermediate 6f (9.8 g, 43.16 mmol), bis(pinacolato)diboron (13.14 g, 51.78 mmol), potassium acetate (12.7 g, 129.46 mmol), and [1,1'-bis(diphenylphosphino)ferrocene]palladium dichloride-dichloromethane complex (3.52 g, 4.32 mmol) were dissolved in 1,4-dioxane (100 mL). The mixture was purged with nitrogen and reacted at 80°C for sixteen hours. The reaction solution was filtered, the filter cake was washed three times with an appropriate amount of ethyl acetate, and the filtrate was extracted with water and ethyl acetate. The organic phase was washed with aqueous sodium chloride solution, dried overnight over anhydrous sodium sulphate, filtered, and concentrated to afford a crude product, which was separated and purified by silica gel column chromatography (mobile phase: PE/EA = 10/1) to afford intermediate 6g. LC-MS: m/z:297.2 (M+Na)⁺. ¹H NMR (400 MHz, DMSO-*d₆*) δ 7.58 (d, *J =* 7.9 Hz, 2H), 7.29 (d, *J =* 8.1 Hz, 2H), 4.68 (t, *J=* 5.6 Hz, 1H), 3.54 (d, *J* = 5.7 Hz, 2H), 1.28 (s, 12H), 0.89-0.83 (m, 2H), 0.74 (t, *J* = 2.9 Hz, 2H).

Step 6: To a microwave tube were added intermediate 2g (80 mg, 0.25 mmol), intermediate 6g (75.40 mg, 0.28 mmol), [1,1'-bis(diphenylphosphino)ferrocene]palladium dichloride-dichloromethane complex (20.42 mg, 0.03 mmol), potassium carbonate (86.37 mg, 0.63 mmol), 1,4-dioxane (3 mL) and water (0.75 mL). The mixture was reacted at 100°C under microwave conditions for two hours. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 6. LC-MS: m/z: 370.0 (M+H)⁺. ¹H NMR (400 MHz, DMSO-*d₆*) δ 8.27 (s, 1H), 8.09 (s, 1H), 7.65 (s, 1H), 7.41-7.32 (m, 5H), 4.75 (s, 1H), 3.59 (s, 2H), 0.89-0.85 (m, 2H), 0.83-0.79 (m, 2H).

### Example 7: Synthesis of compound 7

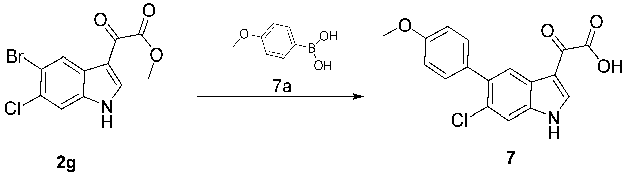

Step 1: To a microwave tube were added intermediate 2g (50 mg, 0.16 mmol), intermediate 7a (26.74 mg, 0.18 mmol), [1,1'-bis(diphenylphosphino)ferrocene]palladium dichloride-dichloromethane complex (13.07 mg, 0.02 mmol), potassium carbonate (55.28 mg, 0.40 mmol), 1,4-dioxane (2 mL) and water (0.5 mL). The mixture was reacted at 100°C under microwave conditions for two hours. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 7. LC-MS: m/z: 330.0 (M+H)⁺. ¹H NMR (400 MHz, DMSO-*d₆*) δ 8.25 (s, 1H), 8.10 (s, 1H), 7.64 (s, 1H), 7.37-7.34 (m, 2H), 7.21 (s, 1H), 7.04-7.00 (m, 2H), 3.81 (s, 3H).

### Example 8: Synthesis of compound 8

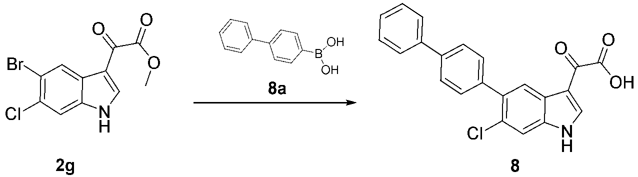

Step 1: To a microwave tube were added intermediate 2g (50 mg, 0.16 mmol), intermediate 8a (34.85 mg, 0.18 mmol), [1,1'-bis(diphenylphosphino)ferrocene]palladium dichloride-dichloromethane complex (13.07 mg, 0.02 mmol), potassium carbonate (55.28 mg, 0.40 mmol), 1,4-dioxane (2 mL) and water (0.5 mL). The mixture was reacted at 100°C under microwave conditions for two hours. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 8. LC-MS: m/z: 376.0 (M+H)⁺. ¹H NMR (400 MHz, DMSO-*d₆*) δ 12.25(s, 1H), 8.37 (s, 1H), 8.17 (s, 1H), 7.78-7.74 (m, 4H), 7.71 (s, 1H), 7.55-7.50 (m, 4H) 7.42-7.38 (m, 1H).

### Example 9: Synthesis of compound 9

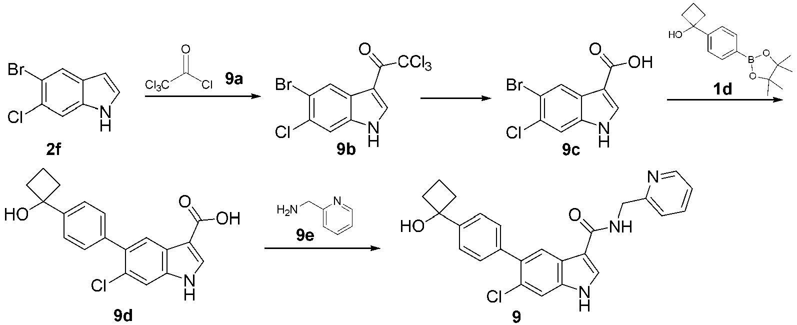

Step 1: At room temperature, intermediate 2f (5.0 g, 21.7 mmol), 4-dimethylaminopyridine (270 mg, 2.17 mmol), pyridine (4.46 g, 56.4 mmol) and tetrahydrofuran (60 mL) were added to a 250 mL single-necked flask. In an ice bath, intermediate 9a (9.47 g, 52.1 mmol) was slowly added dropwise. The final reaction solution was stirred and reacted at 60°C for 8 hours. After the reaction was completed, the reaction solution was quenched with water (100 mL), and the mixture was extracted with ethyl acetate (50 mL x 3). The organic phases were combined, dried over anhydrous sodium sulphate, and concentrated to afford a crude product, and the crude product was separated and purified by silica gel column chromatography to afford intermediate 9b. LC-MS: m/z: 375.8 (M+H)⁺. ¹H NMR (400 MHz, DMSO-*d*₆) δ 12.75 (s, 1H), 8.71-8.65 (m, 1H), 8.50-8.45 (m, 1H), 7.86-7.82 (m, 1H).

Step 2: At room temperature, intermediate 9b (6.3 g, 16.1 mmol) dissolved in mixed solvents of methanol (25 mL) and water (30 mL) was added to a 100 mL single-necked flask, and then sodium hydroxide (1.68 g, 41.9 mmol) was added. The reaction solution was reacted at 60°C for 18 hours. After the reaction was completed, the reaction solution was concentrated under reduced pressure to remove methanol, followed by extraction with ethyl acetate (30 mL x 3), and the aqueous phase was retained. Dilute hydrochloric acid was added to the aqueous phase to adjust the pH to acidic, followed by extraction with ethyl acetate (30 mL x 3). The organic phases were combined, dried over anhydrous sodium sulphate, and concentrated to afford a crude product, and the crude product was subjected to slurrying treatment to afford intermediate 9c. LC-MS: m/z: 275.9 (M+H)⁺. ¹H NMR (400 MHz, DMSO-*d*₆) δ 12.31 (s, 1H), 12.08 (s, 1H), 8.30 (s, 1H), 8.12 (d, *J=* 2.6 Hz, 1H), 7.75 (s, 1H).

Step 3: At room temperature, intermediate 9c (600 mg, 2.19 mmol), intermediate 1d (719.13 mg, 2.62 mmol), [1,1'-bis(diphenylphosphino)ferrocene]palladium dichloride (159.94 mg, 0.22 mmol), potassium carbonate (604.15 mg, 4.37 mmol), N,N-dimethylformamide (5 mL) and water (1 mL) were sequentially added to a 25 mL single-necked flask. After purging with nitrogen three times, the reaction solution was reacted at 80°C for 18 hours. After the reaction was completed, the reaction solution was concentrated, and the crude product was separated and purified by silica gel column chromatography to afford intermediate 9d. LC-MS: m/z: 324.1 (M + H -18)⁺.

Step 4: At room temperature, intermediate 9d (50 mg, 0.15 mmol), HATU (65 mg, 0.18 mmol) and N,N-diisopropylethylamine (38 mg, 0.30 mmol) dissolved in N,N-dimethylformamide (2.0 mL) were added to a 25 mL single-necked flask, followed by dropwise addition of 2-aminomethylpyridine (intermediate 9e) (15.8 mg, 0.15 mmol). The reaction solution was stirred and reacted at room temperature for 2 hours. After the reaction was completed, the reaction solution was concentrated, and the crude product was separated and purified by high performance liquid chromatography (mobile phase: A: 0.05% TFA/H₂O B: ACN, chromatographic column: Waters-SunFire-C₁₈-10 µm-19*250 mm) to finally afford compound 9. LC-MS: m/z: 432.2 (M+H)⁺. ¹H NMR (400 MHz, DMSO-*d*₆) δ 11.77 (d, *J* = 2.9 Hz, 1H), 8.73 (t, *J* = 5.9 Hz, 1H), 8.63 (d, *J=* 5.0 Hz, 1H), 8.19 (d, *J* = 2.9 Hz, 1H), 8.12 (s, 1H), 8.08-8.00 (m, 1H), 7.64 (s, 1H), 7.58 (d, *J* = 8.0 Hz, 1H), 7.57-7.53 (m, 2H), 7.53-7.48 (m, 1H), 7.41-7.37 (m, 2H), 4.64 (d, *J* = 5.7 Hz, 2H), 2.47-2.39 (m, 2H), 2.34-2.25 (m, 2H), 2.00-1.88 (m, 1H), 1.74-1.63 (m, 1H).

### Example 10: Synthesis of compound 10

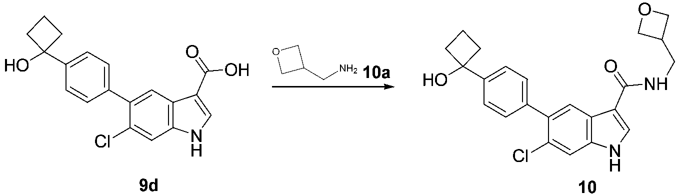

Step 1: At room temperature, intermediate 9d (100 mg, 0.29 mmol) dissolved in super-dry *N,N*-dimethylformamide (3.0 mL) was added to a 25 mL single-necked flask, and then HATU (111.25 mg, 0.29 mmol), *N,N*-diisopropylethylamine (0.1 mL, 0.59 mmol) and intermediate 10a (28.04 mg, 0.32 mmol) were sequentially added. The mixture was reacted for 2 hours until completion. After the reaction was completed, the reaction solution was concentrated, and the crude product was separated and purified by silica gel column chromatography to afford crude compound 10, which was then separated and purified by high performance liquid chromatography (mobile phase: A: 0.05% TFA/H₂O B: ACN, chromatographic column: Waters-SunFire-C₁₈-10 µm-19*250 mm) to finally afford compound 10. LC-MS: m/z: 411.2 (M+H)⁺. ¹H NMR (400 MHz, DMSO-*d*₆) δ 11.68 (s, 1H), 8.11 (d, *J* = 2.0 Hz, 2H), 8.07 (s, 1H), 7.61 (s, 1H), 7.59-7.53 (m, 2H), 7.43-7.35 (m, 2H), 5.53 (s, 1H), 4.62 (dd, *J* = 7.8, 6.0 Hz, 2H), 4.35 (t, *J=* 6.0 Hz, 2H), 3.51 (t, *J=* 6.3 Hz, 2H), 3.19-3.11 (m, 1H), 2.48-2.39 (m, 2H), 2.34-2.27 (m, 2H), 2.00-1.91 (m, 1H), 1.76-1.62 (m, 1H).

### Example 11: Synthesis of compound 11

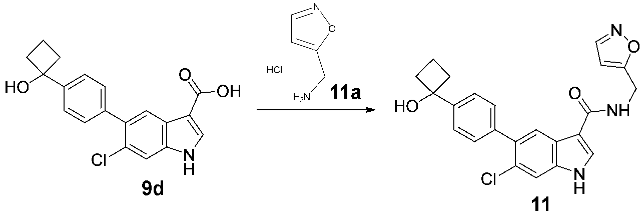

Step 1: At room temperature, intermediate 9d (100 mg, 0.29 mmol) dissolved in super-dry *N,N-*dimethylformamide (3.0 mL) was added to a 25 mL single-necked flask, and then intermediate 11a (39.02 mg, 0.29 mmol), HATU (111.25 mg, 0.29 mmol), and *N,N*-diisopropylethylamine (0.15 mL, 0.88 mmol) were added. The mixture was reacted at room temperature for 2 hours until completion. After the reaction was completed, the reaction solution was concentrated, and the crude product was separated and purified by silica gel column chromatography to afford crude compound 11, which was then separated and purified by high performance liquid chromatography (mobile phase: A: 0.05% TFA/H₂O B: ACN, chromatographic column: Waters-SunFire-C₁₈-10 µm-19*250 mm) to finally afford compound 11. LC-MS: m/z: 422.2 (M+H)⁺. ¹H NMR (400 MHz, DMSO-*d*₆) δ 11.78-11.72 (m, 1H), 8.66 (t, *J=* 5.8 Hz, 1H), 8.47 (d, *J=* 2.0 Hz, 1H), 8.14 (d, *J=* 3.2 Hz, 1H), 8.12 (s, 1H), 7.62 (s, 1H), 7.58-7.53 (m, 2H), 7.42-7.37 (m, 2H), 6.34 (d, *J=* 1.6 Hz, 1H), 4.60 (d, *J* = 5.8 Hz, 2H), 2.48-2.38 (m, 2H), 2.36-2.24 (m, 2H), 2.02-1.87 (m, 1H), 1.75-1.63 (m, 1H).

### Example 12: Synthesis of compound 12

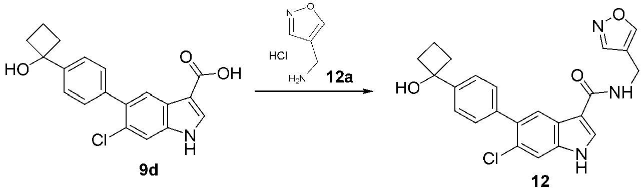

Step 1: At room temperature, intermediate 9d (100 mg, 0.29 mmol) dissolved in super-dry N,N-dimethylformamide (3.0 mL) was added to a 25 mL single-necked flask, and then intermediate 12a (39.02 mg, 0.29 mmol), HATU (111.25 mg, 0.29 mmol), and N,N-diisopropylethylamine (0.15 mL, 0.88 mmol) were added. The mixture was reacted at room temperature for 2 hours until completion. After the reaction was completed, the reaction solution was concentrated, and the crude product was separated and purified by silica gel column chromatography, and then separated and purified by high performance liquid chromatography (mobile phase: A: 0.05% TFA/H₂O B: ACN, chromatographic column: Waters-SunFire-C₁₈-10 µm-19*250 mm) to finally afford compound 12. LC-MS: m/z: 422.2 (M+H)⁺. ¹H NMR (400 MHz, DMSO-*d*₆) δ 11.71 (d, *J* = 2.4 Hz, 1H), 8.84 (s, 1H), 8.55 (s, 1H), 8.43 (t, *J=* 5.6 Hz, 1H), 8.13 (s, 1H), 8.09 (d, *J=* 2.8 Hz, 1H), 7.61 (s, 1H), 7.59-7.53 (m, 2H), 7.43-7.37 (m, 2H), 4.32 (d, *J=* 5.6 Hz, 2H), 2.47-2.40 (m, 2H), 2.36-2.24 (m, 2H), 2.01-1.89 (m, 1H), 1.76-1.64 (m, 1H).

### Example 13: Synthesis of compound 13

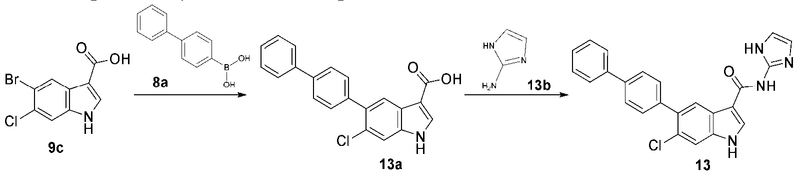

Step 1: At room temperature, intermediate 9c (280 mg, 1.02 mmol), intermediate 8a (242.4 mg, 1.22 mmol), [1,1'-bis(diphenylphosphino)ferrocene]palladium dichloride (74.64 mg, 0.1 mmol), potassium carbonate (281.94 mg, 2.04 mmol), N,N-dimethylformamide (5 mL) and water (1 mL) were sequentially added to a 25 mL single-necked flask. After purging with nitrogen three times, the reaction solution was reacted at 90°C for 3 hours. After the reaction was completed, the reaction solution was concentrated, and the crude product was separated and purified by silica gel column chromatography to afford intermediate 13a. LC-MS: m/z: 348.1 (M+H)⁺.

Step 2: At room temperature, intermediate 13a (120 mg, 0.35 mmol) and *N,N-*dimethylformamide (0.01 mL, 0.13 mmol) dissolved in super-dry dichloromethane (5 mL) were added to a 50 mL single-necked flask, and oxalyl chloride (0.09 mL, 1.04 mmol) was slowly added in an ice bath. The mixture was naturally warmed to room temperature and reacted for 2 hours. After the reaction was completed, the reaction solution was concentrated to afford a residue, which was directly used in the next reaction. At room temperature, the above residue (50 mg, 0.14 mmol) dissolved in super-dry tetrahydrofuran (3.0 mL) was added to a 25 mL single-necked flask, and intermediate 13b (22.69 mg, 0.27 mmol) and triethylamine (0.06 mL, 0.41 mmol) were sequentially added in an ice bath. The final reaction solution was reacted at room temperature for 2 hours. After the reaction was completed, the reaction solution was concentrated, and the crude product was separated and purified by high performance liquid chromatography (mobile phase: A: 0.05% TFA/H₂O B: ACN, chromatographic column: Waters-SunFire-C₁₈-10 µm-19*250 mm) to finally afford compound 13. LC-MS: m/z: 413.2 (M+H)⁺. ¹H NMR (400 MHz, DMSO-*d*₆) δ 12.74 (d, *J* = 3.3 Hz, 1H), 8.42 (s, 2H), 8.40 (d, *J =* 3.3 Hz, 1H), 8.07 (s, 1H), 7.85-7.72 (m, 5H), 7.60 (d, *J* = 2.7 Hz, 1H), 7.58-7.47 (m, 4H), 7.45-7.37 (m, 1H), 7.10 (d, *J* = 2.7 Hz, 1H).

### Example 14: Synthesis of compound 14

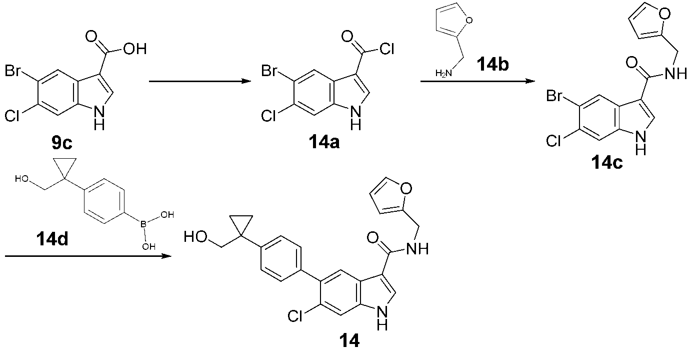

Step 1: At room temperature, intermediate 9c (600 mg, 2.19 mmol) dissolved in super-dry dichloromethane (40.0 mL) was added to a 100 mL single-necked flask. After cooling to 0°C in an ice bath, oxalyl chloride (0.37 mL, 4.38 mmol) and *N,N-*dimethylformamide (0.033 mL, 0.44 mmol) were sequentially added. The mixture was naturally warmed to room temperature and reacted for 2 hours until completion. After the reaction was completed, the reaction solution was concentrated to afford intermediate 14a, which was directly used in the next reaction.

Step 2: At room temperature, intermediate 14a (100 mg, 0.34 mmol) dissolved in super-dry dichloromethane (20.0 mL) was added to a 50 mL single-necked flask. After cooling to 0°C in an ice bath, intermediate 14b (49.53 mg, 0.51 mmol) and *N,N*-diisopropylethylamine (0.22 mL, 1.36 mmol) were sequentially added. The mixture was naturally warmed to room temperature and reacted for 2 hours until completion. After the reaction was completed, the reaction solution was concentrated, and the crude product was separated and purified by silica gel column chromatography to afford intermediate 14c. LC-MS: m/z: 353.0 (M+H)⁺. ¹H NMR (400 MHz, DMSO-*d*₆) δ 11.82 (s, 1H), 8.53 (t, *J* = 5.6 Hz, 1H), 8.49 (s, 1H), 8.16 (d, *J=* 2.6 Hz, 1H), 7.71 (s, 1H), 7.58 (d, *J=* 1.0 Hz, 1H), 6.40 (dd, *J* = 3.2, 2.0 Hz, 1H), 6.28 (d, *J* = 3.0 Hz, 1H), 4.45 (d, *J* = 6.0 Hz, 1H).

Step 3: At room temperature, intermediate 14c (77 mg, 0.22 mmol), intermediate 14d (62.72 mg, 0.33 mmol), [1,1'-bis(diphenylphosphino)ferrocene]palladium dichloride (31.87 mg, 0.04 mmol), potassium carbonate (60.19 mg, 0.44 mmol), dioxane (8.0 mL), and water (1.6 mL) were added to a 50 mL single-necked flask. The mixture was reacted at 90°C under nitrogen atmosphere for 2 hours until completion. After the reaction was completed, the reaction solution was concentrated, and the crude product was separated and purified by silica gel column chromatography to afford crude compound 14, which was then separated and purified by high performance liquid chromatography (mobile phase: A: H₂O B: ACN, chromatographic column: Waters-SunFire-C₁₈-10 µm-19*250 mm) to finally afford compound 14. LC-MS: m/z: 421.2 (M+H)⁺. ¹H NMR (400 MHz, DMSO-*d*₆) δ 11.67 (d, *J* = 1.6 Hz, 1H), 8.43 (t, *J* = 5.6 Hz, 1H), 8.16-8.09 (m, 2H), 7.59 (s, 1H), 7.56 (dd, *J* = 1.6, 0.6 Hz, 1H), 7.40-7.35 (m, 2H), 7.35-7.30 (m, 2H), 6.39 (dd, *J* = 3.2, 2.0 Hz, 1H), 6.26 (d, *J* = 2.8 Hz, 1H), 4.70 (t, *J=* 5.6 Hz, 1H), 4.44 (d, *J* = 5.6 Hz, 2H), 3.59 (d, *J* = 5.6 Hz, 2H), 0.89-0.87 (m, 2H), 0.81-0.78 (m, 2H).

### Example 15: Synthesis of compound 15

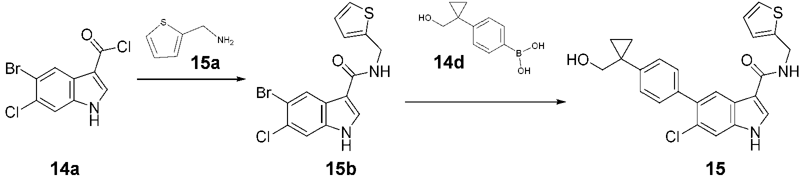

Step 1: At room temperature, intermediate 14a (100 mg, 0.34 mmol) dissolved in super-dry dichloromethane (20.0 mL) was added to a 50 mL single-necked flask. After cooling to 0°C in an ice bath, intermediate 15a (57.95 mg, 0.51 mmol) and N,N-diisopropylethylamine (0.22 mL, 1.36 mmol) were sequentially added. The mixture was naturally warmed to room temperature and reacted for 2 hours until completion. After the reaction was completed, the reaction solution was concentrated, and the crude product was separated and purified by silica gel column chromatography to afford intermediate 15b. ¹H NMR (400 MHz, DMSO-*d*₆) δ 11.82 (d, *J* = 2.4 Hz, 1H), 8.70 (t, *J* = 6.0 Hz, 1H), 8.49 (s, 1H), 8.14 (d, *J=* 3.2 Hz, 1H), 7.72 (s, 1H), 7.38 (dd, *J* = 5.0, 1.2 Hz, 1H), 7.04 - 7.00 (m, 1H), 6.96 (dd, *J* = 5.2, 3.6 Hz, 1H), 4.62 (d, *J=* 6.0 Hz, 2H).

Step 2: At room temperature, intermediate 15b (52 mg, 0.14 mmol), intermediate 14d (40.52 mg, 0.21 mmol), [1,1'-bis(diphenylphosphino)ferrocene]palladium dichloride (20.59 mg, 0.03 mmol), potassium carbonate (38.88 mg, 0.28 mmol), dioxane (7.0 mL), and water (1.4 mL) were added to a 50 mL single-necked flask. The flask was evacuated and backfilled with nitrogen, and the mixture was reacted at 90°C under nitrogen atmosphere for 2 hours until completion. After the reaction was completed, the reaction solution was concentrated, and the crude product was separated and purified by silica gel column chromatography to afford crude compound 15, which was then separated and purified by high performance liquid chromatography (mobile phase: A: H₂O B: ACN, chromatographic column: Waters-SunFire-C₁₈-10 µm-19*250 mm) to finally afford compound 15. LC-MS: m/z: 437.2 (M+H)⁺. ¹H NMR (400 MHz, DMSO-*d*₆) δ 11.69 (d, *J* = 2.0 Hz, 1H), 8.60 (t, *J=* 6.0 Hz, 1H), 8.12 (s, 1H), 8.10 (d, *J=* 2.8 Hz, 1H), 7.60 (s, 1H), 7.40-7.32 (m, 5H), 7.01 (d, *J* = 2.4 Hz, 1H), 6.95 (dd, *J* = 5.0, 3.4 Hz, 1H), 4.70 (t, *J=* 5.6 Hz, 1H), 4.61 (d, *J=* 5.8 Hz, 2H), 3.59 (d, *J=* 5.7 Hz, 2H), 0.91-0.85 (m, 2H), 0.82-0.75 (m, 2H).

### Example 16: Synthesis of compound 16

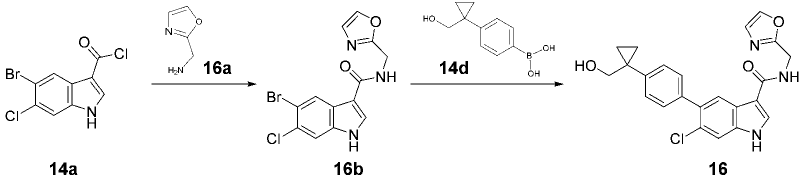

Step 1: At room temperature, intermediate 14a (100 mg, 0.34 mmol) dissolved in super-dry dichloromethane (20.0 mL) was added to a 50 mL single-necked flask. After cooling to 0°C in an ice bath, intermediate 16a (68.90 mg, 0.51 mmol) and N,N-diisopropylethylamine (0.22 mL, 1.36 mmol) were sequentially added. The mixture was naturally warmed to room temperature and reacted for 2 hours until completion. After the reaction was completed, the reaction solution was concentrated, and the crude product was separated and purified by silica gel column chromatography to afford intermediate 16b. ¹H NMR (400 MHz, DMSO-*d*₆) δ 11.85 (s, 1H), 8.73 (t, *J* = 5.8 Hz, 1H), 8.46 (s, 1H), 8.17 (d, *J* = 2.4 Hz, 1H), 8.05 (s, 1H), 7.73 (s, 1H), 7.16 (s, 1H), 4.57 (d, *J=* 5.8 Hz, 2H).

Step 2: At room temperature, intermediate 16b (68 mg, 0.19 mmol), intermediate 14d (55.24 mg, 0.29 mmol), [1,1'-bis(diphenylphosphino)ferrocene]palladium dichloride (28.06 mg, 0.04 mmol), potassium carbonate (53.01 mg, 0.38 mmol), dioxane (7.0 mL), and water (1.4 mL) were added to a 50 mL single-necked flask. The flask was evacuated and backfilled with nitrogen, and the mixture was reacted at 90°C under nitrogen atmosphere for 2 hours until completion. After the reaction was completed, the reaction solution was concentrated, and the crude product was separated and purified by silica gel column chromatography to afford crude compound 16, which was then separated and purified by high performance liquid chromatography (mobile phase: A: H₂O B: ACN, chromatographic column: Waters-SunFire-C₁₈-10 µm-19*250 mm) to finally afford compound 16. LC-MS: m/z: 422.2 (M+H)⁺. ¹H NMR (400 MHz, DMSO-*d*₆) δ 11.72 (br, 1H), 8.63 (t, *J* = 5.8 Hz, 1H), 8.14 (d, *J* = 2.8 Hz, 1H), 8.10 (s, 1H), 8.03 (s, 1H), 7.61 (s, 1H), 7.39-7.30 (m, 4H), 7.14 (s, 1H), 4.70 (t, *J* = 5.6 Hz, 1H), 4.56 (d, *J=* 5.8 Hz, 2H), 3.58 (d, *J* = 6.0 Hz, 2H), 0.91-0.84 (m, 2H), 0.82-0.75 (m, 2H).

### Example 17: Synthesis of compound 17

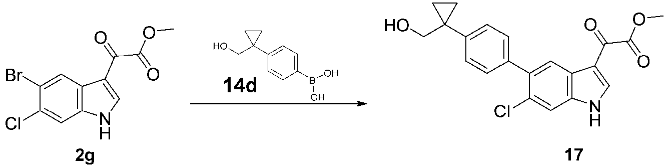

Step 1: Under nitrogen atmosphere, intermediate 2g (200 mg, 0.63 mmol), intermediate 14d (133.46 mg, 0.70 mmol), [1,1'-bis(diphenylphosphino)ferrocene]palladium dichloride-dichloromethane complex (77.39 mg, 0.15 mmol), potassium carbonate (218.3 mg, 1.58 mmol) and N,N-dimethylformamide (4 mL) were added to a 8 mL microwave tube. The mixture was reacted at 110°C under microwave conditions for three hours until completion. The reaction solution was concentrated to afford a crude product, and the crude product was separated and purified by high performance liquid chromatography (mobile phase: A: 0.1% FA/H2O B: ACN, chromatographic column: Waters-CORTECS-C18-2.7 µm-4.6*30 mm) to afford compound 17. LC-MS: m/z: 384.0 (M+H)⁺, ¹H NMR (400 MHz, DMSO-*d*₆) δ 12.57 (s, 1H), 8.53 (s, 1H), 8.08 (s, 1H), 7.72 (s, 1H), 7.37 (q, *J=* 8.2 Hz, 4H), 4.72 (s, 1H), 3.89 (s, 3H), 3.59 (s, 2H), 0.88 (q, *J=* 4.1 Hz, 2H), 0.80 (q, *J* = 4.0 Hz, 2H).

### Example 18: Synthesis of compound 18

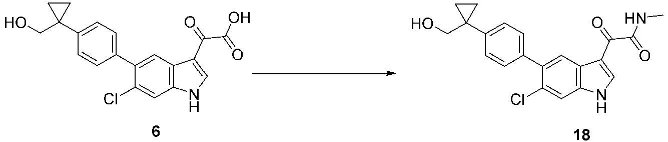

Step 1: Compound 6 (50 mg, 0.14 mmol), methylamine hydrochloride (13.69 mg, 0.20 mmol), HATU (77.12 mg, 0.2 mmol), N,N-diisopropylethylamine (52.43 mg, 0.41 mmol) and N,N-dimethylformamide (2 mL) were added to a 10 mL roundbottom flask. The mixture was reacted at room temperature for five hours until completion. The reaction solution was concentrated to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound 18. LC-MS: m/z: 383.1 (M+H)⁺, ¹H NMR (400 MHz,DMSO-d6) δ 12.31 (s, 1H), 8.84 (s, 1H), 8.67 (d, *J* = 4.8 Hz, 1H), 8.15 (s, 1H), 7.71 (s, 1H), 7.40-7.33 (m, 4H), 4.73 (t, *J* = 6.0 Hz, 1H), 3.58 (d, *J=* 5.6 Hz, 2H), 2.74 (d, *J=* 4.8 Hz, 3H), 0.90-0.79 (m, 4H).

### Example 19: Synthesis of compound 19

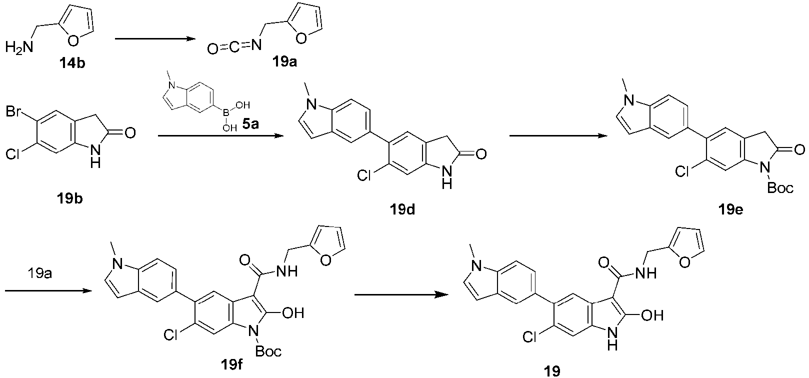

Step 1: Under nitrogen atmosphere, intermediate 14b (2 g, 20.59 mmol) and dichloromethane (40 mL) were added to a 250 mL three-necked flask. The mixture was cooled to 0°C, and a solution of triphosgene (6.11 g, 20.59 mmol) in dichloromethane (40 mL) was slowly added to the three-necked flask at around 0°C. Subsequently, a solution of triethylamine (4.38 g, 43.25 mmol) in dichloromethane (20 mL) was slowly added dropwise to the three-necked flask at around 0°C. The mixture was slowly warmed to 25°C and reacted for 15 hours until completion. The reaction solution was filtered, the filtrate was concentrated under reduced pressure to afford a crude product, and the crude product was separated and purified by silica gel column chromatography to afford intermediate 19a. ¹H NMR (400 MHz, CDCl₃) δ 7.37 (dd, *J* = 5.7, 0.9 Hz, 1H), 6.32 (dd, *J* = 3.2, 1.9 Hz, 1H), 6.26 (d, *J=* 3.2 Hz, 1H), 5.22 (s, 2H).

Step 2: Under nitrogen atmosphere, intermediate 19b (9 g, 36.5 mmol), intermediate 5a (9.86 g, 38.3 mmol), [1,1'-bis(diphenylphosphino)ferrocene]palladium dichloride-dichloromethane complex (2.98 g, 3.65 mmol), potassium carbonate (12.62 g, 91.28 mmol), dioxane (135 mL) and water (27 mL) were added to a 250 mL single-necked flask. The mixture was heated to 100°Cand reacted for 15 hours until completion. After the reaction was completed, the reaction solution was cooled and poured into water (1000 mL), and the mixture was extracted with dichloromethane (100 mL x 3). The organic phase was dried over anhydrous sodium sulphate, and filtered to afford the filtrate. The filtrate was concentrated under reduced pressure to afford a crude product, and the crude product was slurried with dichloromethane and methanol to afford intermediate 19d. ¹H NMR (400 MHz, DMSO-*d6*) δ 10.51 (s, 1H), 7.51 (d, *J* = 1.9 Hz, 1H), 7.46 (d, *J* = 8.5 Hz, 1H), 7.36 (d, *J* = 3.1 Hz, 1H), 7.24 (s, 1H), 7.14 (dd, *J* = 8.4, 1.7 Hz, 1H), 6.92 (s, 1H), 6.45 (d, *J=* 3.1 Hz, 1H), 3.81 (s, 3H), 3.52 (s, 2H).

Step 3: Under nitrogen atmosphere, intermediate 19d (7 g, 23.59 mmol), di-tert-butyl dicarbonate (7.72 g, 35.38 mmol), sodium carbonate (6.25 g, 58.97 mmol) and tetrahydrofuran (140 mL) were added to a 250 mL single-necked flask. The mixture was heated to 70°C and reacted for 8 hours until completion. After the reaction was completed, the reaction solution was cooled and poured into water (800 mL), and the mixture was extracted with dichloromethane (80 mL x 3). The organic phase was dried over anhydrous sodium sulphate, and filtered to afford the filtrate. The filtrate was concentrated under reduced pressure to afford a crude product, and the crude product was separated and purified by silica gel column chromatography to afford intermediate 19e. LC-MS: m/z: 397.2 (M+H)⁺. ¹H NMR (400 MHz, DMSO-d6) δ 7.84 (s, 1H), 7.55 (d, *J* = 1.7 Hz, 1H), 7.50 (d, *J* = 8.4 Hz, 1H), 7.41-7.32 (m, 2H), 7.17 (m, 1H), 6.47 (m, 1H), 3.87-3.71 (m, 5H), 1.58 (s, 9H).

Step 4: Under nitrogen atmosphere, intermediate 19e (1 g, 2.52 mmol) and N,N-dimethylformamide (15 mL) were added to a 50 mL three-necked flask. The mixture was cooled to 0°C, and sodium hydride (0.3 g, 7.56 mmol) was added in portions. The reaction solution was warmed to 25°C and stirred for 0.5 h. After cooling to 0°C, a solution of intermediate 19a (0.62 g, 5.04 mmol) in tetrahydrofuran (5 mL) was slowly added dropwise. The reaction solution was warmed to 25°C and stirred for 15 h until completion. The reaction solution was poured into ice-cold saturated ammonium chloride solution (100 mL), and the mixture was extracted with dichloromethane (80 mL x 3). The organic phase was dried over anhydrous sodium sulphate, and filtered to afford the filtrate. Subsequently, the filtrate was concentrated under reduced pressure to afford a crude product, and the crude product was separated and purified by silica gel column chromatography to afford intermediate 19f. LC-MS: m/z: 420.2 (M-100+H)⁺.

Step 5: Intermediate 19f (40 mg, 0.08 mmol), tetrahydrofuran (0.5 mL) and methanol (0.1 mL) were added to an 8 mL flat-bottom reaction flask, and then a solution of lithium hydroxide monohydrate (12.91 mg, 0.31 mmol) in water (0.2 mL) was slowly added. The reaction solution was warmed to 50°C and reacted for 5 hours until completion. After the reaction was completed, the reaction solution was cooled and adjusted to about pH = 7 with 0.5 M hydrochloric acid. The crude product was separated and purified by high performance liquid chromatography (mobile phase: A: 0.1% FA/H2O B: ACN, chromatographic column: Waters-CORTECS-C18-2.7 µm-4.6*30 mm) to afford compound 19. LC-MS: m/z: 420.2 (M+H)⁺. ¹H NMR (400 MHz, DMSO-*d6*) δ 10.70 (s, 1H), 8.82 (t, *J* = 5.7 Hz, 1H), 7.55 (d, *J=* 1.9 Hz, 1H), 7.49 (dd, *J* = 5.1, 3.3 Hz, 2H), 7.37 (d, *J* = 3.1 Hz, 1H), 7.20 (s, 1H), 7.13 (dd, *J* = 8.5, 1.6 Hz, 1H), 6.96 (s, 1H), 6.47 (d, *J* = 3.1 Hz, 1H), 6.39 (t, *J* = 2.6 Hz, 1H), 6.27 (d, *J* = 3.2 Hz, 1H), 4.49 (s, 1H), 4.42-4.25 (m, 2H), 3.82 (s, 3H).

### Example 20: Synthesis of compound 20

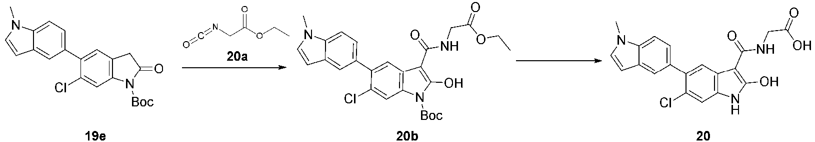

Step 1: Under nitrogen atmosphere, intermediate 19e (500 mg, 1.26 mmol) and N,N-dimethylformamide (15 mL) were added to a 50 mL three-necked flask. The mixture was cooled to 0°C, and sodium hydride (75.59 mg, 1.89 mmol) was added in portions. The reaction solution was warmed to 25°C and stirred for 0.5 h. After cooling to 0°C, a solution of intermediate 20a (244.01 mg, 1.89 mmol) in tetrahydrofuran (0.5 mL) was slowly added dropwise. The reaction solution was warmed to 25°C and stirred for 15 h until completion. The reaction solution was poured into ice-cold saturated ammonium chloride solution (50 mL), and the mixture was extracted with dichloromethane (15 mL x 3) for phase separation. The organic phase was dried over anhydrous sodium sulphate, and filtered to afford the filtrate. Subsequently, the filtrate was concentrated under reduced pressure to afford a crude product, and the crude product was separated and purified by silica gel column chromatography to afford intermediate 20b. ¹H NMR (400 MHz, DMSO-*d6*) δ 8.58 (s, 1H), 7.77 (s, 1H), 7.67 (d, *J* = 6.4 Hz, 1H), 7.52 (s, 1H), 7.45 (t, *J* = 6.9 Hz, 1H), 7.38-7.30 (m, 1H), 7.18 *(d, J=* 8.5 Hz, 1H), 6.45 (s, 1H), 4.21-3.88 (m, 4H), 3.82 (s, 3H), 1.59 (d, *J* = 9.9 Hz, 9H), 1.19 (dt, *J=* 14.1, 7.3 Hz, 3H).

Step 2: Intermediate 20b (50 mg, 0.1 mmol), tetrahydrofuran (0.5 mL) and methanol (0.1 mL) were added to an 8 mL flat-bottom reaction flask, and then a solution of lithium hydroxide monohydrate (16.78 mg, 0.4 mmol) in water (0.2 mL) was slowly added. The reaction solution was warmed to 50°C and reacted for 15 hours until completion. After the reaction was completed, the reaction solution was cooled and adjusted to about pH = 7 with 0.5 M hydrochloric acid. The crude product was separated and purified by high performance liquid chromatography (mobile phase: A: 0.1% FA/H2O B: ACN, chromatographic column: Waters-CORTECS-C18-2.7 µm-4.6*30 mm) to afford compound 20. LC-MS: m/z: 398.2 (M+H)⁺. ¹H NMR (400 MHz, DMSO-*d6*) δ 13.19 (s, 1H), 9.47 (s, 1H), 8.65 (s, 1H), 7.50 (s, 2H), 7.42 (d, *J=* 8.6 Hz, 1H), 7.32 (d, *J=* 3.0 Hz, 1H), 7.18 (d, *J=* 8.5 Hz, 1H), 6.75 (s, 1H), 6.43 (d, *J=* 3.0 Hz, 1H), 3.88 (s, 2H), 3.81 (s, 3H).

### Example 21: Synthesis of compound 21

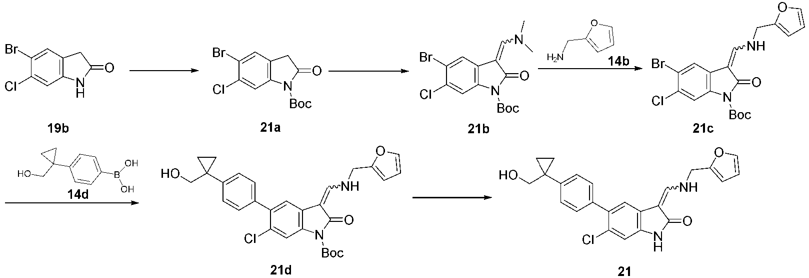

Step 1: At room temperature, intermediate 19b (5 g, 20.28 mmol), di-tert-butyl dicarbonate (11.07 g, 50.70 mmol), sodium bicarbonate (5.96 g, 70.98 mmol) and tetrahydrofuran (200 mL) were added to a 500 mL single-necked flask. The mixture was reacted at 100°C for 5 hours until completion. Water (500 mL) was then added, and the mixture was extracted with ethyl acetate (50 mL x 3). The organic phase was concentrated under reduced pressure to afford a crude product, and the crude product was slurried with petroleum ether/dichloromethane (V : V = 10 : 1), to afford intermediate 21a. LC-MS: m/z: 368.0 (M + Na)⁺. ¹H NMR (400 MHz, DMSO-*d6*) δ 7.87 (s, 1H), 7.72 (d, *J* = 1.2 Hz, 1H), 3.74 (d, *J* = 1.2Hz, 2H), 1.56 (s, 9H).

Step 2: At room temperature, intermediate 21a (1000 mg, 2.89 mmol), chloroform (10 mL) and N,N-dimethylformamide dimethyl acetal (413 mg, 3.46 mmol) were sequentially added to a 50 mL reaction flask. After purging with nitrogen 3 times, the mixture was stirred and reacted at 70°C for 16 hours. After the reaction was completed, the reaction solution was concentrated under reduced pressure, and the resulting residue was separated and purified by silica gel column chromatography to afford intermediate 21b. LC-MS: m/z: 345.0 (M + H -56)⁺. ¹H NMR (400 MHz, DMSO-*d*₆) δ 7.91 (s, 1H), 7.77 (s, 1H), 7.76 (s, 1H), 3.54 (s, 3H), 3.32 (s, 3H), 1.55 (s, 9H).

Step 3: At room temperature, intermediate 21b (630 mg, 1.57 mmol), ethanol (10 mL) and intermediate 14b (183 mg, 1.88 mmol) were sequentially added to a 25 mL reaction flask. After purging with nitrogen 3 times, the mixture was stirred and reacted at room temperature for 16 hours. After the reaction was completed, the reaction solution was filtered, and the resulting filter cake was purified by slurrying with petroleum ether/ethyl acetate to afford intermediate 21c. LC-MS: m/z: 397.0 (M+ H -56)⁺. ¹H NMR (400 MHz, DMSO-*d*₆) δ 9.53-9.31 (m, 1H), 8.33 *(d, J=* 13.9 Hz, 1H), 7.92-7.77 (m, 2H), 7.75-7.60 (m, 1H), 6.50-6.33 (m, 2H), 4.58 (d, *J* = 6.0 Hz, 2H), 1.56 (s, 9H).

Step 4: At room temperature, intermediate 21c (150 mg, 0.33 mmol), intermediate 14d (109 mg, 0.40 mmol), [1,1'-bis(diphenylphosphino)ferrocene]palladium dichloride-dichloromethane complex (27 mg, 0.03 mmol), potassium carbonate (137 mg, 0.99 mmol), dioxane (3 mL) and water (0.60 mL) were added to a 10 mL microwave tube. The mixture was subjected to a microwave reaction at 100°C under nitrogen atmosphere for two hours. After the reaction was completed, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford intermediate 21d. LC-MS: m/z: 465.1 (M + H-56)⁺. ¹H NMR (400 MHz, DMSO-*d*₆) δ 9.40-8.55 (m, 1H), 8.30-7.77 (m, 2H), 7.66-7.47 (m, 2H), 7.42-7.27 (m, 4H), 6.47-6.41 (m, 1H), 6.41-6.36 (m, 1H), 4.75-4.67 (m, 1H), 4.62-4.50 (m, 2H), 3.58 (d, *J* = 5.2 Hz, 2H), 1.58 (s, 9H), 0.91-0.81 (m, 2H), 0.81-0.73 (m, 2H).

Step 5: At room temperature, intermediate 21d (150 mg, 0.29 mmol), methanol (3 mL) and hydrogen chloride methanol solution (1 mL, 4 M) were added to a 10 mL reaction flask. The mixture was stirred and reacted at room temperature for 16 hours. After the reaction was completed, the reaction solution was filtered, and the filtrate was separated and purified by high performance liquid chromatography (mobile phase: A: 0.1% FA/H2O B: ACN, chromatographic column: Waters-CORTECS-C18-2.7 µm-4.6*30 mm) to afford compound 21. LC-MS: m/z: 421.2 (M+H)⁺. ¹H NMR (400 MHz, DMSO-*d*₆) δ 10.36 (s, 1H), 9.02-8.93 (m, 1H), 8.06 (d, *J* = 13.3 Hz, 1H), 7.65 (d, *J* = 12.8 Hz, 1H), 7.38-7.28 (m, 4H), 6.86 (d, *J* = 14.7 Hz, 1H), 6.47-6.34 (m, 2H), 4.69 (q, *J* = 5.4 Hz, 1H), 4.52 (t, *J* = 5.4 Hz, 2H), 3.57 (t, *J=* 5.0 Hz, 2H), 0.92-0.70 (m, 4H).

### Example 22: Synthesis of compound 22

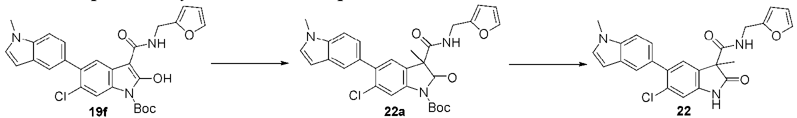

Step 1: Under nitrogen atmosphere, intermediate 19f (80 mg, 0.15 mmol), potassium carbonate (63.79 mg, 0.46 mmol), iodomethane (43.68 mg, 0.31 mmol) and N,N-dimethylformamide (2 mL) were added to an 8 mL flat-bottom flask. The reaction mixture was heated to 50°C and reacted for 4 hours until completion. After the reaction was completed, water (20 mL) was added, and the mixture was extracted with dichloromethane (2 mL x 3) for phase separation. The organic phase was dried over anhydrous sodium sulphate, and filtered to afford the filtrate. Subsequently, the filtrate was concentrated under reduced pressure to afford intermediate 22a. LC-MS: m/z: 434.2 (M-100+H)⁺.

Step 2: Intermediate 22a (70 mg, 0.13 mmol)|, trifluoroacetic acid (0.5 mL) and dichloromethane (5 mL) were added to an 8 mL flat-bottom reaction flask. The mixture was reacted at 25°C for 4 hours until completion. After the reaction was completed, saturated aqueous sodium bicarbonate solution (10 mL) was added, and the mixture was extracted with dichloromethane (3 mL x 3) for phase separation. The organic phase was dried over anhydrous sodium sulphate, and filtered to afford the filtrate. Subsequently, the filtrate was concentrated under reduced pressure to afford a crude product. The crude product was separated and purified by high performance liquid chromatography (mobile phase: A: 0.1% FA/H2O B: ACN, chromatographic column: Waters-CORTECS-C18-2.7 µm-4.6*30 mm) to afford compound 22. LC-MS: m/z: 434.2 (M+H)⁺. ¹H NMR (400 MHz, DMSO-*d6*) δ 10.75 (s, 1H), 8.46 (s, 1H), 8.16 (t, J = 5.8 Hz, 1H), 7.51 (d, J = 9.9 Hz, 1H), 7.49 (d, J = 7.8 Hz, 1H), 7.37 (d, J = 3.1 Hz, 1H), 7.33 (s, 1H), 7.15 (dd, *J=* 8.3, 1.8 Hz, 1H), 6.99 (s, 1H), 6.47 (d, *J=* 3.0 Hz, 1H), 6.37 (dd, *J* = 3.2, 1.9 Hz, 1H), 6.12 (d, *J=* 3.2 Hz, 1H), 4.25 (d, *J* = 5.8 Hz, 2H), 3.82 (s, 3H), 1.56 (s, 3H).

### Example 23: Synthesis of compound 23

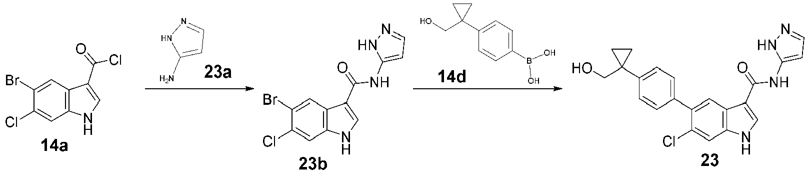

Step 1: At room temperature, intermediate 14a (160 mg, 0.55 mmol) dissolved in dry dichloromethane (20.0 mL) was added to a 50 mL single-necked flask. After cooling to 0°C in an ice bath, intermediate 23a (45.41 mg, 0.55 mmol) and *N,N-*diisopropylethylamine (0.36 mL, 2.19 mmol) were sequentially added. The mixture was naturally warmed to room temperature and reacted for 2 hours until completion. After the reaction was completed, the reaction solution was concentrated, and the crude product was separated and purified by silica gel column chromatography to afford intermediate 23b. LC-MS: m/z: 339.0 (M+H)⁺.

Step 2: At room temperature, intermediate 23b (60 mg, 0.18 mmol), intermediate 14d (50.89 mg, 0.27 mmol), [1,1'-bis(diphenylphosphino)ferrocene]palladium dichloride (25.86 mg, 0.04 mmol), potassium carbonate (48.84 mg, 0.35 mmol), dioxane (7.0 mL), and water (1.2 mL) were added to a 50 mL single-necked flask. The flask was evacuated and backfilled with nitrogen, and the mixture was reacted at 90°C under nitrogen atmosphere for 2 hours until completion. After the reaction was completed, the reaction solution was concentrated to afford a crude product, and the crude product was separated and purified by silica gel column chromatography to afford crude compound 23, which was then separated and purified by high performance liquid chromatography (mobile phase: A: H₂O B: ACN, chromatographic column: Waters-SunFire-C₁₈-10 µm-19*250 mm) to afford compound 23. LC-MS: m/z: 407.20 (M+H)⁺, ¹H NMR (400 MHz, DMSO-*d*₆) δ 12.30 (s, 1H), 11.79 (s, 1H), 10.39 (s, 1H), 8.44 (s, 1H), 8.19 (s, 1H), 7.67-7.54 (m, 2H), 7.41-7.32 (m, 4H), 6.62 (s, 1H), 4.71 (t, *J=* 5.6 Hz, 1H), 3.59 (d, *J* = 5.6 Hz, 2H), 0.91-0.85 (m, 2H), 0.83-0.76 (m, 2H).

### Example 24: Synthesis of compound 24

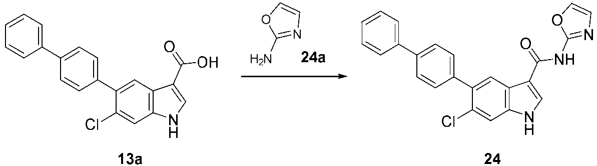

Step 1: At room temperature, intermediate 13a (100 mg, 0.35 mmol) and *N,N-*dimethylformamide (0.02 mL, 0.29 mmol) dissolved in super-dry dichloromethane (5 mL) were added to a 50 mL single-necked flask, and oxalyl chloride (0.07 mL, 0.86 mmol) was slowly added in an ice bath. The mixture was naturally warmed to room temperature and reacted for 2 hours. After the reaction was completed, the reaction solution was concentrated to afford a residue, which was directly used in the next reaction. At room temperature, the above residue was dissolved in super-dry dichloromethane (4.0 mL), and intermediate 24a (24.17 mg, 0.29 mmol) and triethylamine (0.08 mL, 0.58 mmol) were sequentially added in an ice bath. The final reaction solution was reacted at room temperature for 2 hours. After the reaction was completed, the reaction solution was concentrated, and the crude product was separated and purified by high performance liquid chromatography (mobile phase: A: 0.05% TFA/H₂O B: ACN, chromatographic column: Waters-SunFire-C₁₈-10 µm-19*250 mm) to finally afford compound 24. LC-MS: m/z: 414.2 (M+H)⁺, ¹H NMR (400 MHz, DMSO-*d*₆) δ 12.04 (s, 1H), 11.13 (s, 1H), 8.43 (d, *J=* 3.0 Hz, 1H), 8.19 (s, 1H), 7.92 (s, 1H), 7.81-7.69 (m, 5H), 7.60-7.46 (m, 4H), 7.39 (t, *J* = 7.4 Hz, 1H), 7.15 (s, 1H).

### Example 25: Synthesis of compound 25

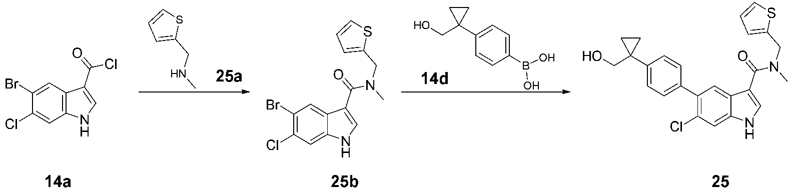

Step 1: At room temperature, intermediate 14a (160 mg, 0.55 mmol) dissolved in super-dry tetrahydrofuran (20.0 mL) was added to a 50 mL single-necked flask. After cooling to 0°C in an ice bath, intermediate 25a (139.02 mg, 1.09 mmol) and N,N-diisopropylethylamine (0.36 mL, 2.19 mmol) were sequentially added. The mixture was naturally warmed to room temperature and reacted for 2 hours until completion. After the reaction was completed, the reaction solution was concentrated, and the crude product was separated and purified by silica gel column chromatography to afford intermediate 25b. LC-MS: m/z: 383.00 (M+H)⁺.

Step 2: At room temperature, intermediate 25b (100 mg, 0.26 mmol), intermediate 14d (75.07 mg, 0.39 mmol), [1,1'-bis(diphenylphosphino)ferrocene]palladium dichloride (38.14 mg, 0.05 mmol), potassium carbonate (72.04 mg, 0.52 mmol), dioxane (7.0 mL), and water (1.2 mL) were added to a 50 mL single-necked flask. The flask was evacuated and backfilled with nitrogen, and the mixture was reacted at 90°C under nitrogen atmosphere for 2 hours until completion. After the reaction was completed, the reaction solution was concentrated, and the crude product was separated and purified by silica gel column chromatography to afford crude compound 25, which was then separated and purified by high performance liquid chromatography (mobile phase: A: H₂O B: ACN, chromatographic column: Waters-SunFire-C₁₈-10 µm-19*250 mm) to finally afford compound 25. LC-MS: m/z: 451.20 (M+H)⁺. ¹H NMR (400 MHz, DMSO-*d*₆) δ 11.75 (d, *J=* 2.0 Hz, 1H), 7.85 (d, *J* = 2.4 Hz, 1H), 7.80 (s, 1H), 7.60 (s, 1H), 7.43 (dd, *J=* 5.1, 1.1 Hz, 1H), 7.39-7.35 (m, 2H), 7.35 - 7.30 (m, 2H), 7.07 (d, *J* = 2.9 Hz, 1H), 6.98 (dd, *J* = 4.8, 1.2 Hz, 1H), 4.83 (s, 2H), 4.60 (s, 1H), 3.59 (s, 2H), 3.10 (s, 3H), 0.91-0.84 (m, 2H), 0.84-0.76 (m, 2H).

### Example 26: Synthesis of compound 26

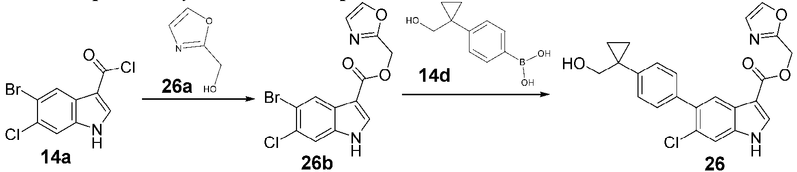

Step 1: At room temperature, intermediate 14a (160 mg, 0.55 mmol) dissolved in super-dry tetrahydrofuran (20.0 mL) was added to a 50 mL single-necked flask. After cooling to 0°C in an ice bath, intermediate 26a (59.95 mg, 0.61 mmol), *N,N-*diisopropylethylamine (0.46 mL, 2.75 mmol), and 4-dimethylaminopyridine (13.44 mg, 0.11 mmol) were sequentially added. The mixture was naturally warmed to room temperature and reacted for 2 hours until completion. After the reaction was completed, the reaction solution was concentrated, and the crude product was separated and purified by silica gel column chromatography to afford intermediate 26b. LC-MS: m/z: 355.00 (M+H)⁺.

Step 2: At room temperature, intermediate 26b (67 mg, 0.19 mmol), intermediate 14d (54.27 mg, 0.28 mmol), [1,1'-bis(diphenylphosphino)ferrocene]palladium dichloride (27.58 mg, 0.04 mmol), potassium carbonate (52.08 mg, 0.38 mmol), dioxane (8.0 mL), and water (1.6 mL) were added to a 50 mL single-necked flask. The flask was evacuated and backfilled with nitrogen, and the mixture was reacted at 90°C under nitrogen atmosphere for 2 hours until completion. After the reaction was completed, the reaction solution was concentrated, and the crude product was separated and purified by silica gel column chromatography to afford crude compound 26, which was then separated and purified by high performance liquid chromatography (mobile phase: A: H₂O B: ACN, chromatographic column: Waters-SunFire-C₁₈-10 µm-19*250 mm) to finally afford compound 26. LC-MS: m/z: 423.20 (M+H)⁺, ¹H NMR (400 MHz, DMSO-*d*₆) δ 12.18 *(d, J=* 2.7 Hz, 1H), 8.23 (d, *J* = 3.0 Hz, 1H), 8.13 (s, 1H), 7.92 (s, 1H), 7.66 (s, 1H), 7.41 - 7.36 (m, 2H), 7.35 - 7.31 (m, 2H), 7.24 (s, 1H), 5.40 (s, 2H), 3.59 (s, 2H), 0.90 - 0.85 (m, 2H), 0.83 - 0.77 (m, 2H).

### Example 27: Synthesis of compound 27

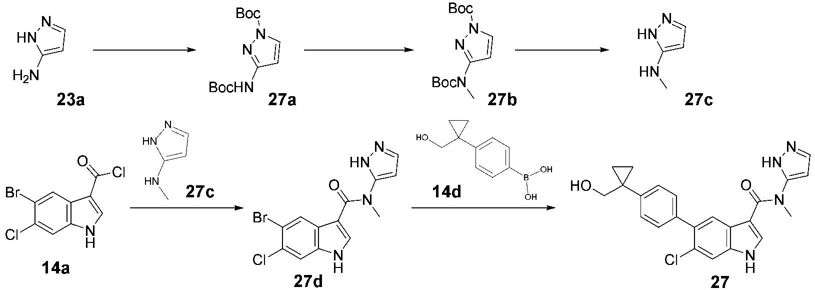

Step 1: At room temperature, intermediate 23a (5 g, 60.18 mmol) dissolved in super-dry tetrahydrofuran (100.0 mL) was added to a 250 mL single-necked flask. After cooling to 0°C in an ice bath, di-tert-butyl dicarbonate (28.33 mL, 132.40 mmol) and 4-dimethylaminopyridine (2.21 g, 18.05 mmol) were sequentially added. The mixture was naturally warmed to room temperature and reacted for 3 hours until completion. After the reaction was completed, the reaction solution was poured into water (500 mL). The mixture was extracted with ethyl acetate (50 mL x 3), dried over anhydrous sodium sulphate, suction-filtered, and concentrated to afford a crude product, and the crude product was separated and purified by silica gel column chromatography to afford intermediate 27a. LC-MS: m/z: 306.2 (M + Na)⁺.

Step 2: At 0°C, super-dry *N,N*-dimethylformamide (10.0 mL), intermediate 27a (1 g, 3.53 mmol) and sodium hydride (0.14 g, 3.53 mmol, 60%) were added to a 50 mL single-necked flask, followed by iodomethane (0.22 mL, 3.53 mmol). The mixture was naturally warmed to room temperature and reacted for 1 hour until completion. After the reaction was completed, the reaction solution was poured into water (100 mL), and the mixture was extracted with ethyl acetate (5 mL x 3). The organic phase was dried over anhydrous sodium sulphate, filtered, and concentrated to afford a crude product, and the crude product was separated and purified by silica gel column chromatography to afford intermediate 27b. LC-MS: m/z: 320.2 (M + Na)⁺. ¹H NMR (400 MHz, DMSO-*d*₆) δ 8.14 (d, *J=* 2.9 Hz, 1H), 6.69 (s, 1H), 3.27 (s, 3H), 1.56 (s, 9H), 1.49 (s, 9H).

Step 3: At room temperature, intermediate 27b (800 mg, 2.69 mmol) dissolved in super-dry dichloromethane (7.0 mL) was added to a 50 mL single-necked flask, followed by trifluoroacetic acid (2 mL). The mixture was reacted for 1 hour until completion. After the reaction was completed, the reaction solution was concentrated to afford a crude product, and the crude product was separated and purified by silica gel column chromatography to afford intermediate 27c. ¹H NMR (400 MHz, DMSO-*d*₆) δ 7.82 (d, *J=* 2.7 Hz, 1H), 5.77 (d, *J=* 2.7 Hz, 1H), 2.77 (s, 3H).

Step 4: At room temperature, intermediate 14a (340 mg, 1.16 mmol) dissolved in tetrahydrofuran (20.0 mL) was added to a 25 mL single-necked flask. After cooling to 0°C in an ice bath, intermediate 27c (169.08 mg, 1.74 mmol), N,N-diisopropylethylamine (0.96 mL, 5.80 mmol), and 4-dimethylaminopyridine (14.18 mg, 0.12 mmol) were sequentially added. The mixture was naturally warmed to room temperature and reacted for 2 hours until completion. After the reaction was completed, the reaction solution was concentrated to afford a crude product, and the crude product was separated and purified by silica gel column chromatography to afford intermediate 27d. LC-MS: m/z: 353.0 (M+H)⁺.

Step 5: At room temperature, intermediate 27d (100 mg, 0.28 mmol), intermediate 14d (80.65 mg, 0.42 mmol), [1,1'-bis(diphenylphosphino)ferrocene]palladium dichloride (40.98 mg, 0.06 mmol), potassium carbonate (78.17 mg, 0.57 mmol), dioxane (8.0 mL), and water (1.6 mL) were added to a 25 mL single-necked flask. The flask was evacuated and backfilled with nitrogen, and the mixture was reacted at 90°C under nitrogen atmosphere for 2 hours until completion. After the reaction was completed, the reaction solution was concentrated to afford a crude product, and the crude product was separated and purified by high performance liquid chromatography (mobile phase: A: 0.05% TFA/H₂O B: ACN, chromatographic column: Waters-SunFire-C₁₈-10 µm-19*250 mm) to finally afford compound 27. LC-MS: m/z: 421.2 (M+H)⁺. ¹H NMR (400 MHz, DMSO-*d*₆) δ 11.50 (s, 1H), 7.90 (s, 1H), 7.72 (d, *J=* 1.9 Hz, 1H), 7.52 (s, 1H), 7.39 - 7.28 (m, 4H), 6.72 (s, 1H), 6.13 (d, *J* = 2.2 Hz, 1H), 3.59 (s, 2H), 3.30 (s, 3H), 0.87 (d, *J=* 4.1 Hz, 2H), 0.82 - 0.78 (m, 2H).

### Example 28: Synthesis of compound 28

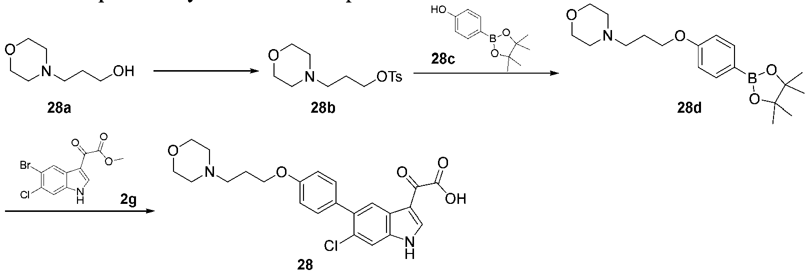

Step 1: At 0°C, intermediate 28a (1 g, 6.89 mmol), p-toluenesulphonyl chloride (1.445 g, 7.58 mmol) and dichloromethane (25 mL) were added to a 100 mL single-necked flask, and triethylamine (1.39 g, 13.78 mmol) was added under stirring. The mixture was reacted at room temperature for 16 hours until completion. The reaction solution was poured into ice water (200 ml), and the mixture was extracted with dichloromethane (20 ml x 3). The organic phase was dried over anhydrous sodium sulphate, filtered, and concentrated to afford intermediate 28b. LC-MS: m/z: 300.0 (M+H)⁺. ¹H NMR (400 MHz, DMSO-*d6*) δ 7.51-7.44 (m, 2H), 7.11 (d, J = 7.9 Hz, 2H), 4.28-4.23 (m, 2H), 3.77 (d, J = 4.7 Hz, 2H), 3.67 (t, J = 6.5 Hz, 1H), 3.58 (s, 1H), 3.51 (d, J = 5.0 Hz, 2H), 3.09 (q, J = 7.8 Hz, 2H), 2.47-2.37 (m, 2H), 2.29 (s, 3H), 1.18 (d, J = 7.3 Hz, 2H).

Step 2: At room temperature, intermediate 28b (161.67 mg, 0.54 mmol), intermediate 28c (100 mg, 0.45 mmol), potassium carbonate (186.57 mg, 1.35 mmol) and N,N-dimethylformamide (3 mL) were added to a 50 mL single-necked flask. The mixture was warmed to 70°C and reacted for five hours until completion. The reaction solution was poured into water (30 ml), and the mixture was extracted with ethyl acetate (5 ml). The organic phase was washed with saturated aqueous sodium chloride solution (10 mL x 3), dried over anhydrous sodium sulphate, filtered, and concentrated to afford a crude product, and the crude product was separated and purified by silica gel column chromatography to afford intermediate 28d. LC-MS: m/z: 348.2 (M+H)⁺. ¹H NMR (400 MHz, DMSO-*d6*) δ 7.63-7.55 (m, 2H), 6.95-6.88 (m, 2H), 4.03 (t, J = 6.4 Hz, 2H), 3.58-3.55 (m, 4H), 2.41-2.32 (m, 6H), 1.90-1.85 (m, 2H), 1.27 (s, 12H).

Step 3: Under nitrogen atmosphere, intermediate 28d (111 mg, 0.32 mmol), intermediate 2g (100 mg, 0.32 mmol), [1,1'-bis(diphenylphosphino)ferrocene]palladium dichloride-dichloromethane complex (26.10 mg, 0.03 mmol), potassium carbonate (110.44 mg, 0.80 mmol), dioxane (3 mL) and water (0.75 mL) were added to a 8 mL microwave tube. The mixture was reacted at 100°C under microwave conditions for two hours until completion. Subsequently, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 28. LC-MS: m/z: 442.9 (M+H)⁺. ¹H NMR (400 MHz, DMSO-*d6*) δ 12.29 (s, 1H), 8.42 (d, J = 2.9 Hz, 1H), 8.09 (s, 1H), 7.68 (s, 1H), 7.35 (d, J = 8.6 Hz, 2H), 7.05-6.98 (m, 2H), 4.08 (t, J = 6.2 Hz, 2H), 3.64 (t, J = 4.6 Hz, 4H), 2.61 (d, J = 20.9 Hz, 6H), 1.98 (q, J = 6.8 Hz, 2H).

### Example 29: Synthesis of compound 29

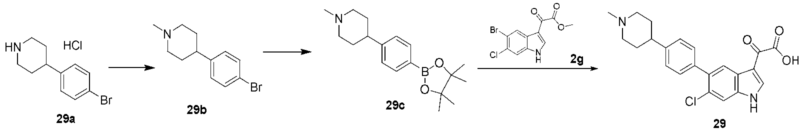

Step 1: At 25°C, intermediate 29a (1 g, 3.62 mmol), paraformaldehyde (1.47 g, 18.08 mmol), tetrahydrofuran (10 mL) and methanol (10 mL) were added to a 100 mL three-necked flask. Subsequently, sodium triacetoxyborohydride (1.53 g, 7.23 mmol) was added to the reaction system in three portions. The mixture was reacted for 15 hours until completion. The reaction solution was filtered, and the filtrate was concentrated under reduced pressure to afford intermediate 29b. LC-MS: m/z: 254.0 (M+H)⁺.

Step 2: Under nitrogen atmosphere, intermediate 29b (400 mg, 1.57 mmol), bis(pinacolato)diboron (479.6 mg, 1.89 mmol), [1,1'-bis(diphenylphosphino)ferrocene]palladium dichloride (115.15 mg, 0.16 mmol), potassium acetate (308.9 mg, 3.15 mmol) and dioxane (4 mL) were added to a 15 mL single-necked flask. The mixture was heated to 90°C and reacted for 15 hours until completion. After the reaction was completed, the reaction system was cooled and filtered, the filtrate was concentrated under reduced pressure to afford a crude product, and the crude product was separated and purified by silica gel column chromatography to afford intermediate 29c. ¹H NMR (400 MHz, CDCl₃) δ 7.75 (d, J = 8.1 Hz, 2H), 7.24 (d, J = 8.0 Hz, 2H), 3.04 (d, J = 11.2 Hz, 2H), 2.51 (td, J = 10.9, 5.0 Hz, 1H), 2.37 (s, 3H), 2.13 (td, J = 11.2, 3.8 Hz, 2H), 1.93 - 1.76 (m, 4H), 1.33 (s, 12H).

Step 3: Under nitrogen atmosphere, intermediate 29c (95.17 mg, 0.32 mmol), intermediate 2g (100 mg, 0.32 mmol), [1,1'-bis(diphenylphosphino)ferrocene]palladium dichloride (23.12 mg, 0.03 mmol), potassium carbonate (109.15 mg, 0.79 mmol), N,N-dimethylformamide (4 mL) and water (0.4 mL) were added to a 8 mL microwave tube. The mixture was heated to 100°C and reacted for 4 hours until completion. After the reaction was completed, the reaction system was cooled and filtered, and the filtrate was directly separated and purified by high performance liquid chromatography (mobile phase: A: 0.1% FA/H₂O B: ACN, chromatographic column: Waters-CORTECS-C18-2.7 µm-4.6*30 mm) to afford compound 29. LC-MS: m/z: 397.2 (M+H)⁺. ¹H NMR (400 MHz, DMSO-*d6*) δ 11.89 (s, 1H), 8.22 (s, 2H), 8.11 (s, 1H), 7.62 (s, 1H), 7.34 (d, J = 4.2 Hz, 4H), 2.94 (d, J =11.0 Hz, 2H), 2.26 (s, 3H), 2.08 (t, J = 11.4 Hz, 2H), 1.86-1.69 (m, 4H).

### Example 30: Synthesis of compound 30

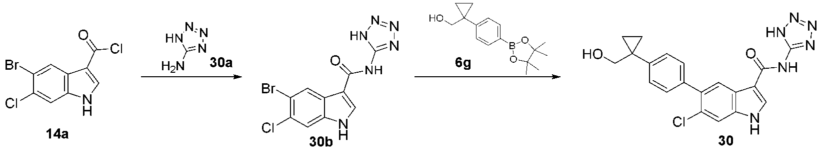

Step 1: Under nitrogen atmosphere, intermediate 14a (533.59 mg, 1.82 mmol), tetrahydrofuran (5 mL), triethylamine (1.01 mL, 7.29 mmol) and intermediate 30a (309.91 mg, 3.64 mmol) were added to a 100 mL three-necked flask. The mixture was reacted at room temperature for 15 hours, and then the above reaction solution was poured into water (100 mL). The mixture was extracted with dichloromethane : methanol (V : V = 10 : 1) (100 mL x 3). The organic phase was dried over anhydrous sodium sulphate, and filtered, the filtrate was subjected to rotary evaporation to dryness to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford intermediate 30b. LC-MS: m/z: 340.8(M+H)⁺.

Step 2: Under nitrogen atmosphere, intermediate 30b (117 mg, 0.34 mmol), potassium carbonate (118.35 mg, 0.86 mmol), dioxane (2 mL), water (0.4 mL), N,N-dimethylformamide (1 mL), [1,1'-bis(diphenylphosphino)ferrocene]palladium dichloride (25 mg, 0.03 mmol) and intermediate 6g (122.09 mg, 0.45 mmol) were added to a 100 mL three-necked flask. The mixture was reacted at 100°C for 5 hours. The reaction solution was added to water (100 mL), and the mixture was extracted with ethyl acetate (100 mL x 3). The organic phase was dried over anhydrous sodium sulphate, and filtered, the filtrate was subjected to rotary evaporation to dryness to afford a crude product, and the crude product was separated and purified by high performance liquid chromatography (mobile phase: A: 0.1% FA/H2O B: ACN, chromatographic column: Waters-CORTECS-C18-2.7 µm-4.6*30 mm) to afford compound 30. LC-MS: m/z: 409.0 (M+H)⁺. ¹H NMR (400 MHz, DMSO-*d*₆) δ 15.81 (s, 1H), 12.08 (s, 1H), 11.88 (s, 1H), 8.58 (s, 1H), 8.20 (s, 1H), 7.69 (s, 1H), 7.40-7.35 (m, 4H), 4.72 (t, *J* = 4.0 Hz, 1H), 3.55 (d, *J* = 8.0 Hz, 2H), 0.90-0.79 (m, 4H).

### Example 31: Synthesis of compound 31

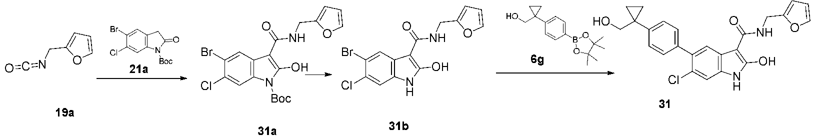

Step 1: Under nitrogen atmosphere, intermediate 21a (1.6 g, 4.62 mmol) and N,N-dimethylformamide (25 mL) were added to a 250 mL three-necked flask. The mixture was cooled to 0°C, and sodium hydride (0.28 g, 6.92 mmol) was added under stirring. The resulting mixture was stirred for 30 minutes, and then intermediate 19a (1.14 g, 9.23 mmol) was added. Subsequently, the mixture was warmed to room temperature and reacted for 16 hours until completion. The reaction solution was poured into ice water (30 ml), and the mixture was extracted with ethyl acetate (300 ml) three times. The organic phase was dried over anhydrous sodium sulphate, filtered, and concentrated under reduced pressure to afford a crude product. Subsequently, the solid was filtered, the filtrate was concentrated under reduced pressure to afford a crude product, and the crude product was separated and purified by silica gel column chromatography (dichloromethane/methanol system) to afford intermediate 31a. LC-MS: m/z: 413.0(M-55)⁺.

Step 2: At room temperature, intermediate 31a (620 mg, 1.32 mmol) and dichloromethane (4 mL) were added to a 50 mL single-necked flask, and trifluoroacetic acid (2 mL) was added under stirring. The mixture was stirred at room temperature for 2 hours until completion. The reaction solution was concentrated under reduced pressure to afford crude intermediate 31b, which was directly used in the next step. LC-MS: m/z: 369.0(M+1)⁺.

Step 3: Under nitrogen atmosphere, intermediate 31b (100 mg, 0.27 mmol), intermediate 6g (74.18 mg, 0.27 mmol), [1,1'-bis(diphenylphosphino)ferrocene]palladium dichloride-dichloromethane complex (22.1 mg, 0.03 mmol), potassium carbonate (93.48 mg, 0.68 mmol), dioxane (3 mL) and water (0.75 mL) were added to a 5 mL microwave tube. The mixture was reacted at 100°C for 2 hours until completion. Subsequently, the solid was filtered, and the filtrate was separated and purified by high performance liquid chromatography (mobile phase: A: 0.1% FA/H2O B: ACN, chromatographic column: Waters-CORTECS-C18-2.7 µm-4.6*30 mm) to afford compound 31. LC-MS: m/z: 437.2(M+1)⁺. ¹H-NMR (400 MHz, DMSO-*d*₆) δ 10.72 (s, 1H), 8.81 (t, *J=* 5.6 Hz, 1H), 7.56 (d, *J* = 1.8 Hz, 1H), 7.36 (d, *J* = 8.3 Hz, 2H), 7.26 (d, *J* = 8.2 Hz, 2H), 7.16 (s, 1H), 6.96 (s, 1H), 6.40 (dd, *J* = 3.2, 1.9 Hz, 1H), 6.27 (d, *J* = 3.2 Hz, 1H), 4.48 (d, *J=* 7.3 Hz, 1H), 4.32 (d, *J=* 5.6 Hz, 2H), 3.57 (s, 2H), 0.86 (t, *J* = 2.8 Hz, 2H), 0.78 (q, *J* = 4.0 Hz, 2H).

### Example 32: Synthesis of compound 32

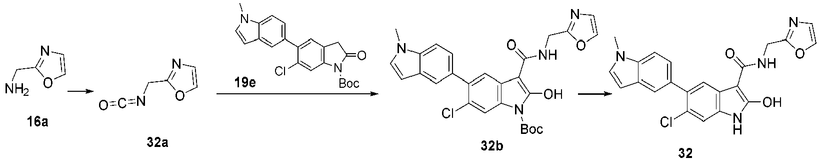

Step 1: Under nitrogen atmosphere at 0°C, intermediate 16a (100 mg, 1.02 mmol), toluene (5 mL), triethylamine (0.30 mL, 2.14 mmol) and triphosgene (151 mg, 0.51 mmol) were added to a 100 mL three-necked flask. The mixture was reacted for 1 hour, and then the above reaction solution was filtered, and the filtrate was concentrated under reduced pressure to afford crude intermediate 32a, which was directly used in the next reaction.

Step 2: At 0°C, intermediate 32a (200 mg, 0.50 mmol), N,N-dimethylformamide (4 mL) and sodium hydride (200.00 mg, 5.00 mmol, 60%) were added to a 100 mL three-necked flask. The mixture was stirred at 0°C for 0.5 hours, and intermediate 19e (125.08 mg, 1.01 mmol) was slowly added to the above reaction solution. The resulting mixture was reacted for 1 hour, and then the above reaction solution was poured into water (100 mL). The mixture was extracted with dichloromethane (100 mL x 3), and the organic phase was dried over anhydrous sodium sulphate, and filtered. The filtrate was concentrated under reduced pressure to afford a crude product, and the crude product was separated and purified by silica gel column chromatography to afford intermediate 32b. LC-MS: m/z: 421.2(M-100)⁺.

Step 3: Under nitrogen atmosphere, methanol (0.5 mL), tetrahydrofuran (0.5 mL), intermediate 32b (50 mg, 0.10 mmol) and lithium hydroxide (17 mg, 0.38 mmol) were added to a 100 mL three-necked flask. The mixture was reacted at 40°C for 1 hour, and the above reaction solution was adjusted to pH = 6 with dilute hydrochloric acid, and separated and purified by high performance liquid chromatography (mobile phase: A: 0.1% FA/H2O B: ACN, chromatographic column: Waters-CORTECS-C18-2.7 µm-4.6*30 mm) to afford compound 32. LC-MS: m/z: 421.2 (M+H)⁺. ¹H NMR (400 MHz, DMSO-*d*₆) δ 10.71 (s, 1H), 9.02 (s, 1H), 8.02-8.00 (m, 1H),7.51-7.49 (m, 2H), 7.43-7.37 (m, 1H), 7.32-7.29 (m, 1H), 7.19-7.11 (m, 2H), 6.96 (s, 1H),6.47-6.43 (m, 1H), 4.54-4.43 (m, 3H), 3.82 (s, 3H).

### Example 33: Synthesis of compound 33

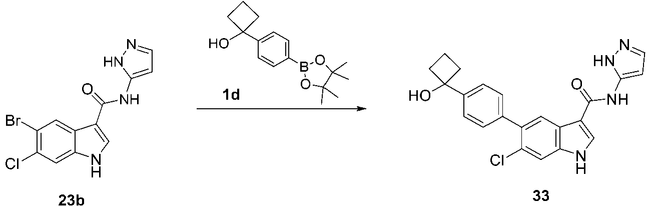

Step 1: Under nitrogen atmosphere, dioxane (4 mL), water (0.8 mL), intermediate 23b (70 mg, 0.21 mmol), potassium carbonate (71 mg, 0.52 mmol), [1,1'-bis(diphenylphosphino)ferrocene]palladium dichloride (15 mg, 0.02 mmol) and intermediate 1d (67.82 mg, 0.25 mmol) were added to a 100 mL three-necked flask. The mixture was reacted at 100°C for 2 hours, and the above reaction solution was poured into water (100 mL), and extracted with ethyl acetate (100 mL x 3). The organic phase was dried over anhydrous sodium sulphate, and filtered, and the filtrate was concentrated under reduced pressure to afford a crude product, which was separated and purified by high performance liquid chromatography (mobile phase: A: 0.1% FA/H2O B: ACN, chromatographic column: Waters-CORTECS-C18-2.7 µm-4.6*30 mm) to afford compound 33. LC-MS: m/z: 407.0 (M+H)⁺. ¹H NMR (400 MHz, DMSO-*d*₆) δ 12.30 (s, 1H), 11.82 (s, 1H), 10.41 (s, 1H), 8.45 (s, 1H), 8.21 (s, 1H), 7.62-7.55 (m, 4H), 7.43-7.40 (m, 2H), 6.61 (s, 1H), 5.53 (s, 1H), 2.46-2.42 (m, 2H), 2.34-2.31 (m, 2H), 2.29-1.93 (m, 1H),1.71-1.68 (m, 1H).

### Example 34: Synthesis of compound 34

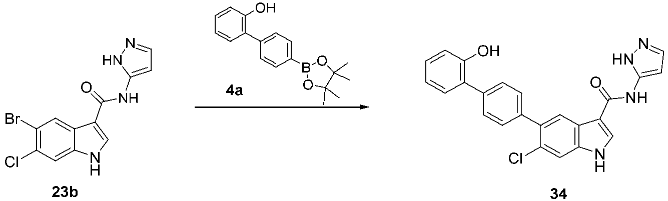

Step 1: Under nitrogen atmosphere, dioxane (4 mL), water (0.8 mL), intermediate 23b (70 mg, 0.21 mmol), potassium carbonate (71 mg, 0.52 mmol), [1,1'-bis(diphenylphosphino)ferrocene]palladium dichloride (15 mg, 0.02 mmol) and intermediate 4a (73.26 mg, 0.25 mmol) were added to a 100 mL three-necked flask. The mixture was reacted at 100°C for 2 hours, and the reaction solution was added to water (100 mL), and extracted with ethyl acetate (100 mL x 3). The organic phase was dried over anhydrous sodium sulphate, and filtered, and the filtrate was concentrated under reduced pressure to afford a crude product, which was separated and purified by high performance liquid chromatography (mobile phase: A: 0.1% FA/H2O B: ACN, chromatographic column: Waters-CORTECS-C18-2.7 µm-4.6*30 mm) to afford compound 34. LC-MS: m/z: 429.0 (M+H)⁺. ¹H NMR (400 MHz, DMSO-*d*₆) δ 12.30 (s, 1H), 11.82 (d, *J =* 4.0 Hz, 1H), 9.59 (s, 1H), 8.47 (d, *J* = 4.0 Hz, 1H), 8.26 (s, 1H), 7.66-7.60 (m, 4H), 7.49-7.46 (m, 2H), 7.35-7.33 (m, 1H), 7.20-7.16 (m, 1H), 6.99-6.91 (m, 1H), 6.90-6.89 (m, 1H), 6.61 (s, 1H),1.71-1.68 (m, 1H).

### Example 35: Synthesis of compound 35

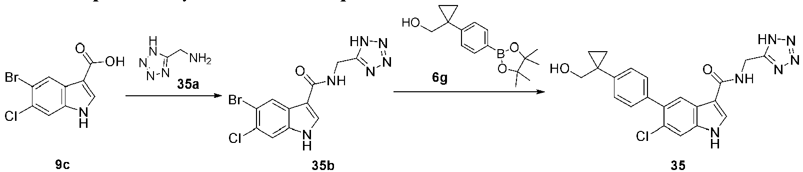

Step 1: Intermediate 9c (100 mg, 0.36 mmol), intermediate 35a (53.51 mg, 0.54 mmol), N,N-dimethylformamide (3 mL), triethylamine (147 mg, 1.46 mmol) and benzotriazole-N,N,N',N'-tetramethyluronium hexafluorophosphate (173 mg, 0.46 mmol) were added to a 100 mL three-necked flask. The mixture was reacted at 25°C for 4 hours, and the reaction solution was separated and purified by high performance liquid chromatography (mobile phase: A: 0.1% FA/H2O B: ACN, chromatographic column: Waters-CORTECS-C18-2.7 µm-4.6*30 mm) to afford intermediate 35b. LC-MS: m/z: 355.0(M+H)⁺.

Step 2: Under nitrogen atmosphere, dioxane (2 mL), water (0.4 mL), intermediate 35b (50 mg, 0.14 mmol), potassium carbonate (49 mg, 0.35 mmol), [1,1'-bis(diphenylphosphino)ferrocene]palladium dichloride (11 mg, 0.01 mmol) and intermediate 6g (50.12 mg, 0.18 mmol) were added to a 100 mL three-necked flask. The mixture was reacted at 100°C for 2 hours, and the above reaction solution was added to water (100 mL), and extracted with ethyl acetate (10 mL x 3). The organic phase was dried over anhydrous sodium sulphate, and filtered, the filtrate was concentrated under reduced pressure to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound 35. LC-MS: m/z: 421.0 (M-H)⁻. ¹H NMR (400 MHz, DMSO-*d*₆) δ 11.70 (s, 1H), 8.48 (s, 1H), 8.15 (s, 1H), 8.12 (s, 1H), 7.60 (s, 1H), 7.38-7.32 (m, 4H), 4.73-4.65 (m, 3H), 3.58 (d, J = 5.2 Hz, 2H), 0.87-0.79 (m, 4H).

### Example 36: Synthesis of compound 36

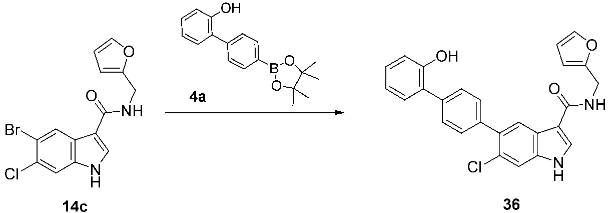

Step 1: Dioxane (2 mL), water (0.4 mL), intermediate 14c (66 mg, 0.19 mmol), potassium carbonate (64.49 mg, 0.47 mmol), [1,1'-bis(diphenylphosphino)ferrocene]palladium dichloride (27.31 mg, 0.04 mmol) and intermediate 4a (66 mg, 0.22 mmol) were added to a 100 mL three-necked flask. The mixture was reacted at 100°C for 2 hours, and the above reaction solution was added to water (100 mL), and extracted with ethyl acetate (10 mL x 3). The organic phase was dried over anhydrous sodium sulphate, and filtered, the filtrate was concentrated under reduced pressure to afford a crude product, and the crude product was separated and purified by high performance liquid chromatography (mobile phase: A: 0.1% FA/H2O B: ACN, chromatographic column: Waters-CORTECS-C18-2.7 µm-4.6*30 mm) to afford compound 36. LC-MS: m/z: 443.2 (M+H)⁺. ¹H NMR (400 MHz, DMSO-*d*₆) δ 11.72(s, 1H), 9.59 (s, 1H), 8.47 (t, *J=* 4.0 Hz, 1H), 8.19-8.14 (m, 2H), 7.64-7.63 (m, 3H), 7.62-7.57 (m, 1H), 7.47-7.45 (m, 2H), 7.35-7.32 (m, 1H), 7.20-7.16 (m, 1H), 6.98-6.88 (m, 2H), 6.40-6.38 (m, 1H), 6.27-6.26 (m, 1H), 4.45 (d, *J =* 4.0 Hz, 2H).

### Example 37: Synthesis of compound 37

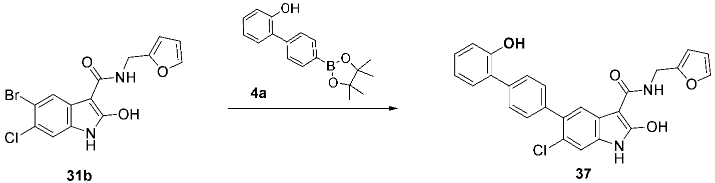

Step 1: Under nitrogen atmosphere at room temperature, dioxane (6 mL), water (1.2 mL), intermediate 31b (250 mg, 0.68 mmol), intermediate 4a (220.36 mg, 0.74 mmol), [1,1'-bis(diphenylphosphino)ferrocene]palladium dichloride-dichloromethane complex (55.37 mg, 0.07 mmol) and potassium carbonate (233.70 mg, 1.69 mmol) were added to a 5 mL microwave tube. The mixture was reacted at 100°C for 16 hours until completion. Subsequently, the reaction solution was filtered, and the filtrate was separated and purified by high performance liquid chromatography (mobile phase: A: 0.1% FA/H2O B: ACN, chromatographic column: Waters-CORTECS-C18-2.7 µm-4.6*30 mm) to afford compound 37. LC-MS: m/z: 459.2(M+1)⁺. 1H NMR (400 MHz, DMSO-d6) δ 10.75 (s, 1H), 9.58 (d, *J* = 9.1 Hz, 1H), 8.83 (t, *J=* 5.7 Hz, 1H), 7.63 (d, *J* = 8.2 Hz, 2H), 7.56 (d, *J=* 1.8 Hz, 1H), 7.47-7.36 (m, 2H), 7.32 (dd, *J* = 7.7, 1.7 Hz, 1H), 7.25-7.14 (m, 2H), 7.01-6.94 (m, 2H), 6.90 (t, *J=* 7.4 Hz, 1H), 6.41-6.39 (m, 1H), 6.28 (d, *J=* 3.1 Hz, 1H), 4.50 (d, *J=* 11.5 Hz, 1H), 4.33 (d, *J=* 5.6 Hz, 1H).

### Biological tests:

### Test example 1: In vitro assay for AMPK (α1β1γ1) enzyme agonist activity

Test compounds were dissolved in DMSO to prepare 10 mM stock solution, which was then diluted to the predetermined test concentrations using kinase buffer (50 mM HEPES, 1 mM EDTA, 10 mM MgCl₂, 0.01% Brij-35, pH = 7.5). Into a 384-well plate were added 5 µL of AMPKα1β1γ1 (CARNA, 02-113), 2.5 µL of the diluted test compound solution, and 2.5 µL of a mixture of Ulight-CRBN and ATP, resulting in the following final concentrations: AMPKα1β1γ1 at 1.6 mg/L, Ulight-CRBN at 5 nM, and ATP at 0.1 mM. The components were mixed evenly, and reacted at room temperature for 1 hour. Subsequently, 5 µL of LANCE Detection Buffer (CR97-100, PerkinElmer) containing 40 mM EDTA was added. After standing for 5 minutes, 5 µL of 4× Eu-anti-phospho-CREB Antibody (TRF0200-M, PerkinElmer) was added, and the mixture was allowed to stand at room temperature for 1 h. The values of samples in each well were read using a microplate reader in TR-FRET mode (excitation wavelength: 320 nm; emission wavelengths: 665 nm and 615 nm). By comparing the values with those of the blank control group (1% DMSO) and the positive control group (1 µM A769662), the relative activity of AMPKα1β1γ1 at each test compound concentration (4.57 nM, 13.72 nM, 41.15 nM, 123.46 nM, 370.37 nM, 1111.11 nM, 3333.33 nM, and 10000 nM) was calculated, with the activity of 1 µM A769662 set as 100%. The data were analysed in GraphPad Prism 9 using a Dose-response-Stimulation four-parameter regression model to obtain the Top and EC₅₀ values for the test compounds. The test results of some compounds are shown in Table 1. 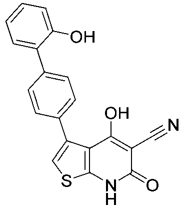 has a structure of A769662.

**Table 1**

| Compound No. | EC₅₀ (nM) | Top value | Compound No. | EC₅₀ (nM) | Top value |
|---|---|---|---|---|---|
| Compound 1 | C | ++ | Compound 18 | B | ++ |
| Compound 2 | A | +++ | Compound 19 | B | ++++ |
| Compound 3 | C | ++ | Compound 20 | B | ++++ |
| Compound 4 | A | ++ | Compound 21 | C | + |
| Compound 5 | A | +++ | Compound 22 | C | |
| Compound 6 | A | ++++ | Compound 23 | A | ++++ |
| Compound 7 | B | +++ | Compound 24 | C | ++++ |
| Compound 8 | A | + | Compound 25 | C | + |
| Compound 9 | C | ++++ | Compound 27 | C | ++++ |
| Compound 10 | C | ++ | Compound 31 | B | ++++ |
| Compound 11 | B | +++ | Compound 32 | B | +++ |
| Compound 12 | C | ++++ | Compound 33 | B | ++++ |
| Compound 13 | C | ++++ | Compound 34 | B | ++++ |
| Compound 14 | C | ++++ | Compound 35 | A | ++++ |
| Compound 15 | C | ++++ | Compound 36 | A | ++ |
| Compound 16 | B | +++ | Compound 37 | B | ++++ |
| Compound 17 | A | +++ | | | |

In Table 1, A indicates EC50 < 200 nM; B indicates 200 nM ≤ EC50 < 800 nM; C indicates 800 nM ≤ EC50; with the AMPK (α1β1γ1) enzyme agonist activity of 1 µM A769662 set as 100%, ++++ indicates the Top value of the test compound's AMPK (α1β1γ1) enzyme agonist activity ≥ 100%; +++ indicates the Top value of the test compound's AMPK (α1β1γ1) enzyme agonist activity ≥ 70% and < 100%; ++ indicates the Top value of the test compound's AMPK (α1β1γ1) enzyme agonist activity ≥ 50% and < 70%; + indicates the Top value of the test compound's AMPK (α1β1γ1) enzyme agonist activity < 50%.

The test results show that the compounds of the present invention exhibit good AMPK agonist activity, with some compounds demonstrating more excellent AMPK agonist activity.

It should be finally noted that the above embodiments are merely used for illustrating rather than limiting the technical solution of the present invention; although the present invention has been illustrated in detail with reference to the preferred embodiments, those of ordinary skill in the art should understand that the specific embodiments of the present invention may still be modified, or some technical features thereof may be equivalently substituted; without departing from the spirit of the technical solution of the present invention, all such modifications and substitutions shall fall within the scope of the technical solution of the present invention.

## Claims

1. A compound of formula (I), or an enantiomer, a diastereomer, a racemate, a stereoisomer, a geometric isomer, a tautomer, a polymorph, a solvate, a hydrate, an N-oxide, an isotopically labelled compound, a metabolite, an ester, a prodrug or a pharmaceutically acceptable salt thereof, 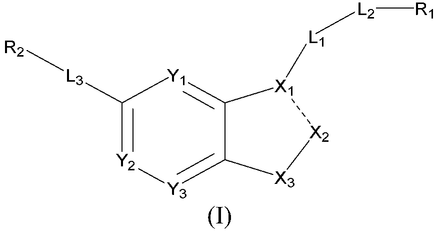
**characterised in that** 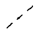 is selected from a single bond and a double bond, preferably, 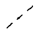 is a double bond;
when 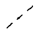 is a single bond, X₁ is selected from CH and N, and X₂ is selected from CR_{X2a}R_{X2b} and C(=O); or
X₁ is selected from CR_{X1} and N, and X₂ is selected from CR_{X2a}R_{X2b} and C(=O);
or X₁ is C, L₁ is -CR_{L1b}-, and X₁ and L₁ are connected via a double bond;
when 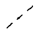 is a double bond, X₁ is C, and X₂ is selected from CR_{X2a} and N; X₃ is selected from NR_{X3}, O and S;
Y₁ is selected from CR_{Y1}, N and an N-oxide;
Y₂ is selected from CR_{Y2} and N;
Y₃ is selected from CR_{Y3} and N;
L₁ is selected from -C(=O)- and -CR_{L1a}R_{L1b}-
L₂ is selected from a bond and -C(=O)-;
L₃ is selected from a bond, -O-, -NR_{L3}-, -S-, -C₁₋₆ alkylene-O-, -C₁₋₆ alkylene- NR_{L3}-, -C₁₋₆ alkylene-S-, -C₁₋₆ alkylene-, -C₂₋₆ alkenylene- and -C₂₋₆ alkynylene-;
R₁ is selected from -OR₁ₐ, -NR_{1b}R_{1c}, tetrazolyl and oxodihydrooxadiazolyl 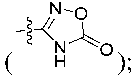
R₂ is selected from C₆₋₁₀ aryl, C₃₋₁₀ cyclic group, 4- to 10-membered heterocyclyl and 5- to 10-membered heteroaryl, and the C₆₋₁₀ aryl, C₃₋₁₀ cyclic group, 4- to 10-membered heterocyclyl and 5- to 10-membered heteroaryl are optionally substituted with 1, 2, 3, 4, or 5 groups each independently selected from R₂ₐ, R_{2b}, R_{2c}, R_{2d} and R₂ₑ;
Rₓ₁ is selected from H, C₁₋₆ alkyl and C₁₋₆ haloalkyl;
R_{X2a} and R_{X2b} are each independently selected from H, halogen, -NH₂, C₁₋₆ alkyl, C₁₋₆ haloalkyl, -NH-C₁₋₆ alkyl, -N(C₁₋₆ alkyl)-C₁₋₆ alkyl, C₁₋₆ alkoxy, C₁₋₆ haloalkoxy, -S-C₁₋₆ alkyl and -S-C₁₋₆ haloalkyl; or
R_{X2a} and R_{X2b} are each independently selected from H, halogen, hydroxyl, - NH₂, C₁₋₆ alkyl, C₁₋₆ haloalkyl, -NH-C₁₋₆ alkyl, -N(C₁₋₆ alkyl)-C₁₋₆ alkyl, C₁₋₆ alkoxy, C₁₋₆ haloalkoxy, -S-C₁₋₆ alkyl and -S-C₁₋₆ haloalkyl;
R_{X3} is selected from H, C₁₋₆ alkyl, C₁₋₆ haloalkyl, C₃₋₆ cycloalkyl and C₃₋₆ halocycloalkyl;
R_{Y1} is selected from H, D, halogen, hydroxyl, carboxyl, -CN, C₁₋₆ alkyl, C₁₋₆ haloalkyl, C₁₋₆ alkoxy, C₁₋₆ haloalkoxy, C₂₋₆ alkenyl, C₂₋₆ alkynyl, C₃₋₁₀ cycloalkyl and 4- to 10-membered heterocycloalkyl;
R_{Y2} and R_{Y3} are each independently selected from H, D, halogen, -CN, hydroxyl, carboxyl, C₁₋₆ alkyl, C₁₋₆ haloalkyl, C₁₋₆ alkoxy, C₁₋₆ haloalkoxy, C₂₋₆ alkenyl, C₂₋₆ alkynyl, C₃₋₁₀ cycloalkyl and 4- to 10-membered heterocycloalkyl; or
R_{Y2} and R_{Y3}, together with the atoms to which they are attached, form phenyl or 5- to 6-membered heteroaryl;
R_{L1a} and R_{L1b} are each independently selected from H, D, halogen, C₁₋₆ alkyl, C₁₋₆ haloalkyl, C₁₋₆ alkoxy and C₁₋₆ haloalkoxy; or
R_{L1a} and R_{L1b}, together with the atoms to which they are attached, form C₃₋₆ cycloalkyl;
R_{L3} is selected from H, D and C₁₋₄ alkyl;
R₁ₐ, R_{1b} and R_{1c} are each independently selected from H, C₁₋₆ alkyl, C₃₋₁₀ cycloalkyl, 4- to 10-membered heterocyclyl, 5- to 10-membered heteroaryl, C₆₋₁₀ aryl, -C₁₋₆ alkylene-C₃₋₁₀ cycloalkyl, -C₁₋₆ alkylene-4- to 10-membered heterocyclyl, -C₁₋₆ alkylene-C₆₋₁₀ aryl, -C₁₋₆ alkylene-5- to 10-membered heteroaryl, -C₁₋₆ haloalkylene-C₃₋₁₀ cycloalkyl, -C₁₋₆ haloalkylene-4- to 10-membered heterocyclyl, - C₁₋₆ haloalkylene-C₆₋₁₀ aryl and -C₁₋₆ haloalkylene-5- to 10-membered heteroaryl; or
R₁ₐ, R_{1b} and R_{1c} are each independently selected from H, C₁₋₆ alkyl, C₃₋₁₀ cycloalkyl, 4- to 10-membered heterocyclyl, 5- to 10-membered heteroaryl, C₆₋₁₀ aryl, -C₁₋₆ alkylene-C₃₋₁₀ cycloalkyl, -C₁₋₆ alkylene-4- to 10-membered heterocyclyl, -C₁₋₆ alkylene-C₆₋₁₀ aryl, -C₁₋₆ alkylene-5- to 10-membered heteroaryl, -C₁₋₆ haloalkylene-C₃₋₁₀ cycloalkyl, -C₁₋₆ haloalkylene-4- to 10-membered heterocyclyl, - C₁₋₆ haloalkylene-C₆₋₁₀ aryl and -C₁₋₆ haloalkylene-5- to 10-membered heteroaryl; wherein the C₁₋₆ alkyl, C₃₋₁₀ cycloalkyl, 4- to 10-membered heterocyclyl, 5- to 10-membered heteroaryl, C₆₋₁₀ aryl, -C₁₋₆ alkylene-C₃₋₁₀ cycloalkyl, -C₁₋₆ alkylene-4- to 10-membered heterocyclyl, -C₁₋₆ alkylene-C₆₋₁₀ aryl, -C₁₋₆ alkylene-5- to 10-membered heteroaryl, -C₁₋₆ haloalkylene-C₃₋₁₀ cycloalkyl, -C₁₋₆ haloalkylene-4- to 10-membered heterocyclyl, -C₁₋₆ haloalkylene-C₆₋₁₀ aryl and -C₁₋₆ haloalkylene-5-to 10-membered heteroaryl are optionally substituted with one or more substituents selected from oxo, hydroxyl, cyano, carboxyl, halogen, C₁₋₆ alkyl, C₁₋₆ haloalkyl, C₁₋₆ alkoxy, C₁₋₆ haloalkoxy, C₃₋₆ cycloalkyl, 4- to 7-membered heterocyclyl, C₆₋₁₀ aryl, and 5- to 10-membered heteroaryl;
R₂ₐ, R_{2b}, R_{2c}, R_{2d} and R₂ₑ are each independently selected from H, halogen, - OH, C₁₋₆ alkyl, C₁₋₆ haloalkyl, -NHC₁₋₆ alkyl, -N(C₁₋₆ alkyl)-C₁₋₆ alkyl, C₁₋₆ alkoxy, C₁₋₆ haloalkoxy, C₃₋₁₀ cyclic group, 4- to 10-membered heterocyclyl, C₆₋₁₀ aryl, 5- to 10-membered heteroaryl, -C₆₋₁₀ arylene-4- to 10-membered heterocyclyl, -C₆₋₁₀ arylene-5- to 10-membered heteroaryl, -C₆₋₁₀ arylene-C₆₋₁₀ aryl and -5- to 10-membered heteroarylene-5- to 10-membered heteroaryl, and the C₁₋₆ alkyl, C₁₋₆ haloalkyl, -NHC₁₋₆ alkyl, -N(C₁₋₆ alkyl)-C₁₋₆ alkyl, C₁₋₆ alkoxy, C₁₋₆ haloalkoxy, C₃₋₁₀ cyclic group, 4- to 10-membered heterocyclyl, C₆₋₁₀ aryl, 5- to 10-membered heteroaryl, -C₆₋₁₀ arylene-4- to 10-membered heterocyclyl, -C₆₋₁₀ arylene-5- to 10-membered heteroaryl, -C₆₋₁₀ arylene-C₆₋₁₀ aryl and -5- to 10-membered heteroarylene-5- to 10-membered heteroaryl are optionally substituted with one or more R;
each R is independently selected from halogen, -OH, -CN, -COOH, oxo, C₁₋₆ alkyl, -C₁₋₆ alkylene-OH, C₁₋₆ alkoxy, -C₁₋₆ alkyleneoxy-C₁₋₆ alkoxy, -C(=O)NH-C₁₋₆ alkyl, -C(=O)-C₁₋₆ alkyl, -NHC(=O)-C₁₋₆ alkyl, -N=S(=O)(C₁₋₆ alkyl)-C₁₋₆ alkyl, - NH-S(=O)₂C₁₋₆ alkyl, -S(=O)₂-C₁₋₆ alkyl, -S(=O)₂-4- to 10-membered heterocyclyl, -C(=O)-4- to 10-membered heterocyclyl, 4- to 10-membered heterocyclyl, 5- to 10-membered heteroaryl and 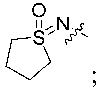
provided that when L₂ is a bond,
1) L₁ is -CR_{L1a}R_{L1b}-, or
2) R₁ is selected from -NR_{1b}R_{1c}, tetrazolyl and oxodihydrooxadiazolyl 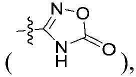 or
3) is a single bond, or
4) R₁ is -OR₁ₐ, wherein R₁ₐ is selected from 5- to 10-membered heteroaryl, -C₁₋₆ alkylene-5- to 10-membered heteroaryl, and -C₁₋₆ alkylene-4- to 10-membered heterocyclyl.

2. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to claim 1, **characterised in that** when 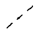 is a single bond, X₁ is selected from CR_{X1} and N, and X₂ is selected from CR_{X2a}R_{X2b} and C(=O), or X₁ is C, L₁ is -CR_{L1b}-, and X₁ and L₁ are connected via a double bond; when 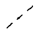 is a double bond, X₁ is C, and X₂ is selected from CR_{X2a} and N; Rₓ₁ is selected from H, C₁₋₃ alkyl, and C₁₋₃ haloalkyl;
preferably, Rₓ₁ is selected from H and C₁₋₃ alkyl;
preferably, Rₓ₁ is C₁₋₃ alkyl;
preferably, 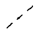 is a single bond, X₁ is C, L₁ is -CR_{L1b}-, and X₁ and L₁ are connected via a double bond;
preferably, 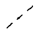 is a single bond, X₁ is selected from CR_{X1} and N, and X₂ is selected from CR_{X2a}R_{X2b} and C(=O);
preferably, 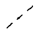 is a single bond, X₁ is N, and X₂ is C(=O); or X₁ is CR_{X1}, and X₂ is CR_{X2a}R_{X2b}; or X₁ is CR_{X1}, and X₂ is C(=O);
preferably, 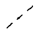 is a double bond, X₁ is C, and X₂ is selected from CR_{X2a} and N;
further preferably, 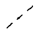 is a double bond, X₁ is C, and X₂ is CR_{X2a};
further preferably, 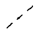 is a double bond, X₁ is C, and X₂ is selected from CH and C(OH).

3. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to claim 1 or 2, **characterised in that** R_{X2a} and R_{X2b} are each independently selected from H, halogen, hydroxyl, C₁₋₄ alkyl, C₁₋₄ haloalkyl, -NH-C₁₋₄ alkyl, -N(C₁₋₄ alkyl)-C₁₋₄ alkyl, C₁₋₄ alkoxy, C₁₋₄ haloalkoxy, -S-C₁₋₄ alkyl and -S-C₁₋₄ haloalkyl;
preferably, R_{X2a} and R_{X2b} are each independently selected from H, halogen, hydroxyl, C₁₋₄ alkoxy, C₁₋₄ haloalkoxy, and -S-C₁₋₄ alkyl;
preferably, R_{X2a} and R_{X2b} are each independently selected from H, halogen, hydroxyl, methyl, ethyl, propyl, butyl, halomethyl, haloethyl, halopropyl, halobutyl, -NH-methyl, -NH-ethyl, -NH-propyl, -NH-butyl, -N(methyl)-methyl, -N(methyl)-ethyl, -N(methyl)-propyl, -N(ethyl)-ethyl, -N(ethyl)-propyl, methoxy, ethoxy, propoxy, butoxy, halomethoxy, haloethoxy, halopropoxy, halobutoxy, -S-methyl, - S-ethyl, -S-propyl, -S-butyl, -S-halomethyl, -S-haloethyl, -S-halopropyl and -S-halobutyl;
further preferably, R_{X2a} and R_{X2b} are each independently selected from H, F, Cl, Br, hydroxyl, -CH₃, -CH₂CH₃, -CH₂F, -CHF₂, -CF₃, -CF₂CF₃, -NHCH₃, -N(CH₃)₂, - N(CH₂CH₃)₂, -OCH₃, -OCH₂CH₃, -OCH₂CH₂CH₃, -OCH(CH₃)₂, -OCF₃, -OCF₂CF₃, -S-CH₃, -S-CH₂CH₃, -S-CH₂CH₂CH₃ and -S-CH(CH₃)₂;
further preferably, R_{X2a} is selected from H, F, Cl, hydroxyl, -OCH₃, -OCF₃ and -SCH₃;
further preferably, R_{X2b} is H;
more preferably, R_{X2a} is selected from H and hydroxyl.

4. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-3, **characterised in that** R_{X3} is selected from H, C₁₋₄ alkyl, C₁₋₄ haloalkyl, C₃₋₅ cycloalkyl and C₃₋₅ halocycloalkyl;
preferably, R_{X3} is selected from H, C₁₋₄ alkyl, and C₁₋₄ haloalkyl;
preferably, R_{X3} is selected from H, methyl, ethyl, propyl, halomethyl, haloethyl, halopropyl, cyclopropyl, cyclobutyl, cyclopentyl, halocyclopropyl, halocyclobutyl and halocyclopentyl;
further preferably, R_{X3} is H;
preferably, X₃ is NR_{X3};
preferably, X₃ is selected from O and S.

5. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-4, **characterised in that** R_{Y1}, R_{Y2} and R_{Y3} are each independently selected from H, D, halogen, -CN, hydroxyl, carboxyl, C₁₋₄ alkyl, C₁₋₄ haloalkyl, C₁₋₄ alkoxy, C₁₋₄ haloalkoxy, C₂₋₄ alkenyl, C₂₋₄ alkynyl, C₃₋₆ cycloalkyl and 4- to 6-membered heterocycloalkyl; or R_{Y2} and R_{Y3}, together with the atoms to which they are attached, form phenyl or 5- to 6-membered heteroaryl;
preferably, Y₁ is selected from CR_{Y1} and N; more preferably, Y₁ is CR_{Y1};
preferably, Y₂ is selected from CR_{Y2} and N; more preferably, Y₂ is CR_{Y2};
preferably, Y₃ is selected from CR_{Y3} and N; more preferably, Y₃ is CR_{Y3};
preferably, R_{Y1}, R_{Y2} and R_{Y3} are each independently selected from H, D, halogen, -CN, hydroxyl, carboxyl, C₁₋₄ alkyl, C₁₋₄ haloalkyl, C₁₋₄ alkoxy, C₁₋₄ haloalkoxy, C₂₋₄ alkenyl, C₂₋₄ alkynyl, C₃₋₆ cycloalkyl and 4- to 6-membered heterocycloalkyl;
preferably, R_{Y1}, R_{Y2} and R_{Y3} are each independently selected from H, D, halogen, -CN, methyl, ethyl, propyl, butyl, halomethyl, haloethyl, halopropyl, halobutyl, methoxy, ethoxy, propoxy, butoxy, halomethoxy, haloethoxy, halopropoxy, halobutoxy, vinyl, propenyl, butenyl, ethynyl, propynyl, butynyl, cyclopropyl, cyclobutyl, cyclopentyl, cyclohexyl, oxetanyl, azetidinyl, tetrahydrofuryl, pyrrolidyl and morpholinyl;
further preferably, R_{Y1}, R_{Y2} and R_{Y3} are each independently selected from H, D, F, Cl, Br, -CN, -CH₃, -CH₂CH₃, -CH₂CH₂CH₃, -CH(CH₃)₂, -CH₂F, -CHF₂, -CF₃, -CF₂CF₃, -OCH₃, -OCH₂CH₃, -OCH₂CH₂CH₃, -OCH(CH₃)₂, -OCF₃, vinyl, propenyl, ethynyl, propynyl, cyclopropyl, cyclobutyl, oxetanyl and azetidinyl;
preferably, R_{Y1} is selected from H, D, halogen, C₁₋₄ alkyl, C₁₋₄ haloalkyl, C₁₋₄ alkoxy, and C₁₋₄ haloalkoxy; more preferably, R_{Y1} is selected from H, D, and halogen;
preferably, R_{Y2} is selected from H, D, halogen, -CN, C₁₋₄ alkyl, C₁₋₄ haloalkyl, C₁₋₄ alkoxy, C₁₋₄ haloalkoxy, C₂₋₄ alkynyl, C₃₋₆ cycloalkyl and 4- to 6-membered heterocycloalkyl; more preferably, R_{Y2} is halogen;
preferably, R_{Y3} is selected from H, D, halogen, and C₁₋₄ alkyl;
further preferably, R_{Y1} is selected from H and F; more preferably, R_{Y1} is H;
further preferably, R_{Y2} is selected from F, Cl, -CN, -CH₃, -CH₂CH₃, -CH(CH₃)₂, -OCH₃, -OCF₃, ethynyl, cyclopropyl and 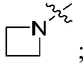 more preferably, R_{Y2} is Cl;
further preferably, R_{Y3} is H.

6. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-5, **characterised in that** R_{Y2} and R_{Y3}, together with the atoms to which they are attached, form phenyl or 5- to 6-membered heteroaryl;
preferably, R_{Y2} and R_{Y3}, together with the atoms to which they are attached, form phenyl or 5-membered heteroaryl;
further preferably, R_{Y2} and R_{Y3}, together with the atoms to which they are attached, form thienyl, furyl, pyrrolyl, thiazolyl, oxazolyl, pyridyl, pyrimidyl, pyrazinyl and phenyl;
further preferably, R_{Y2} and R_{Y3}, together with the atoms to which they are attached, form thienyl and phenyl;
further preferably, R_{Y1} is H.

7. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-6, **characterised in that** structural unit 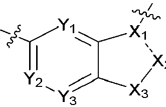 is selected from 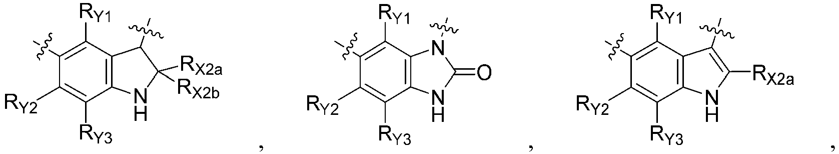 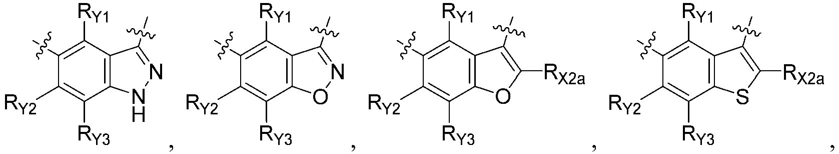 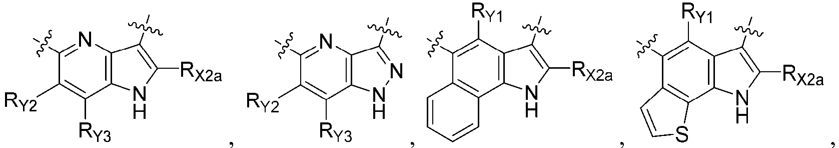 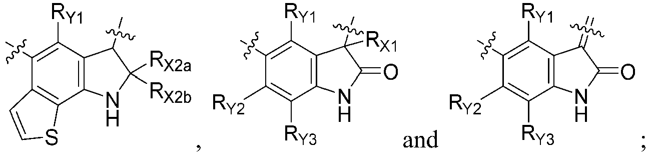
preferably, structural unit 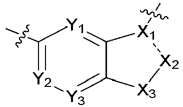 is selected from 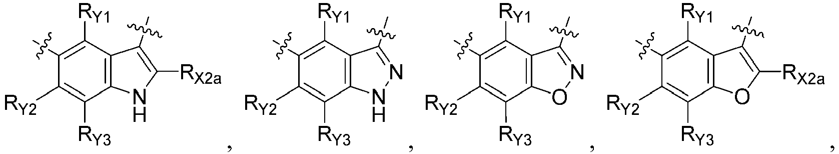 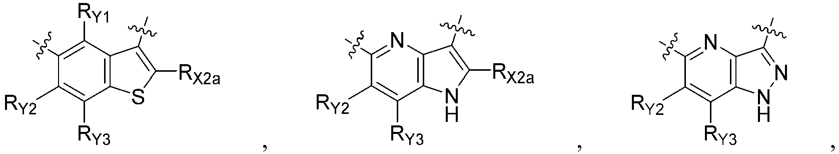 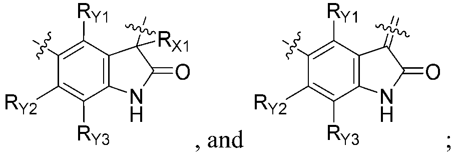
preferably, structural unit 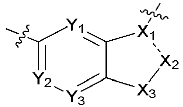 is selected from 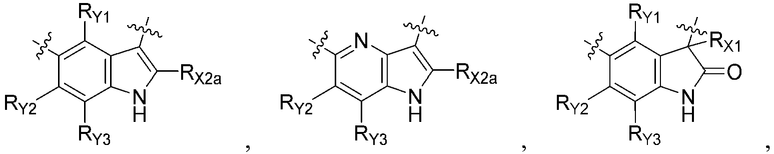 and 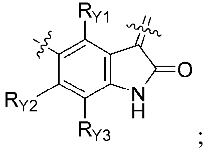
further preferably, structural unit 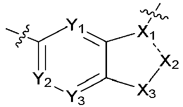 is selected from 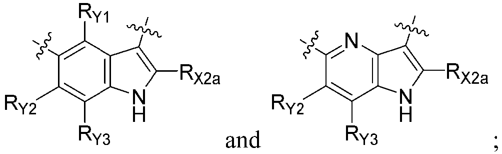
further preferably, structural unit 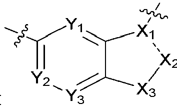 is

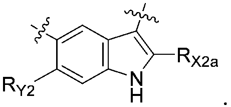

8. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-7, **characterised in that** R_{L1a} and R_{L1b} are each independently selected from H, D, halogen, C₁₋₄ alkyl, C₁₋₄ haloalkyl, C₁₋₄ alkoxy and C₁₋₄ haloalkoxy; or R_{L1a} and R_{L1b}, together with the atoms to which they are attached, form C₃₋₆ cycloalkyl;
preferably, R_{L1a} and R_{L1b} are each independently selected from H, D, halogen, C₁₋₄ alkyl, C₁₋₄ haloalkyl, C₁₋₄ alkoxy and C₁₋₄ haloalkoxy;
preferably, R_{L1a} and R_{L1b} are each independently selected from H, D, and halogen;
preferably, L₁ is -C(=O)-;
preferably, L₁ is -CR_{L1a}R_{L1b}-;
further preferably, R_{L1a} and R_{L1b} are each independently selected from H, D, F, Cl, Br, -CH₃, -CH₂CH₃, -CH₂F, -CHF₂, -CF₃, -CF₂CF₃, -OCH₃, -OCH₂CH₃, - OCH₂CH₂CH₃ and -OCF₃;
further preferably, R_{L1a} and R_{L1b} are each independently selected from H and F;
further preferably, R_{L1a} and R_{L1b} are each independently H;
further preferably, R_{L1a} and R_{L1b}, together with the atoms to which they are attached, form cyclopropyl, cyclobutyl, cyclopentyl or cyclohexyl;
further preferably, the R_{L1a} and R_{L1b}, together with the atoms to which they are attached, form cyclopropyl or cyclobutyl.

9. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-8, **characterised in that** L₁ is selected from -C(=O)-, -CH₂-, -CHF-, =CH- and -CF₂-;
preferably, L₁ is selected from -C(=O)-, -CH₂-, and =CH-;
preferably, L₁ is -C(=O)-.

10. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-9, **characterised in that** L₂ is a bond or L₂ is -C(=O)-.

11. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-10, **characterised in that** R₁ₐ, R_{1b} and R_{1c} are each independently selected from H, C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl; wherein the C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl are optionally substituted with one or more substituents selected from oxo, hydroxyl, cyano, carboxyl, and halogen;
preferably, R₁ₐ is selected from H, C₁₋₃ alkyl, C₃₋₆ cycloalkyl, 4- to 6-membered heterocyclyl, 5- to 6-membered heteroaryl, phenyl, -C₁₋₃ alkylene-4- to 6-membered heterocyclyl, and -C₁₋₃ alkylene-5- to 6-membered heteroaryl;
preferably, R₁ₐ is selected from H and C₁₋₆ alkyl (such as C₁₋₃ alkyl);
further preferably, R₁ₐ is selected from H, methyl, cyclopropyl, cyclobutyl, oxetanyl (such as 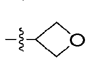 ), oxanyl (such as 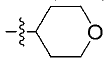 ), pyrazolyl (such as 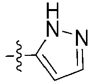 ), oxazolyl (such as 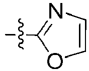 ), phenyl, -C₁₋₃ alkylene-4- to 6-membered heterocyclyl (such as 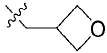 ), and -C₁₋₃ alkylene-5-membered heteroaryl (such as 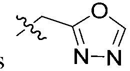 and 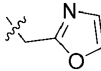 );
further preferably, R₁ₐ is H or methyl;
preferably, R_{1b} is selected from H and C₁₋₃ alkyl; R_{1c} is selected from C₁₋₃ alkyl, C₃₋₆ cycloalkyl, 5- to 6-membered heteroaryl, phenyl, -C₁₋₃ alkylene-4- to 6-membered heterocyclyl, -C₁₋₃ alkylene-phenyl, and -C₁₋₃ alkylene-5- to 6-membered heteroaryl, wherein the C₁₋₃ alkyl is optionally substituted with 1 or 2 substituents selected from oxo, hydroxyl, and carboxyl;
further preferably, R_{1c} is selected from methyl, -CH₂C(=O)OH, cyclopropyl, pyrrolyl (such as 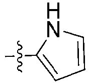 ), furyl (such as 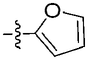 ), thienyl (such as 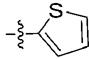 ), imidazolyl (such as 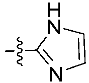 ), pyrazolyl (such as 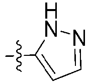 ), oxazolyl (such as 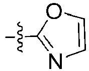 ), tetrazolyl (such as 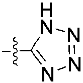 ), phenyl, -CH₂-phenyl, -C₁₋₃ alkylene-4- to 6-membered heterocyclyl (such as 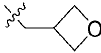 and 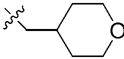 ), and -C₁₋₃ alkylene-5- to 6-membered heteroaryl (such as 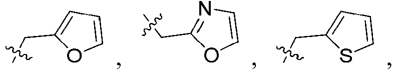 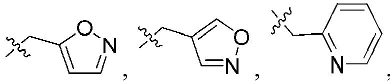 , and 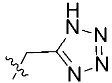 );
preferably, R_{1b} is selected from H and C₁₋₃ alkyl; R_{1c} is selected from C₁₋₃ alkyl, 5-membered heteroaryl, -C₁₋₃ alkylene-4- to 6-membered heterocyclyl, and -C₁₋₃ alkylene-5- to 6-membered heteroaryl, wherein the C₁₋₃ alkyl is optionally substituted with carboxyl;
preferably, R_{1b} is selected from H and C₁₋₃ alkyl; R_{1c} is selected from C₁₋₃ alkyl, 5-membered heteroaryl, and -C₁₋₃ alkylene-5-membered heteroaryl, wherein the C₁₋₃ alkyl is substituted with one carboxyl;
more preferably, R_{1b} is H; R_{1c} is selected from -CH₂C(=O)OH, pyrazolyl (such as 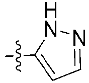 ), and -C₁₋₃ alkylene-5-membered heteroaryl (such as 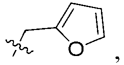 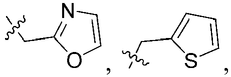 and 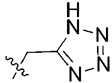 );
preferably, R₁ₐ, R_{1b} and R_{1c} are each independently selected from H, C₁₋₄ alkyl, C₃₋₆ cycloalkyl, 4- to 6-membered heterocyclyl, 5- to 6-membered heteroaryl, phenyl, -C₁₋₄ alkylene-C₃₋₆ cycloalkyl, -C₁₋₄ alkylene-4- to 6-membered heterocyclyl, -C₁₋₄ alkylene-phenyl, -C₁₋₄ alkylene-5- to 6-membered heteroaryl, -C₁₋₄ haloalkylene-C₃₋₆ cycloalkyl, -C₁₋₄ haloalkylene-4- to 6-membered heterocyclyl, -C₁₋₄ haloalkylene-phenyl and -C₁₋₄ haloalkylene-5- to 6-membered heteroaryl; or
R₁ₐ, R_{1b} and R_{1c} are each independently selected from H, C₁₋₄ alkyl, - (CR₁ₐₐR_{1bb})ₘ-C₃₋₆ cycloalkyl, -(CR₁ₐₐR_{1bb})ₘ-4- to 6-membered heterocyclyl, - (CR₁ₐₐR_{1bb})ₘ-5- to 6-membered heteroaryl and -(CR₁ₐₐR_{1bb})ₘ-phenyl, wherein R₁ₐₐ and R_{1bb} are each independently selected from H, F and -CH₃, and m is selected from 0, 1 and 2;
preferably, R₁ₐ, R_{1b} and R_{1c} are each independently selected from H, methyl, ethyl, propyl, butyl, -(CR₁ₐₐR_{1bb})ₘ-cyclopropyl, -(CR₁ₐₐR_{1bb})ₘ-cyclobutyl, - (CR₁ₐₐR_{1bb})ₘ-cyclopentyl, -(CR₁ₐₐR_{1bb})ₘ-cyclohexyl, -(CR₁ₐₐR_{1bb})ₘ-oxetanyl, - (CR₁ₐₐR_{1bb})ₘ-azetidinyl, -(CR₁ₐₐR_{1bb})ₘ-tetrahydrofuryl, -(CR₁ₐₐR_{1bb})ₘ-pyrrolidyl, - (CR₁ₐₐR_{1bb})ₘ-tetrahydropyranyl, -(CR₁ₐₐR_{1bb})ₘ-piperidyl, -(CR₁ₐₐR_{1bb})ₘ-morpholinyl, -(CR₁ₐₐR_{1bb})ₘ-phenyl, -(CR₁ₐₐR_{1bb})ₘ-thienyl, -(CR₁ₐₐR_{1bb})ₘ-pyrazolyl, -(CR₁ₐₐR_{1bb})ₘ-imidazolyl, -(CR₁ₐₐR_{1bb})ₘ-tetrazolyl, -(CR₁ₐₐR_{1bb})ₘ-furyl, - (CR₁ₐₐR_{1bb})ₘ-pyrrolyl, -(CR₁ₐₐR_{1bb})ₘ-thiazolyl, -(CR₁ₐₐR_{1bb})ₘ-oxazolyl, - (CR₁ₐₐR_{1bb})ₘ-isoxazolyl, -(CR₁ₐₐR_{1bb})ₘ-oxadiazolyl, -(CR₁ₐₐR_{1bb})ₘ-pyridyl, - (CR₁ₐₐR_{1bb})ₘ-pyrimidyl and -(CR₁ₐₐR_{1bb})ₘ-pyrazinyl, wherein R₁ₐₐ and R_{1bb} are each independently selected from H, F and -CH₃, and m is selected from 0, 1 and 2;
further preferably, R₁ₐ and R_{1c} are each independently selected from H, -CH₃, cyclopropyl, cyclobutyl, oxetanyl, tetrahydropyranyl, phenyl, pyrazolyl, imidazolyl, oxazolyl, tetrazolyl, pyrrolyl, thienyl, furyl, -CH₂-oxetanyl, -CH₂-tetrahydropyranyl, -CH₂-phenyl, -CH₂-oxadiazolyl, -CH₂-oxazolyl, -CH₂-furyl, -CH₂-thienyl, -CH₂-pyridyl, -CH₂-isoxazolyl, and -CH₂-tetrazolyl;
preferably, R₁ₐ is selected from H, -CH₃, cyclopropyl, cyclobutyl, oxetanyl, tetrahydropyranyl, pyrazolyl, phenyl, -CH₂-oxetanyl, -CH₂-oxadiazolyl, and -CH₂-oxazolyl; R_{1c} is selected from methyl, cyclopropyl, phenyl, -CH₂-phenyl, -CH₂-tetrahydropyranyl, pyrazolyl, imidazolyl, oxazolyl, tetrazolyl, pyrrolyl, thienyl, furyl, -CH₂-oxazolyl, -CH₂-furyl, -CH₂-thienyl, -CH₂-pyridyl, -CH₂-oxetanyl, -CH₂-isoxazolyl, and -CH₂-tetrazolyl;
preferably, R₁ₐ and R_{1c} are each independently selected from H, methyl, pyrazolyl, -CH₂-thienyl, -CH₂-oxazolyl, -CH₂-furyl, and -CH₂-tetrazolyl;
preferably, R₁ₐ is selected from H and methyl; R_{1c} is selected from pyrazolyl, - CH₂-thienyl, -CH₂-oxazolyl, -CH₂-furyl, and -CH₂-tetrazolyl;
further preferably, R_{1b} is H.

12. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-11, **characterised in that** R₁ is selected from -OH, -OCH₃, -O-cyclopropyl, -O-cyclobutyl, 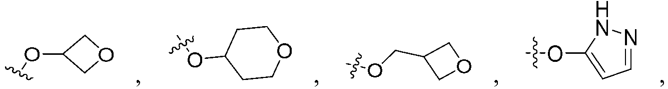 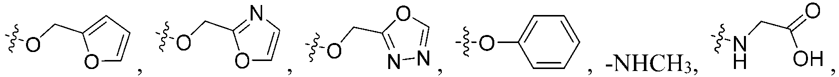 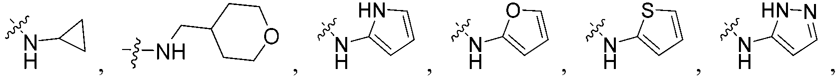 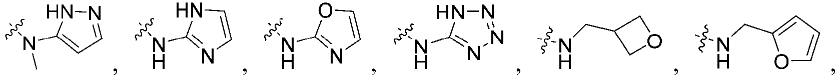 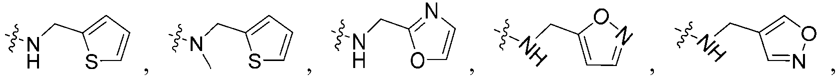 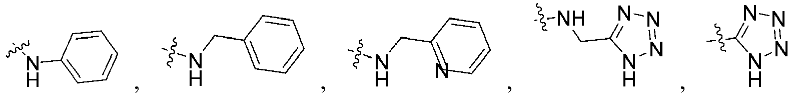 and 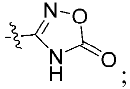
preferably, R₁ is selected from -OR₁ₐ and -NR_{1b}R_{1c};
further preferably, R₁ is selected from -OH, -OCH₃, -O-cyclopropyl, -O-cyclobutyl, 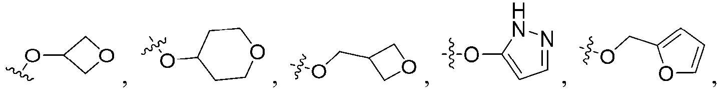 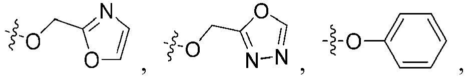 -NHCH₃, 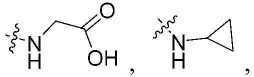 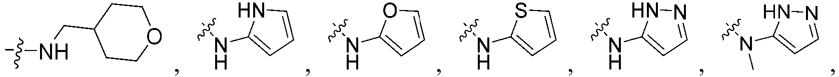 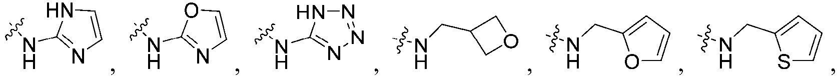 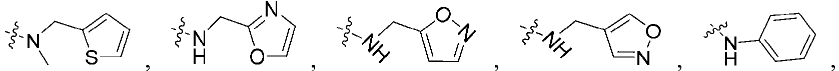 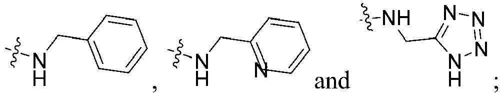
preferably, R₁ is selected from -OH, -OCH₃, -NHCH₃, 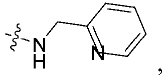 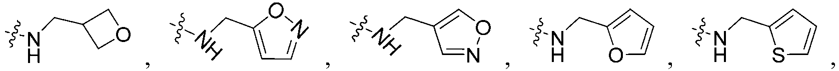 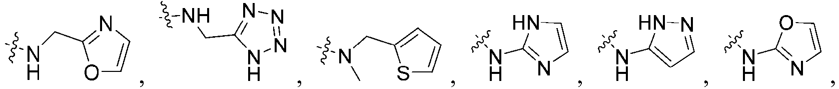 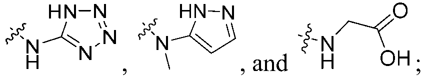
more preferably, R₁ is selected from -OH, -OCH₃,

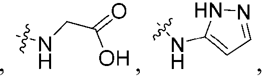

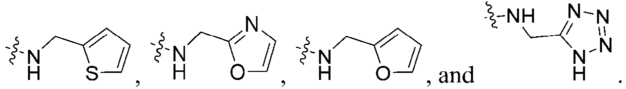

13. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-12, **characterised in that** -L₁-L₂- is selected from -C(=O)C(=O)-, #X1-CR_{L1a}R_{L1b}-C(=O)-$R1, -C(=O)-, -CR_{L1a}R_{L1b}-, or #X1=CR_{L1b}-$R1;
preferably, -L₁-L₂- is selected from -C(=O)C(=O)-, #X1-CH₂-C(=O)-$R1, #X1-CF₂-C(=O)-$R1, -C(=O)-, -CHF-, -CH₂-, and #X1=CH-$R1; wherein #X1 is the point of attachment to X₁, and $R1 is the point of attachment to R₁;
preferably, -L₁-L₂- is selected from -C(=O)C(=O)-, #X1-CH₂-C(=O)-$R1, - C(=O)-, and #X1=CH-$R1;
further preferably, -L₁-L₂- is selected from -C(=O)- and -C(=O)C(=O)-;
more preferably, -L₁-L₂- is -C(=O)-.

14. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-13, **characterised in that** -L₁-L₂-R₁ is selected from -C(=O)-OR₁ₐ, - CR_{L1a}R_{L1b}C(=O)-OR₁ₐ, -C(=O)-C(=O)-OR₁, -C(=O)-C(=O)-NR_{1b}R_{1c}, 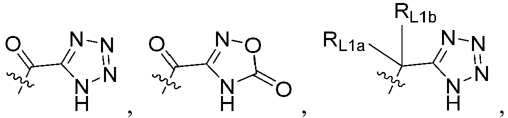 -C(=O)-NR_{1b}R_{1c}, and =CR_{L1b}-NR_{1b}R_{1c};
preferably, -L₁-L₂-R₁ is selected from -CR_{L1a}R_{L1b}-C(=O)-OR₁ₐ, -C(=O)-C(=O)-OR₁ₐ, -C(=O)-C(=O)-NR_{1b}R_{1c}, -C(=O)-NR_{1b}R_{1c}, and =CR_{L1b}-NR_{1b}R_{1c};
preferably, -L₁-L₂-R₁ is selected from -C(=O)-C(=O)-OR₁ₐ and -C(=O)-NR_{1b}R_{1c};
preferably, -L₁-L₂-R₁ is -C(=O)-NR_{1b}R_{1c};
preferably, -L₁-L₂-R₁ is selected from 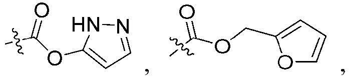 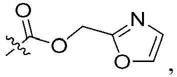 -COOH, -C(=O)-C(=O)-OH, -CH₂-C(=O)-OH, -CF₂-C(=O)-OH, -C(=O)-C(=O)-OCH₃, -C(=O)-C(=O)-NHCH₃, 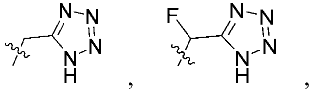 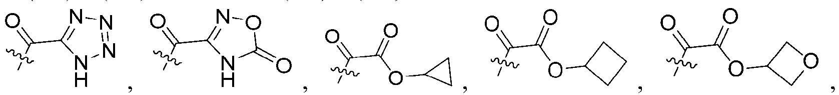 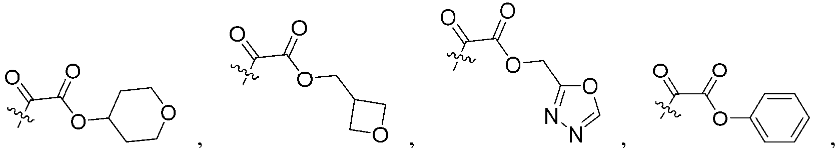 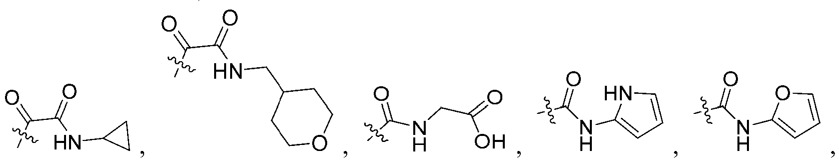 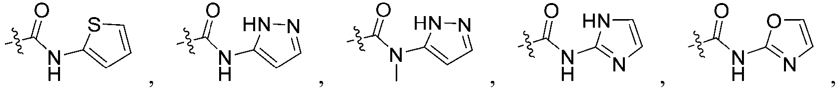 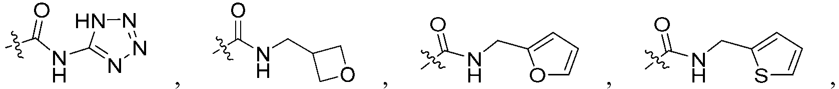 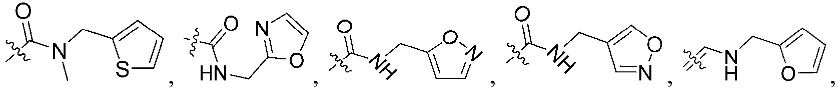 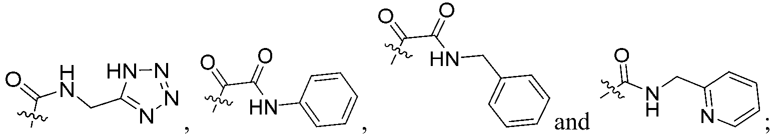
preferably, -L₁-L₂-R₁ is selected from -CH₂-C(=O)-OH, -C(=O)-C(=O)-OH, - C(=O)-C(=O)-OCH₃, -C(=O)-C(=O)-NHCH₃, 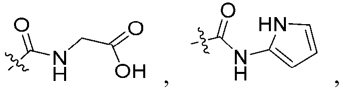 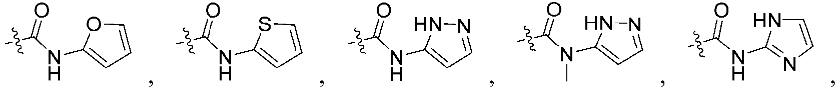 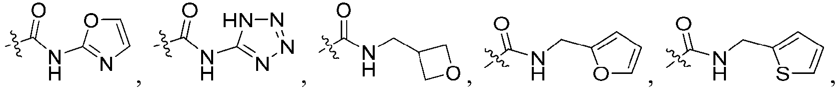 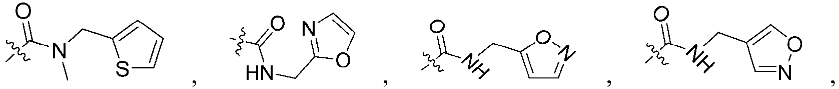 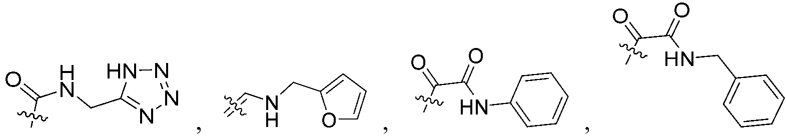 and 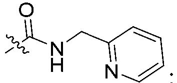
preferably, -L₁-L₂-R₁ is selected from -CH₂-C(=O)-OH, -C(=O)-C(=O)-OH, - C(=O)-C(=O)-OCH₃, -C(=O)-C(=O)-NHCH₃, 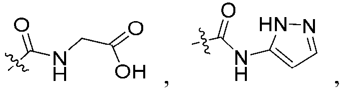 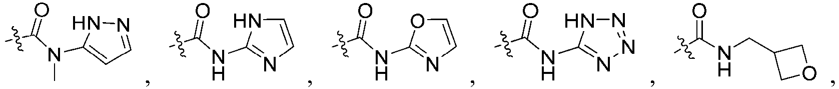 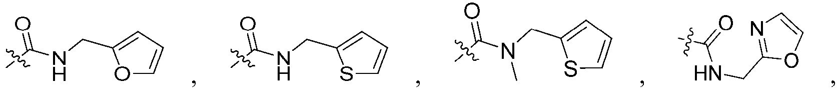 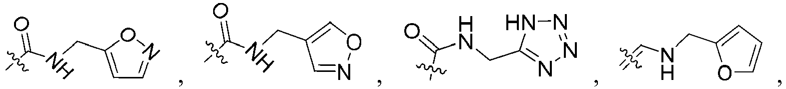
and 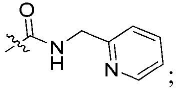
preferably, -L₁-L₂-R₁ is selected from -C(=O)-C(=O)-OH, -C(=O)-C(=O)-OCH₃, 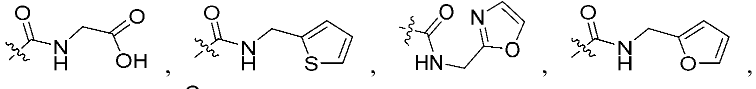 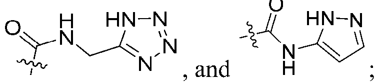
preferably, -L₁-L₂-R₁ is -C(=O)-C(=O)-OH;
preferably, L₁ is -C(=O)-, L₂ is a bond or -C(=O)-, R₁ is selected from -OR₁ₐ and -NR_{1b}R_{1c}, R₁ₐ is C₁₋₃ alkyl, R_{1b} is H, and R_{1c} is selected from C₁₋₃ alkyl, 5-membered heteroaryl (such as pyrrolyl, furyl, thienyl, imidazolyl, pyrazolyl, oxazolyl, and tetrazolyl), and -C₁₋₃ alkylene-5-membered heteroaryl (such as -CH₂-pyrazolyl, -CH₂-oxazolyl, -CH₂-furyl, -CH₂-thienyl, and -CH₂-tetrazolyl), wherein the C₁₋₃ alkyl is optionally substituted with carboxyl;
further preferably, L₂ is a bond.

15. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-14, **characterised in that** L₃ is selected from a bond, -O-, -NR_{L3}-, -S-, #-C₁₋₆ alkylene-O-*, #-C₁₋₆ alkylene-NR_{L3}-*, #-C₁₋₆ alkylene-S-*, -C₁₋₆ alkylene-, -C₂₋₆ alkenylene- and -C₂₋₆ alkynylene-, wherein the end indicated by "#" is attached to R₂, the end indicated by "*" is attached to 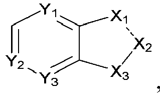 and R_{L3} is as defined in any of claims 1-14; preferably, R_{L3} is selected from H, D, -CH₃, -CH₂CH₃ and - CH(CH₃)₂, further preferably, R_{L3} is H;
preferably, L₃ is selected from a bond, -O-, -NR_{L3}-, -S-, #-C₁₋₄ alkylene-O-*, #-C₁₋₄ alkylene-NR_{L3}-*, #-C₁₋₄ alkylene-S-*, -C₁₋₄ alkylene-, -C₂₋₄ alkenylene- and - C₂₋₄ alkynylene-, wherein the end indicated by "#" is attached to R₂, the end indicated by "*" is attached to 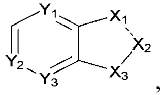 and R_{L3} is as defined in any of claims 1-14; preferably, R_{L3} is selected from H, D, -CH₃, -CH₂CH₃ and -CH(CH₃)₂, further preferably, R_{L3} is H;
preferably, L₃ is selected from a bond, -O-, -NR_{L3}-, -S-, #-(CH₂)ₙ-O-*, #-(CH₂)ₙ-NR_{L3}-*, #-(CH₂)ₙ-S-*, vinylene, propenylene, ethynylene and propynylene, wherein the end indicated by "#" is attached to R₂, the end indicated by "*" is attached to 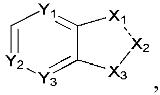 n is selected from 1 and 2, and R_{L3} is as defined in any of claims 1-14; preferably, R_{L3} is selected from H, D, -CH₃, -CH₂CH₃ and - CH(CH₃)₂, further preferably, R_{L3} is H;
further preferably, L₃ is selected from a bond, -O-, -NH-, #-CH₂-NH-*, #-CH₂-S-* and -C≡C-, wherein the end indicated by "#" is attached to R₂, and the end indicated by "*" is attached to 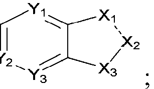
further preferably, L₃ is a bond.

16. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-15, **characterised in that** each R is independently selected from halogen, - OH, -CN, -COOH, oxo, C₁₋₄ alkyl, -C₁₋₄ alkylene-OH, C₁₋₄ alkoxy, -C₁₋₄ alkyleneoxy-C₁₋₄ alkoxy, -C(=O)NH-C₁₋₄ alkyl, -C(=O)-C₁₋₄ alkyl, -NHC(=O)-C₁₋₄ alkyl, -N=S(=O)(C₁₋₄ alkyl)-C₁₋₄ alkyl, -NH-S(=O)₂C₁₋₄ alkyl, -S(=O)₂-C₁₋₄ alkyl, - S(=O)₂-4- to 6-membered heterocyclyl, -C(=O)-4- to 6-membered heterocyclyl, 4-to 6-membered heterocyclyl, 5- to 6-membered heteroaryl and 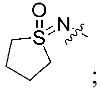
preferably, R is selected from -OH and -C₁₋₄ alkylene-OH;
preferably, R is selected from halogen, -OH, -CN, -COOH, =O, -CH₂OH, methyl, ethyl, propyl, butyl, -ethylene-OH, -propylene-OH, -butylene-OH, methoxy, ethoxy, propoxy, butoxy, -methyleneoxy-methoxy, -methyleneoxy-ethoxy, - methyleneoxy-propoxy, -ethyleneoxy-methoxy, -ethyleneoxy-ethoxy, - ethyleneoxy-propoxy, -C(=O)-NH-methyl, -C(=O)-NH-ethyl, -C(=O)-NH-propyl, - C(=O)-NH-butyl, -N=S(=O)(methyl)-methyl, -N=S(=O)(methyl)-ethyl, - N=S(=O)(methyl)-propyl, -N=S(=O)(methyl)-butyl, -N=S(=O)(ethyl)-ethyl, - N=S(=O)(ethyl)-propyl, -(NH)ₚ-W-methyl, -(NH)ₚ-W-ethyl, -(NH)ₚ-W-propyl, - (NH)ₚ-W-butyl, -(W)_{q}-oxetanyl, -(W)_{q}-azetidinyl, -(W)_{q}-tetrahydrofuryl, -(W)_{q}-pyrrolidyl, -(W)_{q}-tetrahydropyranyl, -(W)_{q}-piperidyl, -(W)_{q}-morpholinyl, thienyl, furyl, pyrrolyl, thiazolyl, oxazolyl, pyridyl, pyrimidyl, pyrazinyl and 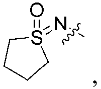 wherein W is C(=O) or S(=O)₂, and each p or q is independently 0 or 1;
further preferably, R is selected from F, -OH, -CN, -COOH, =O, -CH₃, -CH₂OH, -C(CH₃)₂OH, -CH₂C(CH₃)₂OH, -OCH₃, -OCH₂CH₂OCH₃, -C(=O)CH₃, - C(=O)NHCH₃, -NHC(=O)CH₃, -N=S(=O)(CH₃)₂, -NH-S(=O)₂CH₃, -S(=O)₂CH₃,-S(=O)₂-piperidyl (such as 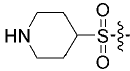 ), -C(=O)-pyrrolidyl (such as 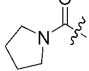 C(=O)-morpholinyl (such as 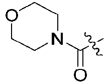 ), morpholinyl (such as 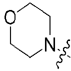 and 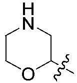 ), pyrazolyl (such as 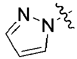 ) and 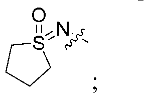 further preferably, R is selected from -OH and -CH₂OH.

17. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-16, **characterised in that** R₂ₐ, R_{2b}, R_{2c}, R_{2d} and R₂ₑ are each independently selected from H, halogen, -OH, C₁₋₄ alkyl, C₁₋₄ haloalkyl, -NHC₁₋₄ alkyl, -N(C₁₋₄ alkyl)-C₁₋₄ alkyl, C₁₋₄ alkoxy, C₁₋₄ haloalkoxy, C₃₋₆ cycloalkyl, C₃₋₆ cycloalkenyl, 4-to 6-membered heterocyclyl, phenyl, 5- to 6-membered heteroaryl, -phenylene-4- to 6-membered heterocyclyl, -phenylene-5- to 6-membered heteroaryl, -phenylene-phenyl and -5- to 6-membered heteroarylene-5- to 6-membered heteroaryl, and the C₁₋₄ alkyl, C₁₋₄ haloalkyl, -NHC₁₋₄ alkyl, -N(C₁₋₄ alkyl)-C₁₋₄ alkyl, C₁₋₄ alkoxy, C₁₋₄ haloalkoxy, C₃₋₆ cycloalkyl, C₃₋₆ cycloalkenyl, 4- to 6-membered heterocyclyl, phenyl, 5- to 6-membered heteroaryl, -phenylene-4- to 6-membered heterocyclyl, - phenylene-5- to 6-membered heteroaryl, -phenylene-phenyl and -5- to 6-membered heteroarylene-5- to 6-membered heteroaryl are optionally substituted with 1, 2 or 3 R;
preferably, R₂ₐ, R_{2b}, R_{2c}, R_{2d} and R₂ₑ are each independently selected from H, C₁₋₄ alkyl, C₁₋₄ alkoxy, C₃₋₆ cycloalkyl, 4- to 6-membered heterocyclyl, phenyl, and 5- to 6-membered heteroaryl; the C₁₋₄ alkyl, C₁₋₄ alkoxy, C₃₋₆ cycloalkyl, 4- to 6-membered heterocyclyl, phenyl, and 5- to 6-membered heteroaryl are optionally substituted with 1, 2 or 3 R;
preferably, R₂ₐ, R_{2b}, R_{2c}, R_{2d} and R₂ₑ are each independently selected from H, C₁₋₄ alkyl, C₁₋₄ alkoxy, C₃₋₆ cycloalkyl, and phenyl; the C₁₋₄ alkyl, C₁₋₄ alkoxy, C₃₋₆ cycloalkyl, and phenyl are optionally substituted with 1, 2 or 3 R;
preferably, R₂ₐ, R_{2b}, R_{2c}, R_{2d} and R₂ₑ are each independently selected from H, C₁₋₄ alkyl, C₃₋₆ cycloalkyl, and phenyl; the C₁₋₄ alkyl, C₃₋₆ cycloalkyl, and phenyl are optionally substituted with 1, 2 or 3 R;
preferably, R₂ₐ, R_{2b}, R_{2c}, R_{2d} and R₂ₑ are each independently selected from H, F, Cl, Br, -OH, methyl, ethyl, propyl, butyl, halomethyl, haloethyl, halopropyl, halobutyl, -NH-methyl, -NH-ethyl, -NH-propyl, -NH-butyl, -N(CH₃)₂, - N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, -(phenylene)ᵣ-oxetanyl, -(phenylene)ᵣ-azetidinyl, -(phenylene)ᵣ-tetrahydrofuryl, -(phenylene)ᵣ-pyrrolidyl, -(phenylene)ᵣ-tetrahydropyranyl, -(phenylene)ᵣ-piperidyl, -(phenylene)ᵣ-morpholinyl, - (phenylene)ᵣ-oxopyrrolidyl, -(phenylene)ᵣ-oxoimidazolyl, -(phenylene)ᵣ-oxopyrazolyl, -(phenylene)ᵣ-oxodihydrotriazolyl, -(phenylene)ᵣ-phenyl, -(V)ᵣ-thienyl, -(V)ᵣ-furyl, -(V)ᵣ-pyrrolyl, -(V)ᵣ-imidazolyl, -(V)ᵣ-pyrazolyl, -(V)ᵣ-thiazolyl, -(V)ᵣ-oxazolyl, -(V)ᵣ-oxadiazolyl, -(V)ᵣ-triazolyl, -(V)ᵣ-tetrazolyl, -(V)ᵣ-pyridyl, - (V)ᵣ-pyrimidyl and -(V)ᵣ-pyrazinyl, and the methyl, ethyl, propyl, butyl, halomethyl, haloethyl, halopropyl, halobutyl, -NH-methyl, -NH-ethyl, -NH-propyl, -NH-butyl, - N(CH₃)₂, -N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, -(phenylene)ᵣ-oxetanyl, - (phenylene)ᵣ-azetidinyl, -(phenylene)ᵣ-tetrahydrofuryl, -(phenylene)ᵣ-pyrrolidyl, - (phenylene)ᵣ-tetrahydropyranyl, -(phenylene)ᵣ-piperidyl, -(phenylene)ᵣ-morpholinyl, -(phenylene)ᵣ-oxopyrrolidyl, -(phenylene)ᵣ-oxoimidazolyl, -(phenylene)ᵣ-oxopyrazolyl, -(phenylene)ᵣ-oxodihydrotriazolyl, -(phenylene)ᵣ-phenyl, -(V)ᵣ-thienyl, -(V)ᵣ-furyl, -(V)ᵣ-pyrrolyl, -(V)ᵣ-imidazolyl, -(V)ᵣ-pyrazolyl, -(V)ᵣ-thiazolyl, -(V)ᵣ-oxazolyl, -(V)ᵣ-oxadiazolyl, -(V)ᵣ-triazolyl, -(V)ᵣ-tetrazolyl, -(V)ᵣ-pyridyl, - (V)ᵣ-pyrimidyl and -(V)ᵣ-pyrazinyl are optionally substituted with 1, 2 or 3 R, wherein V is selected from phenylene and pyridylene, and r is 0 or 1;
further preferably, R₂ₐ, R_{2b}, R_{2c}, R_{2d} and R₂ₑ are each independently selected from H, F, -OH, -CH₃, -CH₂CH₃, -CH₂CH₂CH₃, -CH(CH₃)₂, -C(CH₃)₃, -N(CH₃)₂, - OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, morpholinyl, tetrahydrofuryl, pyrrolidyl, tetrahydropyranyl, piperidyl, phenyl, pyridyl, -phenylene-oxetanyl, -phenylene-morpholinyl, -phenylene-oxopyrrolidyl, -phenylene-oxoimidazolyl, -phenylene-oxopyrazolyl, -phenylene-oxodihydrotriazolyl, -phenylene-phenyl, -phenylene-imidazolyl, -phenylene-pyrazolyl, -phenylene-oxadiazolyl, -phenylene-triazolyl, - phenylene-tetrazolyl, -phenylene-pyrimidyl and -pyridylene-pyrazolyl, and the - CH₃, -CH₂CH₃, -CH₂CH₂CH₃, -CH(CH₃)₂, -C(CH₃)₃, -N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, morpholinyl, tetrahydrofuryl, pyrrolidyl, tetrahydropyranyl, piperidyl, phenyl, pyridyl, -phenylene-oxetanyl, -phenylene-morpholinyl, - phenylene-oxopyrrolidyl, -phenylene-oxoimidazolyl, -phenylene-oxopyrazolyl, - phenylene-oxodihydrotriazolyl, -phenylene-phenyl, -phenylene-imidazolyl, - phenylene-pyrazolyl, -phenylene-oxadiazolyl, -phenylene-triazolyl, -phenylene-tetrazolyl, -phenylene-pyrimidyl and -pyridylene-pyrazolyl are optionally substituted with 1, 2 or 3 R;
preferably, R₂ₐ, R_{2b}, R_{2c}, R_{2d} and R₂ₑ are each independently selected from H, methyl, methoxy, cyclopropyl, cyclobutyl, and phenyl, and the methyl, methoxy, cyclopropyl, cyclobutyl, and phenyl are optionally substituted with 1, 2 or 3 R;
preferably, R₂ₐ, R_{2b}, R_{2c}, R_{2d} and R₂ₑ are each independently selected from H, methyl, cyclopropyl, cyclobutyl, and phenyl, and the methyl, cyclopropyl, cyclobutyl, and phenyl are optionally substituted with 1, 2 or 3 R.

18. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-17, **characterised in that** R₂ is selected from C₃₋₆ cycloalkyl, phenyl, 5- to 6-membered heteroaryl, 9-membered heteroaryl, 9- to 10-membered heterocyclyl and C₉₋₁₀ cyclic group, and the C₃₋₆ cycloalkyl, phenyl, 5- to 6-membered heteroaryl, 9-membered heteroaryl, 9- to 10-membered heterocyclyl and C₉₋₁₀ cyclic group are optionally substituted with 1, 2, 3, 4, or 5 groups each independently selected from R₂ₐ, R_{2b}, R_{2c}, R_{2d} and R₂ₑ;
preferably, R₂ is selected from phenyl and 9-membered heteroaryl, and the phenyl and 9-membered heteroaryl are optionally substituted with 1, 2, 3, 4, or 5 groups each independently selected from R₂ₐ, R_{2b}, R_{2c}, R_{2d} and R₂ₑ; preferably, R₂ is selected from cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, 2,3-dihydrobenzofuryl, 2,3-dihydrobenzodioxanyl and 2,3-dihydroindenyl, and the cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, 2,3-dihydrobenzofuryl, 2,3-dihydrobenzodioxanyl and 2,3-dihydroindenyl are optionally substituted with 1, 2, 3, 4, or 5 groups each independently selected from R₂ₐ, R_{2b}, R_{2c}, R_{2d} and R₂ₑ;
preferably, R₂ is selected from phenyl and indolyl, and the phenyl and indolyl are optionally substituted with 1, 2, 3, 4, or 5 groups each independently selected from R₂ₐ, R_{2b}, R_{2c}, R_{2d} and R₂ₑ;
further preferably, R₂ is selected from cyclopropyl, cyclobutyl, phenyl, thienyl, thiazolyl, oxazolyl, pyridyl, indolyl, indolinyl, 2,3-dihydrobenzofuryl, 2,3-dihydrobenzodioxanyl and 2,3-dihydroindenyl, and the cyclopropyl, cyclobutyl, phenyl, thienyl, thiazolyl, oxazolyl, pyridyl, indolyl, indolinyl, 2,3-dihydrobenzofuryl, 2,3-dihydrobenzodioxanyl and 2,3-dihydroindenyl are optionally substituted with 1, 2, 3, 4, or 5 groups each independently selected from R₂ₐ, R_{2b}, R_{2c}, R_{2d} and R₂ₑ;
further preferably, R₂ is selected from 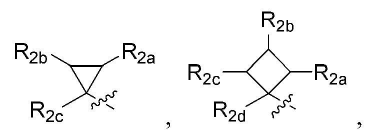 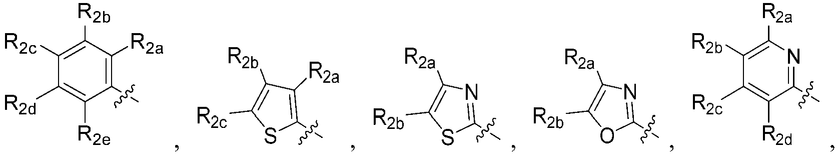 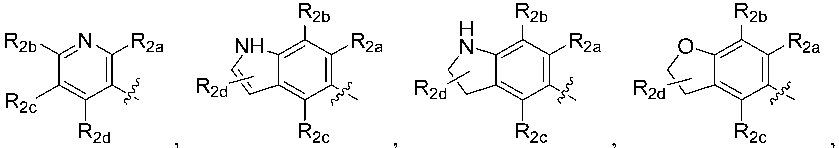 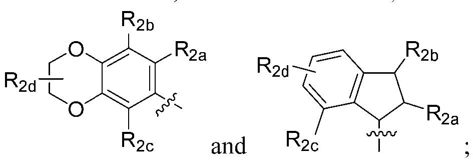
preferably, R₂ is selected from 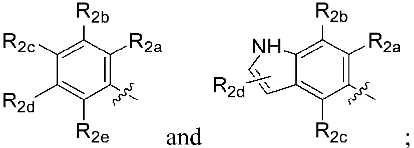
further preferably, the R₂ is selected from 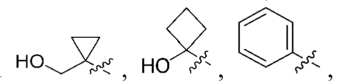 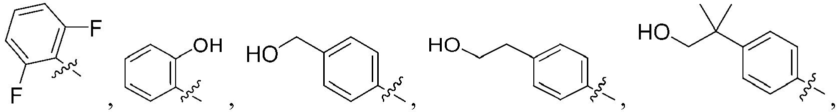 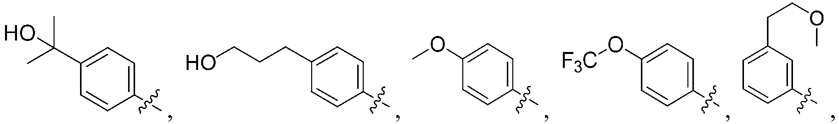 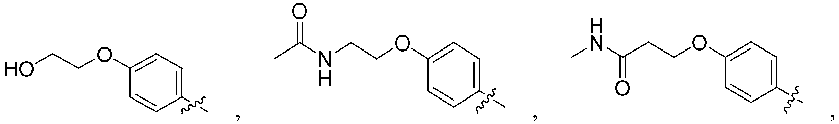 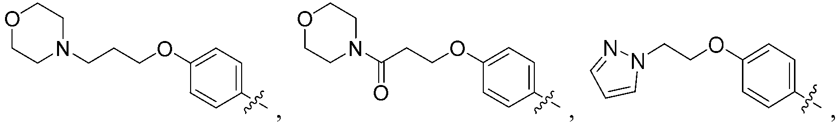 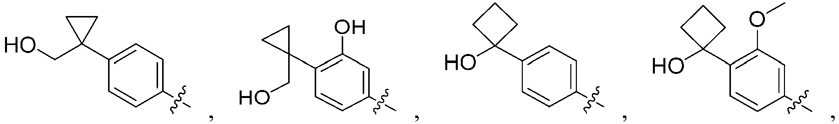 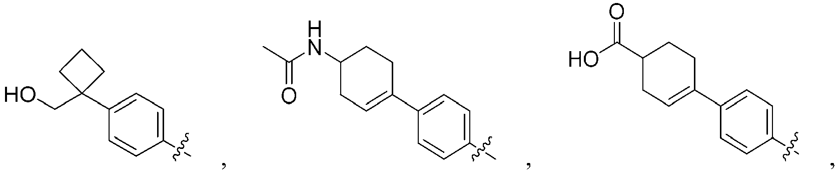 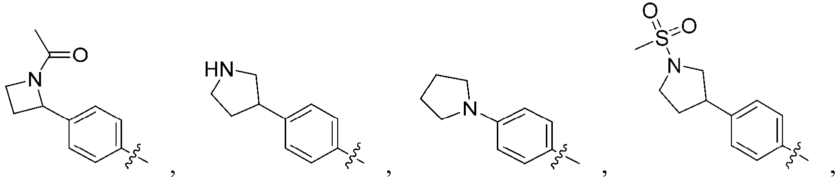 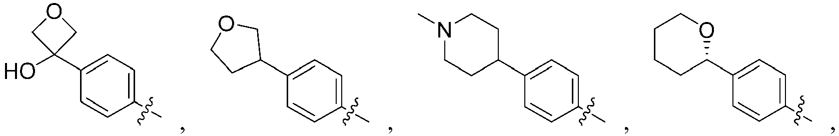 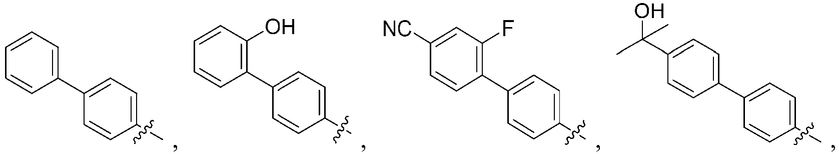 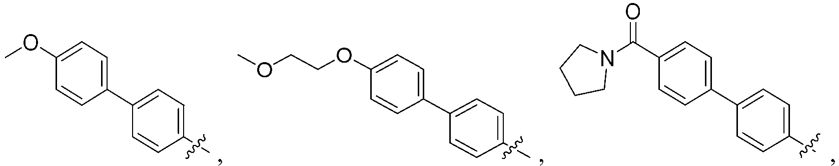 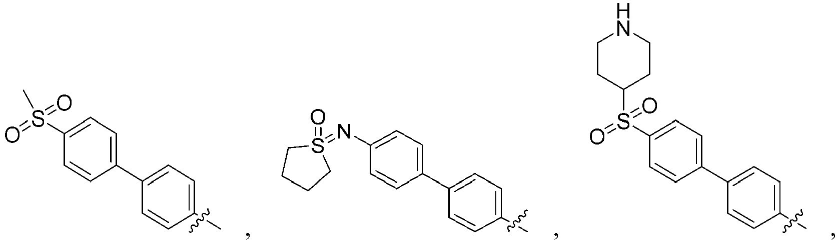 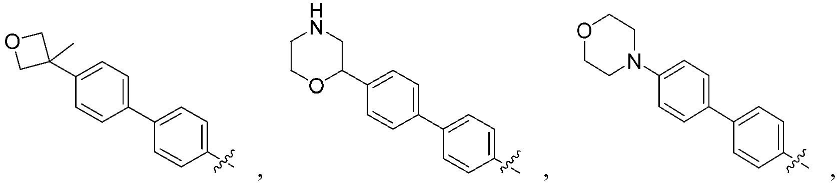 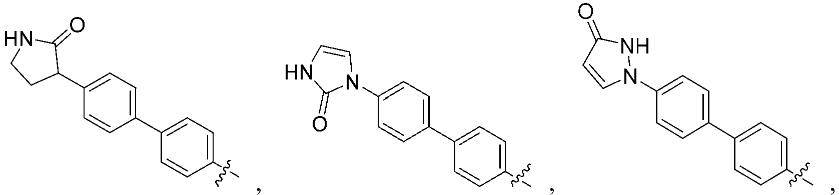 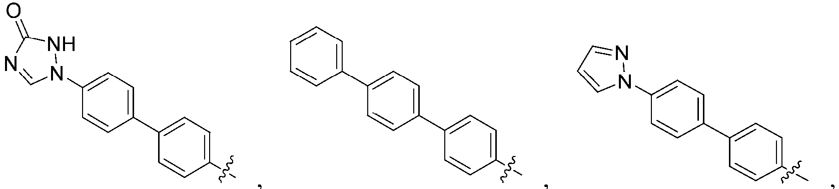 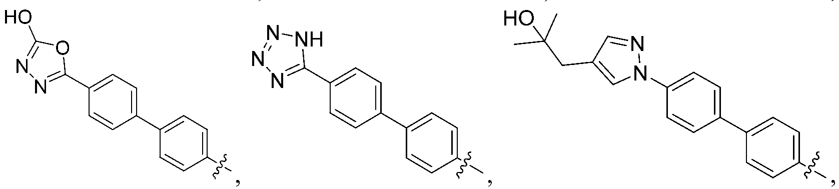 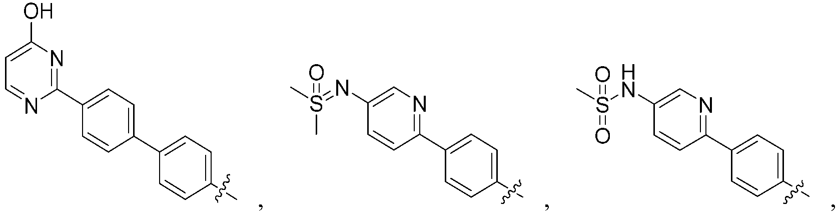 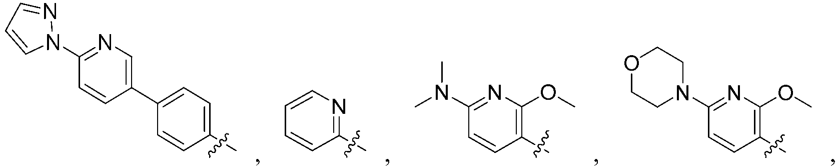 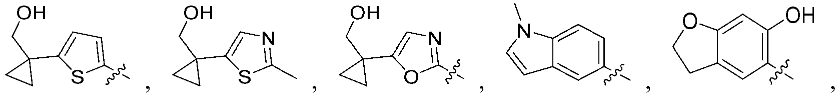 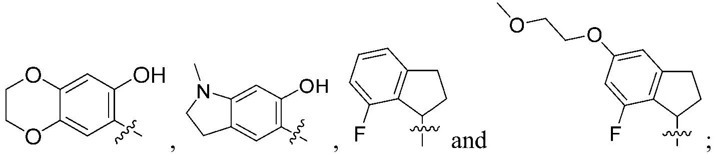
further preferably, the R₂ is selected from 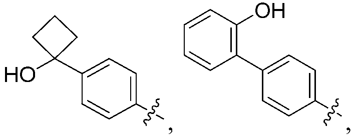 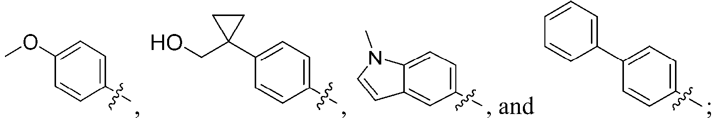
more preferably, the R₂ is selected from

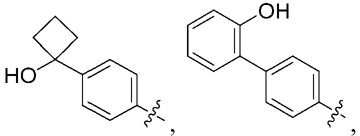

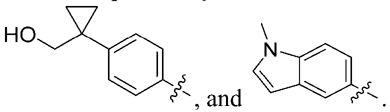

19. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-18, **characterised in that** the compound is selected from formula (I-1) and (I-2), 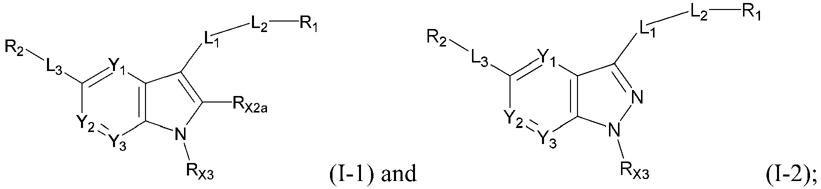
preferably, the compound is selected from formula (I-3), (I-4) and (I-5), 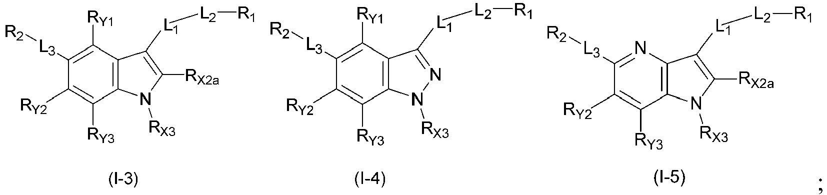
further preferably, the compound is selected from formula (I-6), (I-7), (I-8), (I-9), (I-10), (I-11), (I-12), (I-13), (I-14), (I-15), (I-16), (I-17), (I-18), (I-19), (I-20) and (I-21), 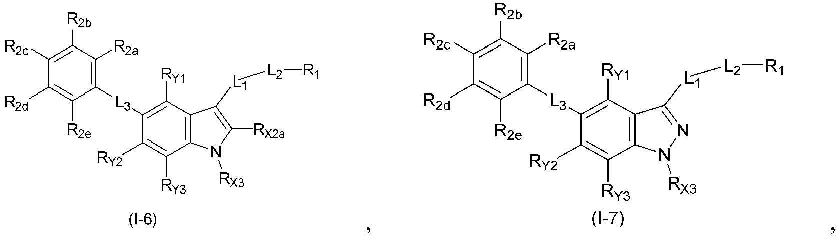 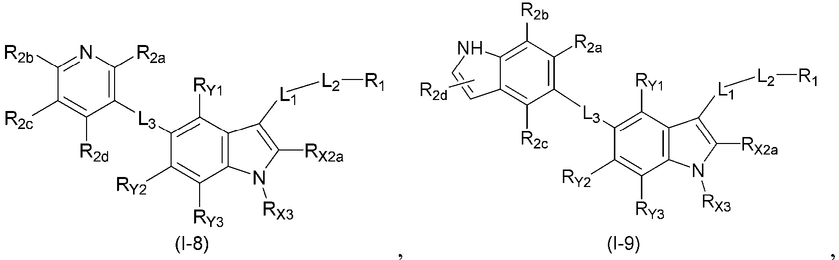 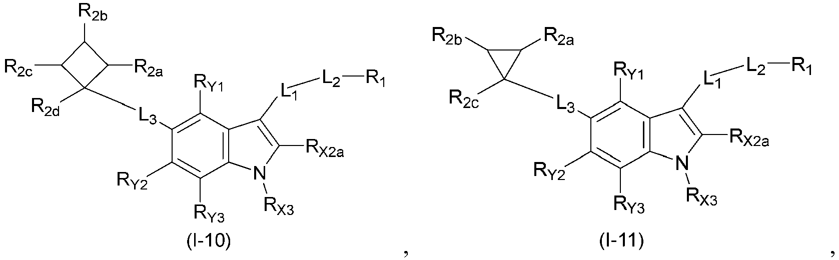 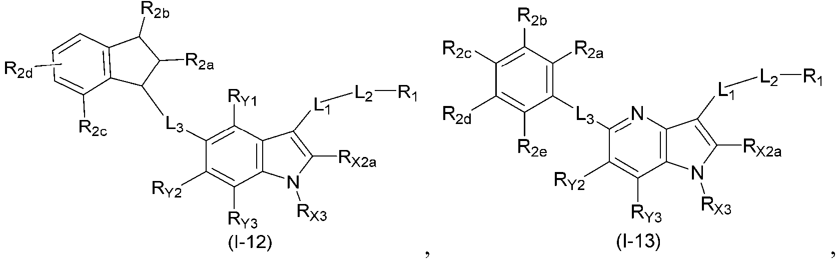 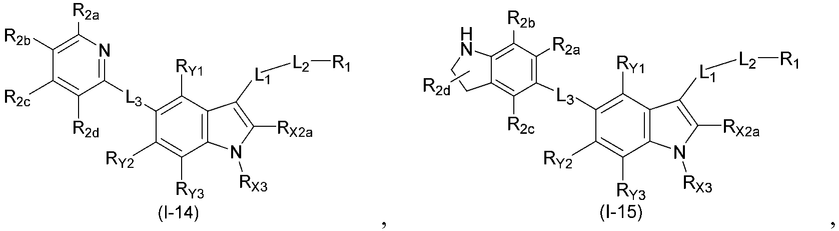 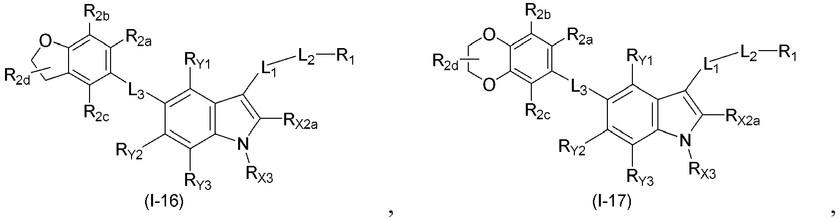 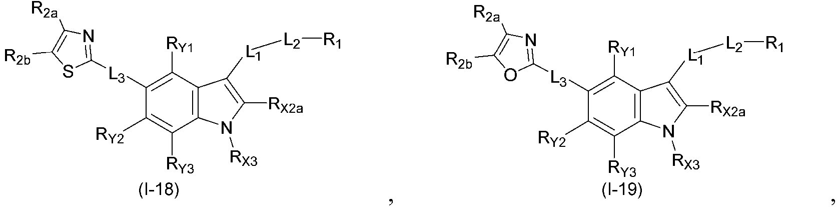 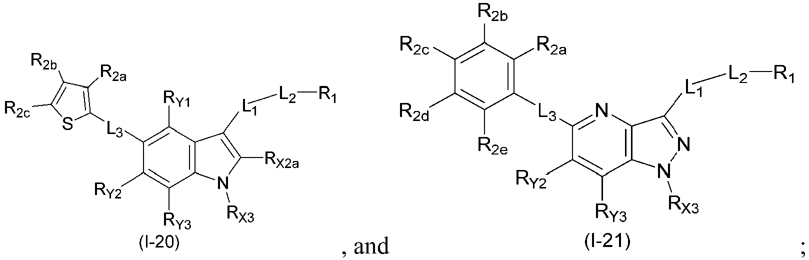
more preferably, the compound is selected from formula (I-A), (I-B) and (I-C), 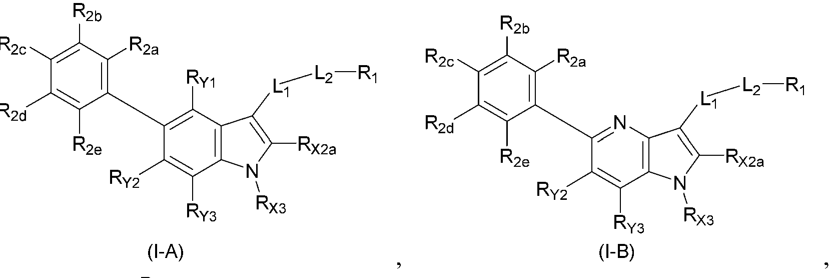 and

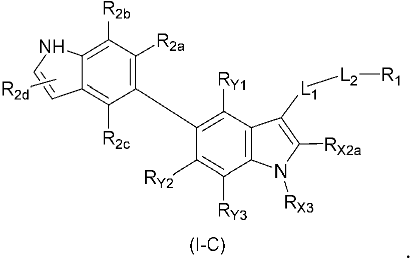

20. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1 to 19, **characterised in that** the compound has a structure as shown in formula (II-1), 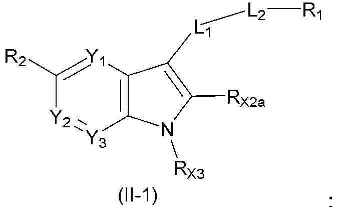
preferably, the compound is selected from formula (II-2') and (II-3), 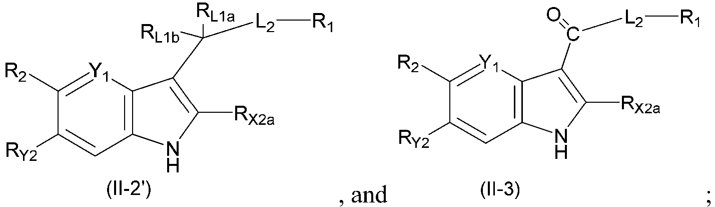
further preferably, the compound is selected from formula (II-4), (II-5), (II-6), (II-7), (II-8) and (II-9),

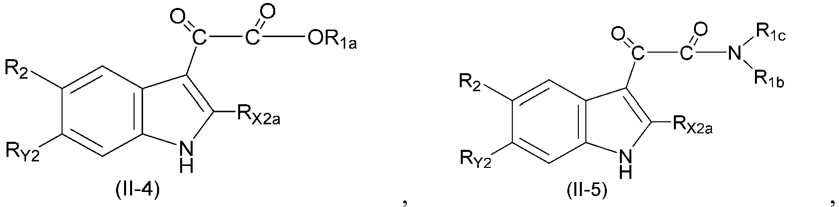

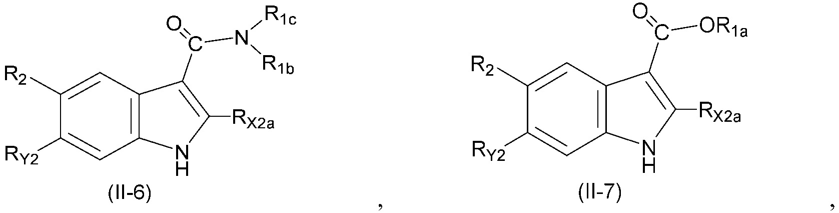

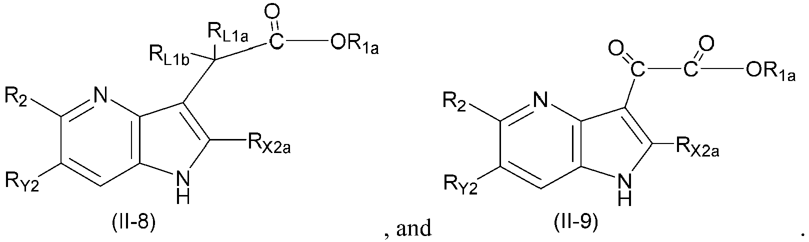

21. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1 to 20, **characterised in that** the compound has a structure as shown in formula (II-6), 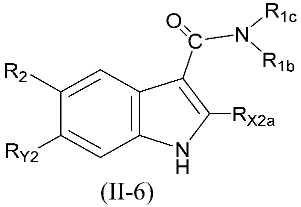
wherein R_{X2a} is selected from H, -OH, and -C₁₋₃ alkoxy;
R_{Y2} is halogen;
R_{1b} is selected from H and C₁₋₃ alkyl;
R_{1c} is selected from C₁₋₃ alkyl, C₃₋₆ cycloalkyl, phenyl, 5- to 6-membered heteroaryl, 4- to 7-membered heterocyclyl, -C₁₋₃ alkylene-C₃₋₆ cycloalkyl, -C₁₋₃ alkylene-phenyl, -C₁₋₃ alkylene-5- to 6-membered heteroaryl, and -C₁₋₃ alkylene-4-to 7-membered heterocyclyl, and the C₁₋₃ alkyl is optionally substituted with carboxyl;
R₂ is selected from phenyl and 5- to 10-membered heteroaryl, wherein the phenyl and 5-10 heteroaryl are optionally substituted with a substituent selected from C₁₋₃ alkyl, C₁₋₃ alkoxy, C₃₋₆ cycloalkyl, and phenyl, and the C₃₋₆ cycloalkyl and phenyl serving as substituents on the phenyl and 5- to 10-membered heteroaryl are optionally substituted with a substituent selected from -OH and -C₁₋₃ alkylene-OH;
preferably, R_{X2a} is selected from H and -OH; more preferably, R_{X2a} is H;
preferably, R_{Y2} is Cl;
preferably, R_{1b} is selected from H and methyl; more preferably, R_{1b} is H;
preferably, R_{1c} is selected from C₁₋₃ alkyl, C₃₋₆ cycloalkyl, phenyl, 5- to 6-membered heteroaryl, -C₁₋₃ alkylene-phenyl, -C₁₋₃ alkylene-5- to 6-membered heteroaryl, and -C₁₋₃ alkylene-4- to 7-membered heterocyclyl, and the C₁₋₃ alkyl is optionally substituted with carboxyl;
preferably, R_{1c} is selected from C₁₋₃ alkyl, 5- to 6-membered heteroaryl, -C₁₋₃ alkylene-5- to 6-membered heteroaryl, and -C₁₋₃ alkylene-4- to 7-membered heterocyclyl, and the C₁₋₃ alkyl is optionally substituted with carboxyl;
preferably, R_{1c} is selected from CH₂C(=O)OH, pyrazolyl (such as 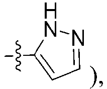 imidazolyl (such as 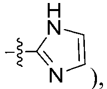 oxazolyl (such as 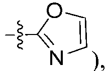 -C₁₋₃ alkylene-pyridyl (such as 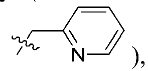 -C₁₋₃ alkylene-furyl (such as 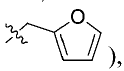 -C₁₋₃ alkylene- thienyl (such as 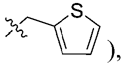 -C₁₋₃ alkylene-oxazolyl (such as 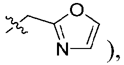 -C₁₋₃ alkylene-isoxazolyl (such as 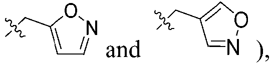 -C₁₋₃ alkylene-tetrazolyl (such as 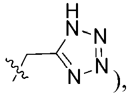 and -C₁₋₃ alkylene-oxetanyl (such as 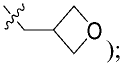
preferably, R₂ is selected from phenyl and 9-membered heteroaryl (such as indolyl), wherein the phenyl and 9-membered heteroaryl are optionally substituted with a substituent selected from C₁₋₃ alkyl, C₁₋₃ alkoxy, C₃₋₆ cycloalkyl, and phenyl, and the C₃₋₆ cycloalkyl and phenyl serving as substituents on the phenyl and 9-membered heteroaryl are optionally substituted with a substituent selected from -OH and -C₁₋₃ alkylene-OH;
further preferably, R₂ is phenyl, the phenyl is substituted with a substituent selected from C₁₋₃ alkyl, C₁₋₃ alkoxy, C₃₋₆ cycloalkyl, and phenyl, and the C₃₋₆ cycloalkyl and phenyl serving as substituents on the phenyl are optionally substituted with a substituent selected from -OH and -C₁₋₃ alkylene-OH;
more preferably, R₂ is selected from 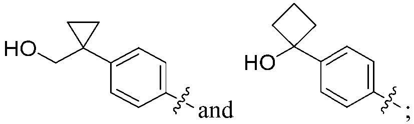
further preferably, R₂ is indolyl, and the indolyl is substituted with C₁₋₃ alkyl;
more preferably, R₂ is

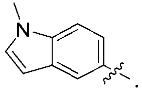

22. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1 to 21, **characterised in that** the compound has a structure as shown in formula (V-1), 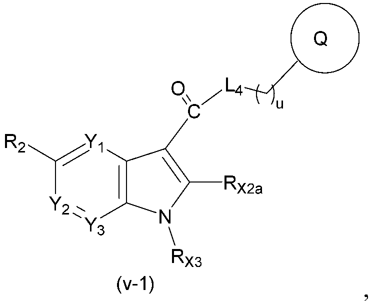
wherein L₄ is selected from -O- and -NR_{1b}-, preferably, L₄ is -NR_{1b}-;
u is selected from 0, 1 and 2, preferably, u is selected from 0 and 1;
Q is selected from C₃₋₁₀ cycloalkyl, 4- to 10-membered heterocyclyl, 5- to 10-membered heteroaryl, and C₆₋₁₀ aryl, and the C₃₋₁₀ cycloalkyl, 4- to 10-membered heterocyclyl, 5- to 10-membered heteroaryl, and C₆₋₁₀ aryl are optionally substituted with one or more substituents selected from oxo, hydroxyl, cyano, carboxyl, halogen, C₁₋₆ alkyl, C₁₋₆ haloalkyl, C₁₋₆ alkoxy, C₁₋₆ haloalkoxy, C₃₋₆ cycloalkyl, 4- to 7-membered heterocyclyl, C₆₋₁₀ aryl, and 5- to 10-membered heteroaryl;
preferably, Q is selected from C₃₋₆ cycloalkyl, 4- to 6-membered heterocyclyl, 5- to 6-membered heteroaryl, and phenyl;
more preferably, Q is selected from 4- to 6-membered heterocyclyl and 5- to 6-membered heteroaryl;
preferably, the compound has a structure as shown in formula (V-2) or (V-3), 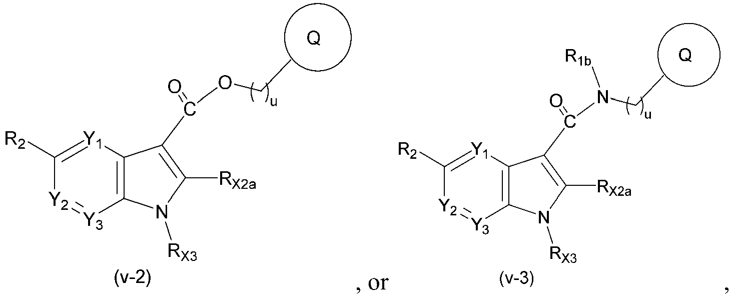
more preferably, the compound has a structure as shown in formula (V-4),

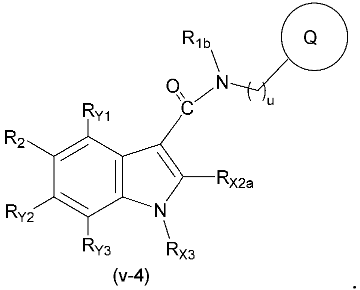

23. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-22, **characterised in that** the compound is formula (VI-1),

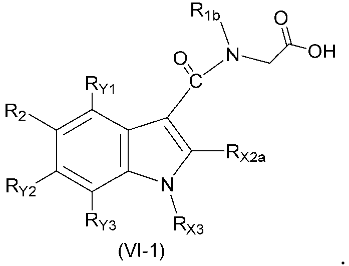

24. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-23, **characterised in that** the compound is selected from formula (III-1) and (III-2), 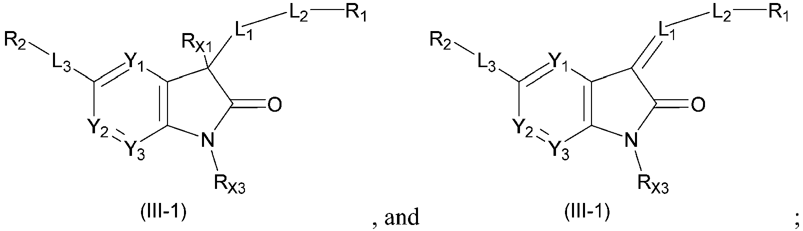
preferably, the compound is selected from formula (III-3) and (III-4), 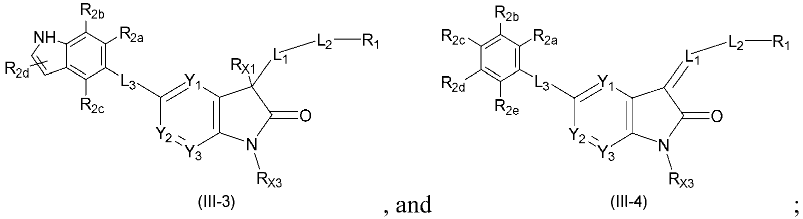
further preferably, the compound is selected from formula (III-5) and (III-6),

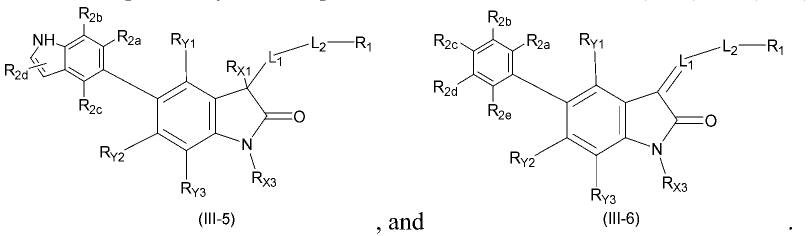

25. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1 to 24, provided that:
1) when structural unit 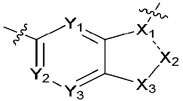 is 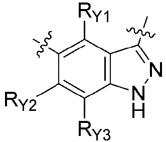 or 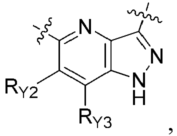 -L₁-L₂-R₁ is not -C(=O)-C(=O)-OH; or
2) when structural unit 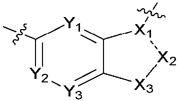 is 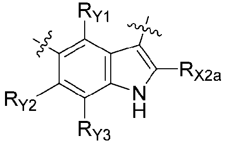 or 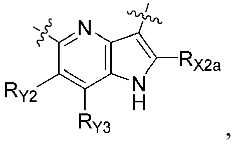 R_{X2a} is not H; or
3) when structural unit 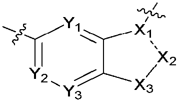 is 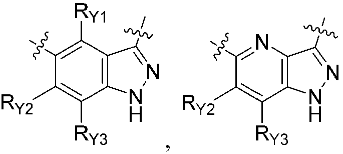 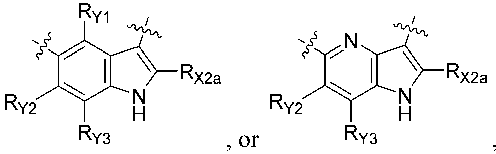 R_{Y2} is not H, halogen, C₁₋₃ alkyl, or -CF₃;
4) when -L₁-L₂-R₁ is -C(=O)-C(=O)-OH, L₃ is not a bond; or
5) when -L₁-L₂-R₁ is -C(=O)-C(=O)-OH, R₂ is not phenyl or 5-membered heteroaryl, and the phenyl or 5-membered heteroaryl is optionally substituted with 1, 2, 3, 4, or 5 groups each independently selected from R₂ₐ, R_{2b}, R_{2c}, R_{2d} and R₂ₑ;
wherein L₁, L₂, L₃, R₁, R₂, R_{Y1}, R_{Y2}, R_{Y3}, R_{X2a}, R₂ₐ, R_{2b}, R_{2c}, R_{2d} and R₂ₑ are as defined in any of claims 1-21;
preferably, provided that:
1) when structural unit 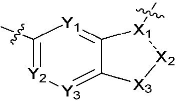 is 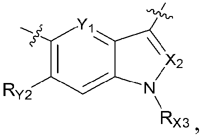 -L₁-L₂-R₁ is not -C(=O)-C(=O)-OH; or
2) when structural unit 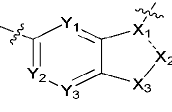 is 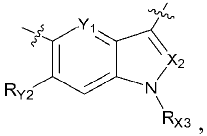 and X₂ is CR_{X2a}, R_{X2a} is not H;
3) when structural unit 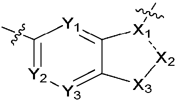 is 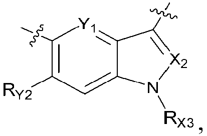 R_{Y2} is not H, halogen, C₁₋₃ alkyl, or -CF₃;
4) when -L₁-L₂-R₁ is -C(=O)-C(=O)-OH, L₃ is not a bond; or
5) when -L₁-L₂-R₁ is -C(=O)-C(=O)-OH, R₂ is not phenyl or 5-membered heteroaryl, and the phenyl or 5-membered heteroaryl is optionally substituted with 1, 2, 3, 4, or 5 groups each independently selected from R₂ₐ, R_{2b}, R_{2c}, R_{2d} and R₂ₑ;
wherein X₂, Y₁, L₁, L₂, L₃, R₁, R₂, R_{X3}, R₂ₐ, R_{2b}, R_{2c}, R_{2d} and R₂ₑ are as defined in any of claims 1-21;
preferably, provided that:
1) -L₁-L₂-R₁ is not -COOR₁ₐ; or
2) -L₁-L₂-R₁ is not -CR_{L1a}R_{L1b}-COOR₁ₐ; or
3) -L₁-L₂-R₁ is not -CO-CO-OR₁ₐ; or
4) -L₁-L₂-R₁ is not -CO-CO-NR_{1b}R_{1c};
preferably, provided that:
the compound is not 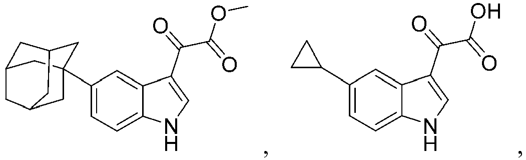 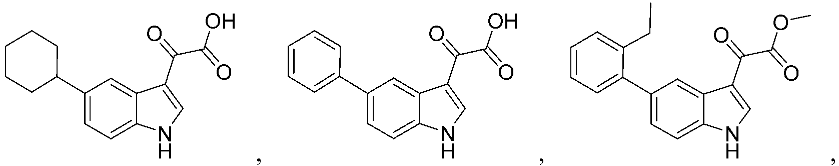 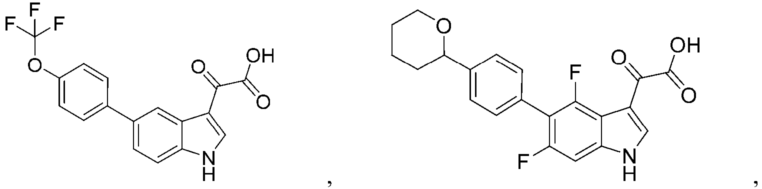 or

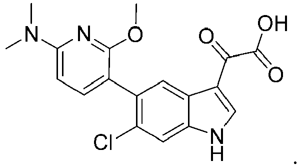

26. The compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-25, **characterised in that** the compound is selected from: 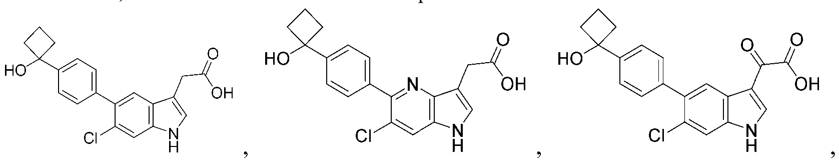 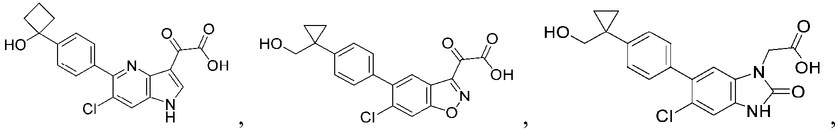 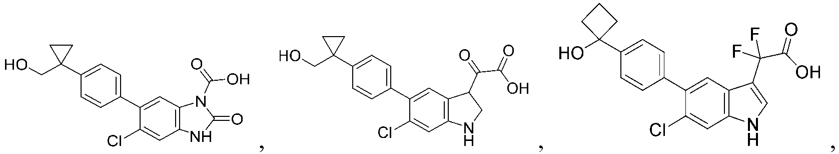 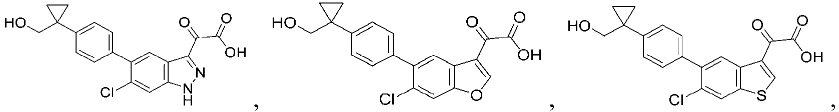 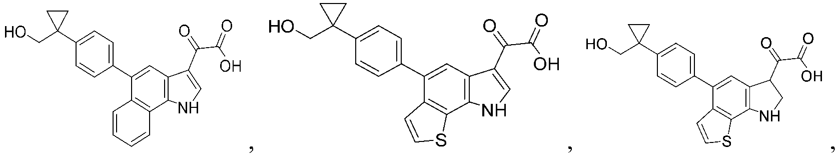 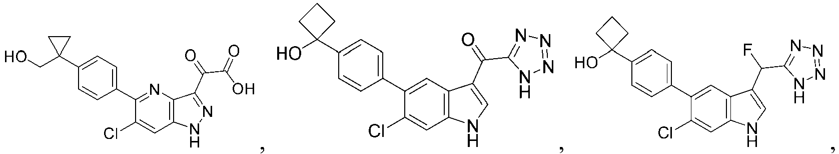 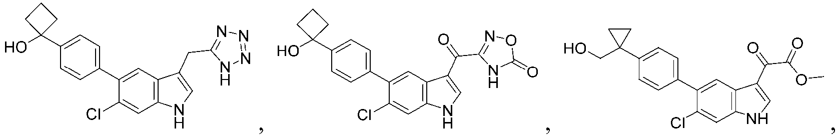 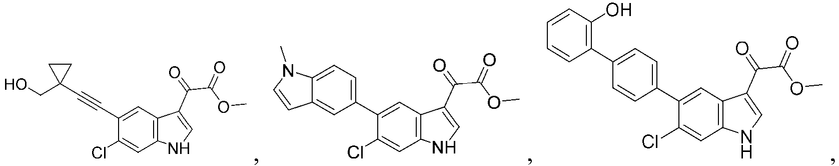 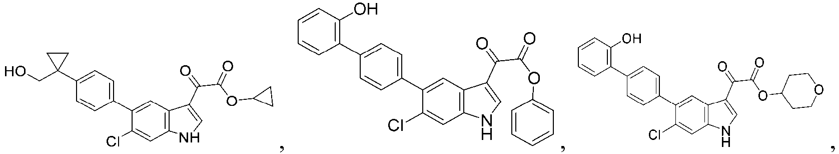 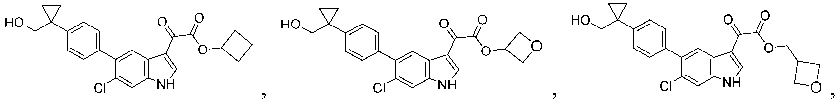 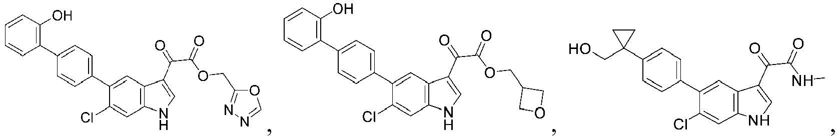 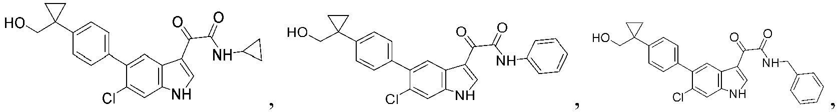 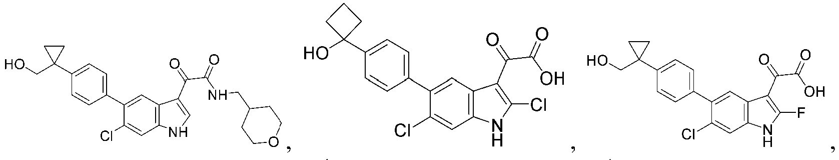 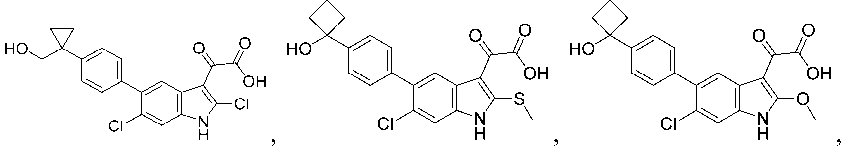 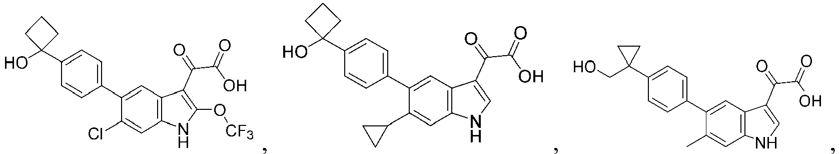 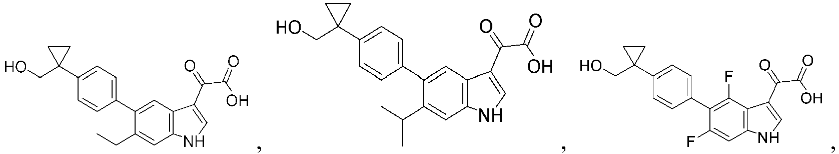 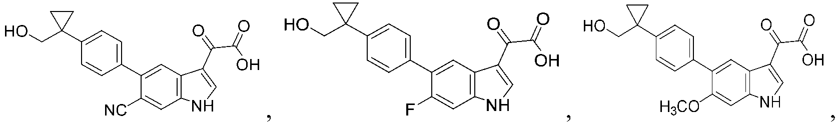 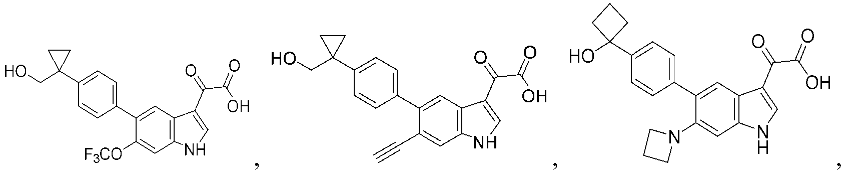 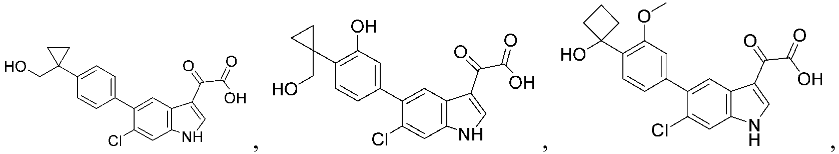 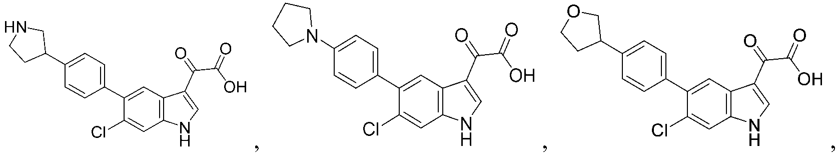 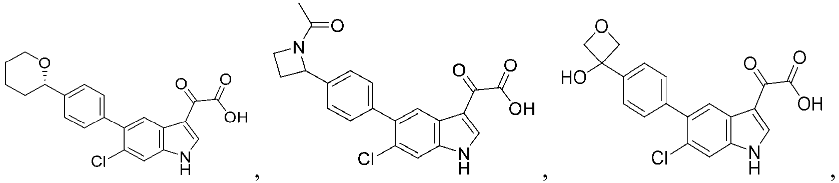 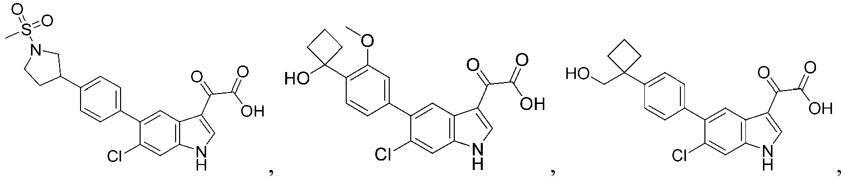 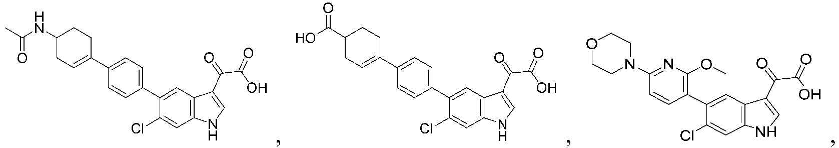 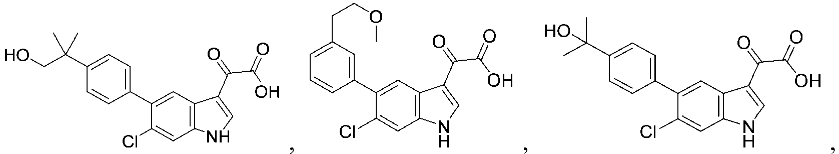 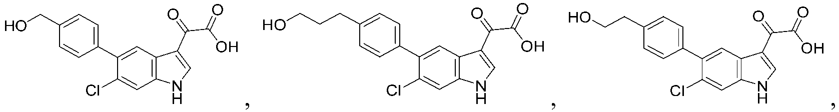 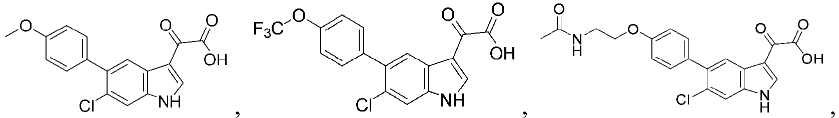 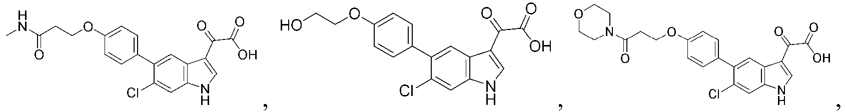 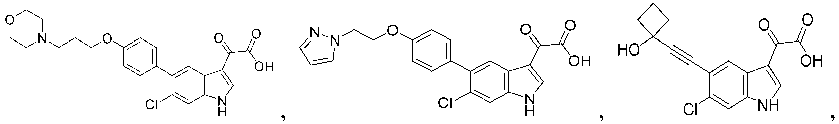 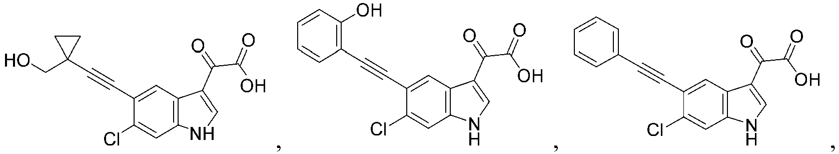 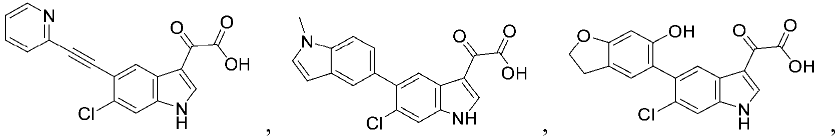 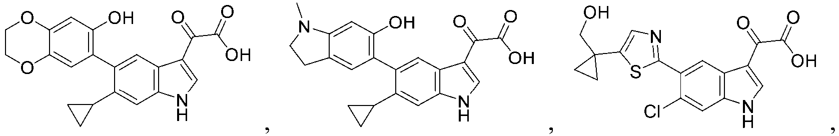 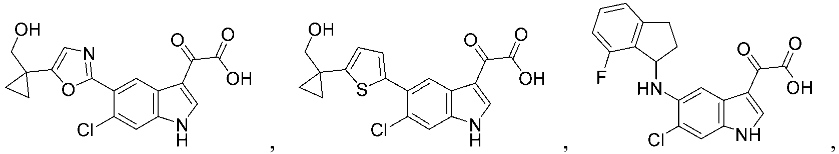 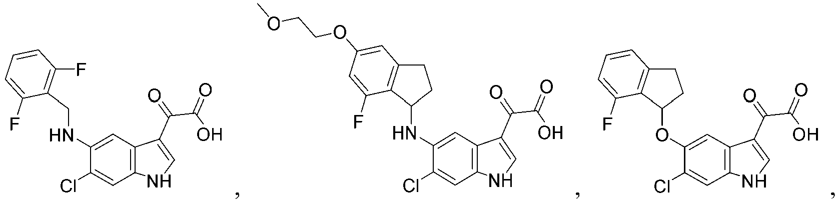 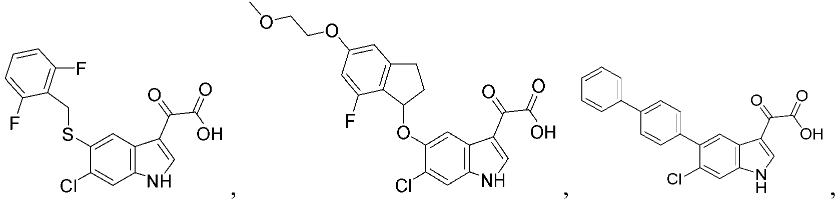 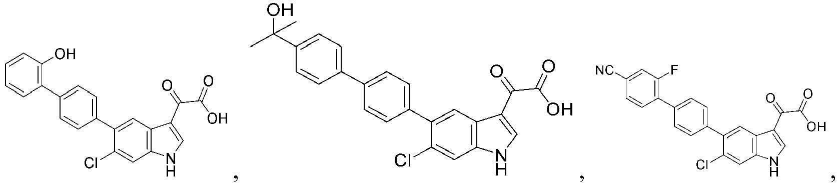 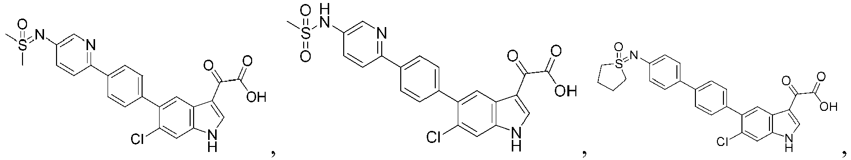 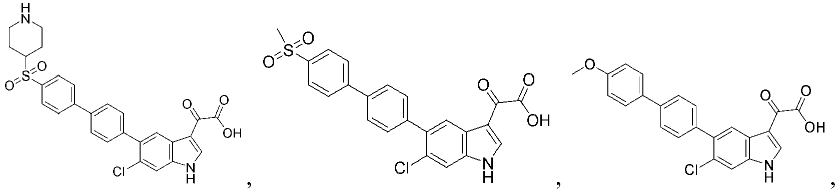 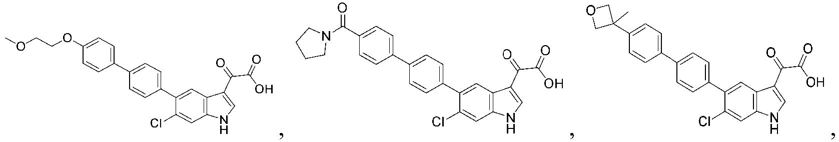 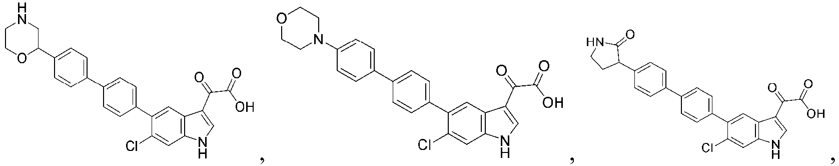 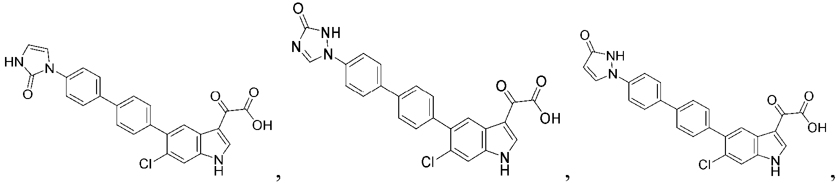 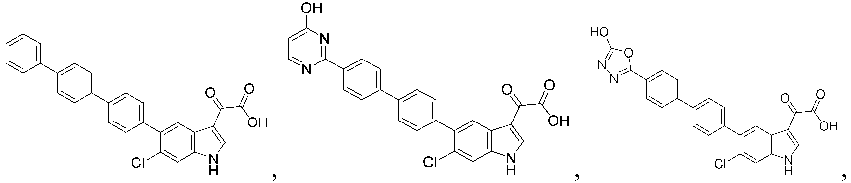 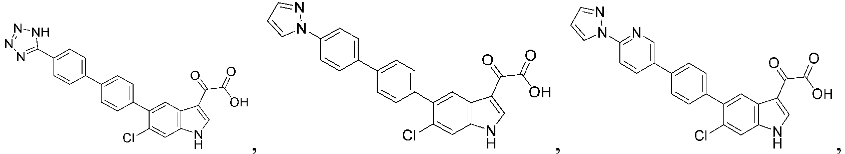 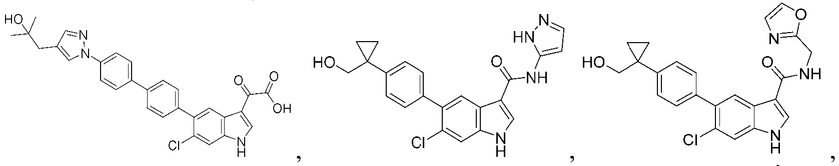 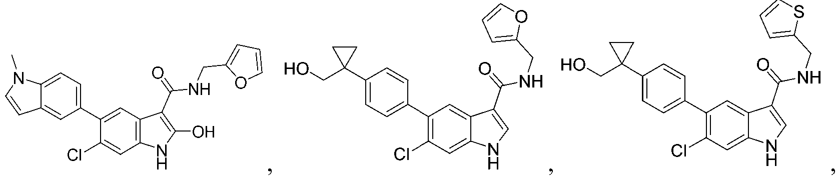 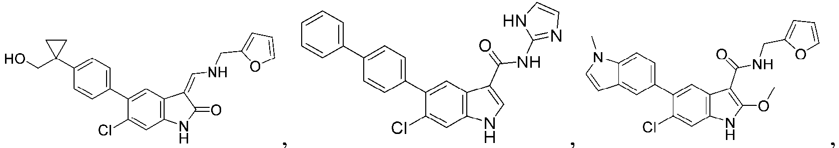 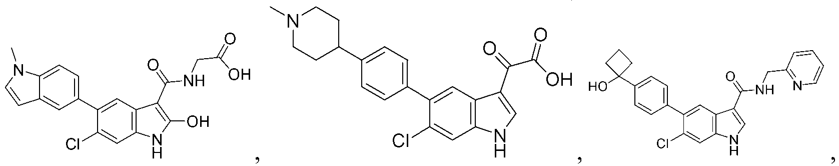 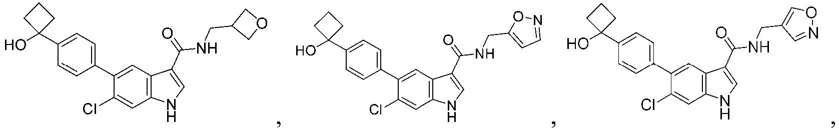 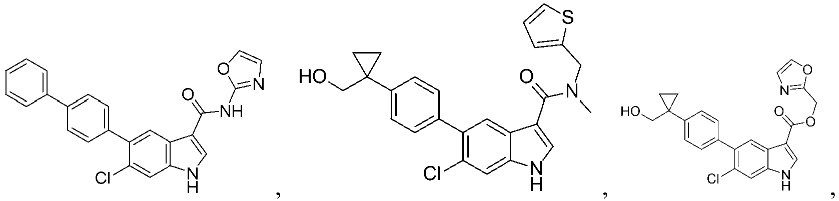 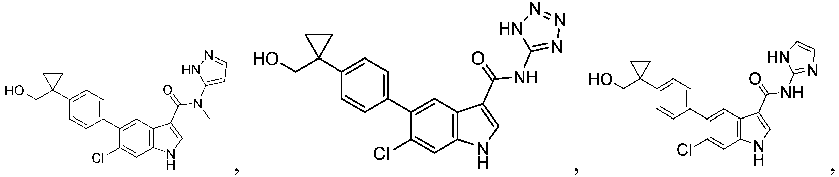 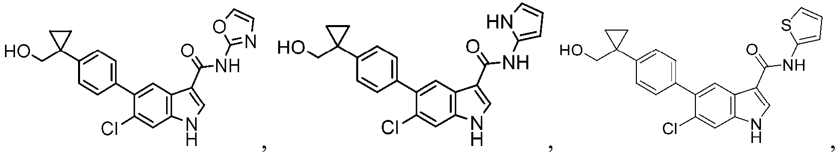 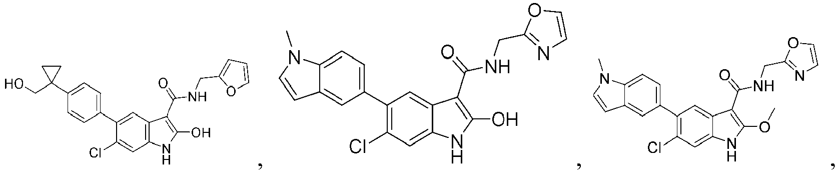 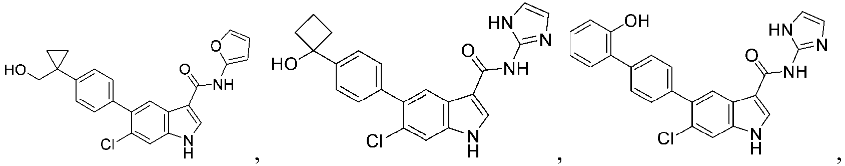 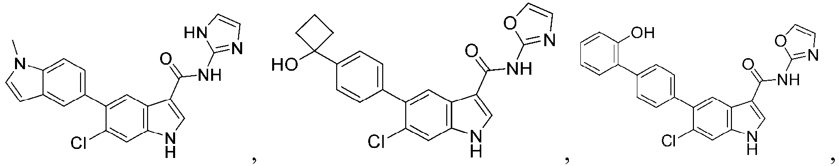 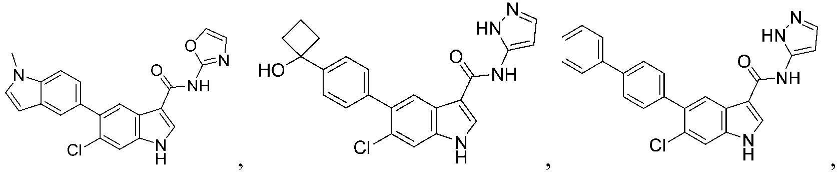 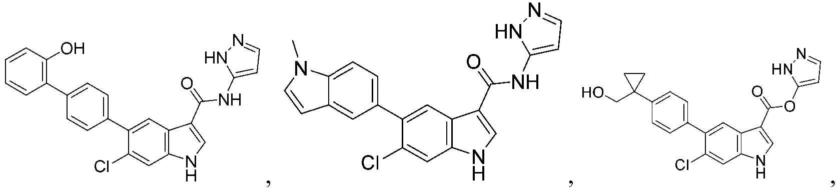 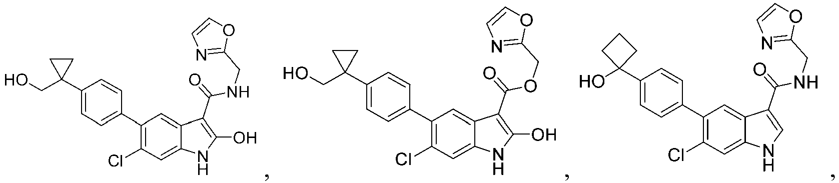 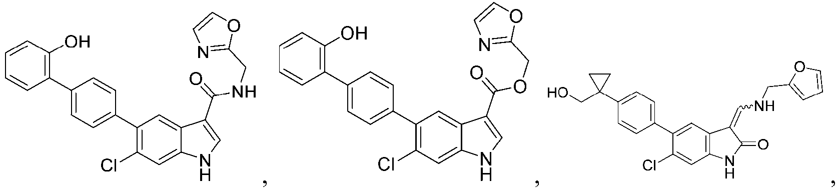 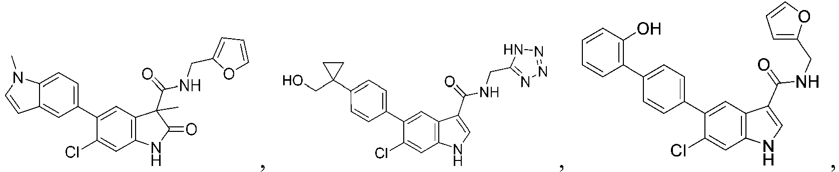 and

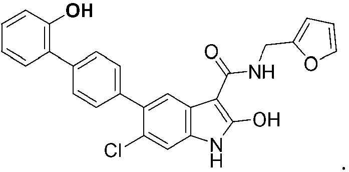

27. A pharmaceutical composition, **characterised by** comprising the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-26, and one or more pharmaceutically acceptable excipients or carriers.

28. Use of the compound, or the enantiomer, the diastereomer, the racemate, the stereoisomer, the geometric isomer, the tautomer, the polymorph, the solvate, the hydrate, the N-oxide, the isotopically labelled compound, the metabolite, the ester, the prodrug or the pharmaceutically acceptable salt thereof according to any of claims 1-26, or the composition according to claim 27 in the preparation of a medicament as an AMPK agonist; or use thereof in the preparation of a medicament for treating and/or preventing type II diabetes, dyslipidaemia, obesity, chronic kidney disease, diabetic nephropathy, acute kidney injury, polycystic kidney disease, or alopecia.
